# EUROPEAN PATENT APPLICATION

(11) **EP 2 746 255 A1**
(43) Date of publication of application: **25.06.2014**
(21) Application number: 13195054.5
(22) Date of filing: 29.11.2013
(51) Int. Cl.: C07D 233/56, C07D 233/68, C07D 233/84, C07D 253/06, A01N 43/50, A01N 43/653

(54) **Substituted [1,2,4]triazole and imidazole compounds**

(30) Priority: 19.12.2012 EP 12198183; 04.01.2013 EP 13150307
(71) Applicant: BASF SE, 67056 Ludwigshafen (DE)
(72) Inventor: Grammenos, Wassilios, 67071 Ludwigshafen (DE); Craig, Ian Robert, 67063 Ludwigshafen (DE); Boudet, Nadege, 69502 Hemsbach (DE); Müller, Bernd, 67227 Frankenthal (DE); Dietz, Jochen, 76227 Karlsruhe (DE); Lauterwasser, Erica May Wilson, 68199 Mannheim (DE); Lohmann, Jan Klaas, 67245 Lambsheim (DE); Grote, Thomas, 67157 Wachenheim (DE); Haden, Egon, 67346 Speyer (DE); Escribano Cuesta, Ana, 68167 Mannheim (DE)
(74) Representative: Maiwald Patentanwalts GmbH

(57) **Abstract**

The present invention relates to compounds of the formula I 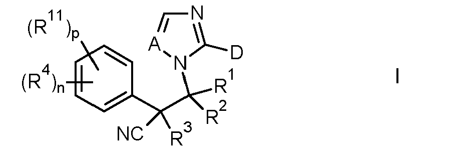 wherein the substituents are as defined in the description and claims, their preparation and their uses.

## Description

The present invention relates to substituted [1,2,4]triazole and imidazole compounds and the N-oxides and the salts thereof for combating phytopathogenic fungi, and to the use and methods for combating phytopathogenic fungi and to seeds coated with at least one such compound. The invention also relates to processes for preparing these compounds, intermediates, processes for preparing such intermediates, and to compositions comprising at least one compound I.

EP 0 251 775 relates to alpha-aryl-alpha-phenylethyl-1 H-1,2,4-triazole-1-propanenitriles. DE 2604047 relates to certain imidazoles and their use as fungicides. US 4,225,723 relates to a process for the preparation of substituted arylcyanoalkyl and diaryl cyanoalkylimidazoles. WO 2004011410 relates to pharmaceutical compounds, some of them being substituted triazole compounds containing a trimethylsilyloxy group.

In many cases, in particular at low application rates, the fungicidal activity of the known fungicidal compounds is unsatisfactory. Based on this, it was an object of the present invention to provide compounds having improved activity and/or a broader activity spectrum against phytopathogenic harmful fungi.

Surprisingly, this object is achieved by the use of the inventive substituted [1,2,4]triazol and imidazole compounds of formula I having favorable fungicidal activity against phytopathogenic fungi.

Accordingly, the present invention relates, in a first aspect, to the compounds of formula I: 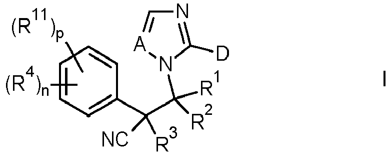 wherein
A is CH or N;
D is H, halogen or SR^{D}, wherein
R^{D} is hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl or CN;
R¹ is halogen, CN, NO₂, OH, SH, C₁-C₆-alkyl, C₁-C₆-alkoxy, C₁-C₆-alkylthio, , C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl),C(=O)-N(C₁-C₄-alkyl)₂, C(=O)-NH(C₃-C₆-cycloalkyl), C(=O)-N(C₃-C₆-cycloalkyl)₂, heteroaryl, phenyl-C₁-C₄-alkyl or heteroaryl-C₁-C₄-alkyl, wherein the heteroaryl radical in the last-mentioned groups is 5- or 6-membered;;
and wherein the aliphatic, alicyclic and aromatic moieties of R¹ are unsubstituted or substituted by one, two, three or four or up to the maximum possible number of R^{1a}; wherein R^{1a} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R² is hydrogen or is selected from the substituents defined for R¹; wherein the aliphatic, alicyclic and aromatic moieties of R² are unsubstituted or substituted by one, two, three or four or up to the maximum possible number of R^{2a}, wherein R^{2a} has the meaning as defined for R^{1a};
or R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle contains one, two, three or four heteroatoms selected from N, O and S, and wherein the carbo-or heterocycle is unsubstituted or carries one, two, three or four substituents R^{12a} independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S);
or R¹ and R² together with the carbon atom to which they are bound (denominated C*) form a group (C*=CR¹⁸R¹⁹), wherein
R¹⁸, R¹⁹ are independently selected from hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R³ is hydrogen, halogen, C₁-C₈-alkyl, C₂-C₈-alkenyl, C₂-C₈-alkinyl, C₁-C₆-alkoxy, C₃-C₈-cycloalkyl, C₃-C₈-cycloalkyl-C₁-C₄-alkyl, phenyl, phenyl-C₁-C₄-alkyl, phenyl-C₂-C₄-alkenyl or phenyl-C₂-C₄-alkynyl; wherein the aliphatic, alicyclic and aromatic moieties of R³ are unsubstituted or substituted by one, two, three or four or up to the maximum possible number of R^{3a}, wherein
R^{3a} is independently selected from halogen, CN, OH, nitro, C₁-C₄-alkyl, C₁-C₄-halogenalkyl, C₃-C₆-cycloalkyl, C₃-C₆-halocycloalkyl, C₁-C₄-alkoxy and C₁-C₄-halogenalkoxy;
p is 0 or 1;
R¹¹ stands for a group Z-Y, wherein said group is bound to the phenyl via Y, wherein
Y is a direct bond or a divalent group selected from the group consisting of -O-, -S-, -SO-, - SO₂-, -NH-, -N(C₁-C₄-alkyl)-, -CR¹²R¹³-, -CR¹²R¹³-CR¹⁴R¹⁵-, -CR¹⁶=CR¹⁷-and -C≡C-; wherein R¹²,R¹³,R¹⁴,R¹⁵,R¹⁶,R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy;
Z is five or six-membered heteroaryl or phenyl, wherein the heteroaryl contains 1, 2, 3 or 4 heteroatoms selected from the group consisting of O, N and S, and wherein the heteroaryl or phenyl is unsubstituted (m=0) or substituted by (R^{L})ₘ, wherein
m is 0, 1, 2, 3 , 4 or 5; and wherein
R^{L} is independently selected from halogen, CN, NO₂, OH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein
R^{La} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio;
R⁴ is independently selected from halogen, CN, NO₂, OH, SH, C₁-C₄-alkyl, C₁C₁-C₄-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, phenyl, phenoxy, a 5- or 6-membered heteroaryl, a 5- or 6-membered heteroaryloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic, alicyclic and aromatic moieties of R⁴ are unsubstituted or substituted by one, two, three or four or up to the maximum possible number of R^{4a}; wherein
R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy;
n is 0, 1, 2, 3 or 4;
with the proviso that for A = CH, R¹=n-C₃H₇, R²=hydrogen, p=0 and n=2 and (R⁴)₂=2,4-Cl₂, R³ is not hydrogen;
and the N-oxides and the agriculturally acceptable salts thereof.

Compounds I, bearing D=halogen or SR^{D}, can be synthesized from compounds I, wherein D is H by simple deprotonation with a base (eg, LDA, BuLi, LHMDS, *i*-PrMgCl, EtMgI, NaH, KH, *t-*BuOK, *t*-BuOK, TMPLi, TMPZnCl, TMPMgCl, (TMP)₂Zn, (TMP)₂Mg, EtONa, EtOK) at low temperature and treatment with an electrophile (e.g. Idodine, ICl, Sulfur, alkyl-disulfide) in a suitable solvent such as Et₂O, MTBE or THF. These compounds can be synthesized for example in analogy with reported methods in: Tetrahedron Letters (2011), 52(36), 4590-4594, W02006/102194, Journal of Organic Chemistry (2009), 74(21), 8309-8313, W02011/113820. Alternatively, compounds I with D= SCN can be obtained by treating a compound I with D equals SH with cyanogen halide such as Br-CN or CL-CN and a base (organic or inorganic base such as K₂CO₃, NaOH, KOH or NEt₃, DBU) in a suitable solvent such as acetone, MeCN or THF. These compounds can be synthesized for example in analogy with reported methods in: W02009/077497, Chemical & Pharmaceutical Bulletin (1964), 12(2), 182-191. Alternatively, compounds I with D= SR^{D} can be obtained by treating a compound I with D equals SH with an alkylation agent such as metyl iodide a base (organic or inorganic base such as K₂CO₃, NaOH, KOH or NEt₃, DBU) in a suitable solvent such as acetone, MeCN or THF. These compounds can be synthesized for example in analogy with reported methods in:_WO2012/047762, Heteroatom Chemistry (2010), 20(7), 405-410, Khimiya Geterotsiklicheskikh Soedinenii (1977), (11), 1561-1563, Indian Journal of Heterocyclic Chemistry (1999), 8(4), 341-342, W02011/113820.

Compounds I with D = H can be synthesized using various synthetic routes outlined in following schemes.

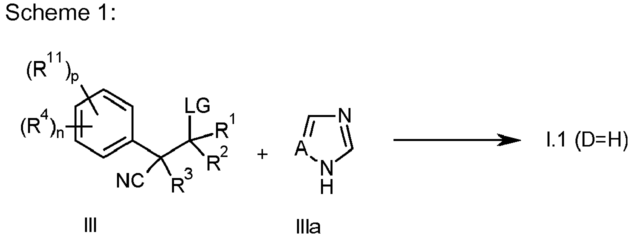

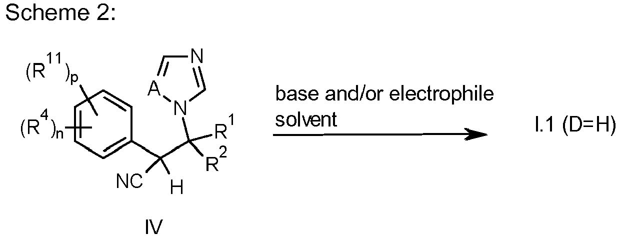

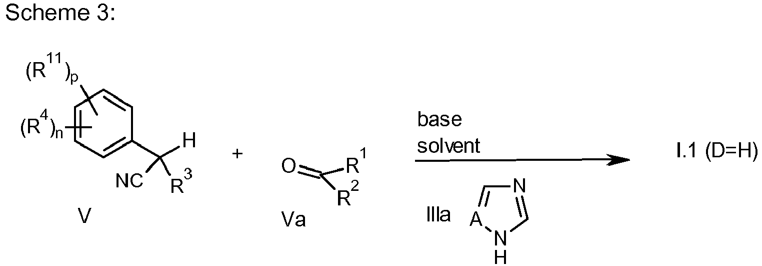

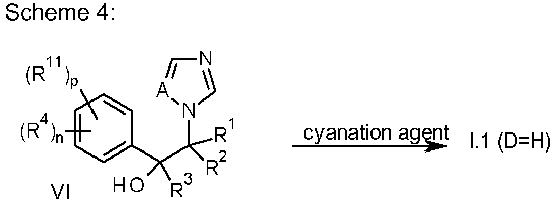

Compounds I.1 can be obtained by substitution of a LG (e.g. halogen, sulfonate, sulfate) in compound III with an azole compound IIIa and a base (e.g .NaOH, KOH, DIEA, NEt₃, DBU, NaH, KH) in an appropriate solvent (e.g. MeCN, DMF, NMP, Acetone, THF, toluene) at elevated temperatures (50-180°C). These compounds can be synthesized for example in analogy with reported methods in: W02011/005520, European Journal of Medicinal Chemistry (2009), 44(7), 3064-3067

Alternatively, compounds I.1 can be obtained by reaction of IV with a base (KH, NaH, NaHCO₃, NaOH, KOH, LDA, MeONa, LiOH, *t*-BuONa, *t*-BuOK, TMPLi, TMPZnCl, TMPMgCl, (TMP)₂Zn, (TMP)₂Mg, NH₃, DBU, BuLi) and/or an electrophile such (Br₂, I₂, allyl-halide, methyl iodide, methyl bromide, cyclopropyl bromide, propargyl bromide, phenyl bromide) in an appropriate organic solvent such as THF, Et₂O, DME, dioxane. These compounds can be synthesized for example in analogy with reported methods in: Journal of Organic Chemistry (2005), 70(6), 2200-2205, Organic Reactions (Hoboken, NJ, United States) (1984), 31. In the case of R³ is an aryl group, these compounds can be obtained via cross-coupling with the corresponding aryl halide, following for example reported procedures in: Journal of the American Chemical Society, 124(32), 9330-9331.

Alternatively, compounds I.1 can be obtained by reaction of V with a base (LDA, KOtBu, BuLi, LTMP, LHMDS or DBU) and a keto compound carrying R¹ an R². Consecutively, the corresponding aldol product can be transferred into a leaving group (such as halide, sulfonate, sulfate) and consequently being replaced by an azole compound IIIa using an appropriate solvent (MeCN, DMF, NMP, a cetone, THF, toluene) and a base (e.g. NaOH, KOH, DIEA, NEt₃, DBU, NaH, KH). These compounds can be synthesized for example in analogy with reported methods in: EP112669, Journal of Organic Chemistry (2005), 70(6), 2200-2205. Compound V can be obtained in analogy with compound IIId.

Alternatively, compounds I.1 can be synthesized starting from an alcohol compound VI, either by direct cyanation of the free alcohol or by transforming the alcohol into a suitable leaving group (such as halide, sulfonate, sulfate) and replacing this LG with a cyanide source such as HCN, NaCN, KCN, Et4N-CN). These compounds can be synthesized for example in analogy with reported methods in: Letters in Organic Chemistry (2005), 2(8), 725-730, Tetrahedron Letters (2007), 48(38), 6779-6784, Journal of Organic Chemistry (2004), 69(7), 2562-2564, Organic Chemistry: An Indian Journal (2008), 4(1), 32-35.

Compound III can be synthesized using the following scheme:

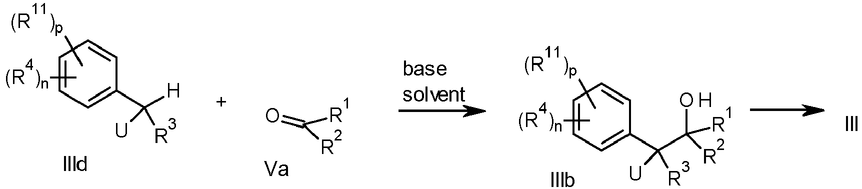

For U=CO₂Me, CHO and CN, compound IIId can perform an aldol addition with aldehydes Va (R²=H) or ketones (R² is not H) Va. If necessary (U is not CN) U can be transformed into CN according to well known procedures. Compounds IIIb can be transformed to compound III via functional group interconversion (e.g. LG = halide, sulfonate, sulfate). These compounds can be synthesized for example in analogy with reported methods in: EP112669, Journal of Organic Chemistry (2005), 70(6), 2200-2205, EP1367058, Journal of Heterocyclic Chemistry (1991), 28(5), 1273-1280. Compounds IIId (U = ester, CHO, CN) can be obtained from the respective alcohol alcohol (IIIe) in analogy with reported method: Letters in Organic Chemistry (2005), 2(8), 725-730, Tetrahedron Letters (2003), 44(8), 1639-1642, Bulletin of the Chemical Society of Japan (1982), 55(2), 643-644.

Compound IIIe can be synthesized for example by the reaction of compounds IIIf and IIIg or IIIh and IIIj (where X can be halide or proton). These reaction take place in the presence of a base (e.g. Na₂CO₃, EtN(*i*-Pr)₂, *t*-BuOK, Cs₂CO₃, NaH, KH, NaOH, KOH, K₂CO₃, NaOEt, KOEt, Et₃N)and/or a catalyst (e.g. Cul, Cu(OAc)₂, PdCl₂, Pd(PPh₃)₄, PdCl₂(PPh₃)₂, Pd(OAc)₂, CuSO₄/O₂. CuO/O₂, CuSO₄/O₂) in an inert organic solvent (e.g. THF, DME, Et₂O, DMF, NMP, DMSO, toluene, acetonitrile). Analogous procedures are reported for example in: W02005/060949, Tetrahedron (2011) 67(5), 1002-1010, Synthesis (2012), 44(10), 1507-1510, Journal of Organic Chemistry (2006), 71(1), 379-381, Organic Letters (2012), 14(5), 1202-1205, Chemistry--A European Journal (2012), 18(19), 6039-6048. Compounds IIIf, IIIg, IIIh and IIIj are commercial available or literature known.

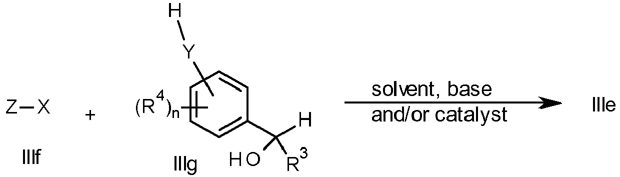

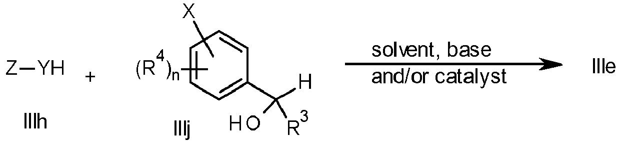

Compounds IVb (U=CO₂Me, CHO and CN) can be obtained by applying an aldol condensation with IVd, IVc and a base(e.g.NaH, LDA, *t*-BuOK, BuLi, LTMP, LHMDS or DBU). These compounds can be synthesized for example in analogy with reported methods in: W02006/060931, Tetrahedron Letters (2010), 51(40), 5246-5251. A 1,4 addition of azole compound IIIa ultimately leads to compounds IV. These compounds can be synthesized for example in analogy with reported methods in: Synthetic Communications (1987), 17(7), 809-815, Medicinal Chemistry (2009), 5(3), 208-215. Compound IVd can be obtained in analogy with compound IIId.

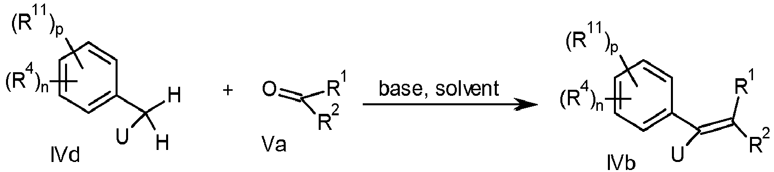

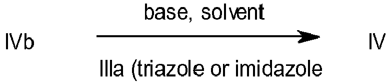

Compound VI can be synthesized for example using the following scheme:

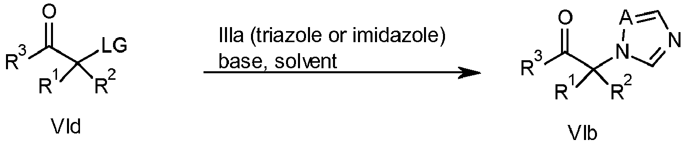

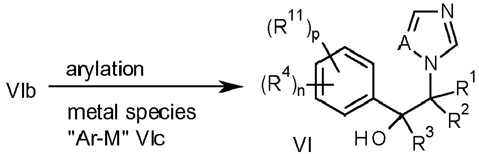

Compound VIb can be synthesized by substitution of a LG (e.g. halogen, sulfonate, sulfate) in compound VId with an azole compound VIe and a base (e.g .NaOH, KOH, DIEA, NEt₃, DBU, NaH, KH) in an appropriate solvent (e.g. MeCN, DMF, NMP, Acetone, THF, toluene) at elevated temperatures (50-180°C). These compounds can be synthesized for example in analogy with reported methods in: W02011/005520, European Journal of Medicinal Chemistry (2009), 44(7), 3064-3067. Compound VI can be obtained for example via arylation of carbonyl compound VIb. This reaction can take places via the addition of aryl metal species VIc and VIb in an inert organic solvent (e.g. THF, DME, Et₂O, DMF, NMP, DMSO, toluene, acetonitrile) preferably. The metal specie can be generated via metal insertion (e.g. e.g. Mg, Zn, Li, Mg/ZnCl₂) or via exchange in the presence of a base (e.g. BuLi, LDA, *i*-PrMgCl, TMPLi, TMPMgCl, TMPZnCl, (TMP)₂Mg, (TMP)₂Zn, *t*-BuOK). These compounds can be prepared for example in analogy to methods described in: for example in analogy to the method described in: Journal of the American Chemical Society (2007), 129(13), 3836-3837, Organic & Biomolecular Chemistry (2009), 7(18), 3660-3662, Liebigs Annalen der Chemie (1987), (4), 349-355, W02004/011410. Compound VIc can be functionalized in analogy with compound IIIe.

The N-oxides may be prepared from the inventive compounds according to conventional oxidation methods, e. g. by treating compounds I with an organic peracid such as metachloroperbenzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the following, the intermediate compounds are further described. A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein.

Compounds of formula III are at least partially new. Consequently, a further embodiment of the present invention are compounds of formula III (see above), wherein the variables are as defined and preferably defined for formula I herein.

Compounds of formula IIIb are at least partially new. Consequently, a further embodiment of the present invention are compounds of formula IIIb (see above), wherein the variables are as defined and preferably defined for formula I herein.

Compounds of formula IIId are at least partially new. Consequently, a further embodiment of the present invention are compounds of formula IIId (see above), wherein the variables are as defined and preferably defined for formula I herein.

Compounds of formula IIIe and are at least partially new. Consequently, a further embodiment of the present invention are compounds of formula IIIe (see above), wherein the variables are as defined and preferably defined for formula I herein.

Compounds of formula IIIg are at least partially new. Consequently, a further embodiment of the present invention are compounds of formula IIIg (see above), wherein the variables are as defined and preferably defined for formula I herein.

Compounds of formula IV are at least partially new. Consequently, a further embodiment of the present invention are compounds of formula IV (see above), wherein the variables are as defined and preferably defined for formula I herein.

Compounds of formula IVb are at least partially new. Consequently, a further embodiment of the present invention are compounds of formula IVb (see above), wherein the variables are as defined and preferably defined for formula I herein.

Compounds of formula V are at least partially new. Consequently, a further embodiment of the present invention are compounds of formula V (see above), wherein the variables are as defined and preferably defined for formula I herein.

Compounds of formula VI are at least partially new. Consequently, a further embodiment of the present invention are compounds of formula VI (see above), wherein the variables are as defined and preferably defined for formula I herein.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).

The term "C₁-C₆-haloalkyl" refers to an alkyl group having 1 or 6 carbon atoms as defined above, wherein some or all of the hydrogen atoms in these groups may be replaced by halogen atoms as mentioned above. Examples are "C₁-C₂-haloalkyl" groups such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₁-C₆-hydroxyalkyl" refers to an alkyl group having 1 or 6 carbon atoms as defined above, wherein some or all of the hydrogen atoms in these groups may be replaced by OH groups.

The term "C₂-C₆-alkenyl" refers to a straight-chain or branched unsaturated hydrocarbon radical having 2 to 6 carbon atoms and a double bond in any position. Examples are "C₂-C₄-alkenyl" groups, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₆-alkynyl" refers to a straight-chain or branched unsaturated hydrocarbon radical having 2 to 6 carbon atoms and containing at least one triple bond. Examples are "C₂-C₄-alkynyl" groups, such as ethynyl, prop-1-ynyl, prop-2-ynyl (propargyl), but-1-ynyl, but-2-ynyl, but-3-ynyl, 1-methyl-prop-2-ynyl.

The term "C₃-C₈-cycloalkyl" refers to monocyclic saturated hydrocarbon radicals having 3 to 8 carbon ring members, such as cyclopropyl, cyclobutyl, cyclopentyl, cyclohexyl, cycloheptyl or cyclooctyl.

The term "C₃-C₈-cycloalkyl-C₁-C₄-alkyl" refers to alkyl having 1 to 4 carbon atoms (as defined above), wherein one hydrogen atom of the alkyl radical is replaced by a cycloalkyl radical having 3 to 8 carbon atoms (as defined above).

The term "C₁-C₆-alkoxy" refers to a straight-chain or branched alkyl group having 1 to 6 carbon atoms which is bonded via an oxygen, at any position in the alkyl group. Examples are "C₁-C₄-alkoxy" groups, such as methoxy, ethoxy, n-propoxy, 1-methylethoxy, butoxy, 1-methyl¬propoxy, 2-methylpropoxy or 1,1-dimethylethoxy.

The term "C₁-C₆-haloalkoxy" refers to a C₁-C₆-alkoxy radical as defined above, wherein some or all of the hydrogen atoms in these groups may be replaced by halogen atoms as mentioned above. Examples are "C₁-C₄-haloalkoxy" groups, such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoro-ethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "phenyl-C₁-C₆-alkyl" refers to alkyl having 1 to 6 carbon atoms (as defined above), wherein one hydrogen atom of the alkyl radical is replaced by a phenyl radical. Likewise, the terms "phenyl-C₂-C₆-alkenyl" and "phenyl-C₂-C₆-alkynyl" refer to alkenyl and alkynyl, respectively, wherein one hydrogen atom of the aforementioned radicals is replaced by a phenyl radical.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₆-alkoxy-C₁-C₄-alkyl" refers to alkyl having 1 to 4 carbon atoms (as defined above), wherein one hydrogen atom of the alkyl radical is replaced by a C₁-C₆-alkoxy group (as defined above).

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₆-alkylsulfinyl" refers to straight-chain or branched alkyl groups having 1 to 6 carbon atoms (as defined above) bonded through a -S(=O)- moiety, at any position in the alkyl group, for example methylsulfinyl and ethylsulfinyl, and the like. Accordingly, the term "C₁-C₆-haloalkylsulfinyl" refers to straight-chain or branched haloalkyl group having 1 to 6 carbon atoms (as defined above), bonded through a -S(=O)- moiety, at any position in the haloalkyl group.

The term "C₁-C₆-alkylsulfonyl" refers to straight-chain or branched alkyl groups having 1 to 6 carbon atoms (as defined above), bonded through a -S(=O)₂- moiety, at any position in the alkyl group, for example methylsulfonyl. Accordingly, the term "C₁-C₆-haloalkylsulfonyl" refers to straight-chain or branched haloalkyl group having 1 to 6 carbon atoms (as defined above), bonded through a -S(=O)₂- moiety, at any position in the haloalkyl group.

The term "C₃-C₈-cycloalkyl-C₃-C₈-cycloalkyl" refers to a cycloalkyl radical having 3 to 8 carbon atoms (as defined above), which is substituted by a further cycloalkyl radical having 3 to 8 carbon atoms.

The term "C₃-C₈-cycloalkoxy" refers to a cycloalkyl radical having 3 to 8 carbon atoms (as defined above), which is bonded via an oxygen.

The term "C(=O)-C₁-C₄-alkyl" refers to a radical which is attached through the carbon atom of the group C(=O) as indicated by the number valence of the carbon atom. The number of valence of carbon is 4, that of nitrogen is 3. Likewise the following terms are to be construed: NH(C₁-C₄-alkyl), N(C₁-C₄-alkyl)₂, NH(C₃-C₆-cycloalkyl), N(C₃-C₆-cycloalkyl)₂, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂.

The term "saturated or partially unsaturated 3-, 4- 5-, 6- or 7-membered carbocycle" is to be understood as meaning both saturated or partially unsaturated carbocycles having 3, 4, 5, 6 or 7 ring members. Examples include cyclopropyl, cyclopentyl, cyclopentenyl, cyclopentadienyl, cyclohexyl, cyclohexenyl, cyclohexadienyl, cycloheptyl, cycloheptenyl, cycloheptadienyl, and the like.

The term "saturated or partially unsaturated 3-, 4-, 5-, 6-, or 7-membered heterocycle, wherein the ring member atoms of the heterocycle include besides carbon atoms 1, 2, 3 or 4 heteroatoms independently selected from the group of N, O and S", is to be understood as meaning both saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine; and
a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetrahydro[2H]azepin-2-,-3-,-4-,-5-,-6- or-7-yl, 2,3,4,7-tetrahydro[1H]azepin-1-,-2-,-3-,-4-,-5-,-6-or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals; and
The term "5-or 6-membered heteroaryl" refers to aromatic ring systems incuding besides carbon atoms, 1, 2, 3 or 4 heteroatoms independently selected from the group consisting of N, O and S, for example,
a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

Agriculturally acceptable salts of the inventive compounds encompass especially the salts of those cations or the acid addition salts of those acids whose cations and anions, respectively, have no adverse effect on the fungicidal action of said compounds. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting such inventive compound with an acid of the corresponding anion, preferably of hydrochloric acid, hydrobromic acid, sulfuric acid, phosphoric acid or nitric acid.

The inventive compounds can be present in atropisomers arising from restricted rotation about a single bond of asymmetric groups. They also form part of the subject matter of the present invention.

Depending on the substitution pattern, the compounds of formula I and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds I also apply to the intermediates.

A according to the invention is N or CH. According to one embodiment A is N. According to a further embodiment A is CH.

D according to the present invention is hydrogen, halogen or SR^{D}, wherein R^{D} is hydrogen, CN, C₁-C₆-alkyl, C₁-C₆-haloalkyl, C₂-C₆-alkenyl, C₂-C₆-haloalkenyl, C₂-C₆-alkynyl or C₂-C₆-haloalkynyl.

In a preferred embodiment D is hydrogen, halogen, SH, SCN or S-CH₂-CH=CH₂ (S-allyl). According to one embodiment D is hydrogen. According to a further embodiment, D is halogen, in particular iodine. According to another preferred embodiment D is SR^{D}. According to a particular embodiment, R^{D} is H. In yet another preferred embodiment R^{D} is CN. In a further preferred embodiment R^{D} is -CH₂-CH=CH₂.

R¹ according to the present invention is halogen, CN, NO₂, OH, SH, C₁-C₆-alkyl, C₁-C₆-alkoxy, C₁-C₆-alkylthio, , C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl),C(=O)-N(C₁-C₄-alkyl)₂, C(=O)-NH(C₃-C₆-cycloalkyl), C(=O)-N(C₃-C₆-cycloalkyl)₂, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R¹ are unsubstituted or substituted by one, two, three or four or up to the maximum possible number of R^{1a}; wherein R^{1a} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl.

R² according to the invention is hydrogen, halogen, CN, NO₂, OH, SH, C₁-C₆-alkyl, C₁-C₆-alkoxy, C₁-C₆-alkylthio, , C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl),C(=O)-N(C₁-C₄-alkyl)₂, C(=O)-NH(C₃-C₆-cycloalkyl), C(=O)-N(C₃-C₆-cycloalkyl)₂, heteroaryl, phenyl-C₁-C₄-alkyl or heteroaryl-C₁-C₄-alkyl, wherein the heteroaryl radical in the last-mentioned groups is 5- or 6-membered; and wherein the aliphatic, alicyclic and aromatic moieties of R² are unsubstituted or substituted by one, two, three or four or up to the maximum possible number of R^{2a}; wherein R^{1a} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl.

Or, R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle contains one, two, three or four heteroatoms selected from N, O and S, and wherein the carbo- or heterocycle is unsubstituted or carries one, two, three or four substituents independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S).

Or R¹ and R² together with the carbon atom to which they are bound (denominated C*) form a group (C*=CR¹⁸R¹⁹), wherein R¹⁸, R¹⁹ are independently selected from hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl.

According to one embodiment of the invention R¹ is halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, wherein the aliphatic and alicyclic moieties of R¹ are in each case unsubstituted or substituted by one, two, three or four or up to the maximum possible number of independently selected substituents R^{1a}; wherein R^{1a} is independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cydoalkyl and C₁-C₄-alkoxy-C₁-C₄-alkyl, in particular from halogen, CN, OH, SH, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₃-6₈-cycloalkyl and C₁-C₄-alkoxy-C₁-C₄-alkyl.

According to another embodiment of the invention R¹ is 5- or 6-membered heteroaryl that is unsubstituted or substituted by one, two, three or four independently selected substituents R^{1a}; as defined and preferably defined herein. According to one embodiment, R¹ is unsubstituted 5-membered heteroaryl. According to a further embodiment, R¹ is 5-membered heteroaryl that is substituted by one, two, three or four independently selected substituents R^{1a}; as defined and preferably defined herein. According to a further embodiment, R¹ is unsubstituted 6-membered heteroaryl. According to a further embodiment, R¹ is 6-membered heteroaryl that is substituted by one, two, three or four independently selected substituents R^{1a}; as defined and preferably defined herein.

According to another embodiment of the invention R¹ is phenyl-C₁-C₄-alkyl, in particular phenyl-C₁-C₂-alkyl, wherein the aliphatic and phenyl moieties are in each case unsubstituted or substituted by one, two, three or four or up to the maximum possible number of independently selected substituents R^{1a} as defined and preferably defined herein, in particular wherein each R^{1a} is selected from Cl, F, CN, OH, C₁-C₂-alkyl, C₁-C₂-haloalkyl, C₁-C₂-alkoxy, C₁-C₂-haloalkoxy, C₁-C₂-alkylthio, C₃-C₆-cycloalkyl or C₁-C₂-alkoxy-C₁-C₂-alkyl.

According to another embodiment of the invention R¹ is heteroaryl-C₁-C₄-alkyl, wherein the heteroaryl radical is 5- or 6-membered, and the aliphatic and the aromatic moieties are unsubstituted or substituted by one, two, three or four or up to the maximum possible number of independently selected substituents R^{1a}; wherein R^{1a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl, in particular Cl, F, CN, OH, C₁-C₂-alkyl, C₁-C₂-haloalkyl, C₁-C₂-alkoxy, C₁-C₂-haloalkoxy, C₁-C₂-alkylthio, C₃-C₆-cycloalkyl or C₁-C₂-alkoxy-C₁-C₂-alkyl. According to one embodiment, R¹ is unsubstituted 5-membered heteroaryl. According to a further embodiment, R¹ is 5-membered heteroaryl that is substituted by one, two, three or four independently selected substituents R^{1a}; as defined and preferably defined herein. According to a further embodiment, R¹ is unsubstituted 6-membered heteroaryl. According to a further embodiment, R¹ is 6-membered heteroaryl that is substituted by one, two, three or four independently selected substituents R^{1a}; as defined and preferably defined herein. Specific embodiments thereof can be found in the below Table P1.

According to a further embodiment R¹ is halogen, CN, OH, SH, C₁-C₄-alkyl, C₁-C₄-hydroxyalkyl, C₁-C₄-haloalkyl, C₁-C₄-alkoxy, C₁-C₄-haloalkoxy, C₁-C₄-alkylthio, C₁-C₄-haloalkylthio, C₁-C₄-alkoxy-C₁-C₄-alkyl, C₂-C₄-alkenyl, C₂-C₄-alkynyl, C₂-C₄-haloalkenyl, C₂-C₄-haloalkynyl, C₃-C₆-cycloalkyl, C₃-C₆-cycloalkyloxy, C₃-C₆-cycloalkyl-C₁-C₄-alkyl, NH₂, NH(C₁-C₄-alkyl) or N(C₁-C₄-alkyl)₂, wherein the aliphatic and alicyclic moieties are in each case unsubstituted or substituted by one, two, three or four independently selected substituents R^{1a} as defined and preferably defined herein. Specific embodiments thereof can be found in the below Table P1.

According to a further embodiment, R¹ is halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl or C₃-C₆-cycloalkyl. Specific embodiments thereof can be found in the below Table P1.

In still a further embodiment R¹ is halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₂-C₆-alkenyl, C₂-C₆-alkynyl or C₃-C₈-cycloalkyl. Specific embodiments thereof can be found in the below Table P1.

In one particular embodiment R¹ is halogen, in particular fluorine, chlorine or bromine. Specific embodiments thereof can be found in the below Table P1.

In one further particular embodiment R¹ is CN.

In one further particular embodiment R¹ is OH.

In one further particular embodiment R¹ is C₁-C₆-alkyl, in particular C₁-C₄-alkyl, that is unsubstituted or substituted by one, two, three or four or up to the maximum possible number of independently selected substituents R^{1a}; wherein R^{1a} is as defined and preferably defined herein. In one specific embodiment thereof, R¹ is C₁-C₆-alkyl, in particular C₁-C₄-alkyl, more particularly methyl, ethyl and 1-methylethyl. In a further specific embodiment thereof, R¹ is C₁-C₆-alkyl, in particular C₁-C₄-alkyl, that is substituted by one, two, three or four or up to the maximum possible number of independently selected substituents R^{1a}, in particular one, two, three or four Cl, F, CN, OH, C₁-C₂-alkoxy, C₁-C₂-haloalkoxy, C₁-C₂-alkylthio or C₃-C₆-cycloalkyl. In a further specific embodiment thereof R¹ is C₁-C₆-haloalkyl, in particular C₁-C₄-haloalkyl, for example CF₃ or CHF₂. In still a further specific embodiment thereof R¹ is hydroxyl-C₁-C₆-alkyl, in particular hydroxy-C₁-C₄-alkyl, more specifically hydroxyl-C₁-C₂-alkyl. In a further specific embodiment thereof R¹ is C₃-C₈-cycloalkyl-C₁-C₄-alkyl, in particular C₃-C₆-cycloalkyl-C₁-C₄-alkyl, wherein the cycloalkyl moiety are unsubstituted or substituted by one, two or three independently selected substituents R^{1a} and wherein the alkyl moiety is unsubstituted or substituted by one, two or three independently selected substituents R^{1a}. A specific embodiemt thereof is where R¹ is C₃-C₆-halocycloalkyl-C₁-C₄-alkyl or C₃-C₆-cycloalkyl-C₁-C₄-haloalkyl. Specific embodiments thereof can also be found in the below Table P1.

In one further particular embodiment R¹ is C₁-C₆-alkoxy, in particular C₁-C₄-alkoxy, that is unsubstituted or substituted by one, two, three or four or up to the maximum possible number of independently selected substituents R^{1a}; wherein R^{1a} is as defined and preferably defined herein. In one specific embodiment thereof, R¹ is C₁-C₆-alkoxy, in particular C₁-C₄-alkoxy, more particularly methoxy, ethoxy or 1-methylethoxy. In a further specific embodiment thereof, R¹ is C₁-C₆-alkoxy, in particular C₁-C₄-alkoxy, that is substituted by one, two, three or four or up to the maximum possible number of independently selected substituents R^{1a}, in particular one, two, three or four Cl, F, CN, OH, C₁-C₂-alkyl, C₁-C₂-alkoxy, C₁-C₂-haloalkoxy, C₁-C₂-alkylthio or C₃-C₆-cycloalkyl. In a further specific embodiment thereof R¹ is C₁-C₆-haloalkoxy, in particular C₁-C₄-haloalkoxy, for example OCF₃ or OCHF₂. Specific embodiments thereof can also be found in the below Table P1.

In another preferred embodiment R¹ is SH, C₁-C₆-alkylthio, C₁-C₆-alkylsulfinyl or C₁-C₆-alkylsulfonyl, in particular SH, C₁-C₄-alkylthio, C₁-C₄-alkylsulfinyl or C₁-C₄-alkylsulfonyl.

In another preferred embodiment R¹ is C₂-C₆-alkenyl, in particular C₂-C₄-alkenyl, such as 2-propenyl.

In another preferred embodiment R¹ is C₂-C₆-alkynyl, in particular C₂-C₄-alkynyl, such as 2-propynyl or 2-butynyl.

According to a further particular embodiment of the invention, R¹ is C₃-C₈-cycloalkyl, in particular C₃-C₆-cycloalkyl, that is unsubstituted or substituted by one, two, three or four or up to the maximum possible number of independently selected substituents R^{1a}; wherein R^{1a} is as defined and preferably defined herein. In one specific embodiment thereof, R¹ is C₃-C₈-cycloalkyl, in particular C₃-C₆-cycloalkyl, more particularly cyclopropyl, cyclobutyl, cyclopentyl or cyclohexyl. In a further specific embodiment thereof, R¹ is C₃-C₈-cycloalkyl, in particular C₃-C₆-cycloalkyl, that is substituted by one, two, three or four or up to the maximum possible number of independently selected substituents R^{1a}, in particular one, two, three or four Cl, F, CN, OH, C₁-C₂-alkyl, C₁-C₂-alkoxy, C₁-C₂-haloalkoxy, C₁-C₂-alkylthio or C₃-C₆-cycloalkyl. In a further specific embodiment thereof R¹ is C₃-C₈-halocycloalkyl, in particular C₃-C₆-halocycloalkyl, for example 1 -Cl-cyclopropyl and 1-F-cyclopropyl. In a further specific embodiment thereof R¹ is C₃-C₈-CN-cyclooalkyl, in particular C₃-C₆-CN-cycloalkyl, for example 1-CN-cyclopropyl. In a further specific embodiment thereof R¹ is C₁-C₂-alkyl-C₃-C₈-cycloalkyl, in particular C₁-C₂-alkyl-C₃-C₆-cycloalkyl, for example 1-CH₃-cyclopropyl.

According to a further particular embodiment of the invention, R¹ is C₃-C₈-cycloalkyloxy, in particular C₃-C₆-cycloalkyloxy, wherein the alicyclic moiety is unsubstituted or substituted by one, two, three or four independently selected substituents R^{1a} as defined and preferably defined herein.

According to a further particular embodiment of the invention, R¹ is NH₂, NH(C₁-C₄-alkyl), N(C₁-C₄-alkyl)₂, NH(C₃-C₆-cycloalkyl) or N(C₃-C₆-cycloalkyl)₂, in particular NH₂, NH(C₁-C₂-alkyl), N(C₁-C₂-alkyl)₂, NH(C₃-C₆-cycloalkyl) or N(C₃-C₆-cycloalkyl)₂, wherein in each case the alicyclic or aliphatic moieties are unsubstituted or substituted by one, two, three or four independently selected substituents R^{1a} as defined and preferably defined herein.Specific embodiments thereof can also be found in the below Table P1.

According to a further particular embodiment of the invention, R¹ is C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, wherein in each case the alicyclic or aliphatic moieties are unsubstituted or substituted by one, two, three or four independently selected substituents R^{1a} as defined and preferably defined herein.

According to a further particular embodiment of the invention, R¹ is C(=O)-C₁-C₄-alkyl, C(=O)OH or C(=O)-O-C₁-C₄-alkyl, wherein in each case the aliphatic moieties are unsubstituted or substituted by one, two, three or four independently selected substituents R^{1a} as defined and preferably defined herein.

According to a further particular embodiment of the invention, R¹ is C(=O)-C₁-C₄-alkyl, in particular C(=O)-C₁-C₂-alkyl, wherein the alkyl is unsubstituted or substituted by one, two, three or four independently selected substituents R^{1a} as defined and preferably defined herein.

According to a further particular embodiment of the invention, R¹ is C(=O)OH or C(=O)-O-C₁-C₄-alkyl, in particular C(=O)-O-C₁-C₂-alkyl, wherein the alkyl is unsubstituted or substituted by one, two, three or four independently selected substituents R^{1a} as defined and preferably defined herein.

According to a further particular embodiment of the invention, R¹ is C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, in particular C(=O)-NH(C₁-C₂-alkyl), C(=O)-N(C₁-C₂-alkyl)₂, C(=O)-NH(C₃-C₆-cycloalkyl) or C(=O)-N(C₃-C₆-cycloalkyl)₂, wherein in each case the alicyclic or aliphatic moieties are unsubstituted or substituted by one, two, three or four independently selected substituents R^{1a} as defined and preferably defined herein.

According to one embodiment, R² is hydrogen.

According to one embodiment of the invention R² is hydrogen, halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, or R² is halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, wherein the aliphatic and alicyclic moieties of R² are in each case unsubstituted or substituted by one, two, three or four or up to the maximum possible number of independently selected substituents R^{2a}; wherein R^{2a} is independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl and C₁-C₄-alkoxy-C₁-C₄-alkyl, in particular from halogen, CN, OH, SH, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₃-C₈-cycloalkyl and C₁-C₄-alkoxy-C₁-C₄-alkyl.

According to another embodiment of the invention R² is hydrogen or 5- or 6-membered heteroaryl that is unsubstituted or substituted by one, two, three or four independently selected substituents R^{2a}; as defined and preferably defined herein. According to one embodiment, R² is hydrogen or unsubstituted 5-membered heteroaryl. According to a further embodiment, R² is hydrogen or 5-membered heteroaryl that is substituted by one, two, three or four independently selected substituents R^{2a}; as defined and preferably defined herein. According to a further embodiment, R² is hydrogen or unsubstituted 6-membered heteroaryl. According to a further embodiment, R² is hydrogen or 6-membered heteroaryl that is substituted by one, two, three or four independently selected substituents R^{2a}; as defined and preferably defined herein.

According to another embodiment of the invention R² is 5- or 6-membered heteroaryl that is unsubstituted or substituted by one, two, three or four independently selected substituents R^{2a}; as defined and preferably defined herein. According to one embodiment, R² is unsubstituted 5-membered heteroaryl. According to a further embodiment, R² is 5-membered heteroaryl that is substituted by one, two, three or four independently selected substituents R^{2a}; as defined and preferably defined herein. According to a further embodiment, R² is unsubstituted 6-membered heteroaryl. According to a further embodiment, R² is 6-membered heteroaryl that is substituted by one, two, three or four independently selected substituents R^{2a}; as defined and preferably defined herein.

According to another embodiment of the invention R² is hydrogen or phenyl-C₁-C₄-alkyl, in particular hydrogen or phenyl-C₁-C₂-alkyl, wherein the aliphatic and phenyl moieties are in each case unsubstituted or substituted by one, two, three or four or up to the maximum possible number of independently selected substituents R^{2a} as defined and preferably defined herein, in particular wherein each R^{2a} is selected from Cl, F, CN, OH, C₁-C₂-alkyl, C₁-C₂-haloalkyl, C₁-C₂-alkoxy, C₁-C₂-haloalkoxy, C₁-C₂-alkylthio, C₃-C₆-cycloalkyl or C₁-C₂-alkoxy-C₁-C₂-alkyl.

According to another embodiment of the invention R² is phenyl-C₁-C₄-alkyl, in particular phenyl-C₁-C₂-alkyl, wherein the aliphatic and phenyl moieties are in each case unsubstituted or substituted by one, two, three or four or up to the maximum possible number of independently selected substituents R^{2a} as defined and preferably defined herein, in particular wherein each R^{2a} is selected from Cl, F, CN, OH, C₁-C₂-alkyl, C₁-C₂-haloalkyl, C₁-C₂-alkoxy, C₁-C₂-haloalkoxy, C₁-C₂-alkylthio, C₃-C₆-cycloalkyl or C₁-C₂-alkoxy-C₁-C₂-alkyl.

According to another embodiment of the invention R² is hydrogen or heteroaryl-C₁-C₄-alkyl, wherein the heteroaryl radical is 5- or 6-membered, and the aliphatic and the aromatic moieties are unsubstituted or substituted by one, two, three or four or up to the maximum possible number of independently selected substituents R^{2a}; wherein R^{2a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl, in particular Cl, F, CN, OH, C₁-C₂-alkyl, C₁-C₂-haloalkyl, C₁-C₂-alkoxy, C₁-C₂-haloalkoxy, C₁-C₂-alkylthio, C₃-C₆-cycloalkyl or C₁-C₂-alkoxy-C₁-C₂-alkyl. According to one embodiment, R² is hydrogen or unsubstituted 5-membered heteroaryl. According to a further embodiment, R² is hydrogen or 5-membered heteroaryl that is substituted by one, two, three or four independently selected substituents R^{2a}; as defined and preferably defined herein. According to a further embodiment, R² is hydrogen or unsubstituted 6-membered heteroaryl. According to a further embodiment, R² is hydrogen or 6-membered heteroaryl that is substituted by one, two, three or four independently selected substituents R^{2a}; as defined and preferably defined herein.

According to another embodiment of the invention R² is heteroaryl-C₁-C₄-alkyl, wherein the heteroaryl radical is 5- or 6-membered, and the aliphatic and the aromatic moieties are unsubstituted or substituted by one, two, three or four or up to the maximum possible number of independently selected substituents R^{2a}; wherein R^{2a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl or C₁-C₄-alkoxy-C₁-C₄-alkyl, in particular Cl, F, CN, OH, C₁-C₂-alkyl, C₁-C₂-haloalkyl, C₁-C₂-alkoxy, C₁-C₂-haloalkoxy, C₁-C₂-alkylthio, C₃-C₆-cycloalkyl or C₁-C₂-alkoxy-C₁-C₂-alkyl. According to one embodiment, R² is unsubstituted 5-membered heteroaryl. According to a further embodiment, R² is 5-membered heteroaryl that is substituted by one, two, three or four independently selected substituents R^{2a}; as defined and preferably defined herein. According to a further embodiment, R² is unsubstituted 6-membered heteroaryl. According to a further embodiment, R² is 6-membered heteroaryl that is substituted by one, two, three or four independently selected substituents R^{2a}; as defined and preferably defined herein. Specific embodiments thereof can be found in the below Table P2.

According to a further embodiment R² is hydrogen, halogen, CN, OH, SH, C₁-C₄-alkyl, C₁-C₄-hydroxyalkyl, C₁-C₄-haloalkyl, C₁-C₄-alkoxy, C₁-C₄-haloalkoxy, C₁-C₄-alkylthio, C₁-C₄-haloalkylthio, C₁-C₄-alkoxy-C₁-C₄-alkyl, C₂-C₄-alkenyl, C₂-C₄-alkynyl, C₂-C₄-haloalkenyl, C₂-C₄-haloalkynyl, C₃-C₆-cycloalkyl, C₃-C₆-cycloalkyloxy, C₃-C₆-cycloalkyl-C₁-C₄-alkyl, NH₂, NH(C₁-C₄-alkyl) or N(C₁-C₄-alkyl)₂, or R² is halogen, CN, OH, SH, C₁-C₄-alkyl, C₁-C₄-hydroxyalkyl, C₁-C₄-haloalkyl, C₁-C₄-alkoxy, C₁-4₆-haloalkoxy, C₁-C₄-alkylthio, C₁-C₄-haloalkylthio, C₁-C₄-alkoxy-C₁-C₄-alkyl, C₂-C₄-alkenyl, C₂-C₄-alkynyl, C₂-C₄-haloalkenyl, C₂-C₄-haloalkynyl, C₃-C₆-cycloalkyl, C₃-C₆-cycloalkyloxy, C₃-C₆-cycloalkyl-C₁-C₄-alkyl, NH₂, NH(C₁-C₄-alkyl) or N(C₁-C₄-alkyl)₂, wherein the aliphatic and alicyclic moieties are in each case unsubstituted or substituted by one, two, three or four independently selected substituents R^{2a} as defined and preferably defined herein. Specific embodiments thereof can be found in the below Table P2.

According to a further embodiment, R² is hydrogen, halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl or C₃-C₈-cycloalkyl. According to a further embodiment, R² is halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl or C₃-C₆-cycloalkyl. Specific embodiments thereof can be found in the below Table P1.

In still a further embodiment R² is hydrogen, halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₂-C₆-alkenyl, C₂-C₆-alkynyl or C₃-C₈-cycloalkyl. According to a further embodiment, R² is halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₂-C₆-alkenyl, C₂-C₆-alkynyl or C₃-C₈-cycloalkyl. Specific embodiments thereof can be found in the below Table P2.

In one particular embodiment R² is hydrogen or halogen. According to a further embodiment, R² is halogen, in particular fluorine, chlorine or bromine. Specific embodiments thereof can be found in the below Table P2.

In one further particular embodiment R² is hydrogen or CN. According to a further embodiment, R² is CN.

In one further particular embodiment R² is hydrogen or OH. According to a further embodiment, R² is OH.

In one further particular embodiment R² is hydrogen or C₁-C₆-alkyl, in particular hydrogen or C₁-C₄-alkyl, wherein the alkyl in each case is unsubstituted or substituted by one, two, three or four or up to the maximum possible number of independently selected substituents R^{2a}; wherein R^{2a} is as defined and preferably defined herein. In one specific embodiment thereof, R² is hydrogen or C₁-C₆-alkyl, in particular hydrogen or C₁-C₄-alkyl, more particularly hydrogen, methyl, ethyl or 1-methylethyl. In a further specific embodiment thereof, R² is hydrogen or C₁-C₆-alkyl, in particular hydrogen or C₁-C₄-alkyl, that is substituted by one, two, three or four or up to the maximum possible number of independently selected substituents R^{2a}, in particular one, two, three or four Cl, F, CN, OH, C₁-C₂-alkoxy, C₁-C₂-haloalkoxy, C₁-C₂-alkylthio or C₃-C₆-cycloalkyl. In a further specific embodiment thereof R² is hydrogen or C₁-C₆-haloalkyl, in particular hydrogen or C₁-C₄-haloalkyl, for example hydrogen, CF₃ or CHF₂. In still a further specific embodiment thereof R² is hydrogen or hydroxyl-C₁-C₆-alkyl, in particular hydrogen or hydroxy-C₁-C₄-alkyl, more specifically hydrogen or hydroxyl-C₁-C₂-alkyl. In a further specific embodiment thereof R² is hydrogen or C₃-C₈-cycloalkyl-C₁-C₄-alkyl, in particular hydrogen or C₃-C₆-cycloalkyl-C₁-C₄-alkyl, wherein the cycloalkyl moiety in each case is unsubstituted or substituted by one, two or three independently selected substituents R^{2a} and wherein the alkyl moiety in each case is unsubstituted or substituted by one, two or three independently selected substituents R^{2a}. A specific embodiment thereof is where R² is hydrogen or C₃-C₆-halocycloalkyl-C₁-C₄-alkyl or hydrogen or C₃-C₆-cycloalkyl-C₁-C₄-haloalkyl. Specific embodiments thereof can be found in the below Table P2.

In one further particular embodiment R² is C₁-C₆-alkyl, in particular C₁-C₄-alkyl, wherein the alkyl in each case is unsubstituted or substituted by one, two, three or four or up to the maximum possible number of independently selected substituents R^{2a}; wherein R^{2a} is as defined and preferably defined herein. In one specific embodiment thereof, R² is C₁-C₆-alkyl, in particular C₁-C₄-alkyl, more particularly methyl, ethyl or 1-methylethyl. In a further specific embodiment thereof, R² is C₁-C₆-alkyl, in particular C₁-C₄-alkyl, that is substituted by one, two, three or four or up to the maximum possible number of independently selected substituents R^{2a}, in particular one, two, three or four Cl, F, CN, OH, C₁-C₂-alkoxy, C₁-C₂-haloalkoxy, C₁-C₂-alkylthio or C₃-C₆-cycloalkyl. In a further specific embodiment thereof R² is C₁-C₆-haloalkyl, in particular C₁-C₄-haloalkyl, for example CF₃ or CHF₂. In still a further specific embodiment thereof R² is hydroxyl-C₁-C₆-alkyl, in particular hydroxy-C₁-C₄-alkyl, more specifically hydroxyl-C₁-C₂-alkyl. In a further specific embodiment thereof R² is C₃-C₈-cycloalkyl-C₁-C₄-alkyl, in particular C₃-C₆-cycloalkyl-C₁-C₄-alkyl, wherein the cycloalkyl moiety is in each case unsubstituted or substituted by one, two or three independently selected substituents R^{2a} and wherein the alkyl moiety is unsubstituted or substituted by one, two or three independently selected substituents R^{2a}. A specific embodiment thereof is where R² is C₃-C₆-halocycloalkyl-C₁-C₄-alkyl or C₃-C₆-cycloalkyl-C₁-C₄-haloalkyl. Specific embodiments thereof can be found in the below Table P2.

In one further particular embodiment R² is hydrogen or C₁-C₆-alkoxy, in particular hydrogen or C₁-C₄-alkoxy, that is in each case unsubstituted or substituted by one, two, three or four or up to the maximum possible number of independently selected substituents R^{2a}; wherein R^{2a} is as defined and preferably defined herein. In one specific embodiment thereof, R² is hydrogen or C₁-C₆-alkoxy, in particular hydrogen or C₁-C₄-alkoxy, more particularly hydrogen, methoxy, ethoxy or 1-methylethoxy. In a further specific embodiment thereof, R² is hydrogen or C₁-C₆-alkoxy, in particular hydrogen or C₁-C₄-alkoxy, that is in each case substituted by one, two, three or four or up to the maximum possible number of independently selected substituents R^{2a}, in particular one, two, three or four Cl, F, CN, OH, C₁-C₂-alkyl, C₁-C₂-alkoxy, C₁-C₂-haloalkoxy, C₁-C₂-alkylthio or C₃-C₆-cycloalkyl. In a further specific embodiment thereof R² is C₁-C₆-haloalkoxy, in particular C₁-C₄-haloalkoxy, for example OCF₃ or OCHF₂. Specific embodiments thereof can be found in the below Table P2.

In one further particular embodiment R² is C₁-C₆-alkoxy, in particular C₁-C₄-alkoxy, that is unsubstituted or substituted by one, two, three or four or up to the maximum possible number of independently selected substituents R^{2a}; wherein R^{2a} is as defined and preferably defined herein. In one specific embodiment thereof, R² is C₁-C₆-alkoxy, in particular C₁-C₄-alkoxy, more particularly methoxy, ethoxy or 1-methylethoxy. In a further specific embodiment thereof, R² is C₁-C₆-alkoxy, in particular C₁-C₄-alkoxy, that is substituted by one, two, three or four or up to the maximum possible number of independently selected substituents R^{2a}, in particular one, two, three or four Cl, F, CN, OH, C₁-C₂-alkyl, C₁-C₂-alkoxy, C₁-C₂-haloalkoxy, C₁-C₂-alkylthio or C₃-C₆-cycloalkyl. In a further specific embodiment thereof R² is C₁-C₆-haloalkoxy, in particular C₁-C₄-haloalkoxy, for example OCF₃ or OCHF₂. Specific embodiments thereof can be found in the below Table P2.

In another embodiment R² is hydrogen, SH, C₁-C₆-alkylthio, C₁-C₆-alkylsulfinyl or C₁-C₆-alkylsulfonyl, in particular hydrogen, SH, C₁-C₄-alkylthio, C₁-C₄-alkylsulfinyl or C₁-C₄-alkylsulfonyl. In another embodiment R² is SH, C₁-C₆-alkylthio, C₁-C₆-alkylsulfinyl or C₁-C₆-alkylsulfonyl, in particular SH, C₁-C₄-alkylthio, C₁-C₄-alkylsulfinyl or C₁-C₄-alkylsulfonyl.

In another preferred embodiment R² is hydrogen or C₂-C₆-alkenyl, in particular hydrogen or C₂-C₄-alkenyl, such as hydrogen or 2-propenyl. In another preferred embodiment R² is C₂-C₆-alkenyl, in particular C₂-C₄-alkenyl, such as 2-propenyl.

In another preferred embodiment R² is hydrogen or C₂-C₆-alkynyl, in particular hydrogen or C₂-C₄-alkynyl, such as hydrogen, 2-propynyl or 2-butynyl. In another preferred embodiment R² is C₂-C₆-alkynyl, in particular C₂-C₄-alkynyl, such as 2-propynyl or 2-butynyl.

According to a further particular embodiment of the invention, R² is hydrogen or C₃-C₈-cycloalkyl, in particular hydrogen or C₃-C₆-cycloalkyl, that is in each case unsubstituted or substituted by one, two, three or four or up to the maximum possible number of independently selected substituents R^{2a}; wherein R^{2a} is as defined and preferably defined herein. In one specific embodiment thereof, R² is hydrogen or C₃-C₈-cycloalkyl, in particular hydrogen or C₃-C₆-cycloalkyl, more particularly hydrogen, cyclopropyl, cyclobutyl, cyclopentyl or cyclohexyl. In a further specific embodiment thereof, R² is hydrogen or C₃-C₈-cycloalkyl, in particular hydrogen or C₃-C₆-cycloalkyl, that is in each case substituted by one, two, three or four or up to the maximum possible number of independently selected substituents R^{2a}, in particular one, two, three or four Cl, F, CN, OH, C₁-C₂-alkyl, C₁-C₂-alkoxy, C₁-C₂-haloalkoxy, C₁-C₂-alkylthio or C₃-C₆-cycloalkyl. In a further specific embodiment thereof R² is hydrogen or C₃-C₈-halocycloalkyl, in particular hydrogen or C₃-C₆-halocycloalkyl, for example hydrogen, 1-Cl-cyclopropyl or 1-F-cyclopropyl. In a further specific embodiment thereof R² is hydrogen or C₃-C₈-CN-cyclooalkyl, in particular hydrogen or C₃-C₆-CN-cycloalkyl, for example hydrogen or 1-CN-cyclopropyl. In a further specific embodiment thereof R² is hydrogen or C₁-C₂-alkyl-C₃-C₈-cycloalkyl, in particular hydrogen or C₁-C₂-alkyl-C₃-C₆-cycloalkyl, for example hydrogen or 1-CH₃-cyclopropyl.

According to a further particular embodiment of the invention, R² is C₃-C₈-cycloalkyl, in particular C₃-C₆-cycloalkyl, that is unsubstituted or substituted by one, two, three or four or up to the maximum possible number of independently selected substituents R^{2a}; wherein R^{2a} is as defined and preferably defined herein. In one specific embodiment thereof, R² is C₃-C₈-cycloalkyl, in particular C₃-C₆-cycloalkyl, more particularly cyclopropyl, cyclobutyl, cyclopentyl or cyclohexyl. In a further specific embodiment thereof, R² is C₃-C₈-cycloalkyl, in particular C₃-C₆-cycloalkyl, that is substituted by one, two, three or four or up to the maximum possible number of independently selected substituents R^{2a}, in particular one, two, three or four Cl, F, CN, OH, C₁-C₂-alkyl, C₁-C₂-alkoxy, C₁-C₂-haloalkoxy, C₁-C₂-alkylthio or C₃-C₆-cycloalkyl. In a further specific embodiment thereof R² is C₃-C₈-halocycloalkyl, in particular C₃-C₆-halocycloalkyl, for example 1 -Cl-cyclopropyl and 1-F-cyclopropyl. In a further specific embodiment thereof R² is C₃-C₈-CN-cyclooalkyl, in particular C₃-C₆-CN-cycloalkyl, for example 1-CN-cyclopropyl. In a further specific embodiment thereof R² is C₁-C₂-alkyl-C₃-C₈-cycloalkyl, in particular C₁-C₂-alkyl-C₃-C₆-cycloalkyl, for example 1-CH₃-cyclopropyl.

According to a further particular embodiment of the invention, R² is hydrogen or C₃-C₈-cycloalkyloxy, in particular hydrogen or C₃-C₆-cycloalkyloxy, wherein the alicyclic moiety is unsubstituted or substituted by one, two, three or four independently selected substituents R^{2a} as defined and preferably defined herein. According to a further particular embodiment of the invention, R² is C₃-C₈-cycloalkyloxy, in particular C₃-C₆-cycloalkyloxy, wherein the alicyclic moiety is unsubstituted or substituted by one, two, three or four independently selected substituents R^{2a} as defined and preferably defined herein.

According to a further particular embodiment of the invention, R² is hydrogen, NH₂, NH(C₁-C₄-alkyl), N(C₁-C₄-alkyl)₂, NH(C₃-C₆-cycloalkyl) or N(C₃-C₆-cycloalkyl)₂, in particular hydrogen, NH₂, NH(C₁-C₂-alkyl), N(C₁-C₂-alkyl)₂, NH(C₃-C₆-cycloalkyl) or N(C₃-C₆-cycloalkyl)₂, wherein in each case the alicyclic or aliphatic moieties are unsubstituted or substituted by one, two, three or four independently selected substituents R^{2a} as defined and preferably defined herein. According to a further particular embodiment of the invention, R² is NH₂, NH(C₁-C₄-alkyl), N(C₁-C₄-alkyl)₂, NH(C₃-C₆-cycloalkyl) or N(C₃-C₆-cycloalkyl)₂, in particular NH₂, NH(C₁-C₂-alkyl), N(C₁-C₂-alkyl)₂, NH(C₃-C₆-cycloalkyl) or N(C₃-C₆-cycloalkyl)₂, wherein in each case the alicyclic or aliphatic moieties are unsubstituted or substituted by one, two, three or four independently selected substituents R^{2a} as defined and preferably defined herein. Specific embodiments thereof can be found in the below Table P2.

According to a further particular embodiment of the invention, R² is hydrogen, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, wherein in each case the alicyclic or aliphatic moieties are unsubstituted or substituted by one, two, three or four independently selected substituents R^{2a} as defined and preferably defined herein. According to a further particular embodiment of the invention, R² is C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, wherein in each case the alicyclic or aliphatic moieties are unsubstituted or substituted by one, two, three or four independently selected substituents R^{2a} as defined and preferably defined herein.

According to a further particular embodiment of the invention, R² is hydrogen, C(=O)-C₁-C₄-alkyl, C(=O)OH or C(=O)-O-C₁-C₄-alkyl, wherein in each case the aliphatic moieties are unsubstituted or substituted by one, two, three or four independently selected substituents R^{2a} as defined and preferably defined herein. According to a further particular embodiment of the invention, R² is C(=O)-C₁-C₄-alkyl, C(=O)OH or C(=O)-O-C₁-C₄-alkyl, wherein in each case the aliphatic moieties are unsubstituted or substituted by one, two, three or four independently selected substituents R^{2a} as defined and preferably defined herein.

According to a further particular embodiment of the invention, R² is hydrogen or C(=O)-C₁-C₄-alkyl, in particular hydrogen or C(=O)-C₁-C₂-alkyl, wherein the alkyl is unsubstituted or substituted by one, two, three or four independently selected substituents R^{2a} as defined and preferably defined herein. According to a further particular embodiment of the invention, R² is C(=O)-C₁-C₄-alkyl, in particular hydrogen or C(=O)-C₁-C₂-alkyl, wherein the alkyl is unsubstituted or substituted by one, two, three or four independently selected substituents R^{2a} as defined and preferably defined herein.

According to a further particular embodiment of the invention, R² is hydrogen, C(=O)OH or C(=O)-O-C₁-C₄-alkyl, in particular hydrogen or C(=O)-O-C₁-C₂-alkyl, wherein the alkyl in each case is unsubstituted or substituted by one, two, three or four independently selected substituents R^{2a} as defined and preferably defined herein. According to a further particular embodiment of the invention, R² is C(=O)OH or C(=O)-O-C₁-C₄-alkyl, in particular C(=O)-O-C₁-C₂-alkyl, wherein the alkyl is unsubstituted or substituted by one, two, three or four independently selected substituents R^{2a} as defined and preferably defined herein.

According to a further particular embodiment of the invention, R² is hydrogen, C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, in particular hydrogen, C(=O)-NH(C₁-C₂-alkyl), C(=O)-N(C₁-C₂-alkyl)₂, C(=O)-NH(C₃-C₆-cycloalkyl) or C(=O)-N(C₃-C₆-cycloalkyl)₂, wherein in each case the alicyclic or aliphatic moieties are unsubstituted or substituted by one, two, three or four independently selected substituents R^{2a} as defined and preferably defined herein. According to a further particular embodiment of the invention, R² is C(=O)-NH(C₁-C₄-alkyl), C(=O)-N(C₁-C₄-alkyl)₂, C(=O)-NH(C₃-C₆-cycloalkyl) or C(=O)-N(C₃-C₆-cycloalkyl)₂, in particular C(=O)-NH(C₁-C₂-alkyl), C(=O)-N(C₁-C₂-alkyl)₂, C(=O)-NH(C₃-C₆-cycloalkyl) or C(=O)-N(C₃-C₆-cycloalkyl)₂, wherein in each case the alicyclic or aliphatic moieties are unsubstituted or substituted by one, two, three or four independently selected substituents R^{2a} as defined and preferably defined herein.

According to one specific embodiment, R¹ is selected from halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl and C₃-C₈-cycloalkyl and R² is hydrogen.

According to a further specific embodiment, R¹ and R² are independently selected from halogen, CN, OH, NH₂, N(CH₃)₂, C₁-C₆-alkyl, C₁-C₆-haloalkyl, C₁-C₆-hydroxyalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, C₂-C₆-alkenyl, C₂-C₆-alkynyl and C₃-C₈-cycloalkyl.

According to a further embodiment, R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle contains one, two, three or four heteroatoms selected from N, O and S, and wherein the carbo- or heterocycle is unsubstituted or carries one, two, three or four substituents R^{12a} independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio,C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S).

According to one particular embodiment of the invention R¹ and R² together with the carbon atom to which they are bound form a saturated or partially unsaturated three-, four-, five-, six- or seven-membered carbocycle which is unsubstituted or carries one, two, three or four independently selected substituents R^{12a} as defined and preferably defined below. According to one specific embodiment, one or two CH₂ groups of the carbocycle may be replaced by a group independently selected from C(=O) and C(=S) to form for example cyclopropanone, cyclopentanone, cyclopropanethione, cyclopentanethione, 5-oxazolone, cyclohexane-1,4-dione or cyclohexane-1,4-dithione.

In one preferred embodiment R¹ and R² together with the carbon atom to which they are bound form a saturated carbocycle, in particular C₃-C₇-cycloalkyl, specifically selected from the group consisting of cyclopropyl, cyclobutyl, cyclopentyl, cyclohexyl and cycloheptyl, wherein the carbocycle in each case is unsubstituted or carries one, two, three or four independently selected substituents R^{12a} as defined and preferably defined below, in particular independently selected from Cl, F, Br, CN, OH, C₁-C₄-alkyl, C₁-C₄-haloalkyl, C₁-C₄-alkoxy, C₁-C₄-haloalkoxy, C₁-C₄-alkoxy-C₁-C₄-alkyl, phenyl and phenoxy.

In a specific embodiment thereof, R¹ and R² together with the carbon atom to which they are bound form a cyclopropyl ring which is unsubstituted or carries one, two, three or four independently selected substituents R^{12a} as defined and preferably defined herein.

According to a further specific embodiment R¹ and R² together with the carbon atom to which they are bound form a cyclobutyl ring which is unsubstituted or carries one, two, three or four independently selected substituents R^{12a} as defined and preferably defined herein.

In still a further specific embodiment of the invention R¹ and R² together with the carbon atom to which they are bound form a cyclopentyl ring which is unsubstituted or carries one, two, three or four independently selected substituents R^{12a} as defined and preferably defined herein.

In yet another preferred embodiment R¹ and R² together with the carbon atom to which they are bound form a cyclohexyl ring which is unsubstituted or carries one, two, three or four independently selected substituents R^{12a} as defined and preferably defined herein.

In another preferred embodiment R¹ and R² together with the carbon atom to which they are bound form a partially unsaturated carbocycle, in particular selected from the group consisting of cyclopentenyl, cyclopentadienyl and cyclohexenyl and wherein the carbocycle is unsubstituted or carries one, two, three or four independently selected substituents R^{12a} as defined and preferably defined below.

According to still another embodiment of the invention R¹ and R² together with the carbon atom to which they are bound form a saturated or partially unsaturated three-, four-, five-, six- or seven-membered heterocycle, in particular a three-, four- or five-membered heterocycle, wherein the heterocycle includes beside carbon atoms one, two, three or four, in particular one or two, heteroatoms independently selected from the group consisting of N, O and S, and wherein the heterocycle is unsubstituted or carries one, two, three or four independently selected substituents R^{12a} as defined and preferably defined below.

In one specific embodiment thereof R¹ and R² form a heterocycle selected from the group consisting of oxirane, aziridine, thiirane, oxetane, azetidine, thiethane, 2-tetrahydrofuranyl, 3-tetrahydrofuranyl, 2-tetrahydrothienyl and 3-tetrahydrothienyl, wherein the heterocycle is unsubstituted or carries one, two, three or four independently selected substituents R^{12a} as defined and preferably defined below.

In a further specific embodiment R¹ and R² together with the carbon atom to which they are bound form an oxirane which is unsubstituted or carries one, two, three or four independently selected substituents R^{12a} as defined and preferably defined below.

In still a further specific embodiment of the invention R¹ and R² together with the carbon atom to which they are bound form an oxetane which is unsubstituted or carries one, two, three or four independently selected substituents R^{12a} as defined and preferably defined below.

R^{12a} according to the invention is in each case independently selected from the group consisting of halogen, CN, NO₂, OH, SH, NH₂, C₁-C₆-alkyl,C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy. In a preferred embodiment R^{12a} is halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio,C₁-C₆-haloalkylthio or C₁-C₄-alkoxy-C₁-C₄-alkyl. In another preferred embodiment R¹² is halogen, CN, C₁-C₆-alkyl,C₁-C₆-haloalkyl, C₁-C₆-alkoxy or C₁-C₆-haloalkoxy. In another preferred embodiment R^{1a2} is halogen, CN or C₁-C₆-alkyl. In yet another preferred embodiment R^{12a} is halogen, in particular fluorine. In a further preferred embodiment R^{12a} is chlorine. In still another preferred aspect of the invention R^{12a} is C₁-C₆-alkyl,in particular C₁-C₄-alkyl, even more preferably methyl.

According to still another embodiment of the invention R¹ and R² together with the carbon atom to which they are bound (denominated C*) form a group (C*=CR¹⁸R¹⁹), wherein R¹⁸, R¹⁹ are independently selected from hydrogen, halogen, CN, NO₂, OH, SH, NH₂, C₁-C₆-alkyl,C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl.

R¹⁸ and R¹⁹ according to the invention are independently selected from the group consisting of hydrogen, halogen, CN, NO₂, OH, SH, NH₂, C₁-C₆-alkyl,C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl. In another preferred embodiment R¹⁸ and R¹⁹ are independently selected from the group consisting of hydrogen, halogen and C₁-C₆-alkyl. In a further preferred embodiment R¹⁸ and R¹⁹ are hydrogen.

Particularly preferred embodiments of R¹ according to the invention are in Table P1 below, wherein each line of lines P1-1 to P1-30 corresponds to one particular embodiment of the invention, wherein P1-1 to P1-30 are also in any combination a preferred embodiment of the present invention.

**Table P1:**

| **line** | **R¹** |
|---|---|
| P1-1 | CH₃ |
| P1-2 | CH₂CH₃ |
| P1-3 | CH₂CH₂CH₃ |
| P1-4 | CH(CH₃)₂ |
| P1-5 | C(CH₃)₃ |
| P1-6 | CH(CH₃)CH₂CH₃ |
| P1-7 | CH₂CH(CH₃)₂ |
| P1-8 | CH₂CH₂CH₂CH₃ |
| P1-9 | CF₃ |
| P1-10 | CHF₂ |
| P1-11 | CH₂F |
| P1-12 | CHCl₂ |
| P1-13 | CH₂Cl |
| P1-14 | CH₂OH |
| P1-15 | CH₂CH₂OH |
| P1-16 | CH₂CH₂CH₂OH |
| P1-17 | CH(CH₃)CH₂OH |
| P1-18 | CH₂CH(CH₃)OH |
| P1-19 | CH₂CH₂CH₂CH₂OH |
| P1-20 | F |
| P1-21 | Cl |
| P1-22 | Br |
| P1-23 | OCH₃ |
| P1-24 | OCH₂CH₃ |
| P1-25 | CH₂CH=CH₂ |
| P1-26 | CN |
| P1-27 | SCH₃ |
| P1-28 | C≡CH |
| P1-29 | CH₂C≡CH |
| P1-30 | C≡CCH₃ |

Particularly preferred embodiments of R² according to the invention are in Table P2 below, wherein each line of lines P2-1 to P2-31 corresponds to one particular embodiment of the invention, wherein P2-1 to P2-31 are also in any combination a preferred embodiment of the present invention.

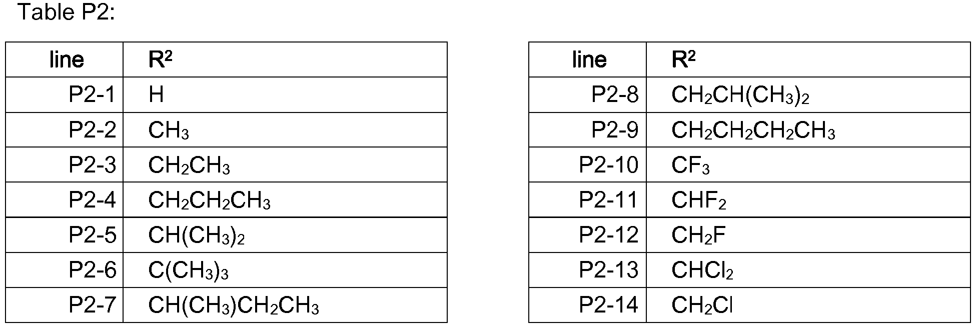

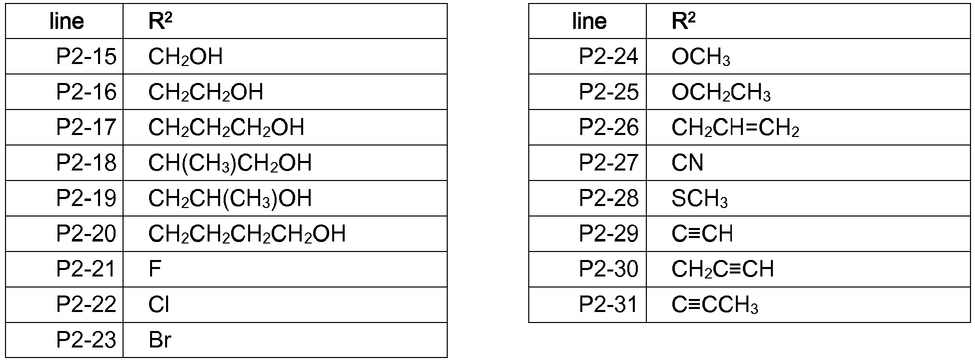

Particularly preferred embodiments of R¹ in combination with R² according to the invention are in Table P12 below, wherein each line of lines P12-1 to P12-29 corresponds to one particular embodiment of the invention, wherein P12-1 to P12-29 are also in any combination a preferred embodiment of the present invention.

**Table P12:**

| **line** | **R¹** | **R²** |
|---|---|---|
| P12-1 | CH₃ | H |
| P12-2 | C₂H₅ | H |
| P12-3 | CH₂CH₂CH₃ | H |
| P12-4 | CH(CH₃)₂ | H |
| P12-5 | CH₂CHCH₂ | H |
| P12-6 | CH₂CH₂OH | H |
| P12-7 | CH₂CH₂CH₂OH | H |
| P12-8 | OCH₃ | H |
| P12-9 | OC₂H₅ | H |
| P12-10 | OCH(CH₃)₂ | H |
| P12-11 | F | H |
| P12-12 | Cl | H |
| P12-13 | Br | H |
| P12-14 | OH | H |
| P12-15 | NH₂ | H |
| P12-16 | N(CH₃)₂ | H |
| P12-17 | CH₃ | CH₃ |
| P12-18 | CH₂CH₃ | CH₃ |
| P12-19 | CH₂CH₃ | CH₂CH₃ |
| P12-20 | 1-triazolyl | H |
| P12-21 | 1-imidazolyl | H |
| P12-22 | 2-pyridinyl | H |
| P12-23 | 3-pyridinyl | H |
| P12-24 | 4-pyridinyl | H |
| P12-25 | =CH₂ | |
| P12-26 | cyclopropyl | |
| P12-27 | cyclobutyl | |
| P12-28 | cyclopentyl | |
| P12-29 | cyclohexyl | |

R³ according to the present invention is hydrogen, halogen, C₁-C₈-alkyl, C₂-C₈-alkenyl, C₂-C₈-alkinyl, C₁-C₆-alkoxy, C₃-C₈-cycloalkyl, C₃-C₈-cycloalkyl-C₁-C₄-alkyl, phenyl, phenyl-C₁-C₄-alkyl, phenyl-C₂-C₄-alkenyl or phenyl-C₂-C₄-alkynyl; wherein the aliphatic, alicyclic and aromatic moieties of R³ are unsubstituted or substituted by one, two, three or four or up to the maximum possible number of R^{3a}, wherein R^{3a} is independently selected from halogen, CN, nitro, C₁-C₄-alkyl, C₁-C₄-halogenalkyl, C₃-C₆-cycloalkyl, C₃-C₆-halocycloalkyl, C₁-C₄-alkoxy and C₁-C₄-halogenalkoxy, with the above proviso.

According to one embodiment, R³ is H with the proviso above. According to one specific embodiment, A at the same time is N.

According to a further embodiment of the invention, R³ is halogen, in particular F, Cl, Br or I, more specifically F or Cl. Specific embodiments thereof can be found in the below Table P3.

According to a further embodiment of the invention, R³ is selected from C₁-C₆-alkyl,C₂-C₆-alkenyl, C₂-C₆-alkynyl, C₃-C₈-cycloalkyl, C₃-C₈-cycloalkyl-C₁-C₄-alkyl, phenyl, phenyl-C₁-C₄-alkyl, phenyl-C₂-C₄-alkenyl and phenyl-C₂-C₄-alkynyl, wherein the R³ are in each case unsubstituted or are substituted by R^{3a} as defined and preferably defined herein. Specific embodiments thereof can be found in the below Table P3.

According to a further embodiment of the invention, R³ is selected from C₁-C₆-alkyl,C₂-C₆-alkenyl, C₂-C₆-alkynyl, C₃-C₈-cycloalkyl, C₃-C₈-cycloalkyl-C₁-C₄-alkyl, wherein the R³ are in each case unsubstituted or are substituted by R^{3a} as defined and preferably defined herein. Specific embodiments thereof can be found in the below Table P3.

According to one particular embodiment, R³ is C₁-C₆-alkyl,in particular C₁-C₄-alkyl, such as CH₃, C₂H₅, CH(CH₃)₂ or C(CH₃)₃. A further embodiment relates to compounds, wherein R³ is C₁-C₆-alkyl, in particular C₁-C₄-alkyl, that is substituted by one, two or three or up to the maximum possible number of identical or different groups R^{3a}, as defined and preferably defined herein. According to a specific embodiment thereof, R³ is C₁-C₆-haloalkyl, in particular C₁-C₄-haloalkyl, more particularly C₁-C₂-haloalkyl such as CF₃ or CHF₂. According to a further specific embodiment thereof, R³ is C₁-C₄-alkoxy-C₁-C₆-alkyl, in particular C₁-C₄-alkoxy-C₁-C₄-alkyl, such as CH₂-OCH₃. Further specific embodiments thereof can be found in the below Table P3.

According to still another embodiment, R³ is C₃-C₈-cycloalkyl-C₁-C₆-alkyl, in particular C₃-C₆-cycloalkyl-C₁-C₄-alkyl. A further embodiment relates to compounds, wherein R³ is C₃-C₈-cycloalkyl-C₁-C₆-alkyl, in particular C₃-C₆-cycloalkyl-C₁-C₄-alkyl, that is substituted by one, two or three or up to the maximum possible number of identical or different groups R^{3a} in the alkyl moiety and/or substituted by one, two, three four or five or up to the maximum possible number of identical or different groups R^{3a} in the cycloalkyl moiety. R^{3a} are in each case as defined and preferably defined herein. Specific embodiments thereof can be found in the below Table P3.

According to another embodiment, R³ is C₂-C₆-alkenyl, in particular C₂-C₄-alkenyl, such as CH=CH₂, CH₂CH=CH₂, CH=CHCH₃ or C(CH₃)=CH₂. A further embodiment relates to compounds, wherein R³ is C₂-C₆-alkenyl, in particular C₂-C₄-alkenyl, that is substituted by one, two or three or up to the maximum possible number of identical or different groups R^{3a} as defined and preferably defined herein. According to a specific embodiment thereof, R³ is C₂-C₆-haloalkenyl, in particular C₂-C₄-haloalkenyl. According to a further specific embodiment thereof, R³ is C₃-C₈-cycloalkyl-C₂-C₆-alkenyl or C₃-C₈-halocycloalkyl-C₂-C₆-alkenyl, in particular C₃-C₆-cycloalkyl-C₂-C₄-alkenyl or C₃-C₆-halocycloalkyl-C₂-C₄-alkenyl. Further specific embodiments thereof can be found in the below Table P3.

According to still another embodiment, R³ is C₂-C₆-alkynyl, in particular C₂-C₄-alkynyl, such as C≡CH, C≡CCH₃, CH₂-C≡C-H or CH₂-C≡C-CH₃. A further embodiment relates to compounds, wherein R³ is C₂-C₆-alkynyl, in particular C₂-C₄-alkynyl, that is substituted by one, two or three or up to the maximum possible number of identical or different groups R^{3a}, as defined and preferably defined herein. According to a specific embodiment thereof, R³ is C₂-C₆-haloalkynyl, in particular C₂-C₄-haloalkynyl. According to a further specific embodiment thereof, R³ is C₃-C₈-cycloalkyl-C₂-C₆-alkynyl or C₃-C₈-halocycloalkyl-C₂-C₆-alkynyl, in particular C₃-C₆-cycloalkyl-C₂-C₄-alkynyl or C₃-C₆-halocycloalkyl-C₂-C₄-alkynyl. Further specific embodiments thereof can be found in the below Table P3.

According to still another embodiment, R³ is phenyl-C₁-C₄-alkyl,in particular phenyl-C₁-C₂-alkyl, such as benzyl, wherein the alkyl moiety in each case is unsubstituted or carries one, two or three R^{3a} as defined and preferably defined herein, in particular selected from halogen, in particular F and Cl, C₁-C₄-alkoxy, in particular OCH₃, and CN, and wherein the phenyl in each case is unsubstituted or carries one, two or three R^{3a} as as defined and preferably defined herein, in particular selected from halogen, in particular Cl and F, C₁-C₄-alkoxy, in particular OCH₃, C₁-C₄-alkyl, in particular CH₃ or C₂H₅, and CN. Specific embodiments thereof can be found in the below Table P3.

According to still another embodiment, R³ is phenyl-C₂-C₄-alkenyl, in particular phenyl-C₂-C₃-alkenyl, such as phenylethenyl, wherein the alkenyl moiety in each case is unsubstituted or carries one, two or three R^{3a} as defined and preferably defined herein, in particular selected from halogen, in particular F and Cl, C₁-C₄-alkoxy, in particular OCH₃, and CN, and wherein the phenyl in each case is unsubstituted or carries one, two or three R^{32b} as defined and preferably defined herein, in particular selected from halogen, in particular Cl and F, C₁-C₄-alkoxy, in particular OCH₃, C₁-C₄-alkyl, in particular CH₃ or C₂H₅, and CN.According to still another embodiment, R³ is phenyl-C₂-C₄-alkynyl, in particular phenyl-C₂-C₃-alkynyl, such as phenylethinyl, wherein the alkynyl moiety in each case is unsubstituted or carries one, two or three R^{3a}, as defined and preferably defined herein, in particular selected from halogen, in particular F and Cl, C₁-C₄-alkoxy, in particular OCH₃, and CN, and wherein the phenyl in each case is unsubstituted or carries one, two or three R^{3a} as defined and preferably defined herein, in particular selected from halogen, in particular Cl and F, C₁-C₄-alkoxy, in particular OCH₃, C₁-C₄-alkyl, in particular CH₃ or C₂H₅, and CN. Specific embodiments thereof can be found in the below Table P3.

According to still another embodiment, R³ is C₃-C₈-cycloalkyl, in particular C₃-C₆-cycloalkyl, such as C₃H₅ (cyclopropyl), C₄H₇ (cyclobutyl), cyclopentyl or cyclohexyl. A further embodiment relates to compounds, wherein R³ is C₃-C₈-cycloalkyl, in particular C₃-C₆-cycloalkyl, such as C₃H₅ (cyclopropyl) or C₄H₇ (cyclobutyl), that is substituted by one, two, three four or five or up to the maximum possible number of identical or different groups R^{3a} as defined and preferably defined herein. According to a specific embodiment thereof, R³ is C₃-C₈-halocycloalkyl, in particular C₃-C₆-halocycloalkyl, such as halocyclopropyl, in particular 1-F-cyclopropyl or 1-Cl-cyclopropyl. According to a further specific embodiment thereof, R³ is C₃-C₈-cycloalkyl-C₃-C₈-cycloalkyl, in particular C₃-C₆-cycloalkyl-C₃-C₆-cycloalkyl, wherein each of said cycloalkyl-cycloalkyl moieties is unsubstituted or carries one, two or three R^{3a} as defined and preferably defined herein, such as 1-cyclopropyl-cyclopropyl or 2-cyclopropyl-cyclopropyl. Specific embodiments thereof can be found in the below Table P3.

According to still another embodiment, R³ is phenyl, wherein the phenyl is unsubstituted or carries one, two, three, four or five independently selected R^{3a} as defined and preferably defined herein, in particular selected from halogen, in particular Cl and F, C₁-C₄-alkoxy, in particular OCH₃, C₁-C₄-alkyl, in particular CH₃ or C₂H₅, and CN. Specific embodiments thereof can be found in the below Table P3.

In a further embodiment of the invention, R³ is selected from hydrogen, C₁-C₆-alkyl, C₂-C₆-alkenyl, C₂-C₆-alkynyl and C₃-C₆-cycloalkyl, wherein the R³ are in each case unsubstituted or are substituted by R^{3a} as defined and preferably defined herein. In each case, the substituents may also have the preferred meanings for the respective substituent as defined above. Specific embodiments thereof can be found in the below Table P3.

Particularly preferred embodiments of R³ according to the invention are in Table P3 below, wherein each line of lines P3-1 to P3-164 corresponds to one particular embodiment of the invention, wherein P3-1 to P3-164 are also in any combination a preferred embodiment of the present invention.

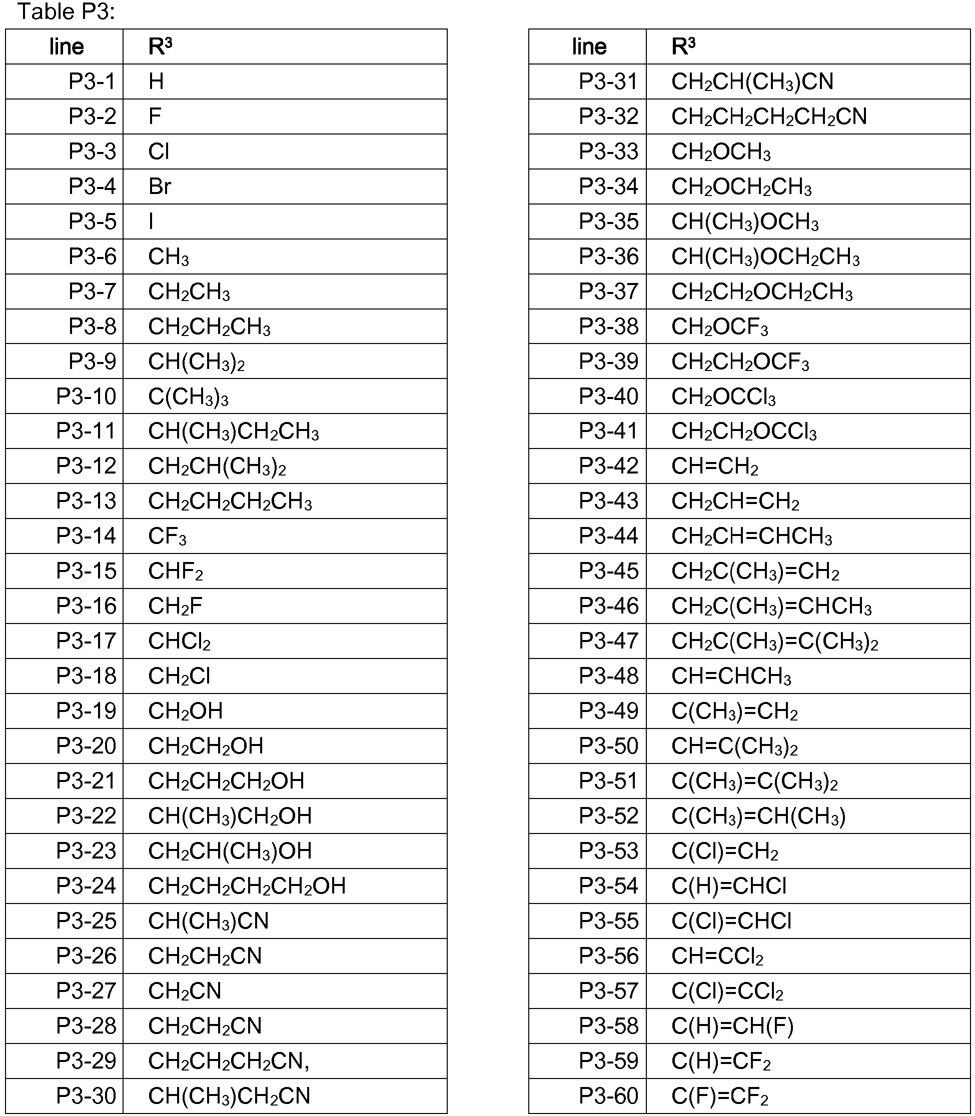

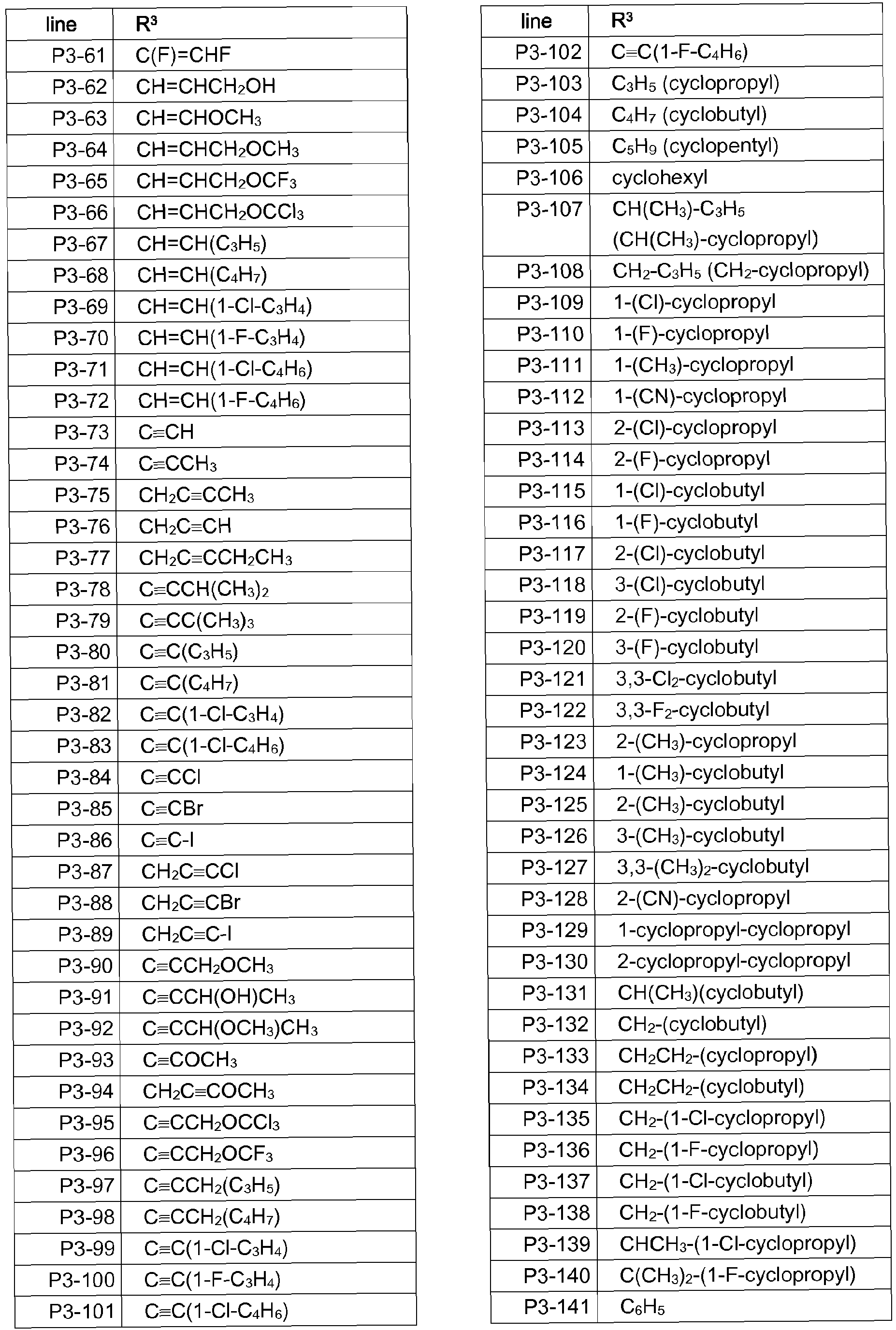

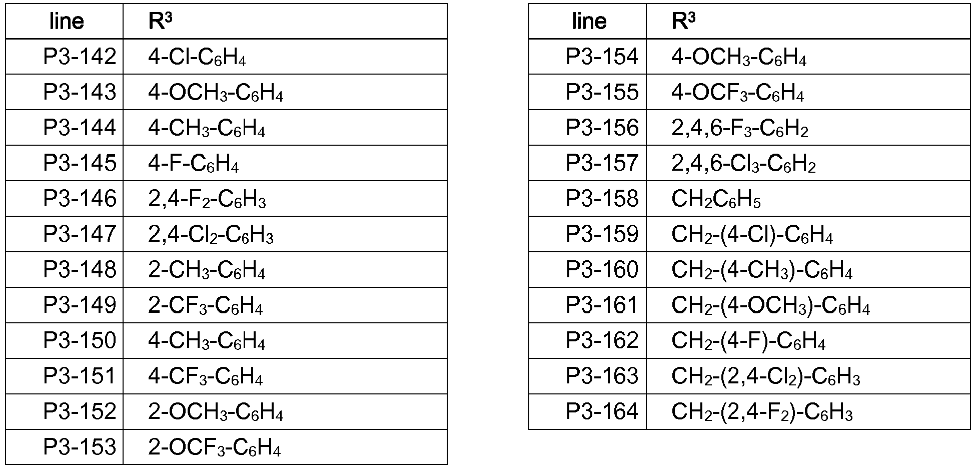

R^{3a} are the possible substituents for R³.

R^{3a} according to the invention is independently selected from halogen, OH, CN, nitro, C₁-C₄-alkyl, C₁-C₄-halogenalkyl, C₁-C₄-alkoxy, C₃-C₈-cycloalkyl, C₃-C₈-halocycloalkyl and C₁-C₄-halogenalkoxy.

According to one embodiment R^{3a} is independently selected from halogen, OH, CN, C₁-C₂-alkyl, C₁-C₂-halogenalkyl, C₁-C₂-alkoxy, C₃-C₆-cycloalkyl, C₃-C₆-halocycloalkyl and C₁-C₂-halogenalkoxy. Specifically, R^{3a} is independently selected from F, Cl, OH, CN, C₁-C₂-alkoxy, C₁-C₂-alkyl, C₁-C₂-halogenalkyl, cyclopropyl, 1-F-cyclopropyl, 1-Cl-cyclopropyl and C₁-C₂-halogenalkoxy.

If R^{3a} are the possible substituents for any cycloalkyl and/or phenyl moiety of R³, the following preferred menaings of R³ are subject of the present invention:
R^{3a} according to this embodiment of the invention is independently selected from halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy.

According to one embodiment thereof R^{3a} is independently selected from halogen, CN, nitro, C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{3a} is independently selected from F, Cl, OH, CN, nitro, CH₃, OCH₃, cyclopropyl, 1-F-cyclopropyl, 1-Cl-cyclopropyl and halogenmethoxy.

According to the invention, p is 0 or 1, i.e. R¹¹ is present or not present.

According to one embodiment, p is 0.

According to a further embodiment, p is 1. R¹¹ stands for the group Z-Y wherein said group is bound to the phenyl via Y, wherein Y is a direct bond or a divalent group selected from the group consisting of -O-, -S-, -SO-, -SO₂-, -NH-, -N(C₁-C₄-alkyl)-, -CR¹²R¹³-, -CR¹²R¹³-CR¹⁴R¹⁵-, - CR¹⁶=CR¹⁷-and -C≡C-; wherein R¹²,R¹³,R¹⁴,R¹⁵,R¹⁶ and R¹¹ are independently selected from hydrogen, halogen, CN, nitro, OH, C₁-C₄-alkyl, C₁-C₄-halogenalkyl, C₁-C₄-alkoxy and C₁-C₄-halogenalkoxy.

According to an embodiment, Y is selected from a direct bond, -O-, -CR¹²R¹³-, -CR¹²R¹³-CR¹⁴R¹⁵-, -CR¹⁶=CR¹⁷-and -C≡C-; wherein R¹²,R¹³,R¹⁴,R¹⁵,R¹⁶ and R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy.

According to one embodiment, Z-Y is attached to the ortho-position (2-position).

According to a further embodiment, Z-Y is attached to the meta-position (3-position).

According to one embodiment, Z-Y is attached to the para-position (4-position).

According to one embodiment, Y is a direct bond. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to a further embodiment, Y is -0-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -S-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -SO-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -SO₂-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -NH-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -N(C₁-C₄-alkyl)-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -CR¹²R¹³-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

R¹² and R¹³ are independently selected from hydrogen, halogen, CN, nitro, OH, C₁-C₄-alkyl, C₁-C₄-halogenalkyl, C₁-C₄-alkoxy and C₁-C₄-halogenalkoxy.

In one preferred embodiment R¹² and R¹³ are independently selected from hydrogen and halogen, in particular hydrogen, fluorine and chlorine. In a further preferred embodiment R¹² and R¹³ are independently selected from hydrogen and C₁-C₄-alkyl, in particular hydrogen, methyl and ethyl. In a preferred embodiment, R¹² and R¹³ are independently selected from hydrogen and C₁-C₄-alkoxy, in particular hydrogen, methoxy and ethoxy. In another preferred embodiment, R¹² and R¹³ are independently selected from hydrogen and CN. In yet another preferred embodiment R¹² and R¹³ are independently selected from hydrogen and OH.

According to still a further embodiment, Y is -CR¹²R¹³-CR¹⁴R¹⁵-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

R¹²,R¹³,R¹⁴ and R¹⁵ are independently selected from hydrogen, halogen, CN, nitro, OH, C₁-C₄-alkyl, C₁-C₄-halogenalkyl, C₁-C₄-alkoxy and C₁-C₄-halogenalkoxy.

In one preferred embodiment R¹²,R¹³,R¹⁴ and R¹⁵ are independently selected from hydrogen and halogen, in particular hydrogen, fluorine and chlorine. In a further preferred embodiment R¹²,R¹³,R¹⁴ and R¹⁵ are independently selected from hydrogen and C₁-C₄-alkyl, in particular hydrogen, methyl and ethyl. In a preferred embodiment, R¹²,R¹³,R¹⁴ and R¹⁵ are independently selected from hydrogen and C₁-C₄-alkoxy, in particular hydrogen, methoxy and ethoxy. In another preferred embodiment, R¹²,R¹³,R¹⁴ and R¹⁵ are independently selected from hydrogen and CN. In yet another preferred embodiment R¹²,R¹³,R¹⁴ and R¹⁵ are independently selected from hydrogen and OH.

According to still a further embodiment, Y is -CR¹⁶=CR¹⁷-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position). R¹⁶ and R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy.

In one preferred embodiment R¹⁶ and R¹⁷ are independently selected from hydrogen and halogen, in particular hydrogen, fluorine and chlorine. In a further preferred embodiment R¹⁶ and R¹⁷ are independently selected from hydrogen and C₁-C₄-alkyl, in particular hydrogen, methyl and ethyl. In a preferred embodiment, R¹⁶ and R¹⁷ are independently selected from hydrogen and C₁-C₄-alkoxy, in particular hydrogen, methoxy and ethoxy. In another preferred embodiment, R¹⁶ and R¹⁷ are independently selected from hydrogen and CN. In yet another preferred embodiment R¹⁶ and R¹⁷ are independently selected from hydrogen and OH.

According to still a further embodiment, Y is -C≡C-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

In general, R¹²,R¹³,R¹⁴,R¹⁵,R¹⁶,R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy.In one preferred embodiment of the invention R¹²,R¹³,R¹⁴,R¹⁵,R¹⁶ and R¹⁷ are independently selected from hydrogen and halogen, in particular hydrogen, fluorine and chlorine. In a further preferred embodiment R¹²,R¹³,R¹⁴,R¹⁵,R¹⁶ and R¹⁷ are independently selected from hydrogen and C₁-C₄-alkyl, in particular hydrogen, methyl and ethyl. In a preferred embodiment, R¹²,R¹³,R¹⁴,R¹⁵,R¹⁶ and R¹⁷ are independently selected from hydrogen and C₁-C₄-alkoxy, in particular hydrogen, methoxy and ethoxy. In another preferred embodiment, R¹²,R¹³,R¹⁴,R¹⁵,R¹⁶ and R¹⁷ are independently selected from hydrogen and CN. In yet another preferred embodiment R¹²,R¹³,R¹⁴,R¹⁵,R¹⁶ and R¹¹ are independently selected from hydrogen and OH.

According to one embodiment, Z is phenyl that is unsubstituted (m=0) or substituted by (R^{L})ₘ. According to the invention, there can be zero, one, two, three, four or five R^{L} present, namely for m is 0, 1, 2, 3, 4 or 5. In particular, m is 0, 1, 2, 3 or 4.

According to one embodiment, m is 0.

According to a further embodiment, m is 1, 2, 3 or 4, in particular 1, 2 or 3, more specifically 1 or 2. According to one specific embodiment thereof, m is 1, according to a further specific embodiment, m is 2.

According to still a further embodiment, m is 2, 3 or 4.

According to still a further embodiment, m is 3.

According to one embodiment of the invention, one R^{L} is attached to the para-position (4-position).

According to a further embodiment of the invention, one R^{L} is attached to the meta-position (3-position).

According to a further embodiment of the invention, one R^{L} is attached to the ortho-position (2-position).

According to a further embodiment of the invention, two R^{L} are attached in 2,4-position.

According to a further embodiment of the invention, two R^{L} are attached in 2,3-position.

According to a further embodiment of the invention, two R^{L} are attached in 2,5-position.

According to a further embodiment of the invention, two R^{L} are attached in 2,6-position.

According to a further embodiment of the invention, two R^{L} are attached in 3,4-position.

According to a further embodiment of the invention, two R^{L} are attached in 3,5-position.

According to a further embodiment of the invention, three R^{L} are attached in 2,4,6-position.

For every R^{L} that is present in the inventive compounds, the following embodiments and preferences apply independently of the meaning of any other R^{L} that may be present in the phenyl ring. Furthermore, the particular embodiments and preferences given herein for R^{L} apply independently for each of m=1, m=2, m=3, m= 4 and m=5.

Each R^{L} is independently selected from halogen, CN, NO₂, OH, C₁-C₆-alkyl,C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cydoalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl,wherein the aliphatic, alicyclic and aromatic moieties of R^{L} are unsubstituted or substituted by one, two, three or four or up to the maximum possible number of R^{La}; wherein R^{La} is independently selected from halogen, CN, NO₂, OH, SH, NH₂, C₁-C₆-alkyl,C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio.

According to one embodiment, R^{L} is independently selected from halogen, CN, NO₂, OH, SH, C₁-C₆-alkyl,C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R^{L} is unsubstituted or further substituted by one, two, three or four independently selected R^{La}, wherein R^{La} is as defined and preferably defined herein.

According to a further embodiment, R^{L} is independently selected from halogen, CN, NO₂, C₁-C₄-alkyl, C₁-C₄-alkoxy, C₂-C₄-alkenyl, C₂-C₄-alkynyl, C₃-C₆-cycloalkyl, C₃-C₆-cycloalkyloxy, NH₂, NH(C₁-C₄₂-alkyl), N(C₁-C₂-alkyl)₂, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl), wherein each of R^{L} is unsubstituted or further substituted by one, two, three or four independently selected R^{La}, wherein R^{La} is as defined and preferably defined herein.

According to a further embodiment, R^{L} is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R^{L} is independently selected from halogen, CN, NO₂, C₁-C₂-alkyl, C₁-C₂-haloalkyl, C₁-C₂-alkoxy, C₁-C₂-haloalkoxy, S(C₁-C₂-alkyl), S(O)(C₁-C₂-alkyl), S(O)₂(C₁-C₂-alkyl), C(=O)(OH) and C(=O)(O-C₁-C₂-alkyl).

According to a further embodiment, R^{L} is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(O)₂(C₁-C₄-alkyl).

According to still a further specific embodiment, R^{L} is independently selected from halogen, in particular from Br, F and Cl, more specifically from F and Cl.

According to a further specific embodiment, R^{L} is CN.

According to one further embodiment R^{L} is NO₂.

According to one further embodiment R^{L} is OH.

According to one further embodiment R^{L} is SH.

According to a further specific embodiment, R^{L} is C₁-C₆-alkyl, in particular C₁-C₄-alkyl, such as CH₃. Further appropriate alkyls are ethyl, n-propyl, i-propyl, n-butyl, i-butyl and t-butyl. According to a further specific embodiment, R^{L} is C₁-C₆-haloalkyl, in particular C₁-C₄-haloalkyl, such as CF₃, CHF₂, CH₂F, CCl₃, CHCl₂ or CH₂Cl.

According to a further specific embodiment R^{L} is C₁-C₆-alkyl, preferably C₁-C₄-alkyl, substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R^{L} is CH₂OH. According to a further specific embodiment R^{L} is C₁-C₆-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH-₂CH₂CH₂CN. In a special embodiment R^{L} is CH₂CH₂CN. In a further special embodiment R⁴ is CH(CH₃)CN. According to a further specific embodiment R^{L} is C₁-C₄-alkoxy-C₁-C₆-alkyl, more preferably C₁-C₄-alkoxy-C₁-C₄-alkyl. In a special embodiment R^{L} is CH₂OCH₃. In a further special embodiment R^{L} is CH₂CH₂OCH₃. In a further special embodiment R^{L} is CH(CH₃)OCH₃. In a further special embodiment R^{L} is CH(CH₃)OCH₂CH₃. In a further special embodiment R^{L} is CH₂CH₂OCH₂CH₃. According to a further specific embodiment R^{L} is C₁-C₄-haloalkoxy-C₁-C₆-alkyl, more preferably C₁-C₄-alkoxy-C₁-C₄-alkyl. In a special embodiment R^{L} is CH₂OCF₃. In a further special embodiment R^{L} is CH₂CH₂OCF₃. In a further special embodiment R^{L} is CH₂OCCl₃. In a further special embodiment R^{L} is CH₂CH₂OCCl₃.

According to a further specific embodiment, R^{L} is C₁-C₆-alkoxy, in particular C₁-C₄-alkoxy, more specifically C₁-C₂-alkoxy such as OCH₃ or OCH₂CH₃.

According to a further specific embodiment, R^{L} is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R^{L} is C₂-C₆-alkenyl or C₂-C₆-haloalkenyl, in particular C₂-C₄-alkenyl or C₂-C₄-haloalkenyl, such as CH=CH₂, CH₂CH=CH₂, CH=CHCH₃ or C(CH₃)=CH₂.

According to a further specific embodiment R^{L} is C₂-C₆-alkenyl, preferably C₂-C₄-alkenyl, substituted by OH, more preferably, CH=CHOH, CH=CHCH₂OH, C(CH₃)=CHOH, CH=C(CH₃)OH. In a special embodiment R^{L} is CH=CHOH. In a further special embodiment R^{L} is CH=CHCH₂OH. According to a further specific embodiment R^{L} is C₁-C₄-alkoxy-C₂-C₆-alkenyl, more preferably C₁-C₄-alkoxy-C₂-C₄-alkenyl. In a special embodiment R^{L} is CH=CHOCH₃. In a further special embodiment R^{L} is CH=CHCH₂OCH₃. According to a further specific embodiment R^{L} is C₁-C₄-haloalkoxy-C₂-C₆-alkenyl, more preferably C₁-C₄-haloalkoxy-C₂-C₄-alkenyl. In a special embodiment R^{L} is CH=CHOCF₃. In a further special embodiment R^{L} is CH=CHCH₂OCF₃. In a further special embodiment R^{L} is CH=CHOCCl₃. In a further special embodiment R^{L} is CH=CHCH₂OCCl₃. According to a further specific embodiment R^{L} is C₃-C₈-cycloalkyl-C₂-C₆-alkenyl, preferably C₃-C₆-cycloalkyl-C₂-C₄-alkenyl. According to a further specific embodiment R^{L} is C₃-C₆-halocycloalkyl-C₂-C₄-alkenyl, preferably C₃-C₈-halocycloalkyl-C₂-C₆-alkenyl.

According to still a further embodiment, R^{L} is C₂-C₆-alkynyl or C₂-C₆-haloalkynyl, in particular C₂-C₄-alkynyl or C₂-C₄-haloalkynyl, such as CΞCH, CH₂CCH or CH₂CCCH₃.

According to a further specific embodiment R^{L} is C₂-C₆-alkynyl, preferably C₂-C₄-alkynyl, substituted by OH, more preferably, CCOH, CH₂CCOH. In a special embodiment R^{L} is CCOH. In a further special embodiment R^{L} is CH₂CCOH. According to a further specific embodiment R^{L} is C₁-C₄-alkoxy-C₂-C₆-alkynyl, more preferably C₁-C₄-alkoxy-C₂-C₄-alkynyl. In a special embodiment R^{L} is CCOCH₃. In a further special embodiment R^{L} is CH₂CCOCH₃. According to a further specific embodiment R^{L} is C₁-C₄-haloalkoxy-C₂-C₆-alkynyl, more preferably C₁-C₄-haloalkoxy-C₂-C₄-alkynyl. In a special embodiment R^{L} is CCOCF₃. In a further special embodiment R^{L} is CH₂CCOCF₃. In a further special embodiment R^{L} is CCOCCl₃. In a further special embodiment

R^{L} is CH₂CCOCCl₃. According to a further specific embodiment R^{L} is C₃-C₈-cycloalkyl-C₂-C₆-alkynyl, preferably C₃-C₆-cycloalkyl-C₂-C₄-alkynyl. According to a further specific embodiment R^{L} is C₃-C₆-halocycloalkyl-C₂-C₄-alkynyl, preferably C₃-C₈-halocycloalkyl-C₂-C₆-alkynyl.

According to one another embodiment R^{L} is C₃-C₈-cycloalkyl, preferably cyclopropyl, cyclobutyl, cyclopentyl or cyclohexyl, in particular cyclopropyl or cyclobutyl. In a special embodiment R^{L} is cyclopropyl. In a further special embodiment R^{L} is cyclobutyl. In a further special embodiment R⁴ is cyclopentyl. In a further special embodiment R^{L} is cyclohexyl.

According to one another embodiment R^{L} is C₃-C₈-cycloalkoxy, preferably C₃-C₆-cycloalkoxy. In a special embodiment R^{L} is O-cyclopropyl.

According to a specific embodiment R^{L} is C₃-C₈-halocycloalkyl, more preferably fully or partially halogenated C₃-C₆-cycloalkyl. In a special embodiment R^{L} is fully or partially halogenated cyclopropyl. In a further special embodiment R^{L} is 1-Cl-cyclopropyl. In a further special embodiment R^{L} is 2-Cl-cyclopropyl. In a further special embodiment R^{L} is 1-F-cyclopropyl. In a further special embodiment R^{L} is 2-F-cyclopropyl. In a further special embodiment R^{L} is fully or partially halogenated cyclobutyl. In a further special embodiment R^{L} is 1-Cl-cyclobutyl. In a further special embodiment R^{L} is 1-F-cyclobutyl. In a further special embodiment R^{L} is 3,3-Cl₂-cyclobutyl. In a further special embodiment R^{L} is 3,3-F₂-cyclobutyl. According to a specific embodiment R^{L} is C₃-C₈-cycloalkyl substituted by C₁-C₄-alkyl, more preferably is C₃-C₆-cycloalkyl substituted by C₁-C₄-alkyl. In a special embodiment R^{L} is 1-CH₃-cyclopropyl. According to a specific embodiment R^{L} is C₃-C₈-cycloalkyl substituted by CN, more preferably is C₃-C₆-cycloalkyl substituted by CN. In a special embodiment R^{L} is 1-CN-cyclopropyl.According to a further specific embodiment R^{L} is C₃-C₈-cycloalkyl-C₃-C₈-cycloalkyl, preferably C₃-C₆-cycloalkyl-C₃-C₆-cycloalkyl. In a special embodiment R^{L} is cyclopropyl-cyclopropyl. In a special embodiment R^{L} is 2-cyclopropyl-cyclopropyl. According to a further specific embodiment R^{L} is C₃-C₈-cycloalkyl-C₃-C₈-halocycloalkyl, preferably C₃-C₆-cycloalkyl-C₃-C₆-halocycloalkyl.

According to one another embodiment R^{L} is C₃-C₈-cycloalkyl-C₁-C₄-alkyl, preferably C₃-C₆-cycloalkyl-C₁-C₄-alkyl. In a special embodiment R^{L} is CH(CH₃)(cyclopropyl). In a further special embodiment R^{L} is CH₂-(cyclopropyl).

According to a further preferred embodiment R^{L} is C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R^{L} is C₃-C₈-cycloalkyl-C₁-C₄-haloalkyl, C₃-C₆-cycloalkyl-C₁-C₄-haloalkyl. According to a specific embodiment R^{L} is C₃-C₈-halocycloalkyl-C₁-C₄-alkyl, C₃-C₆-halocycloalkyl-C₁-C₄-alkyl. In a special embodiment R^{L} is fully or partially halogenated cyclopropyl-C₁-C₄-alkyl. In a further special embodiment R^{L} is 1-Cl-cyclopropyl-C₁-C₄-alkyl. In a further special embodiment R^{L} is 1-F-cyclopropyl-C₁-C₄-alkyl.

According to one another embodiment R^{L} is NH₂.

According to one another embodiment R^{L} is NH(C₁-C₄-alkyl). According to a specific embodiment R^{L} is NH(CH₃). According to a specific embodiment R^{L} is NH(CH₂CH₃). According to a specific embodiment R^{L} is NH(CH₂CH₂CH₃). According to a specific embodiment R^{L} is NH(CH(CH₃)₂). According to a specific embodiment R^{L} is NH(CH₂CH₂CH₂CH₃). According to a specific embodiment R^{L} is NH(C(CH₃)₃).

According to one another embodiment R^{L} is N(C₁-C₄-alkyl)₂. According to a specific embodiment R^{L} is N(CH₃)₂. According to a specific embodiment R^{L} is N(CH₂CH₃)₂. According to a specific embodiment R^{L} is N(CH₂CH₂CH₃)₂. According to a specific embodiment R^{L} is N(CH(CH₃)₂)₂. According to a specific embodiment R^{L} is N(CH₂CH₂CH₂CH₃)₂. According to a specific embodiment R^{L} is NH(C(CH₃)₃)₂.

According to one another embodiment R^{L} is NH(C₃-C₈-cycloalkyl) preferably NH(C₃-C₆-cycloalkyl). According to a specific embodiment R^{L} is NH(cyclopropyl). According to a specific embodiment R^{L} is NH(cyclobutyl). According to a specific embodiment R^{L} is NH(cyclopentyl). According to a specific embodiment R^{L} is NH(cyclohexyl).

According to one another embodiment R^{L} is N(C₃-C₈-cycloalkyl)₂ preferably N(C₃-C₆-cycloalkyl)₂. According to a specific embodiment R^{L} is N(cyclopropyl)₂. According to a specific embodiment R^{L} is N(cyclobutyl)₂. According to a specific embodiment R^{L} is N(cyclopentyl)₂. According to a specific embodiment R^{L} is N(cyclohexyl)₂.

According to still a further embodiment, R^{L} is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R^{L} is C(=O)(OH) or C(=O)(O-C₁-C₄-alkyl), in particular C(=O)(OCH₃).

According to one another embodiment R^{L} is C(=O)(-C₁-C₄-alkyl). According to a specific embodiment R^{L} is C(=O)CH₃.. According to a further specific embodiment R^{L} is C(=O)CH₂CH₃. According to a further specific embodiment R^{L} is C(=O)CH₂CH₂CH₃. According to a further specific embodiment R^{L} is C(=O)CH(CH₃)₂. According to a further specific embodiment R^{L} is C(=O)C(CH₃)₃.

According to one another embodiment R^{L} is C(=O)OH.

According to one another embodiment R^{L} is C(=O)(-O-C₁-C₄-alkyl). According to a specific embodiment R^{L} is C(=O)OCH₃.. According to a further specific embodiment R^{L} is C(=O)OCH₂CH₃. According to a further specific embodiment R^{L} is C(=O)OCH₂CH₂CH₃. According to a further specific embodiment R^{L} is C(=O)OCH(CH₃)₂. According to a further specific embodiment R^{L} is C(=O)OC(CH₃)₃.

According to one another embodiment R^{L} is C(=O)-NH(C₁-C₄-alkyl). According to a specific embodiment R^{L} is C(=O)NHCH₃.. According to a further specific embodiment R^{L} is C(=O)NHCH₂CH₃. According to a further specific embodiment R^{L} is C(=O)NHCH₂CH₂CH₃. According to a further specific embodiment R^{L} is C(=O)NHCH(CH₃)₂. According to a further specific embodiment R^{L} is C(=O)NHC(CH₃)₃.

According to one another embodiment R^{L} is C(=O)-N(C₁-C₄-alkyl)₂. According to a specific embodiment R^{L} is C(=O)N(CH₃)₂. According to a further specific embodiment R^{L} is C(=O)N(CH₂CH₃)₂. According to a further specific embodiment R^{L} is C(=O)N(CH₂CH₂CH₃)₂. According to a further specific embodiment R^{L} is C(=O)N(CH(CH₃)₂)₂. According to a further specific embodiment R^{L} is C(=O)N(C(CH₃)₃)₂.

According to one another embodiment R^{L} is C(=O)-NH(C₃-C₆-cycloalkyl). According to a specific embodiment R^{L} is C(=O)NH(cyclopropyl).. According to a further specific embodiment R^{L} is C(=O)NH(cyclobutyl). According to a further specific embodiment R^{L} is C(=O)NH(cyclopentyl).According to a further specific embodiment R^{L} is C(=O)NH(cyclohexyl).

According to one another embodiment R^{L} is C(=O)-N(C₃-C₆-cycloalkyl)₂. According to a specific embodiment R^{L} is C(=O)N(cyclopropyl)₂. According to a further specific embodiment R^{L} is C(=O)N(cyclobutyl)₂. According to a further specific embodiment R^{L} is C(=O)N(cyclopentyl)₂. According to a further specific embodiment R^{L} is C(=O)N(cyclohexyl)₂.

According to still a further embodiment, R^{L} is selected from S(C₁-C₂-alkyl), S(O)(C₁-C₂-alkyl) and S(O)₂(C₁-C₂-alkyl), in particular SCH₃, S(O)(CH₃) and S(O)₂(CH₃). According to a specific embodiment R^{L} is selected from S(C₁-C₂-haloalkyl), S(O)(C₁-C₂-haloalkyl) and S(O)₂(C₁-C₂-haloalkyl), such as SO₂CF₃.

Particularly preferred embodiments of R^{L} according to the invention are in Table PL below, wherein each line of lines PL-1 to PL-16 corresponds to one particular embodiment of the invention, wherein PL-1 to PL-16 are also in any combination with one another a preferred embodiment of the present invention. Thereby, for every R^{L} that is present in the inventive compounds, these specific embodiments and preferences apply independently of the meaning of any other R^{L} that may be present in the phenyl ring:

**Table PL:**

| **No.** | **R^{L}** |
|---|---|
| PL-1 | Cl |
| PL-2 | F |
| PL-3 | CN |
| PL-4 | NO₂ |
| PL-5 | CH₃ |
| PL-6 | CH₂CH₃ |
| PL-7 | CF₃ |
| PL-8 | CHF₂ |
| PL-9 | OCH₃ |
| PL-10 | OCH₂CH₃ |
| PL-11 | OCF₃ |
| PL-12 | OCHF₂ |
| PL-13 | SCH₃ |
| PL-14 | SOCH₃ |
| PL-15 | SO₂CH₃ |
| PL-16 | CO₂CH₃ |

Particularly preferred embodiments of (R^{L})ₘ if Z is phenyl according to the invention are in Table P4 below, wherein each line of lines P4-1 to P4-155 corresponds to one particular embodiment of the invention, wherein P4-1 to P4-155 are also in any combination a preferred embodiment of the present invention.

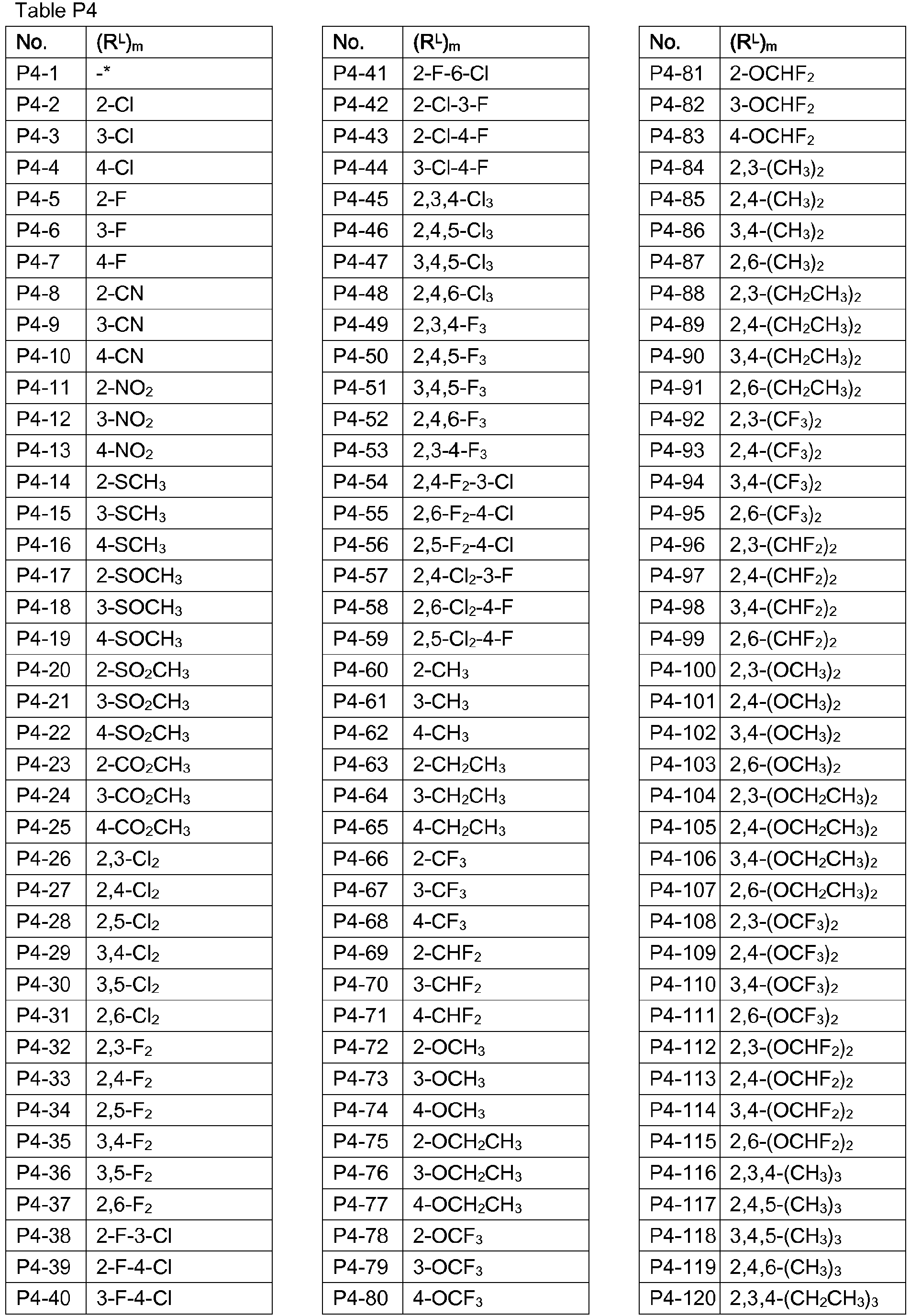

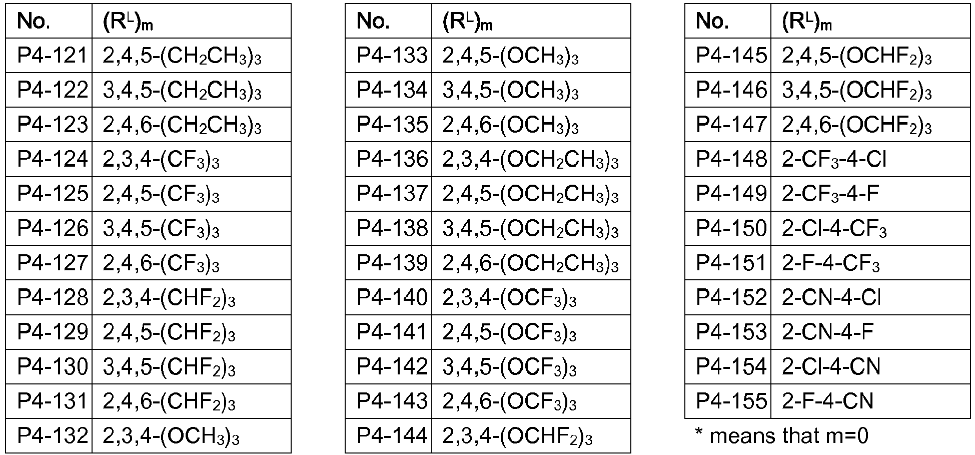

In another embodiment Z is a five- or six-membered heteroaryl that is unsubstituted (m=0) or substituted by (R^{L})ₘ. According to one embodiment thereof, Z is a five-membered heteroaryl which is unsubstituted or carries one, two or three independently selected radicals R^{L} as defined or preferably defined below. According to a further embodiment thereof, Z is a six-membered heteroaryl ahich is unsubstituted or carries one, two or three independently selected radicals R^{L} as defined or preferably defined below.

According to one embodiment thereof, Z is selected from the group consisting of pyrimidin-2-yl, pyrimidin-3-yl, pyrimidin-4-yl, pyridin-2-yl, pyridin-3-yl, pyridin-4-yl, thiazol-2-yl, thiazol-4-yl, thiazol-5-yl, isothiazol-3-yl, isothiazol-4-yl, isothiazol-5-yl, pyrazin-2-yl, pyridazin-3-yl, 1,3,5-triazin-2-yl and 1,2,4-triazin-3-yl; wherein said heteroaryl is unsubstituted or carrie one, two, three or four independently selected radicals R^{L} as defined or preferably defined below.

According to one specific embodiment of the invention Z is selected from the group consisting of pyrimidin-2-yl, pyrimidin-3-yl, pyrimidin-4-yl, pyridin-2-yl, pyridin-3-yl, pyridin-4-yl, thiazol-2-yl, pyrazin-2-yl, pyridazin-3-yl, 1,3,5-triazin-2-yl, and 1,2,4-triazin-3-yl; preferably Z is pyrimidin-2-yl, pyridin-2-yl, pyridin-3-yl, pyridin-4-yl and thiazol-2-yl, that are unsubstituted or carry one, two, three or four independently selected radicals R^{L} as defined or preferably defined below.

According to the invention, there can be zero, one, two, three, four or five R^{L} present, namely for m is 0, 1, 2, 3, 4 or 5. The number of m also depends on the kind of heteroaryl. In particular, m is 0, 1, 2 or 3. According to one embodiment, m is 0. According to a further embodiment, m is 1, 2 or 3, in particular 1 or 2. According to one specific embodiment thereof, m is 1, according to a further specific embodiment, m is 2.

For every R^{L} that is present in the inventive compounds, the following embodiments and preferences apply independently of the meaning of any other R^{L} that may be present in the heteroaryl ring. Furthermore, the particular embodiments and preferences given herein for R^{L} apply independently for each of m=1, m=2, m=3, m= 4 and m=5.

Each R^{L} is independently selected from halogen, CN, NO₂, OH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein R^{La} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio.

According to one embodiment, R^{L} is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R^{L} is unsubstituted or further substituted by one, two, three or four independently selected R^{La}, wherein R^{La} is as defined and preferably defined herein.

According to a further embodiment, R^{L} is independently selected from halogen, CN, NO₂, C₁-C₄-alkyl, C₁-C₄-alkoxy, C₂-C₄-alkenyl, C₂-C₄-alkynyl, C₃-C₆-cycloalkyl, C₃-C₆-cycloalkyloxy, NH₂, NH(C₁-C₄₂-alkyl), N(C₁-C₂-alkyl)₂, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl), wherein each of R^{L} is unsubstituted or further substituted by one, two, three or four independently selected R^{La}, wherein R^{La} is as defined and preferably defined herein.

According to a further embodiment, R^{L} is independently selected from halogen, CN, NO₂, C₁-C₄-alkyl, C₁-C₄-haloalkyl,C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R^{L} is independently selected from halogen, CN, NO₂, C₁-C₂-alkyl, C₁-C₂-haloalkyl, C₁-C₂-alkoxy, C₁-C₂-haloalkoxy, S(C₁-C₂-alkyl), S(O)(C₁-C₂-alkyl), S(O)₂(C₁-C₂-alkyl), C(=O)(OH) and C(=O)(O-C₁-C₂-alkyl).

According to a further embodiment, R^{L} is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(O)₂(C₁-C₄-alkyl).

According to still a further specific embodiment, R^{L} is independently selected from halogen, in particular from Br, F and Cl, more specifically from F and Cl.

According to a further specific embodiment, R^{L} is CN.

According to one further embodiment R^{L} is NO₂.

According to one further embodiment R^{L} is OH.

According to one further embodiment R^{L} is SH.

According to a further specific embodiment, R^{L} is C₁-C₆-alkyl, in particular C₁-C₄-alkyl, such as CH₃. Further appropriate alkyls are ethyl, n-propyl, i-propyl, n-butyl, i-butyl and t-butyl.

According to a further specific embodiment, R^{L} is C₁-C₆-haloalkyl, in particular C₁-C₄-haloalkyl, such as CF₃, CHF₂, CH₂F, CCl₃, CHCl₂ or CH₂Cl.

According to a further specific embodiment R^{L} is C₁-C₆-alkyl, preferably C₁-C₄-alkyl, substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R^{L} is CH₂OH. According to a further specific embodiment R^{L} is C₁-C₆-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH-₂CH₂CH₂CN. In a special embodiment R^{L} is CH₂CH₂CN. In a further special embodiment R⁴ is CH(CH₃)CN. According to a further specific embodiment R^{L} is C₁-C₄-alkoxy-C₁-C₆-alkyl, more preferably C₁-C₄-alkoxy-C₁-C₄-alkyl. In a special embodiment R^{L} is CH₂OCH₃. In a further special embodiment R^{L} is CH₂CH₂OCH₃. In a further special embodiment R^{L} is CH(CH₃)OCH₃. In a further special embodiment R^{L} is CH(CH₃)OCH₂CH₃. In a further special embodiment R^{L} is CH₂CH₂OCH₂CH₃. According to a further specific embodiment R^{L} is C₁-C₄-haloalkoxy-C₁-C₆-alkyl, more preferably C₁-C₄-alkoxy-C₁-C₄-alkyl. In a special embodiment R^{L} is CH₂OCF₃. In a further special embodiment R^{L} is CH₂CH₂OCF₃. In a further special embodiment R^{L} is CH₂OCCl₃. In a further special embodiment R^{L} is CH₂CH₂OCCl₃.

According to a further specific embodiment, R^{L} is C₁-C₆-alkoxy, in particular C₁-C₄-alkoxy, more specifically C₁-C₂-alkoxy such as OCH₃ or OCH₂CH₃.

According to a further specific embodiment, R^{L} is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R^{L} is C₂-C₆-alkenyl or C₂-C₆-haloalkenyl, in particular C₂-C₄-alkenyl or C₂-C₄-haloalkenyl, such as CH=CH₂, CH₂CH=CH₂, CH=CHCH₃ or C(CH₃)=CH₂.

According to a further specific embodiment R^{L} is C₂-C₆-alkenyl, preferably C₂-C₄-alkenyl, substituted by OH, more preferably, CH=CHOH, CH=CHCH₂OH, C(CH₃)=CHOH, CH=C(CH₃)OH. In a special embodiment R^{L} is CH=CHOH. In a further special embodiment R^{L} is CH=CHCH₂OH. According to a further specific embodiment R^{L} is C₁-C₄-alkoxy-C₂-C₆-alkenyl, more preferably C₁-C₄-alkoxy-C₂-C₄-alkenyl. In a special embodiment R^{L} is CH=CHOCH₃. In a further special embodiment R^{L} is CH=CHCH₂OCH₃. According to a further specific embodiment R^{L} is C₁-C₄-haloalkoxy-C₂-C₆-alkenyl, more preferably C₁-C₄-haloalkoxy-C₂-C₄-alkenyl. In a special embodiment R^{L} is CH=CHOCF₃. In a further special embodiment R^{L} is CH=CHCH₂OCF₃. In a further special embodiment R^{L} is CH=CHOCCl₃. In a further special embodiment R^{L} is CH=CHCH₂OCCl₃. According to a further specific embodiment R^{L} is C₃-C₈-cycloalkyl-C₂-C₆-alkenyl, preferably C₃-C₆-cycloalkyl-C₂-C₄-alkenyl. According to a further specific embodiment R^{L} is C₃-C₆-halocycloalkyl-C₂-C₄-alkenyl, preferably C₃-C₈-halocycloalkyl-C₂-C₆-alkenyl.

According to still a further embodiment, R^{L} is C₂-C₆-alkynyl or C₂-C₆-haloalkynyl, in particular C₂-C₄-alkynyl or C₂-C₄-haloalkynyl, such as CΞCH, CH₂CCH or CH₂CCCH₃.

According to a further specific embodiment R^{L} is C₂-C₆-alkynyl, preferably C₂-C₄-alkynyl, substituted by OH, more preferably, CCOH, CH₂CCOH. In a special embodiment R^{L} is CCOH. In a further special embodiment R^{L} is CH₂CCOH. According to a further specific embodiment R^{L} is C₁-C₄-alkoxy-C₂-C₆-alkynyl, more preferably C₁-C₄-alkoxy-C₂-C₄-alkynyl. In a special embodiment R^{L} is CCOCH₃. In a further special embodiment R^{L} is CH₂CCOCH₃. According to a further specific embodiment R^{L} is C₁-C₄-haloalkoxy-C₂-C₆-alkynyl, more preferably C₁-C₄-haloalkoxy-C₂-C₄-alkynyl. In a special embodiment R^{L} is CCOCF₃. In a further special embodiment R^{L} is CH₂CCOCF₃. In a further special embodiment R^{L} is CCOCCl₃. In a further special embodiment

R^{L} is CH₂CCOCCl₃. According to a further specific embodiment R^{L} is C₃-C₈-cycloalkyl-C₂-C₆-alkynyl, preferably C₃-C₆-cycloalkyl-C₂-C₄-alkynyl. According to a further specific embodiment R^{L} is C₃-C₆-halocycloalkyl-C₂-C₄-alkynyl, preferably C₃-C₈-halocycloalkyl-C₂-C₆-alkynyl.

According to one another embodiment R^{L} is C₃-C₈-cycloalkyl, preferably cyclopropyl, cyclobutyl, cyclopentyl or cyclohexyl, in particular cyclopropyl or cyclobutyl. In a special embodiment R^{L} is cyclopropyl. In a further special embodiment R^{L} is cyclobutyl. In a further special embodiment R⁴ is cyclopentyl. In a further special embodiment R^{L} is cyclohexyl.

According to one another embodiment R^{L} is C₃-C₈-cycloalkoxy, preferably C₃-C₆-cycloalkoxy. In a special embodiment R^{L} is O-cyclopropyl.

According to a specific embodiment R^{L} is C₃-C₈-halocycloalkyl, more preferably fully or partially halogenated C₃-C₆-cycloalkyl. In a special embodiment R^{L} is fully or partially halogenated cyclopropyl. In a further special embodiment R^{L} is 1-Cl-cyclopropyl. In a further special embodiment R^{L} is 2-Cl-cyclopropyl. In a further special embodiment R^{L} is 1-F-cyclopropyl. In a further special embodiment R^{L} is 2-F-cyclopropyl. In a further special embodiment R^{L} is fully or partially halogenated cyclobutyl. In a further special embodiment R^{L} is 1-Cl-cyclobutyl. In a further special embodiment R^{L} is 1-F-cyclobutyl. In a further special embodiment R^{L} is 3,3-Cl₂-cyclobutyl. In a further special embodiment R^{L} is 3,3-F₂-cyclobutyl. According to a specific embodiment R^{L} is C₃-C₈-cycloalkyl substituted by C₁-C₄-alkyl, more preferably is C₃-C₆-cycloalkyl substituted by C₁-C₄-alkyl. In a special embodiment R^{L} is 1-CH₃-cyclopropyl. According to a specific embodiment R^{L} is C₃-C₈-cycloalkyl substituted by CN, more preferably is C₃-C₆-cycloalkyl substituted by CN. In a special embodiment R^{L} is 1-CN-cyclopropyl.According to a further specific embodiment R^{L} is C₃-C₈-cycloalkyl-C₃-C₈-cycloalkyl, preferably C₃-C₆-cycloalkyl-C₃-C₆-cycloalkyl. In a special embodiment R^{L} is cyclopropyl-cyclopropyl. In a special embodiment R^{L} is 2-cyclopropyl-cyclopropyl. According to a further specific embodiment R^{L} is C₃-C₈-cycloalkyl-C₃-C₈-halocycloalkyl, preferably C₃-C₆-cycloalkyl-C₃-C₆-halocycloalkyl.

According to one another embodiment R^{L} is C₃-C₈-cycloalkyl-C₁-C₄-alkyl, preferably C₃-C₆-cycloalkyl-C₁-C₄-alkyl. In a special embodiment R^{L} is CH(CH₃)(cyclopropyl). In a further special embodiment R^{L} is CH₂-(cyclopropyl).

According to a further preferred embodiment R^{L} is C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R^{L} is C₃-C₈-cycloalkyl-C₁-C₄-haloalkyl, C₃-C₆-cycloalkyl-C₁-C₄-haloalkyl. According to a specific embodiment R^{L} is C₃-C₈-halocycloalkyl-C₁-C₄-alkyl, C₃-C₆-halocycloalkyl-C₁-C₄-alkyl. In a special embodiment R^{L} is fully or partially halogenated cyclopropyl-C₁-C₄-alkyl. In a further special embodiment R^{L} is 1-Cl-cyclopropyl-C₁-C₄-alkyl. In a further special embodiment R^{L} is 1-F-cyclopropyl-C₁-C₄-alkyl.

According to one another embodiment R^{L} is NH₂.

According to one another embodiment R^{L} is NH(C₁-C₄-alkyl). According to a specific embodiment R^{L} is NH(CH₃). According to a specific embodiment R^{L} is NH(CH₂CH₃). According to a specific embodiment R^{L} is NH(CH₂CH₂CH₃). According to a specific embodiment R^{L} is NH(CH(CH₃)₂). According to a specific embodiment R^{L} is NH(CH₂CH₂CH₂CH₃). According to a specific embodiment R^{L} is NH(C(CH₃)₃).

According to one another embodiment R^{L} is N(C₁-C₄-alkyl)₂. According to a specific embodiment R^{L} is N(CH₃)₂. According to a specific embodiment R^{L} is N(CH₂CH₃)₂. According to a specific embodiment R^{L} is N(CH₂CH₂CH₃)₂. According to a specific embodiment R^{L} is N(CH(CH₃)₂)₂. According to a specific embodiment R^{L} is N(CH₂CH₂CH₂CH₃)₂. According to a specific embodiment R^{L} is NH(C(CH₃)₃)₂.

According to one another embodiment R^{L} is NH(C₃-C₈-cycloalkyl) preferably NH(C₃-C₆-cycloalkyl). According to a specific embodiment R^{L} is NH(cyclopropyl). According to a specific embodiment R^{L} is NH(cyclobutyl). According to a specific embodiment R^{L} is NH(cyclopentyl). According to a specific embodiment R^{L} is NH(cyclohexyl).

According to one another embodiment R^{L} is N(C₃-C₈-cycloalkyl)₂ preferably N(C₃-C₆-cycloalkyl)₂. According to a specific embodiment R^{L} is N(cyclopropyl)₂. According to a specific embodiment R^{L} is N(cyclobutyl)₂. According to a specific embodiment R^{L} is N(cyclopentyl)₂. According to a specific embodiment R^{L} is N(cyclohexyl)₂.

According to still a further embodiment, R^{L} is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R^{L} is C(=O)(OH) or C(=O)(O-C₁-C₄-alkyl), in particular C(=O)(OCH₃).

According to one another embodiment R^{L} is C(=O)(-C₁-C₄-alkyl). According to a specific embodiment R^{L} is C(=O)CH₃.. According to a further specific embodiment R^{L} is C(=O)CH₂CH₃. According to a further specific embodiment R^{L} is C(=O)CH₂CH₂CH₃. According to a further specific embodiment R^{L} is C(=O)CH(CH₃)₂. According to a further specific embodiment R^{L} is C(=O)C(CH₃)₃.

According to one another embodiment R^{L} is C(=O)OH.

According to one another embodiment R^{L} is C(=O)(-O-C₁-C₄-alkyl). According to a specific embodiment R^{L} is C(=O)OCH₃.. According to a further specific embodiment R^{L} is C(=O)OCH₂CH₃. According to a further specific embodiment R^{L} is C(=O)OCH₂CH₂CH₃. According to a further specific embodiment R^{L} is C(=O)OCH(CH₃)₂. According to a further specific embodiment R^{L} is C(=O)OC(CH₃)₃.

According to one another embodiment R^{L} is C(=O)-NH(C₁-C₄-alkyl). According to a specific embodiment R^{L} is C(=O)NHCH₃.. According to a further specific embodiment R^{L} is C(=O)NHCH₂CH₃. According to a further specific embodiment R^{L} is C(=O)NHCH₂CH₂CH₃. According to a further specific embodiment R^{L} is C(=O)NHCH(CH₃)₂. According to a further specific embodiment R^{L} is C(=O)NHC(CH₃)₃.

According to one another embodiment R^{L} is C(=O)-N(C₁-C₄-alkyl)₂. According to a specific embodiment R^{L} is C(=O)N(CH₃)₂. According to a further specific embodiment R^{L} is C(=O)N(CH₂CH₃)₂. According to a further specific embodiment R^{L} is C(=O)N(CH₂CH₂CH₃)₂. According to a further specific embodiment R^{L} is C(=O)N(CH(CH₃)₂)₂. According to a further specific embodiment R^{L} is C(=O)N(C(CH₃)₃)₂.

According to one another embodiment R^{L} is C(=O)-NH(C₃-C₆-cycloalkyl). According to a specific embodiment R^{L} is C(=O)NH(cyclopropyl). According to a further specific embodiment R^{L} is C(=O)NH(cyclobutyl). According to a further specific embodiment R^{L} is C(=O)NH(cyclopentyl).According to a further specific embodiment R^{L} is C(=O)NH(cyclohexyl).

According to one another embodiment R^{L} is C(=O)-N(C₃-C₆-cycloalkyl)₂. According to a specific embodiment R^{L} is C(=O)N(cyclopropyl)₂. According to a further specific embodiment R^{L} is C(=O)N(cyclobutyl)₂. According to a further specific embodiment R^{L} is C(=O)N(cyclopentyl)₂. According to a further specific embodiment R^{L} is C(=O)N(cyclohexyl)₂.

According to still a further embodiment, R^{L} is selected from S(C₁-C₂-alkyl), S(O)(C₁-C₂-alkyl) and S(O)₂(C₁-C₂-alkyl), in particular SCH₃, S(O)(CH₃) and S(O)₂(CH₃). According to a specific embodiment R^{L} is selected from S(C₁-C₂-haloalkyl), S(O)(C₁-C₂-haloalkyl) and S(O)₂(C₁-C₂-haloalkyl), such as SO₂CF₃.

Particularly preferred embodiments of R^{L} present in the heteroaryl according to the invention are in Table PL above, wherein each line of lines PL-1 to PL-16 corresponds to one particular embodiment of the invention, wherein PL-1 to PL-16 are also in any combination with one another a preferred embodiment of the present invention. Thereby, for every R^{L} that is present in the inventive compounds, these specific embodiments and preferences apply independently of the meaning of any other R^{L} that may be present in the heteroaryl ring.

Particularly preferred embodiments of (R^{L})ₘ if Z is heteroaryl according to the invention are in Table H below, wherein each line of lines H-1 to H-155 corresponds to one particular embodiment of the invention, wherein H-1 to H-155 are also in any combination a preferred embodiment of the present invention.

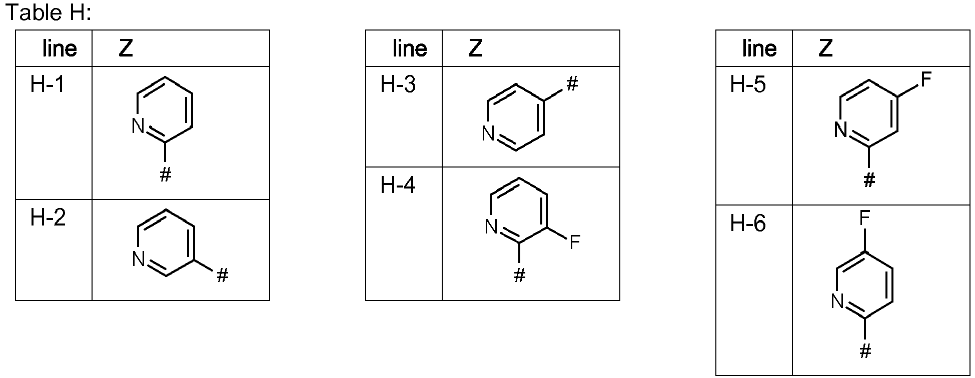

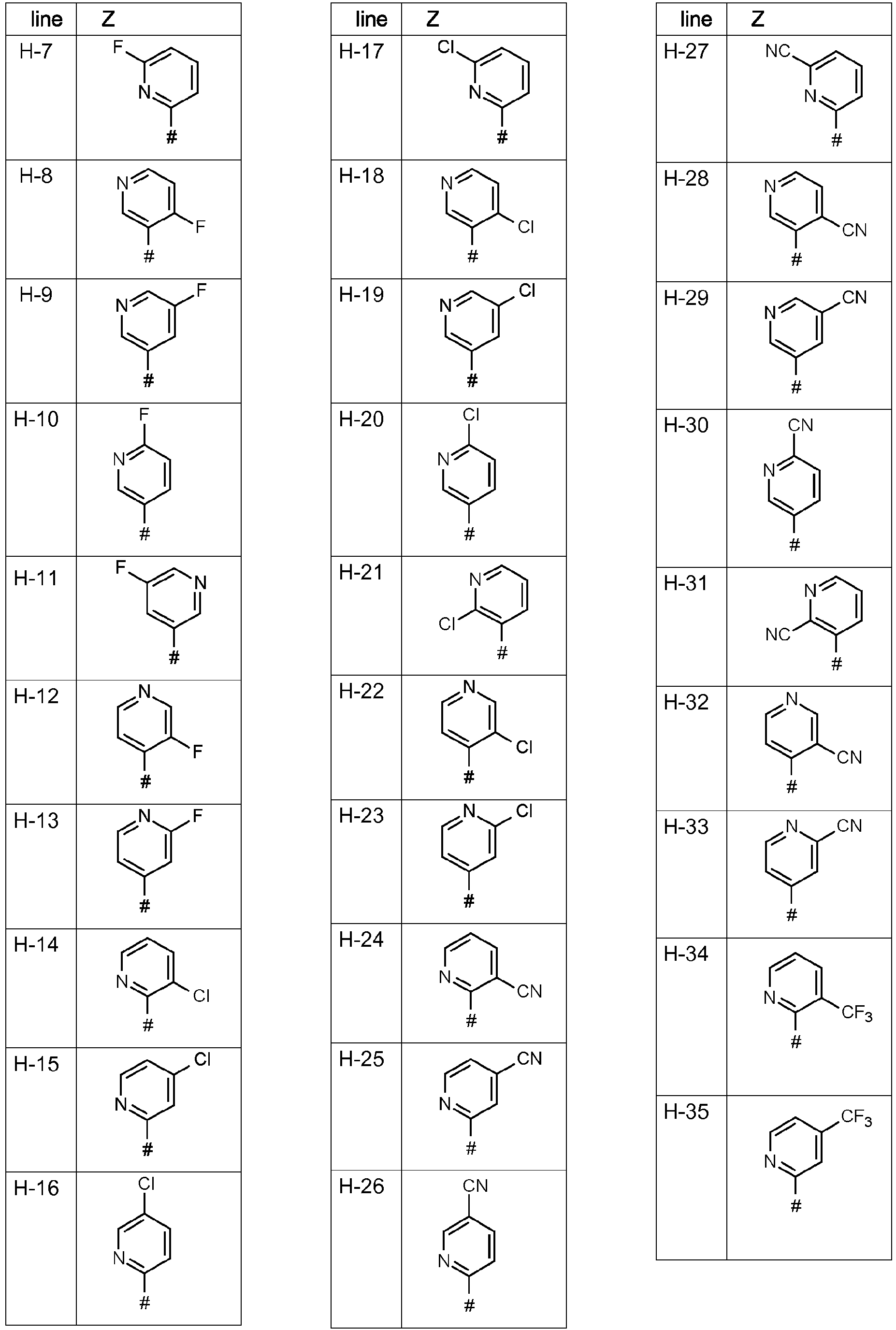

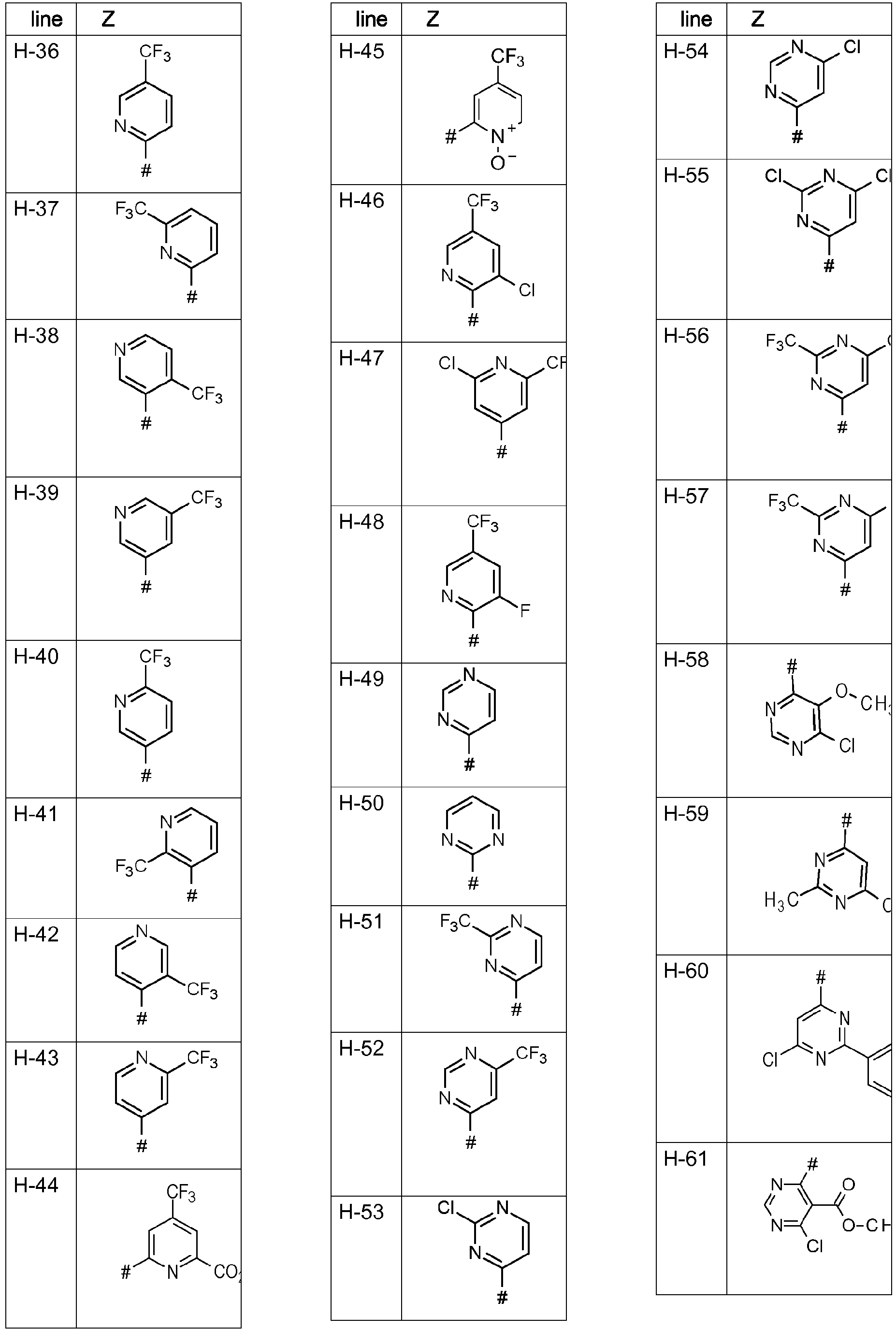

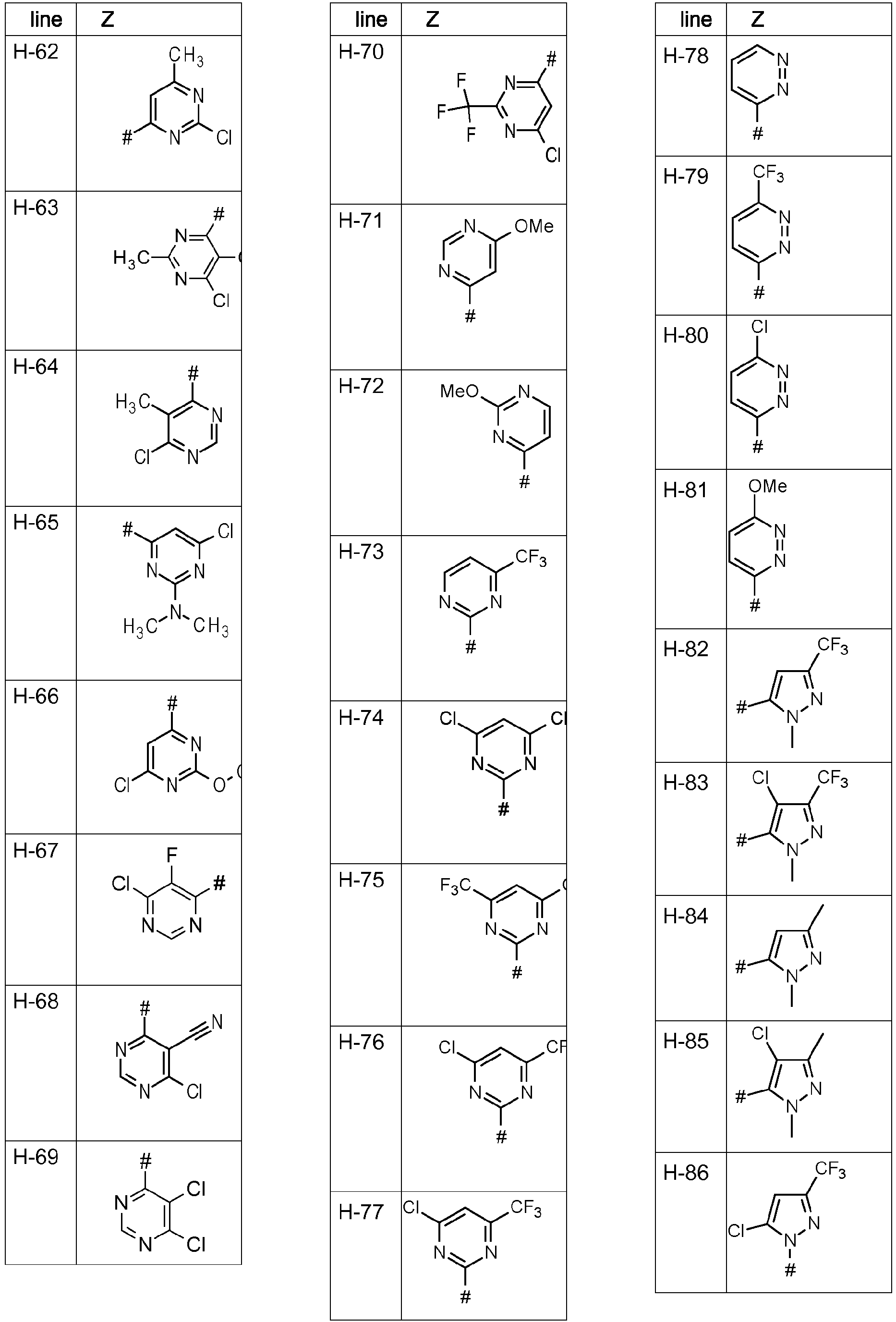

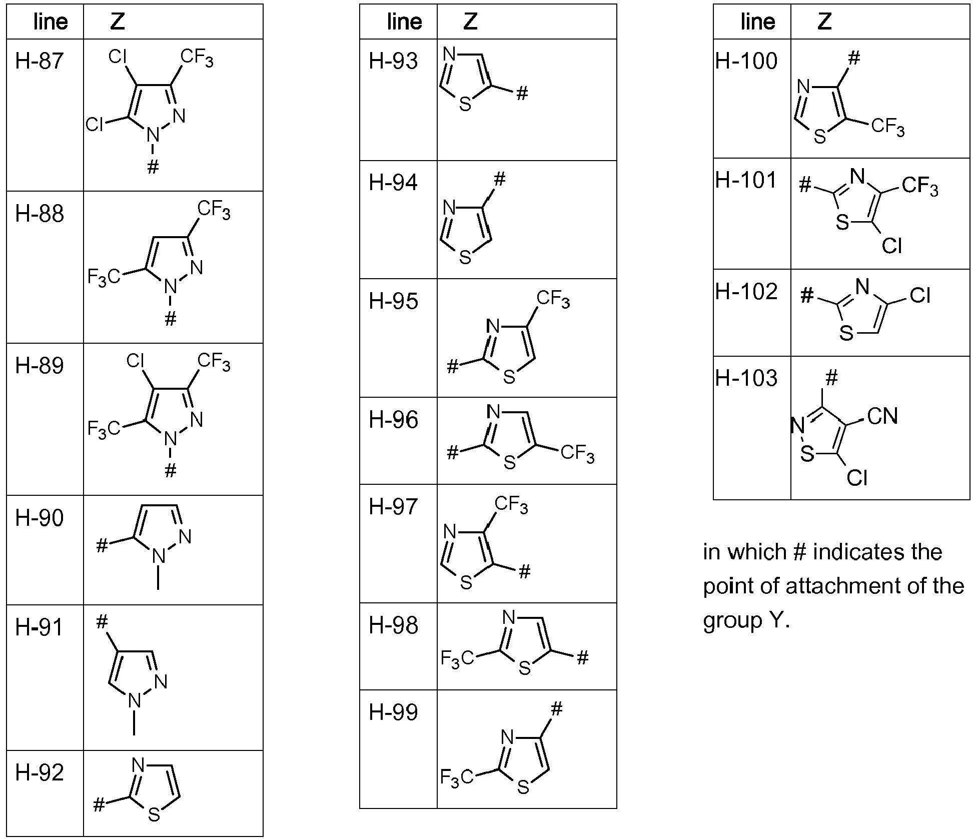

Each R⁴ according to the present invention is independently selected from halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, phenyl, phenoxy, a 5- or 6-membered heteroaryl, a 5- or 6-membered heteroaryloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic, alicyclic and aromatic moieties of R⁴ are unsubstituted or substituted by one, two, three or four or up to the maximum possible number of R^{4a}; wherein R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy.

According to the invention, there can be zero, one, two, three or four R⁴ present, namely for n is 0, 1, 2, 3 or 4.

According to one embodiment, n is 0. According to a further embodiment, n is 1.

According to a further embodiment, n is 2 or 3. According to one specific embodiment thereof, n is 2, according to a further specific embodiment, n is 3.

According to one embodiment of the invention, one R⁴ is attached to the 2-position (R⁴¹). According to one specific embodiment thereof, n is 1, according to a further specific embodiment, n is 2.

According to one embodiment of the invention, one R⁴ is attached to the 3-position (R⁴²). According to one specific embodiment thereof, n is 1, according to a further specific embodiment, n is 2.

According to a further embodiment of the invention, one R⁴ is attached to the 4-position (R⁴³). According to one specific embodiment thereof, n is 1, according to a further specific embodiment, n is 2.

According to a further embodiment of the invention, one R⁴ is attached to the 5-position (R⁴⁴). According to one specific embodiment thereof, n is 1, according to a further specific embodiment, n is 2.

According to still a further embodiment, n is 1, 2 or 3 and one R⁴ is in 2- or 6-position.

According to a further embodiment of the invention, one R⁴ is attached to the 6-position (R⁴⁵). According to one specific embodiment thereof, n is 1, according to a further specific embodiment, n is 2.

According to a further embodiment of the invention, two R⁴ are attached in 2,3-position. According to one specific embodiment thereof, n is 2, according to a further specific embodiment, n is 3.

According to a further embodiment of the invention, two R⁴ are attached in 2,4-position. According to one specific embodiment thereof, n is 2, according to a further specific embodiment, n is 3.

According to a further embodiment of the invention, two R⁴ are attached in 2,5-position. According to one specific embodiment thereof, n is 2, according to a further specific embodiment, n is 3.

According to a further embodiment of the invention, two R⁴ are attached in 2,6-position. According to one specific embodiment thereof, n is 2, according to a further specific embodiment, n is 3.

According to a further embodiment of the invention, two R⁴ are attached in 3,4-position. According to one specific embodiment thereof, n is 2, according to a further specific embodiment, n is 3.

According to a further embodiment of the invention, two R⁴ are attached in 3,5-position. According to one specific embodiment thereof, n is 2, according to a further specific embodiment, n is 3.

According to a further embodiment of the invention, two R³ are attached in 3,6-position. According to one specific embodiment thereof, n is 2, according to a further specific embodiment, n is 3.

For every R⁴ (or R⁴¹, R⁴², R⁴³, R⁴⁴, R⁴⁵, respectively) that is present in the inventive compounds, the following embodiments and preferences apply independently of the meaning of any other R⁴ (or R⁴¹, R⁴², R⁴³ R⁴⁴, R⁴⁵ respectively) that may be present in the phenyl ring. Furthermore, the particular embodiments and preferences given herein for R⁴ (or R⁴¹, R⁴², R⁴³, R⁴⁴, R⁴⁵, respectively) apply independently for each of n=1, n=2, n=3 and n=4.

According to one embodiment, R⁴ is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R⁴ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four R^{4a}, wherein R^{4a} is as defined and preferably defined herein.

According to still a further embodiment, R⁴ is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to still a further embodiment, R⁴ is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(O)₂(C₁-C₄-alkyl).

According to one specific embodiment, R⁴ is halogen, in particular Br, F or Cl, more specifically F or Cl.

According to a further specific embodiment, R⁴ is CN.

According to a further specific embodiment, R⁴ is C₁-C₆-alkyl, in particular C₁-C₄-alkyl, such as CH₃.

According to a further specific embodiment, R⁴ is C₁-C₆-haloalkyl, in particular C₁-C₄-haloalkyl, such as CF₃, CHF₂, CH₂F, CCl₃, CHCl₂ or CH₂Cl.

According to a further specific embodiment, R⁴ is C₁-C₆-alkoxy, in particular C₁-C₄-alkoxy, more specifically C₁-C₂-alkoxy such as OCH₃ or OCH₂CH₃.

According to a further specific embodiment, R⁴ is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R⁴ is C₂-C₆-alkenyl or C₂-C₆-haloalkenyl, in particular C₂-C₄-alkenyl or C₂-C₄-haloalkenyl, such as CH=CH₂.

According to still a further embodiment, R⁴ is C₂-C₆-alkynyl or C₂-C₆-haloalkynyl, in particular C₂-C₄-alkynyl or C₂-C₄-haloalkynyl, such as CΞCH.

According to still a further embodiment, R⁴ is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R⁴ is C(=O)(OH) or C(=O)(O-C₁-C₄-alkyl), in particular C(=O)(OCH₃).

According to still a further embodiment, R⁴ is selected from S(C₁-C₂-alkyl), S(O)(C₁-C₂-alkyl) and S(O)₂(C₁-C₂-alkyl), in particular SCH₃, S(O)(CH₃) and S(O)₂(CH₃).

According to still a further embodiment, R⁴ is unsubstituted phenyl or phenyl that is substituted by one, two, three or four R^{4a}, as defined herein.

According to still a further embodiment, R⁴ is unsubstituted phenoxy or phenoxy that is substituted by one, two, three or four R^{4a}, as defined herein.

According to still a further embodiment, R⁴ is unsubstituted 5- or 6-membered heteroaryl. According to still a further embodiment, R⁴ is 5- or 6-membered heteroaryl that is substituted by one, two or three R^{4a}, as defined herein. According to one specific embodiment, the heteroaryl in each case is 5-membered such as . According to a further specific embodiment, the heteroaryl in each case is 6-membered such as .

According to still a further embodiment, R⁴ is unsubstituted 5- or 6-membered heteroaryloxy. According to still a further embodiment, R⁴ is 5- or 6-membered heteroaryloxy that is substituted by one, two or three R^{4a}, as defined herein. According to one specific embodiment, the heteroaryloxy in each case is 5-membered. According to a further specific embodiment, the heteroaryloxy in each case is 6-membered.

R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy, in particular selected from halogen, CN, C₁-C₂-alkyl, C₁-C₂-haloalkyl, C₃-C₆-cycloalkyl, C₃-C₆-halocycloalkyl, C₁-C₂-alkoxy and C₁-C₂-halogenalkoxy. Specifically, R^{4a} is independently selected from F, Cl, CN, OH, CH₃, halomethyl, cyclopropyl, halocyclopropyl, OCH₃ and halogenmethoxy.

Particularly preferred embodiments of R⁴ according to the invention are in Table P5 below, wherein each line of lines P5-1 to P5-16 corresponds to one particular embodiment of the invention, wherein P5-1 to P5-16 are also in any combination with one another a preferred embodiment of the present invention. Thereby, for every R⁴ that is present in the inventive compounds, these specific embodiments and preferences apply independently of the meaning of any other R⁴ that may be present in the phenyl ring:

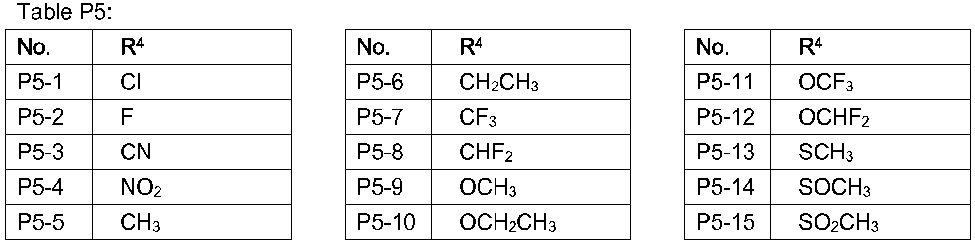

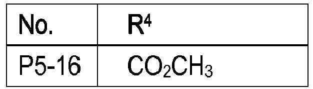

Particularly preferred embodiments of (R⁴)ₙ according to the invention are in Table P6 below, wherein each line of lines P6-1 to P6-155 corresponds to one particular embodiment of the invention, wherein P6-1 to P6-155 are also in any combination a preferred embodiment of the present invention.

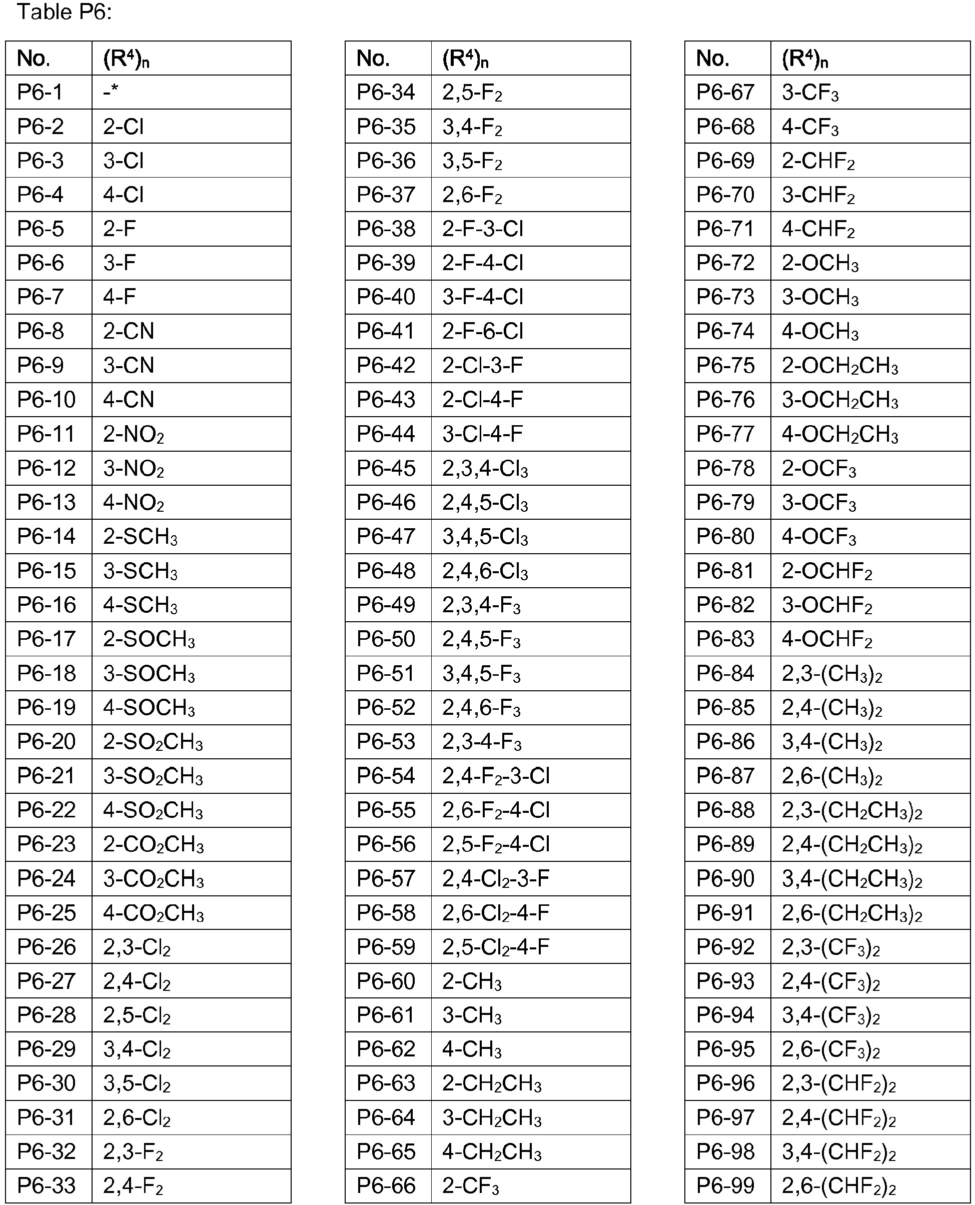

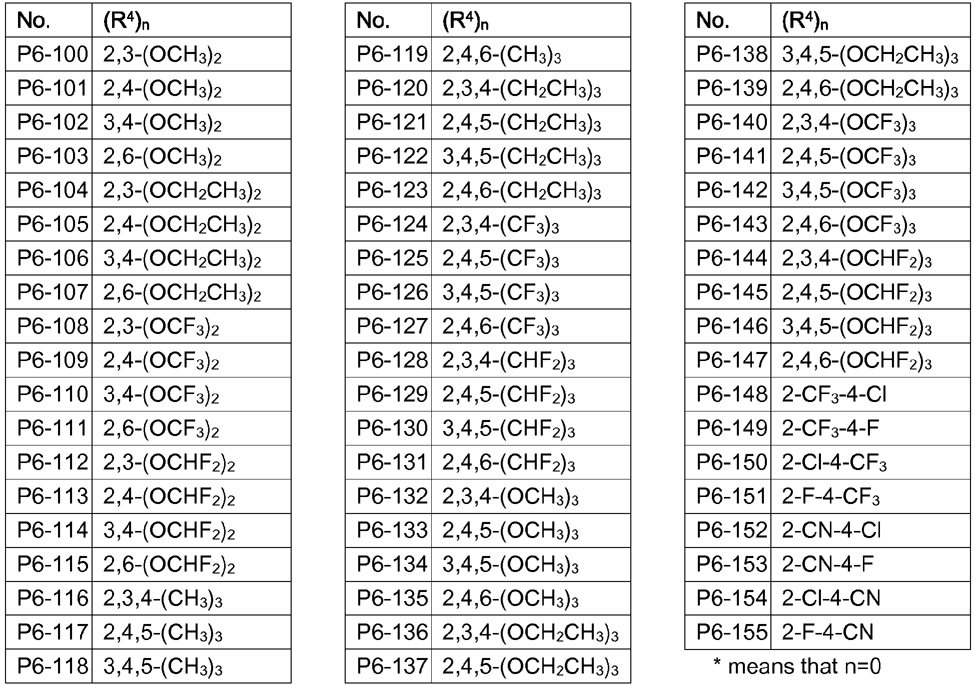

The embodiments of (R⁴)ₙ as outlined in Table P6 above and Table A below are referred to be substituted at the numbered positions of the phenyl substituent in accordance with the following numbering of the atoms of the phenyl ring:

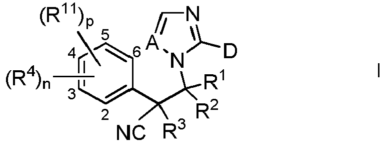

According to one embodiment, the invention relates to compounds of Formula I, wherein p is 0:

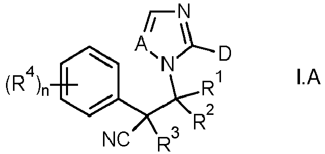

Specific embodiment are compounds I.A1 (D=H, A=N), I.A2 (D=H, A=CH), I.A3 (D=SH, A=N), I.A4 (D=SH, A=CH):

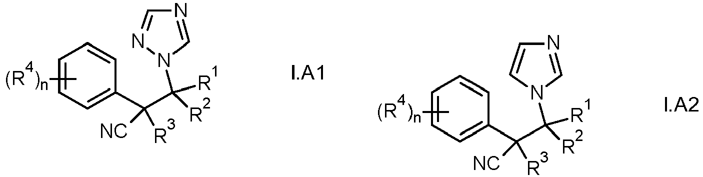

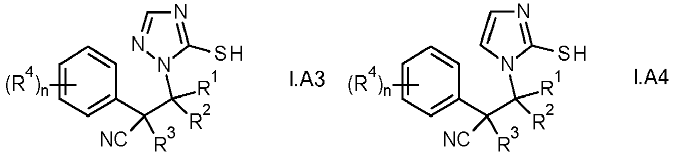

In particular with a view to their use, preference is given to the compounds of the formula I.A, in particular compounds I.A1 (D=H, A=N), I.A2 (D=H, A=CH), I.A3 (D=SH, A=N), I.A4 (D=SH, A=CH), more particularly I.A1 (D=H, A=N) and I.A2 (D=H, A=CH), that are compiled in the Tables 1 a to 64a and Tables 1 b to 64b, below. Each of the groups mentioned for a substituent in the tables is furthermore per se, independently of the combination in which it is mentioned, a particularly preferred aspect of the substituent in question.
- Table 1a: Compounds of the formula I.A1 in which (R⁴)ₙ corresponds to line A-1 of Table A and the meaning for the combination of R¹, R² and R³ for each individual compound corresponds in each case to one line of Table B (compounds I.A1.A1.B1 to I.A1.A1.B442).
- Table 2a: Compounds of the formula I.A1 in which (R⁴)ₙ corresponds to line A-2 of Table A and the meaning for the combination of R¹, R² and R³ for each individual compound corresponds in each case to one line of Table B (compounds I.A1.A2.B1 to I.A1.A2.B442).
- Table 3a: Compounds of the formula I.A1 in which (R⁴)ₙ corresponds to line A-3 of Table A and the meaning for the combination of R¹, R² and R³ for each individual compound corresponds in each case to one line of Table B (compounds I.A1.A3.B1 to I.A1.A3.B442).
- Table 4a: Compounds of the formula I.A1 in which (R⁴)ₙ corresponds to line A-4 of Table A and the meaning for the combination of R¹, R² and R³ for each individual compound corresponds in each case to one line of Table B (compounds I.A1.A4.B1 to I.A1.A4.B442).
- Table 5a: Compounds of the formula I.A1 in which (R⁴)ₙ corresponds to line A-5 of Table A and the meaning for the combination of R¹, R² and R³ for each individual compound corresponds in each case to one line of Table B (compounds I.A1.A5.B1 to I.A1.A5.B442).
- Table 6a: Compounds of the formula I.A1 in which (R⁴)ₙ corresponds to line A-6 of Table A and the meaning for the combination of R¹, R² and R³ for each individual compound corresponds in each case to one line of Table B (compounds I.A1.A6.B1 to I.A1.A6.B442).
- Table 7a: Compounds of the formula I.A1 in which (R⁴)ₙ corresponds to line A-7 of Table A and the meaning for the combination of R¹, R² and R³ for each individual compound corresponds in each case to one line of Table B (compounds I.A1.A7.B1 to I.A1.A7.B442).
- Table 8a: Compounds of the formula I.A1 in which (R⁴)ₙ corresponds to line A-8 of Table A and the meaning for the combination of R¹, R² and R³ for each individual compound corresponds in each case to one line of Table B (compounds I.A1.A8.B1 to I.A1.A8.B442).
- Table 9a: Compounds of the formula I.A1 in which (R⁴)ₙ corresponds to line A-9 of Table A and the meaning for the combination of R¹, R² and R³ for each individual compound corresponds in each case to one line of Table B (compounds I.A1.A9.B1 to I.A1.A9.B442).
- Table 10a: Compounds of the formula I.A1 in which (R⁴)ₙ corresponds to line A-1 0 of Table A and the meaning for the combination of R¹, R² and R³ for each individual compound corresponds in each case to one line of Table B (compounds I.A1.A10.B1 to I.A1.A10.B442).
- Table 11 a: Compounds of the formula I.A1 in which (R⁴)ₙ corresponds to line A-11 of Table A and the meaning for the combination of R¹, R² and R³ for each individual compound corresponds in each case to one line of Table B (compounds I.A1.A11.B1 to I.A1.A11.B442).
- Table 12a: Compounds of the formula I.A1 in which (R⁴)ₙ corresponds to line A-12 of Table A and the meaning for the combination of R¹, R² and R³ for each individual compound corresponds in each case to one line of Table B (compounds I.A1.A12.B1 to I.A1.A12.B442).
- Table 13a: Compounds of the formula I.A1 in which (R⁴)ₙ corresponds to line A-13 of Table A and the meaning for the combination of R¹, R² and R³ for each individual compound corresponds in each case to one line of Table B (compounds I.A1.A13.B1 to I.A1.A13.B442).
- Table 14a: Compounds of the formula I.A1 in which (R⁴)ₙ corresponds to line A-14 of Table A and the meaning for the combination of R¹, R² and R³ for each individual compound corresponds in each case to one line of Table B (compounds I.A1.A14.B1 to I.A1.A14.B442).
- Table 15a: Compounds of the formula I.A1 in which (R⁴)ₙ corresponds to line A-15 of Table A and the meaning for the combination of R¹, R² and R³ for each individual compound corresponds in each case to one line of Table B (compounds I.A1.A15.B1 to I.A1.A15.B442).
- Table 16a: Compounds of the formula I.A1 in which (R⁴)ₙ corresponds to line A-16 of Table A and the meaning for the combination of R¹, R² and R³ for each individual compound corresponds in each case to one line of Table B (compounds I.A1.A16.B1 to I.A1.A16.B442).
- Table 17a: Compounds of the formula I.A1 in which (R⁴)ₙ corresponds to line A-17 of Table A and the meaning for the combination of R¹, R² and R³ for each individual compound corresponds in each case to one line of Table B (compounds I.A1.A17.B1 to I.A1.A17.B442).
- Table 18a: Compounds of the formula I.A1 in which (R⁴)ₙ corresponds to line A-18 of Table A and the meaning for the combination of R¹, R² and R³ for each individual compound corresponds in each case to one line of Table B (compounds I.A1.A18.B1 to I.A1.A18.B442).
- Table 19a: Compounds of the formula I.A1 in which (R⁴)ₙ corresponds to line A-19 of Table A and the meaning for the combination of R¹, R² and R³ for each individual compound corresponds in each case to one line of Table B (compounds I.A1.A19.B1 to I.A1.A19.B442).
- Table 20a: Compounds of the formula I.A1 in which (R⁴)ₙ corresponds to line A-20 of Table A and the meaning for the combination of R¹, R² and R³ for each individual compound corresponds in each case to one line of Table B (compounds I.A1.A20.B1 to I.A1.A20.B442).
- Table 21a: Compounds of the formula I.A1 in which (R⁴)ₙ corresponds to line A-21 of Table A and the meaning for the combination of R¹, R² and R³ for each individual compound corresponds in each case to one line of Table B (compounds I.A1.A21.B1 to I.A1.A21.B442).
- Table 22a: Compounds of the formula I.A1 in which (R⁴)ₙ corresponds to line A-22 of Table A and the meaning for the combination of R¹, R² and R³ for each individual compound corresponds in each case to one line of Table B (compounds I.A1.A22.B1 to I.A1.A22.B442).
- Table 23a: Compounds of the formula I.A1 in which (R⁴)ₙ corresponds to line A-23 of Table A and the meaning for the combination of R¹, R² and R³ for each individual compound corresponds in each case to one line of Table B (compounds I.A1.A23.B1 to I.A1.A23.B442).
- Table 24a: Compounds of the formula I.A1 in which (R⁴)ₙ corresponds to line A-24 of Table A and the meaning for the combination of R¹, R² and R³ for each individual compound corresponds in each case to one line of Table B (compounds I.A1.A24.B1 to I.A1.A24.B442).
- Table 25a: Compounds of the formula I.A1 in which (R⁴)ₙ corresponds to line A-25 of Table A and the meaning for the combination of R¹, R² and R³ for each individual compound corresponds in each case to one line of Table B (compounds I.A1.A25.B1 to I.A1.A25.B442).
- Table 26a: Compounds of the formula I.A1 in which (R⁴)ₙ corresponds to line A-26 of Table A and the meaning for the combination of R¹, R² and R³ for each individual compound corresponds in each case to one line of Table B (compounds I.A1.A26.B1 to I.A1.A26.B442).
- Table 27a: Compounds of the formula I.A1 in which (R⁴)ₙ corresponds to line A-27 of Table A and the meaning for the combination of R¹, R² and R³ for each individual compound corresponds in each case to one line of Table B (compounds I.A1.A27.B1 to I.A1.A27.B442).
- Table 28a: Compounds of the formula I.A1 in which (R⁴)ₙ corresponds to line A-28 of Table A and the meaning for the combination of R¹, R² and R³ for each individual compound corresponds in each case to one line of Table B (compounds I.A1.A28.B1 to I.A1.A28.B442).
- Table 29a: Compounds of the formula I.A1 in which (R⁴)ₙ corresponds to line A-29 of Table A and the meaning for the combination of R¹, R² and R³ for each individual compound corresponds in each case to one line of Table B (compounds I.A1.A29.B1 to I.A1.A29.B442).
- Table 30a: Compounds of the formula I.A1 in which (R⁴)ₙ corresponds to line A-30 of Table A and the meaning for the combination of R¹, R² and R³ for each individual compound corresponds in each case to one line of Table B (compounds I.A1.A30.B1 to I.A1.A30.B442).
- Table 31a: Compounds of the formula I.A1 in which (R⁴)ₙ corresponds to line A-31 of Table A and the meaning for the combination of R¹, R² and R³ for each individual compound corresponds in each case to one line of Table B (compounds I.A1.A31.B1 to I.A1.A31.B442).
- Table 32a: Compounds of the formula I.A1 in which (R⁴)ₙ corresponds to line A-32 of Table A and the meaning for the combination of R¹, R² and R³ for each individual compound corresponds in each case to one line of Table B (compounds I.A1.A32.B1 to I.A1.A32.B442).
- Table 33a: Compounds of the formula I.A1 in which (R⁴)ₙ corresponds to line A-33 of Table A and the meaning for the combination of R¹, R² and R³ for each individual compound corresponds in each case to one line of Table B (compounds I.A1.A33.B1 to I.A1.A33.B442).
- Table 34a: Compounds of the formula I.A1 in which (R⁴)ₙ corresponds to line A-34 of Table A and the meaning for the combination of R¹, R² and R³ for each individual compound corresponds in each case to one line of Table B (compounds I.A1.A34.B1 to I.A1.A34.B442).
- Table 35a: Compounds of the formula I.A1 in which (R⁴)ₙ corresponds to line A-35 of Table A and the meaning for the combination of R¹, R² and R³ for each individual compound corresponds in each case to one line of Table B (compounds I.A1.A35.B1 to I.A1.A35.B442).
- Table 36a: Compounds of the formula I.A1 in which (R⁴)ₙ corresponds to line A-36 of Table A and the meaning for the combination of R¹, R² and R³ for each individual compound corresponds in each case to one line of Table B (compounds I.A1.A36.B1 to I.A1.A36.B442).
- Table 37a: Compounds of the formula I.A1 in which (R⁴)ₙ corresponds to line A-37 of Table A and the meaning for the combination of R¹, R² and R³ for each individual compound corresponds in each case to one line of Table B (compounds I.A1.A37.B1 to I.A1.A37.B442).
- Table 38a: Compounds of the formula I.A1 in which (R⁴)ₙ corresponds to line A-38 of Table A and the meaning for the combination of R¹, R² and R³ for each individual compound corresponds in each case to one line of Table B (compounds I.A1.A38.B1 to I.A1.A38.B442).
- Table 39a: Compounds of the formula I.A1 in which (R⁴)ₙ corresponds to line A-39 of Table A and the meaning for the combination of R¹, R² and R³ for each individual compound corresponds in each case to one line of Table B (compounds I.A1.A39.B1 to I.A1.A39.B442).
- Table 40a: Compounds of the formula I.A1 in which (R⁴)ₙ corresponds to line A-40 of Table A and the meaning for the combination of R¹, R² and R³ for each individual compound corresponds in each case to one line of Table B (compounds I.A1.A40.B1 to I.A1.A40.B442).
- Table 41 a: Compounds of the formula I.A1 in which (R⁴)ₙ corresponds to line A-41 of Table A and the meaning for the combination of R¹, R² and R³ for each individual compound corresponds in each case to one line of Table B (compounds I.A1.A41.B1 to I.A1.A41.B442).
- Table 42a: Compounds of the formula I.A1 in which (R⁴)ₙ corresponds to line A-42 of Table A and the meaning for the combination of R¹, R² and R³ for each individual compound corresponds in each case to one line of Table B (compounds I.A1.A42.B1 to I.A1.A42.B442).
- Table 43a: Compounds of the formula I.A1 in which (R⁴)ₙ corresponds to line A-43 of Table A and the meaning for the combination of R¹, R² and R³ for each individual compound corresponds in each case to one line of Table B (compounds I.A1.A43.B1 to I.A1.A43.B442).
- Table 44a: Compounds of the formula I.A1 in which (R⁴)ₙ corresponds to line A-44 of Table A and the meaning for the combination of R¹, R² and R³ for each individual compound corresponds in each case to one line of Table B (compounds I.A1.A44.B1 to I.A1.A44.B442).
- Table 45a: Compounds of the formula I.A1 in which (R⁴)ₙ corresponds to line A-45 of Table A and the meaning for the combination of R¹, R² and R³ for each individual compound corresponds in each case to one line of Table B (compounds I.A1.A45.B1 to I.A1.A45.B442).
- Table 46a: Compounds of the formula I.A1 in which (R⁴)ₙ corresponds to line A-46 of Table A and the meaning for the combination of R¹, R² and R³ for each individual compound corresponds in each case to one line of Table B (compounds I.A1.A46.B1 to I.A1.A46.B442).
- Table 47a: Compounds of the formula I.A1 in which (R⁴)ₙ corresponds to line A-47 of Table A and the meaning for the combination of R¹, R² and R³ for each individual compound corresponds in each case to one line of Table B (compounds I.A1.A47.B1 to I.A1.A47.B442).
- Table 48a: Compounds of the formula I.A1 in which (R⁴)ₙ corresponds to line A-48 of Table A and the meaning for the combination of R¹, R² and R³ for each individual compound corresponds in each case to one line of Table B (compounds I.A1.A48.B1 to I.A1.A48.B442).
- Table 49a: Compounds of the formula I.A1 in which (R⁴)ₙ corresponds to line A-49 of Table A and the meaning for the combination of R¹, R² and R³ for each individual compound corresponds in each case to one line of Table B (compounds I.A1.A49.B1 to I.A1.A49.B442).
- Table 50a: Compounds of the formula I.A1 in which (R⁴)ₙ corresponds to line A-50 of Table A and the meaning for the combination of R¹, R² and R³ for each individual compound corresponds in each case to one line of Table B (compounds I.A1.A50.B1 to I.A1.A50.B442).
- Table 51a: Compounds of the formula I.A1 in which (R⁴)ₙ corresponds to line A-51 of Table A and the meaning for the combination of R¹, R² and R³ for each individual compound corresponds in each case to one line of Table B (compounds I.A1.A51.B1 to I.A1.A51.B442).
- Table 52a: Compounds of the formula I.A1 in which (R⁴)ₙ corresponds to line A-52 of Table A and the meaning for the combination of R¹, R² and R³ for each individual compound corresponds in each case to one line of Table B (compounds I.A1.A52.B1 to I.A1.A52.B442).
- Table 53a: Compounds of the formula I.A1 in which (R⁴)ₙ corresponds to line A-53 of Table A and the meaning for the combination of R¹, R² and R³ for each individual compound corresponds in each case to one line of Table B (compounds I.A1.A53.B1 to I.A1.A53.B442).
- Table 54a: Compounds of the formula I.A1 in which (R⁴)ₙ corresponds to line A-54 of Table A and the meaning for the combination of R¹, R² and R³ for each individual compound corresponds in each case to one line of Table B (compounds I.A1.A54.B1 to I.A1.A54.B442).
- Table 55a: Compounds of the formula I.A1 in which (R⁴)ₙ corresponds to line A-55 of Table A and the meaning for the combination of R¹, R² and R³ for each individual compound corresponds in each case to one line of Table B (compounds I.A1.A55.B1 to I.A1.A55.B442).
- Table 56a: Compounds of the formula I.A1 in which (R⁴)ₙ corresponds to line A-56 of Table A and the meaning for the combination of R¹, R² and R³ for each individual compound corresponds in each case to one line of Table B (compounds I.A1.A56.B1 to I.A1.A56.B442).
- Table 57a: Compounds of the formula I.A1 in which (R⁴)ₙ corresponds to line A-57 of Table A and the meaning for the combination of R¹, R² and R³ for each individual compound corresponds in each case to one line of Table B (compounds I.A1.A57.B1 to I.A1.A57.B442).
- Table 58a: Compounds of the formula I.A1 in which (R⁴)ₙ corresponds to line A-58 of Table A and the meaning for the combination of R¹, R² and R³ for each individual compound corresponds in each case to one line of Table B (compounds I.A1.A58.B1 to I.A1.A58.B442).
- Table 59a: Compounds of the formula I.A1 in which (R⁴)ₙ corresponds to line A-59 of Table A and the meaning for the combination of R¹, R² and R³ for each individual compound corresponds in each case to one line of Table B (compounds I.A1.A59.B1 to I.A1.A59.B442).
- Table 60a: Compounds of the formula I.A1 in which (R⁴)ₙ corresponds to line A-60 of Table A and the meaning for the combination of R¹, R² and R³ for each individual compound corresponds in each case to one line of Table B (compounds I.A1.A60.B1 to I.A1.A60.B442).
- Table 61a: Compounds of the formula I.A1 in which (R⁴)ₙ corresponds to line A-61 of Table A and the meaning for the combination of R¹, R² and R³ for each individual compound corresponds in each case to one line of Table B (compounds I.A1.A61.B1 to I.A1.A61.B442).
- Table 62a: Compounds of the formula I.A1 in which (R⁴)ₙ corresponds to line A-62 of Table A and the meaning for the combination of R¹, R² and R³ for each individual compound corresponds in each case to one line of Table B (compounds I.A1.A62.B1 to I.A1.A62.B442).
- Table 63a: Compounds of the formula I.A1 in which (R⁴)ₙ corresponds to line A-63 of Table A and the meaning for the combination of R¹, R² and R³ for each individual compound corresponds in each case to one line of Table B (compounds I.A1.A63.B1 to I.A1.A63.B442).
- Table 64a: Compounds of the formula I.A1 in which (R⁴)ₙ corresponds to line A-64 of Table A and the meaning for the combination of R¹, R² and R³ for each individual compound corresponds in each case to one line of Table B (compounds I.A1.A64.B1 to I.A1.A64.B442).
- Table 1 b: Compounds of the formula I.A2 in which (R⁴)ₙ corresponds to line A-1 of Table A and the meaning for the combination of R¹, R² and R³ for each individual compound corresponds in each case to one line of Table B (compounds I.A2.A1.B1 to I.A2.A1.B442).
- Table 2b: Compounds of the formula I.A2 in which (R⁴)ₙ corresponds to line A-2 of Table A and the meaning for the combination of R¹, R² and R³ for each individual compound corresponds in each case to one line of Table B (compounds I.A2.A2.B1 to 1.A2.A2.B442).
- Table 3b: Compounds of the formula I.A2 in which (R⁴)ₙ corresponds to line A-3 of Table A and the meaning for the combination of R¹, R² and R³ for each individual compound corresponds in each case to one line of Table B (compounds I.A2.A3.B1 to 1.A2.A3.B442).
- Table 4b: Compounds of the formula I.A2 in which (R⁴)ₙ corresponds to line A-4 of Table A and the meaning for the combination of R¹, R² and R³ for each individual compound corresponds in each case to one line of Table B (compounds I.A2.A4.B1 to I.A2.A4.B442).
- Table 5b: Compounds of the formula I.A2 in which (R⁴)ₙ corresponds to line A-5 of Table A and the meaning for the combination of R¹, R² and R³ for each individual compound corresponds in each case to one line of Table B (compounds I.A2.A5.B1 to I.A2.A5.B442).
- Table 6b: Compounds of the formula I.A2 in which (R⁴)ₙ corresponds to line A-6 of Table A and the meaning for the combination of R¹, R² and R³ for each individual compound corresponds in each case to one line of Table B (compounds I.A2.A6.B1 to I.A2.A6.B442).
- Table 7b: Compounds of the formula I.A2 in which (R⁴)ₙ corresponds to line A-7 of Table A and the meaning for the combination of R¹, R² and R³ for each individual compound corresponds in each case to one line of Table B (compounds I.A2.A7.B1 to I.A2.A7.B442).
- Table 8b: Compounds of the formula I.A2 in which (R⁴)ₙ corresponds to line A-8 of Table A and the meaning for the combination of R¹, R² and R³ for each individual compound corresponds in each case to one line of Table B (compounds I.A2.A8.B1 to I.A2.A8.B442).
- Table 9b: Compounds of the formula I.A2 in which (R⁴)ₙ corresponds to line A-9 of Table A and the meaning for the combination of R¹, R² and R³ for each individual compound corresponds in each case to one line of Table B (compounds I.A2.A9.B1 to I.A2.A9.B442).
- Table 10b: Compounds of the formula I.A2 in which (R⁴)ₙ corresponds to line A-10 of Table A and the meaning for the combination of R¹, R² and R³ for each individual compound corresponds in each case to one line of Table B (compounds I.A2.A10.B1 to I.A2.A10.B442).
- Table 11b: Compounds of the formula I.A2 in which (R⁴)ₙ corresponds to line A-11 of Table A and the meaning for the combination of R¹, R² and R³ for each individual compound corresponds in each case to one line of Table B (compounds I.A2.A11.B1 to I.A2.A11.B442).
- Table 12b: Compounds of the formula I.A2 in which (R⁴)ₙ corresponds to line A-12 of Table A and the meaning for the combination of R¹, R² and R³ for each individual compound corresponds in each case to one line of Table B (compounds I.A2.A12.B1 to I.A2.A12.B442).
- Table 13b: Compounds of the formula I.A2 in which (R⁴)ₙ corresponds to line A-13 of Table A and the meaning for the combination of R¹, R² and R³ for each individual compound corresponds in each case to one line of Table B (compounds I.A2.A13.B1 to I.A2.A13.B442).
- Table 14b: Compounds of the formula I.A2 in which (R⁴)ₙ corresponds to line A-14 of Table A and the meaning for the combination of R¹, R² and R³ for each individual compound corresponds in each case to one line of Table B (compounds I.A2.A14.B1 to I.A2.A14.B442).
- Table 15b: Compounds of the formula I.A2 in which (R⁴)ₙ corresponds to line A-15 of Table A and the meaning for the combination of R¹, R² and R³ for each individual compound corresponds in each case to one line of Table B (compounds I.A2.A15.B1 to I.A2.A15.B442).
- Table 16b: Compounds of the formula I.A2 in which (R⁴)ₙ corresponds to line A-16 of Table A and the meaning for the combination of R¹, R² and R³ for each individual compound corresponds in each case to one line of Table B (compounds I.A2.A16.B1 to I.A2.A16.B442).
- Table 17b: Compounds of the formula I.A2 in which (R⁴)ₙ corresponds to line A-17 of Table A and the meaning for the combination of R¹, R² and R³ for each individual compound corresponds in each case to one line of Table B (compounds I.A2.A17.B1 to I.A2.A17.B442).
- Table 18b: Compounds of the formula I.A2 in which (R⁴)ₙ corresponds to line A-18 of Table A and the meaning for the combination of R¹, R² and R³ for each individual compound corresponds in each case to one line of Table B (compounds I.A2.A18.B1 to I.A2.A18.B442).
- Table 19b: Compounds of the formula I.A2 in which (R⁴)ₙ corresponds to line A-19 of Table A and the meaning for the combination of R¹, R² and R³ for each individual compound corresponds in each case to one line of Table B (compounds I.A2.A19.B1 to I.A2.A19.B442).
- Table 20b: Compounds of the formula I.A2 in which (R⁴)ₙ corresponds to line A-20 of Table A and the meaning for the combination of R¹, R² and R³ for each individual compound corresponds in each case to one line of Table B (compounds I.A2.A20.B1 to I.A2.A20.B442).
- Table 21b: Compounds of the formula I.A2 in which (R⁴)ₙ corresponds to line A-21 of Table A and the meaning for the combination of R¹, R² and R³ for each individual compound corresponds in each case to one line of Table B (compounds I.A2.A21.B1 to I.A2.A21.B442).
- Table 22b: Compounds of the formula I.A2 in which (R⁴)ₙ corresponds to line A-22 of Table A and the meaning for the combination of R¹, R² and R³ for each individual compound corresponds in each case to one line of Table B (compounds I.A2.A22.B1 to I.A2.A22.B442).
- Table 23b: Compounds of the formula I.A2 in which (R⁴)ₙ corresponds to line A-23 of Table A and the meaning for the combination of R¹, R² and R³ for each individual compound corresponds in each case to one line of Table B (compounds I.A2.A23.B1 to I.A2.A23.B442).
- Table 24b: Compounds of the formula I.A2 in which (R⁴)ₙ corresponds to line A-24 of Table A and the meaning for the combination of R¹, R² and R³ for each individual compound corresponds in each case to one line of Table B (compounds I.A2.A24.B1 to I.A2.A24.B442).
- Table 25b: Compounds of the formula I.A2 in which (R⁴)ₙ corresponds to line A-25 of Table A and the meaning for the combination of R¹, R² and R³ for each individual compound corresponds in each case to one line of Table B (compounds I.A2.A25.B1 to I.A2.A25.B442).
- Table 26b: Compounds of the formula I.A2 in which (R⁴)ₙ corresponds to line A-26 of Table A and the meaning for the combination of R¹, R² and R³ for each individual compound corresponds in each case to one line of Table B (compounds I.A2.A26.B1 to I.A2.A26.B442).
- Table 27b: Compounds of the formula I.A2 in which (R⁴)ₙ corresponds to line A-27 of Table A and the meaning for the combination of R¹, R² and R³ for each individual compound corresponds in each case to one line of Table B (compounds I.A2.A27.B1 to I.A2.A27.B442).
- Table 28b: Compounds of the formula I.A2 in which (R⁴)ₙ corresponds to line A-28 of Table A and the meaning for the combination of R¹, R² and R³ for each individual compound corresponds in each case to one line of Table B (compounds I.A2.A28.B1 to I.A2.A28.B442).
- Table 29b: Compounds of the formula I.A2 in which (R⁴)ₙ corresponds to line A-29 of Table A and the meaning for the combination of R¹, R² and R³ for each individual compound corresponds in each case to one line of Table B (compounds I.A2.A29.B1 to I.A2.A29.B442).
- Table 30b: Compounds of the formula I.A2 in which (R⁴)ₙ corresponds to line A-30 of Table A and the meaning for the combination of R¹, R² and R³ for each individual compound corresponds in each case to one line of Table B (compounds I.A2.A30.B1 to I.A2.A30.B442).
- Table 31b: Compounds of the formula I.A2 in which (R⁴)ₙ corresponds to line A-31 of Table A and the meaning for the combination of R¹, R² and R³ for each individual compound corresponds in each case to one line of Table B (compounds I.A2.A31.B1 to I.A2.A31.B442).
- Table 32b: Compounds of the formula I.A2 in which (R⁴)ₙ corresponds to line A-32 of Table A and the meaning for the combination of R¹, R² and R³ for each individual compound corresponds in each case to one line of Table B (compounds I.A2.A32.B1 to I.A2.A32.B442).
- Table 33b: Compounds of the formula I.A2 in which (R⁴)ₙ corresponds to line A-33 of Table A and the meaning for the combination of R¹, R² and R³ for each individual compound corresponds in each case to one line of Table B (compounds I.A2.A33.B1 to I.A2.A33.B442).
- Table 34b: Compounds of the formula I.A2 in which (R⁴)ₙ corresponds to line A-34 of Table A and the meaning for the combination of R¹, R² and R³ for each individual compound corresponds in each case to one line of Table B (compounds I.A2.A34.B1 to I.A2.A34.B442).
- Table 35b: Compounds of the formula I.A2 in which (R⁴)ₙ corresponds to line A-35 of Table A and the meaning for the combination of R¹, R² and R³ for each individual compound corresponds in each case to one line of Table B (compounds I.A2.A35.B1 to I.A2.A35.B442).
- Table 36b: Compounds of the formula I.A2 in which (R⁴)ₙ corresponds to line A-36 of Table A and the meaning for the combination of R¹, R² and R³ for each individual compound corresponds in each case to one line of Table B (compounds I.A2.A36.B1 to I.A2.A36.B442).
- Table 37b: Compounds of the formula I.A2 in which (R⁴)ₙ corresponds to line A-37 of Table A and the meaning for the combination of R¹, R² and R³ for each individual compound corresponds in each case to one line of Table B (compounds I.A2.A37.B1 to I.A2.A37.B442).
- Table 38b: Compounds of the formula I.A2 in which (R⁴)ₙ corresponds to line A-38 of Table A and the meaning for the combination of R¹, R² and R³ for each individual compound corresponds in each case to one line of Table B (compounds I.A2.A38.B1 to I.A2.A38.B442).
- Table 39b: Compounds of the formula I.A2 in which (R⁴)ₙ corresponds to line A-39 of Table A and the meaning for the combination of R¹, R² and R³ for each individual compound corresponds in each case to one line of Table B (compounds I.A2.A39.B1 to I.A2.A39.B442).
- Table 40b: Compounds of the formula I.A2 in which (R⁴)ₙ corresponds to line A-40 of Table A and the meaning for the combination of R¹, R² and R³ for each individual compound corresponds in each case to one line of Table B (compounds I.A2.A40.B1 to I.A2.A40.B442).
- Table 41b: Compounds of the formula I.A2 in which (R⁴)ₙ corresponds to line A-41 of Table A and the meaning for the combination of R¹, R² and R³ for each individual compound corresponds in each case to one line of Table B (compounds I.A2.A41.B1 to I.A2.A41.B442).
- Table 42b: Compounds of the formula I.A2 in which (R⁴)ₙ corresponds to line A-42 of Table A and the meaning for the combination of R¹, R² and R³ for each individual compound corresponds in each case to one line of Table B (compounds I.A2.A42.B1 to I.A2.A42.B442).
- Table 43b: Compounds of the formula I.A2 in which (R⁴)ₙ corresponds to line A-43 of Table A and the meaning for the combination of R¹, R² and R³ for each individual compound corresponds in each case to one line of Table B (compounds I.A2.A43.B1 to I.A2.A43.B442).
- Table 44b: Compounds of the formula I.A2 in which (R⁴)ₙ corresponds to line A-44 of Table A and the meaning for the combination of R¹, R² and R³ for each individual compound corresponds in each case to one line of Table B (compounds I.A2.A44.B1 to I.A2.A44.B442).
- Table 45b: Compounds of the formula I.A2 in which (R⁴)ₙ corresponds to line A-45 of Table A and the meaning for the combination of R¹, R² and R³ for each individual compound corresponds in each case to one line of Table B (compounds I.A2.A45.B1 to I.A2.A45.B442).
- Table 46b: Compounds of the formula I.A2 in which (R⁴)ₙ corresponds to line A-46 of Table A and the meaning for the combination of R¹, R² and R³ for each individual compound corresponds in each case to one line of Table B (compounds I.A2.A46.B1 to I.A2.A46.B442).
- Table 47b: Compounds of the formula I.A2 in which (R⁴)ₙ corresponds to line A-47 of Table A and the meaning for the combination of R¹, R² and R³ for each individual compound corresponds in each case to one line of Table B (compounds I.A2.A47.B1 to I.A2.A47.B442).
- Table 48b: Compounds of the formula I.A2 in which (R⁴)ₙ corresponds to line A-48 of Table A and the meaning for the combination of R¹, R² and R³ for each individual compound corresponds in each case to one line of Table B (compounds I.A2.A48.B1 to I.A2.A48.B442).
- Table 49b: Compounds of the formula I.A2 in which (R⁴)ₙ corresponds to line A-49 of Table A and the meaning for the combination of R¹, R² and R³ for each individual compound corresponds in each case to one line of Table B (compounds I.A2.A49.B1 to I.A2.A49.B442).
- Table 50b: Compounds of the formula I.A2 in which (R⁴)ₙ corresponds to line A-50 of Table A and the meaning for the combination of R¹, R² and R³ for each individual compound corresponds in each case to one line of Table B (compounds I.A2.A50.B1 to I.A2.A50.B442).
- Table 51b: Compounds of the formula I.A2 in which (R⁴)ₙ corresponds to line A-51 of Table A and the meaning for the combination of R¹, R² and R³ for each individual compound corresponds in each case to one line of Table B (compounds LA2.A51.B1 to I.A2.A51.B442).
- Table 52b: Compounds of the formula I.A2 in which (R⁴)ₙ corresponds to line A-52 of Table A and the meaning for the combination of R¹, R² and R³ for each individual compound corresponds in each case to one line of Table B (compounds I.A2.A52.B1 to I.A2.A52.B442).
- Table 53b: Compounds of the formula I.A2 in which (R⁴)ₙ corresponds to line A-53 of Table A and the meaning for the combination of R¹, R² and R³ for each individual compound corresponds in each case to one line of Table B (compounds I.A2.A53.B1 to I.A2.A53.B442).
- Table 54b: Compounds of the formula I.A2 in which (R⁴)ₙ corresponds to line A-54 of Table A and the meaning for the combination of R¹, R² and R³ for each individual compound corresponds in each case to one line of Table B (compounds I.A2.A54.B1 to I.A2.A54.B442).
- Table 55b: Compounds of the formula I.A2 in which (R⁴)ₙ corresponds to line A-55 of Table A and the meaning for the combination of R¹, R² and R³ for each individual compound corresponds in each case to one line of Table B (compounds I.A2.A55.B1 to I.A2.A55.B442).
- Table 56b: Compounds of the formula I.A2 in which (R⁴)ₙ corresponds to line A-56 of Table A and the meaning for the combination of R¹, R² and R³ for each individual compound corresponds in each case to one line of Table B (compounds I.A2.A56.B1 to I.A2.A56.B442).
- Table 57b: Compounds of the formula I.A2 in which (R⁴)ₙ corresponds to line A-57 of Table A and the meaning for the combination of R¹, R² and R³ for each individual compound corresponds in each case to one line of Table B (compounds I.A2.A57.B1 to I.A2.A57.B442).
- Table 58b: Compounds of the formula I.A2 in which (R⁴)ₙ corresponds to line A-58 of Table A and the meaning for the combination of R¹, R² and R³ for each individual compound corresponds in each case to one line of Table B (compounds I.A2.A58.B1 to I.A2.A58.B442).
- Table 59b: Compounds of the formula I.A2 in which (R⁴)ₙ corresponds to line A-59 of Table A and the meaning for the combination of R¹, R² and R³ for each individual compound corresponds in each case to one line of Table B (compounds I.A2.A59.B1 to I.A2.A59.B442).
- Table 60b: Compounds of the formula I.A2 in which (R⁴)ₙ corresponds to line A-60 of Table A and the meaning for the combination of R¹, R² and R³ for each individual compound corresponds in each case to one line of Table B (compounds I.A2.A60.B1 to I.A2.A60.B442).
- Table 61b: Compounds of the formula I.A2 in which (R⁴)ₙ corresponds to line A-61 of Table A and the meaning for the combination of R¹, R² and R³ for each individual compound corresponds in each case to one line of Table B (compounds I.A2.A61.B1 to I.A2.A61.B442).
- Table 62b: Compounds of the formula I.A2 in which (R⁴)ₙ corresponds to line A-62 of Table A and the meaning for the combination of R¹, R² and R³ for each individual compound corresponds in each case to one line of Table B (compounds I.A2.A62.B1 to I.A2.A62.B442).
- Table 63b: Compounds of the formula I.A2 in which (R⁴)ₙ corresponds to line A-63 of Table A and the meaning for the combination of R¹, R² and R³ for each individual compound corresponds in each case to one line of Table B (compounds I.A2.A63.B1 to I.A2.A63.B442).
- Table 64b: Compounds of the formula I.A2 in which (R⁴)ₙ corresponds to line A-64 of Table A and the meaning for the combination of R¹, R² and R³ for each individual compound corresponds in each case to one line of Table B (compounds I.A2.A64.B1 to I.A2.A64.B442).

**Table A:**

| line | **(R⁴)ₙ** |
|---|---|
| A-1 | -* |
| A-2 | 2-Cl |
| A-3 | 3-Cl |
| A-4 | 4-Cl |
| A-5 | 2-F |
| A-6 | 3-F |
| A-7 | 4-F |
| A-8 | 2-CN |
| A-9 | 3-CN |
| A-10 | 4-CN |
| A-11 | 2-NO₂ |
| A-12 | 3-NO₂ |
| A-13 | 4-NO₂ |
| A-14 | 2-SCH₃ |
| A-15 | 3-SCH₃ |
| A-16 | 4-SCH₃ |
| A-17 | 2-SOCH₃ |
| A-18 | 3-SOCH₃ |
| A-19 | 4-SOCH₃ |
| A-20 | 2-SO₂CH₃ |
| A-21 | 3-SO₂CH₃ |
| A-22 | 4-SO₂CH₃ |
| A-23 | 2-CO₂CH₃ |
| A-24 | 3-CO₂CH₃ |
| A-25 | 4-CO₂CH₃ |
| A-26 | 2-CH₃ |
| A-27 | 3-CH₃ |
| A-28 | 4-CH₃ |
| A-29 | 2-CF₃ |
| A-30 | 3-CF₃ |
| A-31 | 4-CF₃ |
| A-32 | 2-CHF₂ |
| A-33 | 3-CHF₂ |
| A-34 | 4-CHF₂ |
| A-35 | 2-OCH₃ |
| A-36 | 3-OCH₃ |
| A-37 | 4-OCH₃ |
| A-38 | 2-OCF₃ |
| A-39 | 3-OCF₃ |
| A-40 | 4-OCF₃ |
| A-41 | 2-OCHF₂ |
| A-42 | 3-OCHF₂ |
| A-43 | 4-OCHF₂ |
| A-44 | 2,4,6-(CH₃)₃ |
| A-45 | 2,3-Cl₂ |
| A-46 | 2,4-Cl₂ |
| A-47 | 2,5-Cl₂ |
| A-48 | 3,4-Cl₂ |
| A-49 | 3,5-Cl₂ |
| A-50 | 2,6-Cl₂ |
| A-51 | 2,3-F₂ |
| A-52 | 2,4-F₂ |
| A-53 | 2,5-F₂ |
| A-54 | 3,4-F₂ |
| A-55 | 3,5-F₂ |
| A-56 | 2,6-F₂ |
| A-57 | 2-CF₃-4-Cl |
| A-58 | 2-CF₃-4-F |
| A-59 | 2-Cl-4-CF₃ |
| A-60 | 2-F-4-CF₃ |
| A-61 | 2-CN-4-Cl |
| A-62 | 2-CN-4-F |
| A-63 | 2-Cl-4-CN |
| A-64 | 2-F-4-CN |

| | |
|---|---|
| * means that n=0 | |

**Table B:**

| **line** | **R¹** | **R²** | **R³** |
|---|---|---|---|
| B-1 | CH₃ | H | H |
| B-2 | C₂H₅ | H | H |
| B-3 | CH₂CH₂CH₃ | H | H |
| B-4 | CH(CH₃)₂ | H | H |
| B-5 | CH₂CHCH₂ | H | H |
| B-6 | CH₂CH₂OH | H | H |
| B-7 | CH₂CH₂CH₂OH | H | H |
| B-8 | F | H | H |
| B-9 | Cl | H | H |
| B-10 | Br | H | H |
| B-11 | OH | H | H |
| B-12 | NH₂ | H | H |
| B-13 | N(CH₃)₂ | H | H |
| B-14 | CH₃ | CH₃ | H |
| B-15 | CH₂CH₃ | CH₃ | H |
| B-16 | CH₂CH₃ | CH₂CH₃ | H |
| B-17 | 1-triazolyl | H | H |
| B-18 | 1-imidazolyl | H | H |
| B-19 | 2-pyridinyl | H | H |
| B-20 | 3-pyridinyl | H | H |
| B-21 | 4-pyridinyl | H | H |
| B-22 | =CH₂ | | H |
| B-23 | cyclopropyl | | H |
| B-24 | cyclobutyl | | H |
| B-25 | cyclopentyl | | H |
| B-26 | cyclohexyl | | H |
| B-27 | CH₃ | H | CH₃ |
| B-28 | C₂H₅ | H | CH₃ |
| B-29 | CH₂CH₂CH₃ | H | CH₃ |
| B-30 | CH(CH₃)₂ | H | CH₃ |
| B-31 | CH₂CHCH₂ | H | CH₃ |
| B-32 | CH₂CH₂OH | H | CH₃ |
| B-33 | CH₂CH₂CH₂OH | H | CH₃ |
| B-34 | F | H | CH₃ |
| B-35 | Cl | H | CH₃ |
| B-36 | Br | H | CH₃ |
| B-37 | OH | H | CH₃ |
| B-38 | NH₂ | H | CH₃ |
| B-39 | N(CH₃)₂ | H | CH₃ |
| B-40 | CH₃ | CH₃ | CH₃ |
| B-41 | CH₂CH₃ | CH₃ | CH₃ |
| B-42 | CH₂CH₃ | CH₂CH₃ | CH₃ |
| B-43 | 1-triazolyl | H | CH₃ |
| B-44 | 1-imidazolyl | H | CH₃ |
| B-45 | 2-pyridinyl | H | CH₃ |
| B-46 | 3-pyridinyl | H | CH₃ |
| B-47 | 4-pyridinyl | H | CH₃ |
| B-48 | =CH₂ | | CH₃ |
| B-49 | cyclopropyl | | CH₃ |
| B-50 | cyclobutyl | | CH₃ |
| B-51 | cyclopentyl | | CH₃ |
| B-52 | cyclohexyl | | CH₃ |
| B-53 | CH₃ | H | CH₂CH₃ |
| B-54 | C₂H₅ | H | CH₂CH₃ |
| B-55 | CH₂CH₂CH₃ | H | CH₂CH₃ |
| B-56 | CH(CH₃)₂ | H | CH₂CH₃ |
| B-57 | CH₂CHCH₂ | H | CH₂CH₃ |
| B-58 | CH₂CH₂OH | H | CH₂CH₃ |
| B-59 | CH₂CH₂CH₂OH | H | CH₂CH₃ |
| B-60 | F | H | CH₂CH₃ |
| B-61 | Cl | H | CH₂CH₃ |
| B-62 | Br | H | CH₂CH₃ |
| B-63 | OH | H | CH₂CH₃ |
| B-64 | NH₂ | H | CH₂CH₃ |
| B-65 | N(CH₃)₂ | H | CH₂CH₃ |
| B-66 | CH₃ | CH₃ | CH₂CH₃ |
| B-67 | CH₂CH₃ | CH₃ | CH₂CH₃ |
| B-68 | CH₂CH₃ | CH₂CH₃ | CH₂CH₃ |
| B-69 | 1-triazolyl | H | CH₂CH₃ |
| B-70 | 1-imidazolyl | H | CH₂CH₃ |
| B-71 | 2-pyridinyl | H | CH₂CH₃ |
| B-72 | 3-pyridinyl | H | CH₂CH₃ |
| B-73 | 4-pyridinyl | H | CH₂CH₃ |
| B-74 | =CH₂ | | CH₂CH₃ |
| B-75 | cyclopropyl | | CH₂CH₃ |
| B-76 | cyclobutyl | | CH₂CH₃ |
| B-77 | cyclopentyl | | CH₂CH₃ |
| B-78 | cyclohexyl | | CH₂CH₃ |
| B-79 | CH₃ | H | CH(CH₃)₂ |
| B-80 | C₂H₅ | H | CH(CH₃)₂ |
| B-81 | CH₂CH₂CH₃ | H | CH(CH₃)₂ |
| B-82 | CH(CH₃)₂ | H | CH(CH₃)₂ |
| B-83 | CH₂CHCH₂ | H | CH(CH₃)₂ |
| B-84 | CH₂CH₂OH | H | CH(CH₃)₂ |
| B-85 | CH₂CH₂CH₂OH | H | CH(CH₃)₂ |
| B-86 | F | H | CH(CH₃)₂ |
| B-87 | Cl | H | CH(CH₃)₂ |
| B-88 | Br | H | CH(CH₃)₂ |
| B-89 | OH | H | CH(CH₃)₂ |
| B-90 | NH₂ | H | CH(CH₃)₂ |
| B-91 | N(CH₃)₂ | H | CH(CH₃)₂ |
| B-92 | CH₃ | CH₃ | CH(CH₃)₂ |
| B-93 | CH₂CH₃ | CH₃ | CH(CH₃)₂ |
| B-94 | CH₂CH₃ | CH₂CH₃ | CH(CH₃)₂ |
| B-95 | 1-triazolyl | H | CH(CH₃)₂ |
| B-96 | 1-imidazolyl | H | CH(CH₃)₂ |
| B-97 | 2-pyridinyl | H | CH(CH₃)₂ |
| B-98 | 3-pyridinyl | H | CH(CH₃)₂ |
| B-99 | 4-pyridinyl | H | CH(CH₃)₂ |
| B-100 | =CH₂ | | CH(CH₃)₂ |
| B-101 | cyclopropyl | | CH(CH₃)₂ |
| B-102 | cyclobutyl | | CH(CH₃)₂ |
| B-103 | cyclopentyl | | CH(CH₃)₂ |
| B-104 | cyclohexyl | | CH(CH₃)₂ |
| B-105 | CH₃ | H | C₃H₅ (cyclopropyl) |
| B-106 | C₂H₅ | H | C₃H₅ (cyclopropyl) |
| B-107 | CH₂CH₂CH₃ | H | C₃H₅ (cyclopropyl) |
| B-108 | CH(CH₃)₂ | H | C₃H₅ (cyclopropyl) |
| B-109 | CH₂CHCH₂ | H | C₃H₅ (cyclopropyl) |
| B-110 | CH₂CH₂OH | H | C₃H₅ (cyclopropyl) |
| B-111 | CH₂CH₂CH₂OH | H | C₃H₅ (cyclopropyl) |
| B-112 | F | H | C₃H₅ (cyclopropyl) |
| B-113 | Cl | H | C₃H₅ (cyclopropyl) |
| B-114 | Br | H | C₃H₅ (cyclopropyl) |
| B-115 | OH | H | C₃H₅ (cyclopropyl) |
| B-116 | NH₂ | H | C₃H₅ (cyclopropyl) |
| B-117 | N(CH₃)₂ | H | C₃H₅ (cyclopropyl) |
| B-118 | CH₃ | CH₃ | C₃H₅ (cyclopropyl) |
| B-119 | CH₂CH₃ | CH₃ | C₃H₅ (cyclopropyl) |
| B-120 | CH₂CH₃ | CH₂CH₃ | C₃H₅ (cyclopropyl) |
| B-121 | 1-triazolyl | H | C₃H₅ (cyclopropyl) |
| B-122 | 1-imidazolyl | H | C₃H₅(cydopropyl) |
| B-123 | 2-pyridinyl | H | C₃H₅ (cyclopropyl) |
| B-124 | 3-pyridinyl | H | C₃H₅ (cyclopropyl) |
| B-125 | 4-pyridinyl | H | C₃H₅ (cyclopropyl) |
| B-126 | =CH₂ | | C₃H₅ (cyclopropyl) |
| B-127 | cyclopropyl | | C₃H₅ (cyclopropyl) |
| B-128 | cyclobutyl | | C₃H₅ (cyclopropyl) |
| B-129 | cyclopentyl | | C₃H₅(cyclopropyl) |
| B-130 | cyclohexyl | | C₃H₅(cyclopropyl) |
| B-131 | CH₃ | H | C₄H₇(cyclobutyl) |
| B-132 | C₂H₅ | H | C₄H₇(cyclobutyl) |
| B-133 | CH₂CH₂CH₃ | H | C₄H₇(cyclobutyl) |
| B-134 | CH(CH₃)₂ | H | C₄H₇(cyclobutyl) |
| B-135 | CH₂CHCH₂ | H | C₄H₇(cyclobutyl) |
| B-136 | CH₂CH₂OH | H | C₄H₇(cyclobutyl) |
| B-137 | CH₂CH₂CH₂OH | H | C₄H₇(cyclobutyl) |
| B-138 | F | H | C₄H₇(cyclobutyl) |
| B-139 | Cl | H | C₄H₇(cyclobutyl) |
| B-140 | Br | H | C₄H₇(cyclobutyl) |
| B-141 | OH | H | C₄H₇(cyclobutyl) |
| B-142 | NH₂ | H | C₄H₇(cyclobutyl) |
| B-143 | N(CH₃)₂ | H | C₄H₇(cyclobutyl) |
| B-144 | CH₃ | CH₃ | C₄H₇(cyclobutyl) |
| B-145 | CH₂CH₃ | CH₃ | C₄H₇(cyclobutyl) |
| B-146 | CH₂CH₃ | CH₂CH₃ | C₄H₇(cyclobutyl) |
| B-147 | 1-triazolyl | H | C₄H₇(cyclobutyl) |
| B-148 | 1-imidazolyl | H | C₄H₇(cyclobutyl) |
| B-149 | 2-pyridinyl | H | C₄H₇(cyclobutyl) |
| B-150 | 3-pyridinyl | H | C₄H₇(cyclobutyl) |
| B-151 | 4-pyridinyl | H | C₄H₇(cyclobutyl) |
| B-152 | =CH₂ | | C₄H₇(cyclobutyl) |
| B-153 | cyclopropyl | | C₄H₇(cyclobutyl) |
| B-154 | cyclobutyl | | C₄H₇(cyclobutyl) |
| B-155 | cyclopentyl | | C₄H₇(cyclobutyl) |
| B-156 | cyclohexyl | | C₄H₇(cyclobutyl) |
| B-157 | CH₃ | H | C≡CCH₃ |
| B-158 | C₂H₅ | H | C≡CCH₃ |
| B-159 | CH₂CH₂CH₃ | H | C≡CCH₃ |
| B-160 | CH(CH₃)₂ | H | C≡CCH₃ |
| B-161 | CH₂CHCH₂ | H | C≡CCH₃ |
| B-162 | CH₂CH₂OH | H | C≡CCH₃ |
| B-163 | CH₂CH₂CH₂OH | H | C≡CCH₃ |
| B-164 | F | H | C≡CCH₃ |
| B-165 | Cl | H | C≡CCH₃ |
| B-166 | Br | H | C≡CCH₃ |
| B-167 | OH | H | C≡CCH₃ |
| B-168 | NH₂ | H | C≡CCH₃ |
| B-169 | N(CH₃)₂ | H | C≡CCH₃ |
| B-170 | CH₃ | CH₃ | C≡CCH₃ |
| B-171 | CH₂CH₃ | CH₃ | C≡CCH₃ |
| B-172 | CH₂CH₃ | CH₂CH₃ | C≡CCH₃ |
| B-173 | 1-triazolyl | H | C≡CCH₃ |
| B-174 | 1-imidazolyl | H | C≡CCH₃ |
| B-175 | 2-pyridinyl | H | C≡CCH₃ |
| B-176 | 3-pyridinyl | H | C≡CCH₃ |
| B-177 | 4-pyridinyl | H | C≡CCH₃ |
| B-178 | =CH₂ | | C≡CCH₃ |
| B-179 | cyclopropyl | | C≡CCH₃ |
| B-180 | cyclobutyl | | C≡CCH₃ |
| B-181 | cyclopentyl | | C≡CCH₃ |
| B-182 | cyclohexyl | | C≡CCH₃ |
| B-183 | CH₃ | H | C(CH₃)₃ |
| B-184 | C₂H₅ | H | C(CH₃)₃ |
| B-185 | CH₂CH₂CH₃ | H | C(CH₃)₃ |
| B-186 | CH(CH₃)₂ | H | C(CH₃)₃ |
| B-187 | CH₂CHCH₂ | H | C(CH₃)₃ |
| B-188 | CH₂CH₂OH | H | C(CH₃)₃ |
| B-189 | CH₂CH₂CH₂OH | H | C(CH₃)₃ |
| B-190 | F | H | C(CH₃)₃ |
| B-191 | Cl | H | C(CH₃)₃ |
| B-192 | Br | H | C(CH₃)₃ |
| B-193 | OH | H | C(CH₃)₃ |
| B-194 | NH₂ | H | C(CH₃)₃ |
| B-195 | N(CH₃)₂ | H | C(CH₃)₃ |
| B-196 | CH₃ | CH₃ | C(CH₃)₃ |
| B-197 | CH₂CH₃ | CH₃ | C(CH₃)₃ |
| B-198 | CH₂CH₃ | CH₂CH₃ | C(CH₃)₃ |
| B-199 | 1-triazolyl | H | C(CH₃)₃ |
| B-200 | 1-imidazolyl | H | C(CH₃)₃ |
| B-201 | 2-pyridinyl | H | C(CH₃)₃ |
| B-202 | 3-pyridinyl | H | C(CH₃)₃ |
| B-203 | 4-pyridinyl | H | C(CH₃)₃ |
| B-204 | =CH₂ | | C(CH₃)₃ |
| B-205 | cyclopropyl | | C(CH₃)₃ |
| B-206 | cyclobutyl | | C(CH₃)₃ |
| B-207 | cyclopentyl | | C(CH₃)₃ |
| B-208 | cyclohexyl | | C(CH₃)₃ |
| B-209 | CH₃ | H | CF₃ |
| B-210 | C₂H₅ | H | CF₃ |
| B-211 | CH₂CH₂CH₃ | H | CF₃ |
| B-212 | CH(CH₃)₂ | H | CF₃ |
| B-213 | CH₂CHCH₂ | H | CF₃ |
| B-214 | CH₂CH₂OH | H | CF₃ |
| B-215 | CH₂CH₂CH₂OH | H | CF₃ |
| B-216 | F | H | CF₃ |
| B-217 | Cl | H | CF₃ |
| B-218 | Br | H | CF₃ |
| B-219 | OH | H | CF₃ |
| B-220 | NH₂ | H | CF₃ |
| B-221 | N(CH₃)₂ | H | CF₃ |
| B-222 | CH₃ | CH₃ | CF₃ |
| B-223 | CH₂CH₃ | CH₃ | CF₃ |
| B-224 | CH₂CH₃ | CH₂CH₃ | CF₃ |
| B-225 | 1-triazolyl | H | CF₃ |
| B-226 | 1-imidazolyl | H | CF₃ |
| B-227 | 2-pyridinyl | H | CF₃ |
| B-228 | 3-pyridinyl | H | CF₃ |
| B-229 | 4-pyridinyl | H | CF₃ |
| B-230 | =CH₂ | | CF₃ |
| B-231 | cyclopropyl | | CF₃ |
| B-232 | cyclobutyl | | CF₃ |
| B-233 | cyclopentyl | | CF₃ |
| B-234 | cyclohexyl | | CF₃ |
| B-235 | CH₃ | H | CHF₂ |
| B-236 | C₂H₅ | H | CHF₂ |
| B-237 | CH₂CH₂CH₃ | H | CHF₂ |
| B-238 | CH(CH₃)₂ | H | CHF₂ |
| B-239 | CH₂CHCH₂ | H | CHF₂ |
| B-240 | CH₂CH₂OH | H | CHF₂ |
| B-241 | CH₂CH₂CH₂OH | H | CHF₂ |
| B-242 | F | H | CHF₂ |
| B-243 | Cl | H | CHF₂ |
| B-244 | Br | H | CHF₂ |
| B-245 | OH | H | CHF₂ |
| B-246 | NH₂ | H | CHF₂ |
| B-247 | N(CH₃)₂ | H | CHF₂ |
| B-248 | CH₃ | CH₃ | CHF₂ |
| B-249 | CH₂CH₃ | CH₃ | CHF₂ |
| B-250 | CH₂CH₃ | CH₂CH₃ | CHF₂ |
| B-251 | 1-triazolyl | H | CHF₂ |
| B-252 | 1-imidazolyl | H | CHF₂ |
| B-253 | 2-pyridinyl | H | CHF₂ |
| B-254 | 3-pyridinyl | H | CHF₂ |
| B-255 | 4-pyridinyl | H | CHF₂ |
| B-256 | =CH₂ | | CHF₂ |
| B-257 | cyclopropyl | | CHF₂ |
| B-258 | cyclobutyl | | CHF₂ |
| B-259 | cyclopentyl | | CHF₂ |
| B-260 | cyclohexyl | | CHF₂ |
| B-261 | CH₃ | H | CH=CHCH₃ |
| B-262 | C₂H₅ | H | CH=CHCH₃ |
| B-263 | CH₂CH₂CH₃ | H | CH=CHCH₃ |
| B-264 | CH(CH₃)₂ | H | CH=CHCH₃ |
| B-265 | CH₂CHCH₂ | H | CH=CHCH₃ |
| B-266 | CH₂CH₂OH | H | CH=CHCH₃ |
| B-267 | CH₂CH₂CH₂OH | H | CH=CHCH₃ |
| B-268 | F | H | CH=CHCH₃ |
| B-269 | Cl | H | CH=CHCH₃ |
| B-270 | Br | H | CH=CHCH₃ |
| B-271 | OH | H | CH=CHCH₃ |
| B-272 | NH₂ | H | CH=CHCH₃ |
| B-273 | N(CH₃)₂ | H | CH=CHCH₃ |
| B-274 | CH₃ | CH₃ | CH=CHCH₃ |
| B-275 | CH₂CH₃ | CH₃ | CH=CHCH₃ |
| B-276 | CH₂CH₃ | CH₂CH₃ | CH=CHCH₃ |
| B-277 | 1-triazolyl | H | CH=CHCH₃ |
| B-278 | 1-imidazolyl | H | CH=CHCH₃ |
| B-279 | 2-pyridinyl | H | CH=CHCH₃ |
| B-280 | 3-pyridinyl | H | CH=CHCH₃ |
| B-281 | 4-pyridinyl | H | CH=CHCH₃ |
| B-282 | =CH₂ | | CH=CHCH₃ |
| B-283 | cyclopropyl | | CH=CHCH₃ |
| B-284 | cyclobutyl | | CH=CHCH₃ |
| B-285 | cyclopentyl | | CH=CHCH₃ |
| B-286 | cyclohexyl | | CH=CHCH₃ |
| B-287 | CH₃ | H | C(CH₃)=CH₂ |
| B-288 | C₂H₅ | H | C(CH₃)=CH₂ |
| B-289 | CH₂CH₂CH₃ | H | C(CH₃)=CH₂ |
| B-290 | CH(CH₃)₂ | H | C(CH₃)=CH₂ |
| B-291 | CH₂CHCH₂ | H | C(CH₃)=CH₂ |
| B-292 | CH₂CH₂OH | H | C(CH₃)=CH₂ |
| B-293 | CH₂CH₂CH₂OH | H | C(CH₃)=CH₂ |
| B-294 | F | H | C(CH₃)=CH₂ |
| B-295 | Cl | H | C(CH₃)=CH₂ |
| B-296 | Br | H | C(CH₃)=CH₂ |
| B-297 | OH | H | C(CH₃)=CH₂ |
| B-298 | NH₂ | H | C(CH₃)=CH₂ |
| B-299 | N(CH₃)₂ | H | C(CH₃)=CH₂ |
| B-300 | CH₃ | CH₃ | C(CH₃)=CH₂ |
| B-301 | CH₂CH₃ | CH₃ | C(CH₃)=CH₂ |
| B-302 | CH₂CH₃ | CH₂CH₃ | C(CH₃)=CH₂ |
| B-303 | 1-triazolyl | H | C(CH₃)=CH₂ |
| B-304 | 1-imidazolyl | H | C(CH₃)=CH₂ |
| B-305 | 2-pyridinyl | H | C(CH₃)=CH₂ |
| B-306 | 3-pyridinyl | H | C(CH₃)=CH₂ |
| B-307 | 4-pyridinyl | H | C(CH₃)=CH₂ |
| B-308 | =CH₂ | | C(CH₃)=CH₂ |
| B-309 | cyclopropyl | | C(CH₃)=CH₂ |
| B-310 | cyclobutyl | | C(CH₃)=CH₂ |
| B-311 | cyclopentyl | | C(CH₃)=CH₂ |
| B-312 | cyclohexyl | | C(CH₃)=CH₂ |
| B-313 | CH₃ | H | 1-(Cl)-cyclopropyl |
| B-314 | C₂H₅ | H | 1-(Cl)-cyclopropyl |
| B-315 | CH₂CH₂CH₃ | H | 1-(Cl)-cyclopropyl |
| B-316 | CH(CH₃)₂ | H | 1-(Cl)-cyclopropyl |
| B-317 | CH₂CHCH₂ | H | 1-(Cl)-cyclopropyl |
| B-318 | CH₂CH₂OH | H | 1-(Cl)-cyclopropyl |
| B-319 | CH₂CH₂CH₂OH | H | 1-(Cl)-cyclopropyl |
| B-320 | F | H | 1-(Cl)-cyclopropyl |
| B-321 | Cl | H | 1-(Cl)-cyclopropyl |
| B-322 | Br | H | 1-(Cl)-cyclopropyl |
| B-323 | OH | H | 1-(Cl)-cyclopropyl |
| B-324 | NH₂ | H | 1-(Cl)-cyclopropyl |
| B-325 | N(CH₃)₂ | H | 1-(Cl)-cyclopropyl |
| B-326 | CH₃ | CH₃ | 1-(Cl)-cyclopropyl |
| B-327 | CH₂CH₃ | CH₃ | 1-(Cl)-cyclopropyl |
| B-328 | CH₂CH₃ | CH₂CH₃ | 1-(Cl)-cyclopropyl |
| B-329 | 1-triazolyl | H | 1-(Cl)-cyclopropyl |
| B-330 | 1-imidazolyl | H | 1-(Cl)-cyclopropyl |
| B-331 | 2-pyridinyl | H | 1-(Cl)-cyclopropyl |
| B-332 | 3-pyridinyl | H | 1-(Cl)-cyclopropyl |
| B-333 | 4-pyridinyl | H | 1-(Cl)-cyclopropyl |
| B-334 | =CH₂ | | 1-(Cl)-cyclopropyl |
| B-335 | cyclopropyl | | 1-(Cl)-cyclopropyl |
| B-336 | cyclobutyl | | 1-(Cl)-cyclopropyl |
| B-337 | cyclopentyl | | 1-(Cl)-cyclopropyl |
| B-338 | cyclohexyl | | 1-(Cl)-cyclopropyl |
| B-339 | CH₃ | H | 1-(F)-cyclopropyl |
| B-340 | C₂H₅ | H | 1-(F)-cyclopropyl |
| B-341 | CH₂CH₂CH₃ | H | 1-(F)-cyclopropyl |
| B-342 | CH(CH₃)₂ | H | 1-(F)-cyclopropyl |
| B-343 | CH₂CHCH₂ | H | 1-(F)-cyclopropyl |
| B-344 | CH₂CH₂OH | H | 1-(F)-cyclopropyl |
| B-345 | CH₂CH₂CH₂OH | H | 1-(F)-cyclopropyl |
| B-346 | F | H | 1-(F)-cyclopropyl |
| B-347 | Cl | H | 1-(F)-cyclopropyl |
| B-348 | Br | H | 1-(F)-cyclopropyl |
| B-349 | OH | H | 1-(F)-cyclopropyl |
| B-350 | NH₂ | H | 1-(F)-cyclopropyl |
| B-351 | N(CH₃)₂ | H | 1-(F)-cyclopropyl |
| B-352 | CH₃ | CH₃ | 1-(F)-cyclopropyl |
| B-353 | CH₂CH₃ | CH₃ | 1-(F)-cyclopropyl |
| B-354 | CH₂CH₃ | CH₂CH₃ | 1-(F)-cyclopropyl |
| B-355 | 1-triazolyl | H | 1-(F)-cyclopropyl |
| B-356 | 1-imidazolyl | H | 1-(F)-cyclopropyl |
| B-357 | 2-pyridinyl | H | 1-(F)-cyclopropyl |
| B-358 | 3-pyridinyl | H | 1-(F)-cyclopropyl |
| B-359 | 4-pyridinyl | H | 1-(F)-cyclopropyl |
| B-360 | =CH₂ | | 1-(F)-cyclopropyl |
| B-361 | cyclopropyl | | 1-(F)-cyclopropyl |
| B-362 | cyclobutyl | | 1-(F)-cyclopropyl |
| B-363 | cyclopentyl | | 1-(F)-cyclopropyl |
| B-364 | cyclohexyl | | 1-(F)-cyclopropyl |
| B-365 | CH₃ | H | CH₂CH₂CH₃ |
| B-366 | C₂H₅ | H | CH₂CH₂CH₃ |
| B-367 | CH₂CH₂CH₃ | H | CH₂CH₂CH₃ |
| B-368 | CH(CH₃)₂ | H | CH₂CH₂CH₃ |
| B-369 | CH₂CHCH₂ | H | CH₂CH₂CH₃ |
| B-370 | CH₂CH₂OH | H | CH₂CH₂CH₃ |
| B-371 | CH₂CH₂CH₂OH | H | CH₂CH₂CH₃ |
| B-372 | F | H | CH₂CH₂CH₃ |
| B-373 | Cl | H | CH₂CH₂CH₃ |
| B-374 | Br | H | CH₂CH₂CH₃ |
| B-375 | OH | H | CH₂CH₂CH₃ |
| B-376 | NH₂ | H | CH₂CH₂CH₃ |
| B-377 | N(CH₃)₂ | H | CH₂CH₂CH₃ |
| B-378 | CH₃ | CH₃ | CH₂CH₂CH₃ |
| B-379 | CH₂CH₃ | CH₃ | CH₂CH₂CH₃ |
| B-380 | CH₂CH₃ | CH₂CH₃ | CH₂CH₂CH₃ |
| B-381 | 1-triazolyl | H | CH₂CH₂CH₃ |
| B-382 | 1-imidazolyl | H | CH₂CH₂CH₃ |
| B-383 | 2-pyridinyl | H | CH₂CH₂CH₃ |
| B-384 | 3-pyridinyl | H | CH₂CH₂CH₃ |
| B-385 | 4-pyridinyl | H | CH₂CH₂CH₃ |
| B-386 | =CH₂ | | CH₂CH₂CH₃ |
| B-387 | cyclopropyl | | CH₂CH₂CH₃ |
| B-388 | cyclobutyl | | CH₂CH₂CH₃ |
| B-389 | cyclopentyl | | CH₂CH₂CH₃ |
| B-390 | cyclohexyl | | CH₂CH₂CH₃ |
| B-391 | CH₃ | H | CH₂CH₂CH₂CH₃ |
| B-392 | C₂H₅ | H | CH₂CH₂CH₂CH₃ |
| B-393 | CH₂CH₂CH₃ | H | CH₂CH₂CH₂CH₃ |
| B-394 | CH(CH₃)₂ | H | CH₂CH₂CH₂CH₃ |
| B-395 | CH₂CHCH₂ | H | CH₂CH₂CH₂CH₃ |
| B-396 | CH₂CH₂OH | H | CH₂CH₂CH₂CH₃ |
| B-397 | CH₂CH₂CH₂OH | H | CH₂CH₂CH₂CH₃ |
| B-398 | F | H | CH₂CH₂CH₂CH₃ |
| B-399 | Cl | H | CH₂CH₂CH₂CH₃ |
| B-400 | Br | H | CH₂CH₂CH₂CH₃ |
| B-401 | OH | H | CH₂CH₂CH₂CH₃ |
| B-402 | NH₂ | H | CH₂CH₂CH₂CH₃ |
| B-403 | N(CH₃)₂ | H | CH₂CH₂CH₂CH₃ |
| B-404 | CH₃ | CH₃ | CH₂CH₂CH₂CH₃ |
| B-405 | CH₂CH₃ | CH₃ | CH₂CH₂CH₂CH₃ |
| B-406 | CH₂CH₃ | CH₂CH₃ | CH₂CH₂CH₂CH₃ |
| B-407 | 1-triazolyl | H | CH₂CH₂CH₂CH₃ |
| B-408 | 1-imidazolyl | H | CH₂CH₂CH₂CH₃ |
| B-409 | 2-pyridinyl | H | CH₂CH₂CH₂CH₃ |
| B-410 | 3-pyridinyl | H | CH₂CH₂CH₂CH₃ |
| B-411 | 4-pyridinyl | H | CH₂CH₂CH₂CH₃ |
| B-412 | =CH₂ | | CH₂CH₂CH₂CH₃ |
| B-413 | cyclopropyl | | CH₂CH₂CH₂CH₃ |
| B-414 | cyclobutyl | | CH₂CH₂CH₂CH₃ |
| B-415 | cyclopentyl | | CH₂CH₂CH₂CH₃ |
| B-416 | cyclohexyl | | CH₂CH₂CH₂CH₃ |
| B-417 | CH₃ | H | CH₂CH=CH₂ |
| B-418 | C₂H₅ | H | CH₂CH=CH₂ |
| B-419 | CH₂CH₂CH₃ | H | CH₂CH=CH₂ |
| B-420 | CH(CH₃)₂ | H | CH₂CH=CH₂ |
| B-421 | CH₂CHCH₂ | H | CH₂CH=CH₂ |
| B-422 | CH₂CH₂OH | H | CH₂CH=CH₂ |
| B-423 | CH₂CH₂CH₂OH | H | CH₂CH=CH₂ |
| B-424 | F | H | CH₂CH=CH₂ |
| B-425 | Cl | H | CH₂CH=CH₂ |
| B-426 | Br | H | CH₂CH=CH₂ |
| B-427 | OH | H | CH₂CH=CH₂ |
| B-428 | NH₂ | H | CH₂CH=CH₂ |
| B-429 | N(CH₃)₂ | H | CH₂CH=CH₂ |
| B-430 | CH₃ | CH₃ | CH₂CH=CH₂ |
| B-431 | CH₂CH₃ | CH₃ | CH₂CH=CH₂ |
| B-432 | CH₂CH₃ | CH₂CH₃ | CH₂CH=CH₂ |
| B-433 | 1-triazolyl | H | CH₂CH=CH₂ |
| B-434 | 1-imidazolyl | H | CH₂CH=CH₂ |
| B-435 | 2-pyridinyl | H | CH₂CH=CH₂ |
| B-436 | 3-pyridinyl | H | CH₂CH=CH₂ |
| B-437 | 4-pyridinyl | H | CH₂CH=CH₂ |
| B-438 | =CH₂ | | CH₂CH=CH₂ |
| B-439 | cyclopropyl | | CH₂CH=CH₂ |
| B-440 | cyclobutyl | | CH₂CH=CH₂ |
| B-441 | cyclopentyl | | CH₂CH=CH₂ |
| B-442 | cyclohexyl | | CH₂CH=CH₂ |

According to a further embodiment, the invention relates to compounds of Formula I.B, wherein pis1:

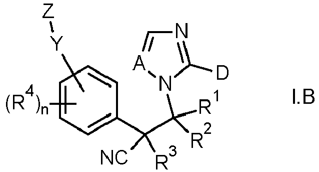

Specific embodiment are compounds I.B1 (D=H, A=N), I.B2 (D=H, A=CH), I.B3 (D=SH, A=N), I.B4 (D=SH, A=CH):

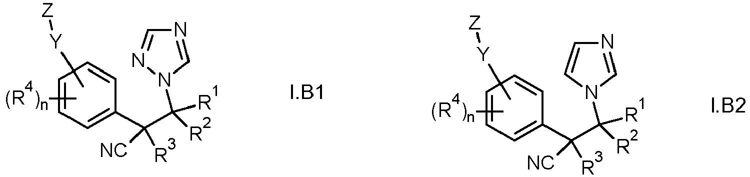

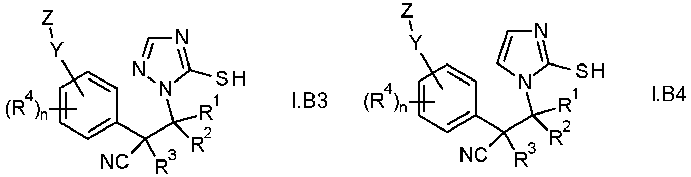

More specific embodiments are compounds I.Ba, I.Bb, I.Bc, I.Bd, I.Be, I.Bf, I.Bg and I.Bh:

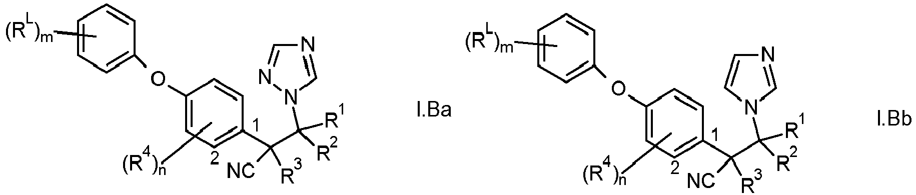

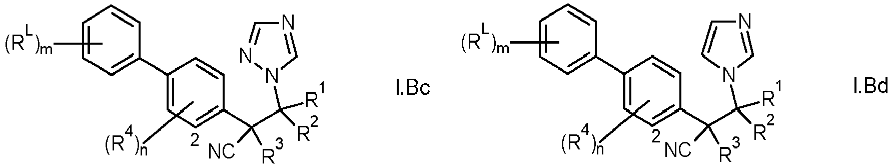

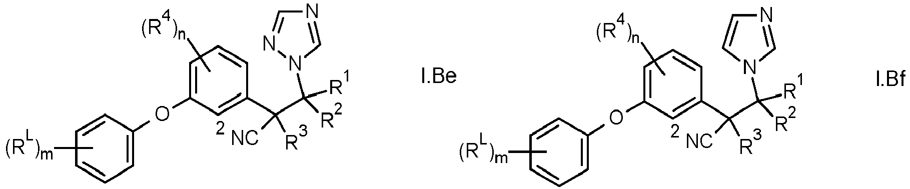

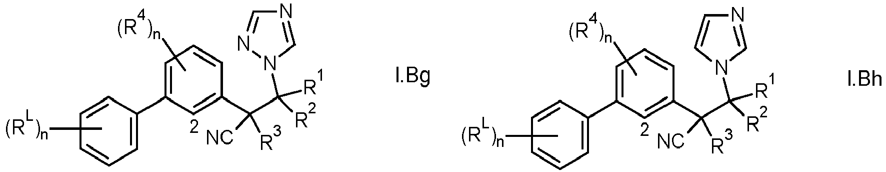

In particular with a view to their use, preference is given to the compounds of the formula I.B, in particular compounds I.B1 (D=H, A=N), I.B2 (D=H, A=CH), I.B3 (D=SH, A=N), I.B4 (D=SH, A=CH), more particularly I.Ba, I.Bb, I.Bc, I.Bd, I.Be, I.Bf, I.Bg and I.Bh.

In particular with a view to their use, preference is given to the compounds of the formula I.B, in particular compounds I.B1 (D=H, A=N), I.B2 (D=H, A=CH), I.B3 (D=SH, A=N), I.B4 (D=SH, A=CH), more particularly I.Ba, I.Bb, I.Bc and I.Bd, that are compiled in the Tables 1c to 140c, Tables 1 d to 140d, Tables 1 e to 140e and Tables 1f to 140f, below. Each of the groups mentioned for a substituent in the tables is furthermore per se, independently of the combination in which it is mentioned, a particularly preferred aspect of the substituent in question.
- Table 1c: Compounds of the formula I.Ba in which the combination of (R⁴)ₙ and (R^{L})ₘ corresponds to line D1-1 of Table D1 and the meaning for the combination of R¹, R² and R³ for each individual compound corresponds in each case to one line of Table B (compounds I.Ba.D1-1.B1 to I.Ba.D1.B442).
- Table 2c: Compounds of the formula I.Ba in which the combination of (R⁴)ₙ and (R^{L})ₘ corresponds to line D1-2 of Table D1 and the meaning for the combination of R¹, R² and R³ for each individual compound corresponds in each case to one line of Table B (compounds I.Ba.D1-2.B1 to I.Ba.D1-2.B442).
- Table 3c: Compounds of the formula I.Ba in which the combination of (R⁴)ₙ and (R^{L})ₘ corresponds to line D1-3 of Table D1 and the meaning for the combination of R¹, R² and R³ for each individual compound corresponds in each case to one line of Table B (compounds I.Ba.D1-3.B1 to I.Ba.D1-3.B442).
- Table 4c: Compounds of the formula I.Ba in which the combination of (R⁴)ₙ and (R^{L})ₘ corresponds to line D1-4 of Table D1 and the meaning for the combination of R¹, R² and R³ for each individual compound corresponds in each case to one line of Table B (compounds I.Ba.D1-4.B1 to I.Ba.D1-4.B442).
- Table 5c: Compounds of the formula I.Ba in which the combination of (R⁴)ₙ and (R^{L})ₘ corresponds to line D1-5 of Table D1 and the meaning for the combination of R¹, R² and R³ for each individual compound corresponds in each case to one line of Table B (compounds I.Ba.D1-5.B1 to I.Ba.D1-5.B442).
- Table 6c: Compounds of the formula I.Ba in which the combination of (R⁴)ₙ and (R^{L})ₘ corresponds to line D1-6 of Table D1 and the meaning for the combination of R¹, R² and R³ for each individual compound corresponds in each case to one line of Table B (compounds I.Ba.D1-6.B1 to I.Ba.D1-6.B442).
- Table 7c: Compounds of the formula I.Ba in which the combination of (R⁴)ₙ and (R^{L})ₘ corresponds to line D1-7 of Table D1 and the meaning for the combination of R¹, R² and R³ for each individual compound corresponds in each case to one line of Table B (compounds I.Ba.D1-7.B1 to I.Ba.D1-7.B442).
- Table 8c: Compounds of the formula I.Ba in which the combination of (R⁴)ₙ and (R^{L})ₘ corresponds to line D1-8 of Table D1 and the meaning for the combination of R¹, R² and R³ for each individual compound corresponds in each case to one line of Table B (compounds I.Ba.D1-8.B1 to I.Ba.D1-8.B442).
- Table 9c: Compounds of the formula I.Ba in which the combination of (R⁴)ₙ and (R^{L})ₘ corresponds to line D1-9 of Table D1 and the meaning for the combination of R¹, R² and R³ for each individual compound corresponds in each case to one line of Table B (compounds I.Ba.D1-9.B1 to I.Ba.D1-9.B442).
- Table 10c: Compounds of the formula I.Ba in which the combination of (R⁴)ₙ and (R^{L})ₘ corresponds to line D1-10 of Table D1 and the meaning for the combination of R¹, R² and R³ for each individual compound corresponds in each case to one line of Table B (compounds I.Ba.D1-10.B1 to I.Ba.D1-10.B442).
- Table 11c: Compounds of the formula I.Ba in which the combination of (R⁴)ₙ and (R^{L})ₘ corresponds to line D1-11 of Table D1 and the meaning for the combination of R¹, R² and R³ for each individual compound corresponds in each case to one line of Table B (compounds I.Ba.D1-11.B1 to I.Ba.D1-11.B442).
- Table 12c: Compounds of the formula I.Ba in which the combination of (R⁴)ₙ and (R^{L})ₘ corresponds to line D1-12 of Table D1 and the meaning for the combination of R¹, R² and R³ for each individual compound corresponds in each case to one line of Table B (compounds I.Ba.D1-12.B1 to I.Ba.D1-12.B442).
- Table 13c: Compounds of the formula I.Ba in which the combination of (R⁴)ₙ and (R^{L})ₘ corresponds to line D1-13 of Table D1 and the meaning for the combination of R¹, R² and R³ for each individual compound corresponds in each case to one line of Table B (compounds I.Ba.D1-13.B1 to I.Ba.D1-13.B442).
- Table 14c: Compounds of the formula I.Ba in which the combination of (R⁴)ₙ and (R^{L})ₘ corresponds to line D1-14 of Table D1 and the meaning for the combination of R¹, R² and R³ for each individual compound corresponds in each case to one line of Table B (compounds I.Ba.D1-14.B1 to I.Ba.D1-14.B442).
- Table 15c: Compounds of the formula I.Ba in which the combination of (R⁴)ₙ and (R^{L})ₘ corresponds to line D1-15 of Table D1 and the meaning for the combination of R¹, R² and R³ for each individual compound corresponds in each case to one line of Table B (compounds I.Ba.D1-15.B1 to I.Ba.D1-15.B442).
- Table 16c: Compounds of the formula I.Ba in which the combination of (R⁴)ₙ and (R^{L})ₘ corresponds to line D1-16 of Table D1 and the meaning for the combination of R¹, R² and R³ for each individual compound corresponds in each case to one line of Table B (compounds I.Ba.D1-16.B1 to I.Ba.D1-16.B442).
- Table 17c: Compounds of the formula I.Ba in which the combination of (R⁴)ₙ and (R^{L})ₘ corresponds to line D1-17 of Table D1 and the meaning for the combination of R¹, R² and R³ for each individual compound corresponds in each case to one line of Table B (compounds I.Ba.D1-17.B1 to I.Ba.D1-17.B442).
- Table 18c: Compounds of the formula I.Ba in which the combination of (R⁴)ₙ and (R^{L})ₘ corresponds to line D1-18 of Table D1 and the meaning for the combination of R¹, R² and R³ for each individual compound corresponds in each case to one line of Table B (compounds I.Ba.D1-18.B1 to I.Ba.D1-18.B442).
- Table 19c: Compounds of the formula I.Ba in which the combination of (R⁴)ₙ and (R^{L})ₘ corresponds to line D1-19 of Table D1 and the meaning for the combination of R¹, R² and R³ for each individual compound corresponds in each case to one line of Table B (compounds I.Ba.D1-19.B1 to I.Ba.D1-19.B442).
- Table 20c: Compounds of the formula I.Ba in which the combination of (R⁴)ₙ and (R^{L})ₘ corresponds to line D1-20 of Table D1 and the meaning for the combination of R¹, R² and R³ for each individual compound corresponds in each case to one line of Table B (compounds I.Ba.D1-20.B1 to I.Ba.D1-20.B442).
- Table 21c: Compounds of the formula I.Ba in which the combination of (R⁴)ₙ and (R^{L})ₘ corresponds to line D1-21 of Table D1 and the meaning for the combination of R¹, R² and R³ for each individual compound corresponds in each case to one line of Table B (compounds I.Ba.D1-21.B1 to I.Ba.D1-21.B442).
- Table 22c: Compounds of the formula I.Ba in which the combination of (R⁴)ₙ and (R^{L})ₘ corresponds to line D1-22 of Table D1 and the meaning for the combination of R¹, R² and R³ for each individual compound corresponds in each case to one line of Table B (compounds I.Ba.D1-22.B1 to I.Ba.D1-22.B442).
- Table 23c: Compounds of the formula I.Ba in which the combination of (R⁴)ₙ and (R^{L})ₘ corresponds to line D1-23 of Table D1 and the meaning for the combination of R¹, R² and R³ for each individual compound corresponds in each case to one line of Table B (compounds I.Ba.D1-23.B1 to I.Ba.D1-23.B442).
- Table 24c: Compounds of the formula I.Ba in which the combination of (R⁴)ₙ and (R^{L})ₘ corresponds to line D1-24 of Table D1 and the meaning for the combination of R¹, R² and R³ for each individual compound corresponds in each case to one line of Table B (compounds I.Ba.D1-24.B1 to I.Ba.D1-24.B442).
- Table 25c: Compounds of the formula I.Ba in which the combination of (R⁴)ₙ and (R^{L})ₘ corresponds to line D1-25 of Table D1 and the meaning for the combination of R¹, R² and R³ for each individual compound corresponds in each case to one line of Table B (compounds I.Ba.D1-25.B1 to I.Ba.D1-25.B442).
- Table 26c: Compounds of the formula I.Ba in which the combination of (R⁴)ₙ and (R^{L})ₘ corresponds to line D1-26 of Table D1 and the meaning for the combination of R¹, R² and R³ for each individual compound corresponds in each case to one line of Table B (compounds I.Ba.D1-26.B1 to I.Ba.D1-26.B442).
- Table 27c: Compounds of the formula I.Ba in which the combination of (R⁴)ₙ and (R^{L})ₘ corresponds to line D1-27 of Table D1 and the meaning for the combination of R¹, R² and R³ for each individual compound corresponds in each case to one line of Table B (compounds I.Ba.D1-27.B1 to I.Ba.D1-27.B442).
- Table 28c: Compounds of the formula I.Ba in which the combination of (R⁴)ₙ and (R^{L})ₘ corresponds to line D1-28 of Table D1 and the meaning for the combination of R¹, R² and R³ for each individual compound corresponds in each case to one line of Table B (compounds I.Ba.D1-28.B1 to I.Ba.D1-28.B442).
- Table 29c: Compounds of the formula I.Ba in which the combination of (R⁴)ₙ and (R^{L})ₘ corresponds to line D1-29 of Table D1 and the meaning for the combination of R¹, R² and R³ for each individual compound corresponds in each case to one line of Table B (compounds I.Ba.D1-29.B1 to I.Ba.D1-29.B442).
- Table 30c: Compounds of the formula I.Ba in which the combination of (R⁴)ₙ and (R^{L})ₘ corresponds to line D1-30 of Table D1 and the meaning for the combination of R¹, R² and R³ for each individual compound corresponds in each case to one line of Table B (compounds I.Ba.D1-30.B1 to I.Ba.D1-30.B442).
- Table 31c: Compounds of the formula I.Ba in which the combination of (R⁴)ₙ and (R^{L})ₘ corresponds to line D1-31 of Table D1 and the meaning for the combination of R¹, R² and R³ for each individual compound corresponds in each case to one line of Table B (compounds I.Ba.D1-31.B1 to I.Ba.D1-31.B442).
- Table 32c: Compounds of the formula I.Ba in which the combination of (R⁴)ₙ and (R^{L})ₘ corresponds to line D1-32 of Table D1 and the meaning for the combination of R¹, R² and R³ for each individual compound corresponds in each case to one line of Table B (compounds I.Ba.D1-32.B1 to I.Ba.D1-32.B442).
- Table 33c: Compounds of the formula I.Ba in which the combination of (R⁴)ₙ and (R^{L})ₘ corresponds to line D1-33 of Table D1 and the meaning for the combination of R¹, R² and R³ for each individual compound corresponds in each case to one line of Table B (compounds I.Ba.D1-33.B1 to I.Ba.D1-33.B442).
- Table 34c: Compounds of the formula I.Ba in which the combination of (R⁴)ₙ and (R^{L})ₘ corresponds to line D1-34 of Table D1 and the meaning for the combination of R¹, R² and R³ for each individual compound corresponds in each case to one line of Table B (compounds I.Ba.D1-34.B1 to I.Ba.D1-34.B442).
- Table 35c: Compounds of the formula I.Ba in which the combination of (R⁴)ₙ and (R^{L})ₘ corresponds to line D1-35 of Table D1 and the meaning for the combination of R¹, R² and R³ for each individual compound corresponds in each case to one line of Table B (compounds I.Ba.D1-35.B1 to I.Ba.D1-35.B442).
- Table 36c: Compounds of the formula I.Ba in which the combination of (R⁴)ₙ and (R^{L})ₘ corresponds to line D1-36 of Table D1 and the meaning for the combination of R¹, R² and R³ for each individual compound corresponds in each case to one line of Table B (compounds I.Ba.D1-36.B1 to I.Ba.D1-36.B442).
- Table 37c: Compounds of the formula I.Ba in which the combination of (R⁴)ₙ and (R^{L})ₘ corresponds to line D1-37 of Table D1 and the meaning for the combination of R¹, R² and R³ for each individual compound corresponds in each case to one line of Table B (compounds I.Ba.D1-37.B1 to I.Ba.D1-37.B442).
- Table 38c: Compounds of the formula I.Ba in which the combination of (R⁴)ₙ and (R^{L})ₘ corresponds to line D1-38 of Table D1 and the meaning for the combination of R¹, R² and R³ for each individual compound corresponds in each case to one line of Table B (compounds I.Ba.D1-38.B1 to I.Ba.D1-38.B442).
- Table 39c: Compounds of the formula I.Ba in which the combination of (R⁴)ₙ and (R^{L})ₘ corresponds to line D1-39 of Table D1 and the meaning for the combination of R¹, R² and R³ for each individual compound corresponds in each case to one line of Table B (compounds I.Ba.D1-39.B1 to I.Ba.D1-39.B442).
- Table 40c: Compounds of the formula I.Ba in which the combination of (R⁴)ₙ and (R^{L})ₘ corresponds to line D1-40 of Table D1 and the meaning for the combination of R¹, R² and R³ for each individual compound corresponds in each case to one line of Table B (compounds I.Ba.D1-40.B1 to I.Ba.D1-40.B442).
- Table 41c: Compounds of the formula I.Ba in which the combination of (R⁴)ₙ and (R^{L})ₘ corresponds to line D1-41 of Table D1 and the meaning for the combination of R¹, R² and R³ for each individual compound corresponds in each case to one line of Table B (compounds I.Ba.D1-41.B1 to I.Ba.D1-41.B442).
- Table 42c: Compounds of the formula I.Ba in which the combination of (R⁴)ₙ and (R^{L})ₘ corresponds to line D1-42 of Table D1 and the meaning for the combination of R¹, R² and R³ for each individual compound corresponds in each case to one line of Table B (compounds I.Ba.D1-42.B1 to I.Ba.D1-42.B442).
- Table 43c: Compounds of the formula I.Ba in which the combination of (R⁴)ₙ and (R^{L})ₘ corresponds to line D1-43 of Table D1 and the meaning for the combination of R¹, R² and R³ for each individual compound corresponds in each case to one line of Table B (compounds I.Ba.D1-43.B1 to I.Ba.D1-43.B442).
- Table 44c: Compounds of the formula I.Ba in which the combination of (R⁴)ₙ and (R^{L})ₘ corresponds to line D1-44 of Table D1 and the meaning for the combination of R¹, R² and R³ for each individual compound corresponds in each case to one line of Table B (compounds I.Ba.D1-44.B1 to I.Ba.D1-44.B442).
- Table 45c: Compounds of the formula I.Ba in which the combination of (R⁴)ₙ and (R^{L})ₘ corresponds to line D1-45 of Table D1 and the meaning for the combination of R¹, R² and R³ for each individual compound corresponds in each case to one line of Table B (compounds I.Ba.D1-45.B1 to I.Ba.D1-45.B442).
- Table 46c: Compounds of the formula I.Ba in which the combination of (R⁴)ₙ and (R^{L})ₘ corresponds to line D1-46 of Table D1 and the meaning for the combination of R¹, R² and R³ for each individual compound corresponds in each case to one line of Table B (compounds I.Ba.D1-46.B1 to I.Ba.D1-46.B442).
- Table 47c: Compounds of the formula I.Ba in which the combination of (R⁴)ₙ and (R^{L})ₘ corresponds to line D1-47 of Table D1 and the meaning for the combination of R¹, R² and R³ for each individual compound corresponds in each case to one line of Table B (compounds I.Ba.D1-47.B1 to I.Ba.D1-47.B442).
- Table 48c: Compounds of the formula I.Ba in which the combination of (R⁴)ₙ and (R^{L})ₘ corresponds to line D1-48 of Table D1 and the meaning for the combination of R¹, R² and R³ for each individual compound corresponds in each case to one line of Table B (compounds I.Ba.D1-48.B1 to I.Ba.D1-48.B442).
- Table 49c: Compounds of the formula I.Ba in which the combination of (R⁴)ₙ and (R^{L})ₘ corresponds to line D1-49 of Table D1 and the meaning for the combination of R¹, R² and R³ for each individual compound corresponds in each case to one line of Table B (compounds I.Ba.D1-49.B1 to I.Ba.D1-49.B442).
- Table 50c: Compounds of the formula I.Ba in which the combination of (R⁴)ₙ and (R^{L})ₘ corresponds to line D1-50 of Table D1 and the meaning for the combination of R¹, R² and R³ for each individual compound corresponds in each case to one line of Table B (compounds I.Ba.D1-50.B1 to I.Ba.D1-50.B442).
- Table 51c: Compounds of the formula I.Ba in which the combination of (R⁴)ₙ and (R^{L})ₘ corresponds to line D1-51 of Table D1 and the meaning for the combination of R¹, R² and R³ for each individual compound corresponds in each case to one line of Table B (compounds I.Ba.D1-51.B1 to I.Ba.D1-51.B442).
- Table 52c: Compounds of the formula I.Ba in which the combination of (R⁴)ₙ and (R^{L})ₘ corresponds to line D1-52 of Table D1 and the meaning for the combination of R¹, R² and R³ for each individual compound corresponds in each case to one line of Table B (compounds I.Ba.D1-52.B1 to I.Ba.D1-52.B442).
- Table 53c: Compounds of the formula I.Ba in which the combination of (R⁴)ₙ and (R^{L})ₘ corresponds to line D1-53 of Table D1 and the meaning for the combination of R¹, R² and R³ for each individual compound corresponds in each case to one line of Table B (compounds I.Ba.D1-53.B1 to I.Ba.D1-53.B442).
- Table 54c: Compounds of the formula I.Ba in which the combination of (R⁴)ₙ and (R^{L})ₘ corresponds to line D1-54 of Table D1 and the meaning for the combination of R¹, R² and R³ for each individual compound corresponds in each case to one line of Table B (compounds I.Ba.D1-54.B1 to I.Ba.D1-54.B442).
- Table 55c: Compounds of the formula I.Ba in which the combination of (R⁴)ₙ and (R^{L})ₘ corresponds to line D1-55 of Table D1 and the meaning for the combination of R¹, R² and R³ for each individual compound corresponds in each case to one line of Table B (compounds I.Ba.D1-55.B1 to I.Ba.D1-55.B442).
- Table 56c: Compounds of the formula I.Ba in which the combination of (R⁴)ₙ and (R^{L})ₘ corresponds to line D1-56 of Table D1 and the meaning for the combination of R¹, R² and R³ for each individual compound corresponds in each case to one line of Table B (compounds I.Ba.D1-56.B1 to I.Ba.D1-56.B442).
- Table 57c: Compounds of the formula I.Ba in which the combination of (R⁴)ₙ and (R^{L})ₘ corresponds to line D1-57 of Table D1 and the meaning for the combination of R¹, R² and R³ for each individual compound corresponds in each case to one line of Table B (compounds I.Ba.D1-57.B1 to I.Ba.D1-57.B442).
- Table 58c: Compounds of the formula I.Ba in which the combination of (R⁴)ₙ and (R^{L})ₘ corresponds to line D1-58 of Table D1 and the meaning for the combination of R¹, R² and R³ for each individual compound corresponds in each case to one line of Table B (compounds I.Ba.D1-58.B1 to I.Ba.D1-58.B442).
- Table 59c: Compounds of the formula I.Ba in which the combination of (R⁴)ₙ and (R^{L})ₘ corresponds to line D1-59 of Table D1 and the meaning for the combination of R¹, R² and R³ for each individual compound corresponds in each case to one line of Table B (compounds I.Ba.D1-59.B1 to I.Ba.D1-59.B442).
- Table 60c: Compounds of the formula I.Ba in which the combination of (R⁴)ₙ and (R^{L})ₘ corresponds to line D1-60 of Table D1 and the meaning for the combination of R¹, R² and R³ for each individual compound corresponds in each case to one line of Table B (compounds I.Ba.D1-60.B1 to I.Ba.D1-60.B442).
- Table 61c: Compounds of the formula I.Ba in which the combination of (R⁴)ₙ and (R^{L})ₘ corresponds to line D1-61 of Table D1 and the meaning for the combination of R¹, R² and R³ for each individual compound corresponds in each case to one line of Table B (compounds I.Ba.D1-61.B1 to I.Ba.D1-61.B442).
- Table 62c: Compounds of the formula I.Ba in which the combination of (R⁴)ₙ and (R^{L})ₘ corresponds to line D1-62 of Table D1 and the meaning for the combination of R¹, R² and R³ for each individual compound corresponds in each case to one line of Table B (compounds I.Ba.D1-62.B1 to I.Ba.D1-62.B442).
- Table 63c: Compounds of the formula I.Ba in which the combination of (R⁴)ₙ and (R^{L})ₘ corresponds to line D1-63 of Table D1 and the meaning for the combination of R¹, R² and R³ for each individual compound corresponds in each case to one line of Table B (compounds I.Ba.D1-63.B1 to I.Ba.D1-63.B442).
- Table 64c: Compounds of the formula I.Ba in which the combination of (R⁴)ₙ and (R^{L})ₘ corresponds to line D1-64 of Table D1 and the meaning for the combination of R¹, R² and R³ for each individual compound corresponds in each case to one line of Table B (compounds I.Ba.D1-64.B1 to I.Ba.D1-64.B442).
- Table 65c: Compounds of the formula I.Ba in which the combination of (R⁴)ₙ and (R^{L})ₘ corresponds to line D1-65 of Table D1 and the meaning for the combination of R¹, R² and R³ for each individual compound corresponds in each case to one line of Table B (compounds I.Ba.D1-65.B1 to I.Ba.D1-65.B442).
- Table 66c: Compounds of the formula I.Ba in which the combination of (R⁴)ₙ and (R^{L})ₘ corresponds to line D1-66 of Table D1 and the meaning for the combination of R¹, R² and R³ for each individual compound corresponds in each case to one line of Table B (compounds I.Ba.D1-66.B1 to I.Ba.D1-66.B442).
- Table 67c: Compounds of the formula I.Ba in which the combination of (R⁴)ₙ and (R^{L})ₘ corresponds to line D1-67 of Table D1 and the meaning for the combination of R¹, R² and R³ for each individual compound corresponds in each case to one line of Table B (compounds I.Ba.D1-67.B1 to I.Ba.D1-67.B442).
- Table 68c: Compounds of the formula I.Ba in which the combination of (R⁴)ₙ and (R^{L})ₘ corresponds to line D1-68 of Table D1 and the meaning for the combination of R¹, R² and R³ for each individual compound corresponds in each case to one line of Table B (compounds I.Ba.D1-68.B1 to I.Ba.D1-68.B442).
- Table 69c: Compounds of the formula I.Ba in which the combination of (R⁴)ₙ and (R^{L})ₘ corresponds to line D1-69 of Table D1 and the meaning for the combination of R¹, R² and R³ for each individual compound corresponds in each case to one line of Table B (compounds I.Ba.D1-69.B1 to I.Ba.D1-69.B442).
- Table 70c: Compounds of the formula I.Ba in which the combination of (R⁴)ₙ and (R^{L})ₘ corresponds to line D1-70 of Table D1 and the meaning for the combination of R¹, R² and R³ for each individual compound corresponds in each case to one line of Table B (compounds I.Ba.D1-70.B1 to I.Ba.D1-70.B442).
- Table 71c: Compounds of the formula I.Ba in which the combination of (R⁴)ₙ and (R^{L})ₘ corresponds to line D2-1 of Table D2 and the meaning for the combination of R¹, R² and R³ for each individual compound corresponds in each case to one line of Table B (compounds I.Ba.D2-1.B1 to I.Ba.D2-1.B442).
- Table 72c: Compounds of the formula I.Ba in which the combination of (R⁴)ₙ and (R^{L})ₘ corresponds to line D2-2 of Table D2 and the meaning for the combination of R¹, R² and R³ for each individual compound corresponds in each case to one line of Table B (compounds I.Ba.D2-2.B1 to I.Ba.D2-2.B442).
- Table 73c: Compounds of the formula I.Ba in which the combination of (R⁴)ₙ and (R^{L})ₘ corresponds to line D2-3 of Table D2 and the meaning for the combination of R¹, R² and R³ for each individual compound corresponds in each case to one line of Table B (compounds I.Ba.D2-3.B1 to I.Ba.D2-3.B442).
- Table 74c: Compounds of the formula I.Ba in which the combination of (R⁴)ₙ and (R^{L})ₘ corresponds to line D2-4 of Table D2 and the meaning for the combination of R¹, R² and R³ for each individual compound corresponds in each case to one line of Table B (compounds I.Ba.D2-4.B1 to I.Ba.D2-4.B442).
- Table 75c: Compounds of the formula I.Ba in which the combination of (R⁴)ₙ and (R^{L})ₘ corresponds to line D2-5 of Table D2 and the meaning for the combination of R¹, R² and R³ for each individual compound corresponds in each case to one line of Table B (compounds I.Ba.D2-5.B1 to I.Ba.D2-5.B442).
- Table 76c: Compounds of the formula I.Ba in which the combination of (R⁴)ₙ and (R^{L})ₘ corresponds to line D2-6 of Table D2 and the meaning for the combination of R¹, R² and R³ for each individual compound corresponds in each case to one line of Table B (compounds I.Ba.D2-6.B1 to I.Ba.D2-6.B442).
- Table 77c: Compounds of the formula I.Ba in which the combination of (R⁴)ₙ and (R^{L})ₘ corresponds to line D2-7 of Table D2 and the meaning for the combination of R¹, R² and R³ for each individual compound corresponds in each case to one line of Table B (compounds I.Ba.D2-7.B1 to I.Ba.D2-7.B442).
- Table 78c: Compounds of the formula I.Ba in which the combination of (R⁴)ₙ and (R^{L})ₘ corresponds to line D2-8 of Table D2 and the meaning for the combination of R¹, R² and R³ for each individual compound corresponds in each case to one line of Table B (compounds I.Ba.D2-8.B1 to I.Ba.D2-8.B442).
- Table 79c: Compounds of the formula I.Ba in which the combination of (R⁴)ₙ and (R^{L})ₘ corresponds to line D2-9 of Table D2 and the meaning for the combination of R¹, R² and R³ for each individual compound corresponds in each case to one line of Table B (compounds I.Ba.D2-9.B1 to I.Ba.D2-9.B442).
- Table 80c: Compounds of the formula I.Ba in which the combination of (R⁴)ₙ and (R^{L})ₘ corresponds to line D2-10 of Table D2 and the meaning for the combination of R¹, R² and R³ for each individual compound corresponds in each case to one line of Table B (compounds I.Ba.D2-10.B1 to I.Ba.D2-10.B442).
- Table 81c: Compounds of the formula I.Ba in which the combination of (R⁴)ₙ and (R^{L})ₘ corresponds to line D2-11 of Table D2 and the meaning for the combination of R¹, R² and R³ for each individual compound corresponds in each case to one line of Table B (compounds I.Ba.D2-11.B1 to I.Ba.D2-11.B442).
- Table 82c: Compounds of the formula I.Ba in which the combination of (R⁴)ₙ and (R^{L})ₘ corresponds to line D2-12 of Table D2 and the meaning for the combination of R¹, R² and R³ for each individual compound corresponds in each case to one line of Table B (compounds I.Ba.D2-12.B1 to I.Ba.D2-12.B442).
- Table 83c: Compounds of the formula I.Ba in which the combination of (R⁴)ₙ and (R^{L})ₘ corresponds to line D2-13 of Table D2 and the meaning for the combination of R¹, R² and R³ for each individual compound corresponds in each case to one line of Table B (compounds I.Ba.D2-13.B1 to I.Ba.D2-13.B442).
- Table 84c: Compounds of the formula I.Ba in which the combination of (R⁴)ₙ and (R^{L})ₘ corresponds to line D2-14 of Table D2 and the meaning for the combination of R¹, R² and R³ for each individual compound corresponds in each case to one line of Table B (compounds I.Ba.D2-14.B1 to I.Ba.D2-14.B442).
- Table 85c: Compounds of the formula I.Ba in which the combination of (R⁴)ₙ and (R^{L})ₘ corresponds to line D2-15 of Table D2 and the meaning for the combination of R¹, R² and R³ for each individual compound corresponds in each case to one line of Table B (compounds I.Ba.D2-15.B1 to I.Ba.D2-15.B442).
- Table 86c: Compounds of the formula I.Ba in which the combination of (R⁴)ₙ and (R^{L})ₘ corresponds to line D2-16 of Table D2 and the meaning for the combination of R¹, R² and R³ for each individual compound corresponds in each case to one line of Table B (compounds I.Ba.D2-16.B1 to I.Ba.D2-16.B442).
- Table 87c: Compounds of the formula I.Ba in which the combination of (R⁴)ₙ and (R^{L})ₘ corresponds to line D2-17 of Table D2 and the meaning for the combination of R¹, R² and R³ for each individual compound corresponds in each case to one line of Table B (compounds I.Ba.D2-17.B1 to I.Ba.D2-17.B442).
- Table 88c: Compounds of the formula I.Ba in which the combination of (R⁴)ₙ and (R^{L})ₘ corresponds to line D2-18 of Table D2 and the meaning for the combination of R¹, R² and R³ for each individual compound corresponds in each case to one line of Table B (compounds I.Ba.D2-18.B1 to I.Ba.D2-18.B442).
- Table 89c: Compounds of the formula I.Ba in which the combination of (R⁴)ₙ and (R^{L})ₘ corresponds to line D2-19 of Table D2 and the meaning for the combination of R¹, R² and R³ for each individual compound corresponds in each case to one line of Table B (compounds I.Ba.D2-19.B1 to I.Ba.D2-19.B442).
- Table 90c: Compounds of the formula I.Ba in which the combination of (R⁴)ₙ and (R^{L})ₘ corresponds to line D2-20 of Table D2 and the meaning for the combination of R¹, R² and R³ for each individual compound corresponds in each case to one line of Table B (compounds I.Ba.D2-20.B1 to I.Ba.D2-20.B442).
- Table 91c: Compounds of the formula I.Ba in which the combination of (R⁴)ₙ and (R^{L})ₘ corresponds to line D2-21 of Table D2 and the meaning for the combination of R¹, R² and R³ for each individual compound corresponds in each case to one line of Table B (compounds I.Ba.D2-21.B1 to I.Ba.D2-21.B442).
- Table 92c: Compounds of the formula I.Ba in which the combination of (R⁴)ₙ and (R^{L})ₘ corresponds to line D2-22 of Table D2 and the meaning for the combination of R¹, R² and R³ for each individual compound corresponds in each case to one line of Table B (compounds I.Ba.D2-22.B1 to I.Ba.D2-22.B442).
- Table 93c: Compounds of the formula I.Ba in which the combination of (R⁴)ₙ and (R^{L})ₘ corresponds to line D2-23 of Table D2 and the meaning for the combination of R¹, R² and R³ for each individual compound corresponds in each case to one line of Table B (compounds I.Ba.D2-23.B1 to I.Ba.D2-23.B442).
- Table 94c: Compounds of the formula I.Ba in which the combination of (R⁴)ₙ and (R^{L})ₘ corresponds to line D2-24 of Table D2 and the meaning for the combination of R¹, R² and R³ for each individual compound corresponds in each case to one line of Table B (compounds I.Ba.D2-24.B1 to I.Ba.D2-24.B442).
- Table 95c: Compounds of the formula I.Ba in which the combination of (R⁴)ₙ and (R^{L})ₘ corresponds to line D2-25 of Table D2 and the meaning for the combination of R¹, R² and R³ for each individual compound corresponds in each case to one line of Table B (compounds I.Ba.D2-25.B1 to I.Ba.D2-25.B442).
- Table 96c: Compounds of the formula I.Ba in which the combination of (R⁴)ₙ and (R^{L})ₘ corresponds to line D2-26 of Table D2 and the meaning for the combination of R¹, R² and R³ for each individual compound corresponds in each case to one line of Table B (compounds I.Ba.D2-26.B1 to I.Ba.D2-26.B442).
- Table 97c: Compounds of the formula I.Ba in which the combination of (R⁴)ₙ and (R^{L})ₘ corresponds to line D2-27 of Table D2 and the meaning for the combination of R¹, R² and R³ for each individual compound corresponds in each case to one line of Table B (compounds I.Ba.D2-27.B1 to I.Ba.D2-27.B442).
- Table 98c: Compounds of the formula I.Ba in which the combination of (R⁴)ₙ and (R^{L})ₘ corresponds to line D2-28 of Table D2 and the meaning for the combination of R¹, R² and R³ for each individual compound corresponds in each case to one line of Table B (compounds I.Ba.D2-28.B1 to I.Ba.D2-28.B442).
- Table 99c: Compounds of the formula I.Ba in which the combination of (R⁴)ₙ and (R^{L})ₘ corresponds to line D2-29 of Table D2 and the meaning for the combination of R¹, R² and R³ for each individual compound corresponds in each case to one line of Table B (compounds I.Ba.D2-29.B1 to I.Ba.D2-29.B442).
- Table 100c: Compounds of the formula I.Ba in which the combination of (R⁴)ₙ and (R^{L})ₘ corresponds to line D2-30 of Table D2 and the meaning for the combination of R¹, R² and R³ for each individual compound corresponds in each case to one line of Table B (compounds I.Ba.D2-30.B1 to I.Ba.D2-30.B442).
- Table 101c: Compounds of the formula I.Ba in which the combination of (R⁴)ₙ and (R^{L})ₘ corresponds to line D2-31 of Table D2 and the meaning for the combination of R¹, R² and R³ for each individual compound corresponds in each case to one line of Table B (compounds I.Ba.D2-31.B1 to I.Ba.D2-31.B442).
- Table 102c: Compounds of the formula I.Ba in which the combination of (R⁴)ₙ and (R^{L})ₘ corresponds to line D2-32 of Table D2 and the meaning for the combination of R¹, R² and R³ for each individual compound corresponds in each case to one line of Table B (compounds I.Ba.D2-32.B1 to I.Ba.D2-32.B442).
- Table 103c: Compounds of the formula I.Ba in which the combination of (R⁴)ₙ and (R^{L})ₘ corresponds to line D2-33 of Table D2 and the meaning for the combination of R¹, R² and R³ for each individual compound corresponds in each case to one line of Table B (compounds I.Ba.D2-33.B1 to I.Ba.D2-33.B442).
- Table 104c: Compounds of the formula I.Ba in which the combination of (R⁴)ₙ and (R^{L})ₘ corresponds to line D2-34 of Table D2 and the meaning for the combination of R¹, R² and R³ for each individual compound corresponds in each case to one line of Table B (compounds I.Ba.D2-34.B1 to I.Ba.D2-34.B442).
- Table 105c: Compounds of the formula I.Ba in which the combination of (R⁴)ₙ and (R^{L})ₘ corresponds to line D2-35 of Table D2 and the meaning for the combination of R¹, R² and R³ for each individual compound corresponds in each case to one line of Table B (compounds I.Ba.D2-35.B1 to I.Ba.D2-35.B442).
- Table 106c: Compounds of the formula I.Ba in which the combination of (R⁴)ₙ and (R^{L})ₘ corresponds to line D2-36 of Table D2 and the meaning for the combination of R¹, R² and R³ for each individual compound corresponds in each case to one line of Table B (compounds I.Ba.D2-36.B1 to I.Ba.D2-36.B442).
- Table 107c: Compounds of the formula I.Ba in which the combination of (R⁴)ₙ and (R^{L})ₘ corresponds to line D2-37 of Table D2 and the meaning for the combination of R¹, R² and R³ for each individual compound corresponds in each case to one line of Table B (compounds I.Ba.D2-37.B1 to I.Ba.D2-37.B442).
- Table 108c: Compounds of the formula I.Ba in which the combination of (R⁴)ₙ and (R^{L})ₘ corresponds to line D2-38 of Table D2 and the meaning for the combination of R¹, R² and R³ for each individual compound corresponds in each case to one line of Table B (compounds I.Ba.D2-38.B1 to I.Ba.D2-38.B442).
- Table 109c: Compounds of the formula I.Ba in which the combination of (R⁴)ₙ and (R^{L})ₘ corresponds to line D2-39 of Table D2 and the meaning for the combination of R¹, R² and R³ for each individual compound corresponds in each case to one line of Table B (compounds I.Ba.D2-39.B1 to I.Ba.D2-39.B442).
- Table 110c: Compounds of the formula I.Ba in which the combination of (R⁴)ₙ and (R^{L})ₘ corresponds to line D2-40 of Table D2 and the meaning for the combination of R¹, R² and R³ for each individual compound corresponds in each case to one line of Table B (compounds I.Ba.D2-40.B1 to I.Ba.D2-40.B442).
- Table 111c: Compounds of the formula I.Ba in which the combination of (R⁴)ₙ and (R^{L})ₘ corresponds to line D2-41 of Table D2 and the meaning for the combination of R¹, R² and R³ for each individual compound corresponds in each case to one line of Table B (compounds I.Ba.D2-41.B1 to I.Ba.D2-41.B442).
- Table 112c: Compounds of the formula I.Ba in which the combination of (R⁴)ₙ and (R^{L})ₘ corresponds to line D2-42 of Table D2 and the meaning for the combination of R¹, R² and R³ for each individual compound corresponds in each case to one line of Table B (compounds I.Ba.D2-42.B1 to I.Ba.D2-42.B442).
- Table 113c: Compounds of the formula I.Ba in which the combination of (R⁴)ₙ and (R^{L})ₘ corresponds to line D2-43 of Table D2 and the meaning for the combination of R¹, R² and R³ for each individual compound corresponds in each case to one line of Table B (compounds I.Ba.D2-43.B1 to I.Ba.D2-43.B442).
- Table 114c: Compounds of the formula I.Ba in which the combination of (R⁴)ₙ and (R^{L})ₘ corresponds to line D2-44 of Table D2 and the meaning for the combination of R¹, R² and R³ for each individual compound corresponds in each case to one line of Table B (compounds I.Ba.D2-44.B1 to I.Ba.D2-44.B442).
- Table 115c: Compounds of the formula I.Ba in which the combination of (R⁴)ₙ and (R^{L})ₘ corresponds to line D2-45 of Table D2 and the meaning for the combination of R¹, R² and R³ for each individual compound corresponds in each case to one line of Table B (compounds I.Ba.D2-45.B1 to I.Ba.D2-45.B442).
- Table 116c: Compounds of the formula I.Ba in which the combination of (R⁴)ₙ and (R^{L})ₘ corresponds to line D2-46 of Table D2 and the meaning for the combination of R¹, R² and R³ for each individual compound corresponds in each case to one line of Table B (compounds I.Ba.D2-46.B1 to I.Ba.D2-46.B442).
- Table 117c: Compounds of the formula I.Ba in which the combination of (R⁴)ₙ and (R^{L})ₘ corresponds to line D2-47 of Table D2 and the meaning for the combination of R¹, R² and R³ for each individual compound corresponds in each case to one line of Table B (compounds I.Ba.D2-47.B1 to I.Ba.D2-47.B442).
- Table 118c: Compounds of the formula I.Ba in which the combination of (R⁴)ₙ and (R^{L})ₘ corresponds to line D2-48 of Table D2 and the meaning for the combination of R¹, R² and R³ for each individual compound corresponds in each case to one line of Table B (compounds I.Ba.D2-48.B1 to I.Ba.D2-48.B442).
- Table 119c: Compounds of the formula I.Ba in which the combination of (R⁴)ₙ and (R^{L})ₘ corresponds to line D2-49 of Table D2 and the meaning for the combination of R¹, R² and R³ for each individual compound corresponds in each case to one line of Table B (compounds I.Ba.D2-49.B1 to I.Ba.D2-49.B442).
- Table 120c: Compounds of the formula I.Ba in which the combination of (R⁴)ₙ and (R^{L})ₘ corresponds to line D2-50 of Table D2 and the meaning for the combination of R¹, R² and R³ for each individual compound corresponds in each case to one line of Table B (compounds I.Ba.D2-50.B1 to I.Ba.D2-50.B442).
- Table 121c: Compounds of the formula I.Ba in which the combination of (R⁴)ₙ and (R^{L})ₘ corresponds to line D2-51 of Table D2 and the meaning for the combination of R¹, R² and R³ for each individual compound corresponds in each case to one line of Table B (compounds I.Ba.D2-51.B1 to I.Ba.D2-51.B442).
- Table 122c: Compounds of the formula I.Ba in which the combination of (R⁴)ₙ and (R^{L})ₘ corresponds to line D2-52 of Table D2 and the meaning for the combination of R¹, R² and R³ for each individual compound corresponds in each case to one line of Table B (compounds I.Ba.D2-52.B1 to I.Ba.D2-52.B442).
- Table 123c: Compounds of the formula I.Ba in which the combination of (R⁴)ₙ and (R^{L})ₘ corresponds to line D2-53 of Table D2 and the meaning for the combination of R¹, R² and R³ for each individual compound corresponds in each case to one line of Table B (compounds I.Ba.D2-53.B1 to I.Ba.D2-53.B442).
- Table 124c: Compounds of the formula I.Ba in which the combination of (R⁴)ₙ and (R^{L})ₘ corresponds to line D2-54 of Table D2 and the meaning for the combination of R¹, R² and R³ for each individual compound corresponds in each case to one line of Table B (compounds I.Ba.D2-54.B1 to I.Ba.D2-54.B442).
- Table 125c: Compounds of the formula I.Ba in which the combination of (R⁴)ₙ and (R^{L})ₘ corresponds to line D2-55 of Table D2 and the meaning for the combination of R¹, R² and R³ for each individual compound corresponds in each case to one line of Table B (compounds I.Ba.D2-55.B1 to I.Ba.D2-55.B442).
- Table 126c: Compounds of the formula I.Ba in which the combination of (R⁴)ₙ and (R^{L})ₘ corresponds to line D2-56 of Table D2 and the meaning for the combination of R¹, R² and R³ for each individual compound corresponds in each case to one line of Table B (compounds I.Ba.D2-56.B1 to I.Ba.D2-56.B442).
- Table 127c: Compounds of the formula I.Ba in which the combination of (R⁴)ₙ and (R^{L})ₘ corresponds to line D2-57 of Table D2 and the meaning for the combination of R¹, R² and R³ for each individual compound corresponds in each case to one line of Table B (compounds I.Ba.D2-57.B1 to I.Ba.D2-57.B442).
- Table 128c: Compounds of the formula I.Ba in which the combination of (R⁴)ₙ and (R^{L})ₘ corresponds to line D2-58 of Table D2 and the meaning for the combination of R¹, R² and R³ for each individual compound corresponds in each case to one line of Table B (compounds I.Ba.D2-58.B1 to I.Ba.D2-58.B442).
- Table 129c: Compounds of the formula I.Ba in which the combination of (R⁴)ₙ and (R^{L})ₘ corresponds to line D2-59 of Table D2 and the meaning for the combination of R¹, R² and R³ for each individual compound corresponds in each case to one line of Table B (compounds I.Ba.D2-59.B1 to I.Ba.D2-59.B442).
- Table 130c: Compounds of the formula I.Ba in which the combination of (R⁴)ₙ and (R^{L})ₘ corresponds to line D2-60 of Table D2 and the meaning for the combination of R¹, R² and R³ for each individual compound corresponds in each case to one line of Table B (compounds I.Ba.D2-60.B1 to I.Ba.D2-60.B442).
- Table 131c: Compounds of the formula I.Ba in which the combination of (R⁴)ₙ and (R^{L})ₘ corresponds to line D2-61 of Table D2 and the meaning for the combination of R¹, R² and R³ for each individual compound corresponds in each case to one line of Table B (compounds I.Ba.D2-61.B1 to I.Ba.D2-61.B442).
- Table 132c: Compounds of the formula I.Ba in which the combination of (R⁴)ₙ and (R^{L})ₘ corresponds to line D2-62 of Table D2 and the meaning for the combination of R¹, R² and R³ for each individual compound corresponds in each case to one line of Table B (compounds I.Ba.D2-62.B1 to I.Ba.D2-62.B442).
- Table 133c: Compounds of the formula I.Ba in which the combination of (R⁴)ₙ and (R^{L})ₘ corresponds to line D2-63 of Table D2 and the meaning for the combination of R¹, R² and R³ for each individual compound corresponds in each case to one line of Table B (compounds I.Ba.D2-63.B1 to I.Ba.D2-63.B442).
- Table 134c: Compounds of the formula I.Ba in which the combination of (R⁴)ₙ and (R^{L})ₘ corresponds to line D2-64 of Table D2 and the meaning for the combination of R¹, R² and R³ for each individual compound corresponds in each case to one line of Table B (compounds I.Ba.D2-64.B1 to I.Ba.D2-64.B442).
- Table 135c: Compounds of the formula I.Ba in which the combination of (R⁴)ₙ and (R^{L})ₘ corresponds to line D2-65 of Table D2 and the meaning for the combination of R¹, R² and R³ for each individual compound corresponds in each case to one line of Table B (compounds I.Ba.D2-65.B1 to I.Ba.D2-65.B442).
- Table 136c: Compounds of the formula I.Ba in which the combination of (R⁴)ₙ and (R^{L})ₘ corresponds to line D2-66 of Table D2 and the meaning for the combination of R¹, R² and R³ for each individual compound corresponds in each case to one line of Table B (compounds I.Ba.D2-66.B1 to I.Ba.D2-66.B442).
- Table 137c: Compounds of the formula I.Ba in which the combination of (R⁴)ₙ and (R^{L})ₘ corresponds to line D2-67 of Table D2 and the meaning for the combination of R¹, R² and R³ for each individual compound corresponds in each case to one line of Table B (compounds I.Ba.D2-67.B1 to I.Ba.D2-67.B442).
- Table 138c: Compounds of the formula I.Ba in which the combination of (R⁴)ₙ and (R^{L})ₘ corresponds to line D2-68 of Table D2 and the meaning for the combination of R¹, R² and R³ for each individual compound corresponds in each case to one line of Table B (compounds I.Ba.D2-68.B1 to I.Ba.D2-68.B442).
- Table 139c: Compounds of the formula I.Ba in which the combination of (R⁴)ₙ and (R^{L})ₘ corresponds to line D2-69 of Table D2 and the meaning for the combination of R¹, R² and R³ for each individual compound corresponds in each case to one line of Table B (compounds I.Ba.D2-69.B1 to I.Ba.D2-69.B442).
- Table 140c: Compounds of the formula I.Ba in which the combination of (R⁴)ₙ and (R^{L})ₘ corresponds to line D2-70 of Table D2 and the meaning for the combination of R¹, R² and R³ for each individual compound corresponds in each case to one line of Table B (compounds I.Ba.D2-70.B1 to I.Ba.D2-70.B442).
- Table 1d: Compounds of the formula I.Bb in which the combination of (R⁴)ₙ and (R^{L})ₘ corresponds to line D1-1 of Table D1 and the meaning for the combination of R¹, R² and R³ for each individual compound corresponds in each case to one line of Table B (compounds I.Bb.D1-1.B1 to I.Bb.D1.B442).
- Table 2d: Compounds of the formula I.Bb in which the combination of (R⁴)ₙ and (R^{L})ₘ corresponds to line D1-2 of Table D1 and the meaning for the combination of R¹, R² and R³ for each individual compound corresponds in each case to one line of Table B (compounds I.Bb.D1-2.B1 to I.Bb.D1-2.B442).
- Table 3d: Compounds of the formula I.Bb in which the combination of (R⁴)ₙ and (R^{L})ₘ corresponds to line D1-3 of Table D1 and the meaning for the combination of R¹, R² and R³ for each individual compound corresponds in each case to one line of Table B (compounds I.Bb.D1-3.B1 to I.Bb.D1-3.B442).
- Table 4d: Compounds of the formula I.Bb in which the combination of (R⁴)ₙ and (R^{L})ₘ corresponds to line D1-4 of Table D1 and the meaning for the combination of R¹, R² and R³ for each individual compound corresponds in each case to one line of Table B (compounds I.Bb.D1-4.B1 to I.Bb.D1-4.B442).
- Table 5d: Compounds of the formula I.Bb in which the combination of (R⁴)ₙ and (R^{L})ₘ corresponds to line D1-5 of Table D1 and the meaning for the combination of R¹, R² and R³ for each individual compound corresponds in each case to one line of Table B (compounds I.Bb.D1-5.B1 to I.Bb.D1-5.B442).
- Table 6d: Compounds of the formula I.Bb in which the combination of (R⁴)ₙ and (R^{L})ₘ corresponds to line D1-6 of Table D1 and the meaning for the combination of R¹, R² and R³ for each individual compound corresponds in each case to one line of Table B (compounds I.Bb.D1-6.B1 to I.Bb.D1-6.B442).
- Table 7d: Compounds of the formula I.Bb in which the combination of (R⁴)ₙ and (R^{L})ₘ corresponds to line D1-7 of Table D1 and the meaning for the combination of R¹, R² and R³ for each individual compound corresponds in each case to one line of Table B (compounds I.Bb.D1-7.B1 to I.Bb.D1-7.B442).
- Table 8d: Compounds of the formula I.Bb in which the combination of (R⁴)ₙ and (R^{L})ₘ corresponds to line D1-8 of Table D1 and the meaning for the combination of R¹, R² and R³ for each individual compound corresponds in each case to one line of Table B (compounds I.Bb.D1-8.B1 to I.Bb.D1-8.B442).
- Table 9d: Compounds of the formula I.Bb in which the combination of (R⁴)ₙ and (R^{L})ₘ corresponds to line D1-9 of Table D1 and the meaning for the combination of R¹, R² and R³ for each individual compound corresponds in each case to one line of Table B (compounds I.Bb.D1-9.B1 to I.Bb.D1-9.B442).
- Table 10d: Compounds of the formula I.Bb in which the combination of (R⁴)ₙ and (R^{L})ₘ corresponds to line D1-10 of Table D1 and the meaning for the combination of R¹, R² and R³ for each individual compound corresponds in each case to one line of Table B (compounds I.Bb.D1-10.B1 to I.Bb.D1-10.B442).
- Table 11d: Compounds of the formula I.Bb in which the combination of (R⁴)ₙ and (R^{L})ₘ corresponds to line D1-11 of Table D1 and the meaning for the combination of R¹, R² and R³ for each individual compound corresponds in each case to one line of Table B (compounds I.Bb.D1-11.B1 to I.Bb.D1-11.B442).
- Table 12d: Compounds of the formula I.Bb in which the combination of (R⁴)ₙ and (R^{L})ₘ corresponds to line D1-12 of Table D1 and the meaning for the combination of R¹, R² and R³ for each individual compound corresponds in each case to one line of Table B (compounds I.Bb.D1-12.B1 to I.Bb.D1-12.B442).
- Table 13d: Compounds of the formula I.Bb in which the combination of (R⁴)ₙ and (R^{L})ₘ corresponds to line D1-13 of Table D1 and the meaning for the combination of R¹, R² and R³ for each individual compound corresponds in each case to one line of Table B (compounds I.Bb.D1-13.B1 to I.Bb.D1-13.B442).
- Table 14d: Compounds of the formula I.Bb in which the combination of (R⁴)ₙ and (R^{L})ₘ corresponds to line D1-14 of Table D1 and the meaning for the combination of R¹, R² and R³ for each individual compound corresponds in each case to one line of Table B (compounds I.Bb.D1-14.B1 to I.Bb.D1-14.B442).
- Table 15d: Compounds of the formula I.Bb in which the combination of (R⁴)ₙ and (R^{L})ₘ corresponds to line D1-15 of Table D1 and the meaning for the combination of R¹, R² and R³ for each individual compound corresponds in each case to one line of Table B (compounds I.Bb.D1-15.B1 to I.Bb.D1-15.B442).
- Table 16d: Compounds of the formula I.Bb in which the combination of (R⁴)ₙ and (R^{L})ₘ corresponds to line D1-16 of Table D1 and the meaning for the combination of R¹, R² and R³ for each individual compound corresponds in each case to one line of Table B (compounds I.Bb.D1-16.B1 to I.Bb.D1-16.B442).
- Table 17d: Compounds of the formula I.Bb in which the combination of (R⁴)ₙ and (R^{L})ₘ corresponds to line D1-17 of Table D1 and the meaning for the combination of R¹, R² and R³ for each individual compound corresponds in each case to one line of Table B (compounds I.Bb.D1-17.B1 to I.Bb.D1-17.B442).
- Table 18d: Compounds of the formula I.Bb in which the combination of (R⁴)ₙ and (R^{L})ₘ corresponds to line D1-18 of Table D1 and the meaning for the combination of R¹, R² and R³ for each individual compound corresponds in each case to one line of Table B (compounds I.Bb.D1-18.B1 to I.Bb.D1-18.B442).
- Table 19d: Compounds of the formula I.Bb in which the combination of (R⁴)ₙ and (R^{L})ₘ corresponds to line D1-19 of Table D1 and the meaning for the combination of R¹, R² and R³ for each individual compound corresponds in each case to one line of Table B (compounds I.Bb.D1-19.B1 to I.Bb.D1-19.B442).
- Table 20d: Compounds of the formula I.Bb in which the combination of (R⁴)ₙ and (R^{L})ₘ corresponds to line D1-20 of Table D1 and the meaning for the combination of R¹, R² and R³ for each individual compound corresponds in each case to one line of Table B (compounds I.Bb.D1-20.B1 to I.Bb.D1-20.B442).
- Table 21d: Compounds of the formula I.Bb in which the combination of (R⁴)ₙ and (R^{L})ₘ corresponds to line D1-21 of Table D1 and the meaning for the combination of R¹, R² and R³ for each individual compound corresponds in each case to one line of Table B (compounds I.Bb.D1-21.B1 to I.Bb.D1-21.B442).
- Table 22d: Compounds of the formula I.Bb in which the combination of (R⁴)ₙ and (R^{L})ₘ corresponds to line D1-22 of Table D1 and the meaning for the combination of R¹, R² and R³ for each individual compound corresponds in each case to one line of Table B (compounds I.Bb.D1-22.B1 to I.Bb.D1-22.B442).
- Table 23d: Compounds of the formula I.Bb in which the combination of (R⁴)ₙ and (R^{L})ₘ corresponds to line D1-23 of Table D1 and the meaning for the combination of R¹, R² and R³ for each individual compound corresponds in each case to one line of Table B (compounds I.Bb.D1-23.B1 to I.Bb.D1-23.B442).
- Table 24d: Compounds of the formula I.Bb in which the combination of (R⁴)ₙ and (R^{L})ₘ corresponds to line D1-24 of Table D1 and the meaning for the combination of R¹, R² and R³ for each individual compound corresponds in each case to one line of Table B (compounds I.Bb.D1-24.B1 to I.Bb.D1-24.B442).
- Table 25d: Compounds of the formula I.Bb in which the combination of (R⁴)ₙ and (R^{L})ₘ corresponds to line D1-25 of Table D1 and the meaning for the combination of R¹, R² and R³ for each individual compound corresponds in each case to one line of Table B (compounds I.Bb.D1-25.B1 to I.Bb.D1-25.B442).
- Table 26d: Compounds of the formula I.Bb in which the combination of (R⁴)ₙ and (R^{L})ₘ corresponds to line D1-26 of Table D1 and the meaning for the combination of R¹, R² and R³ for each individual compound corresponds in each case to one line of Table B (compounds I.Bb.D1-26.B1 to I.Bb.D1-26.B442).
- Table 27d: Compounds of the formula I.Bb in which the combination of (R⁴)ₙ and (R^{L})ₘ corresponds to line D1-27 of Table D1 and the meaning for the combination of R¹, R² and R³ for each individual compound corresponds in each case to one line of Table B (compounds I.Bb.D1-27.B1 to I.Bb.D1-27.B442).
- Table 28d: Compounds of the formula I.Bb in which the combination of (R⁴)ₙ and (R^{L})ₘ corresponds to line D1-28 of Table D1 and the meaning for the combination of R¹, R² and R³ for each individual compound corresponds in each case to one line of Table B (compounds I.Bb.D1-28.B1 to I.Bb.D1-28.B442).
- Table 29d: Compounds of the formula I.Bb in which the combination of (R⁴)ₙ and (R^{L})ₘ corresponds to line D1-29 of Table D1 and the meaning for the combination of R¹, R² and R³ for each individual compound corresponds in each case to one line of Table B (compounds I.Bb.D1-29.B1 to I.Bb.D1-29.B442).
- Table 30d: Compounds of the formula I.Bb in which the combination of (R⁴)ₙ and (R^{L})ₘ corresponds to line D1-30 of Table D1 and the meaning for the combination of R¹, R² and R³ for each individual compound corresponds in each case to one line of Table B (compounds I.Bb.D1-30.B1 to I.Bb.D1-30.B442).
- Table 31d: Compounds of the formula I.Bb in which the combination of (R⁴)ₙ and (R^{L})ₘ corresponds to line D1-31 of Table D1 and the meaning for the combination of R¹, R² and R³ for each individual compound corresponds in each case to one line of Table B (compounds I.Bb.D1-31.B1 to I.Bb.D1-31.B442).
- Table 32d: Compounds of the formula I.Bb in which the combination of (R⁴)ₙ and (R^{L})ₘ corresponds to line D1-32 of Table D1 and the meaning for the combination of R¹, R² and R³ for each individual compound corresponds in each case to one line of Table B (compounds I.Bb.D1-32.B1 to I.Bb.D1-32.B442).
- Table 33d: Compounds of the formula I.Bb in which the combination of (R⁴)ₙ and (R^{L})ₘ corresponds to line D1-33 of Table D1 and the meaning for the combination of R¹, R² and R³ for each individual compound corresponds in each case to one line of Table B (compounds I.Bb.D1-33.B1 to I.Bb.D1-33.B442).
- Table 34d: Compounds of the formula I.Bb in which the combination of (R⁴)ₙ and (R^{L})ₘ corresponds to line D1-34 of Table D1 and the meaning for the combination of R¹, R² and R³ for each individual compound corresponds in each case to one line of Table B (compounds I.Bb.D1-34.B1 to I.Bb.D1-34.B442).
- Table 35d: Compounds of the formula I.Bb in which the combination of (R⁴)ₙ and (R^{L})ₘ corresponds to line D1-35 of Table D1 and the meaning for the combination of R¹, R² and R³ for each individual compound corresponds in each case to one line of Table B (compounds I.Bb.D1-35.B1 to I.Bb.D1-35.B442).
- Table 36d: Compounds of the formula I.Bb in which the combination of (R⁴)ₙ and (R^{L})ₘ corresponds to line D1-36 of Table D1 and the meaning for the combination of R¹, R² and R³ for each individual compound corresponds in each case to one line of Table B (compounds I.Bb.D1-36.B1 to I.Bb.D1-36.B442).
- Table 37d: Compounds of the formula I.Bb in which the combination of (R⁴)ₙ and (R^{L})ₘ corresponds to line D1-37 of Table D1 and the meaning for the combination of R¹, R² and R³ for each individual compound corresponds in each case to one line of Table B (compounds I.Bb.D1-37.B1 to I.Bb.D1-37.B442).
- Table 38d: Compounds of the formula I.Bb in which the combination of (R⁴)ₙ and (R^{L})ₘ corresponds to line D1-38 of Table D1 and the meaning for the combination of R¹, R² and R³ for each individual compound corresponds in each case to one line of Table B (compounds I.Bb.D1-38.B1 to I.Bb.D1-38.B442).
- Table 39d: Compounds of the formula I.Bb in which the combination of (R⁴)ₙ and (R^{L})ₘ corresponds to line D1-39 of Table D1 and the meaning for the combination of R¹, R² and R³ for each individual compound corresponds in each case to one line of Table B (compounds I.Bb.D1-39.B1 to I.Bb.D1-39.B442).
- Table 40d: Compounds of the formula I.Bb in which the combination of (R⁴)ₙ and (R^{L})ₘ corresponds to line D1-40 of Table D1 and the meaning for the combination of R¹, R² and R³ for each individual compound corresponds in each case to one line of Table B (compounds I.Bb.D1-40.B1 to I.Bb.D1-40.B442).
- Table 41d: Compounds of the formula I.Bb in which the combination of (R⁴)ₙ and (R^{L})ₘ corresponds to line D1-41 of Table D1 and the meaning for the combination of R¹, R² and R³ for each individual compound corresponds in each case to one line of Table B (compounds I.Bb.D1-41.B1 to I.Bb.D1-41.B442).
- Table 42d: Compounds of the formula I.Bb in which the combination of (R⁴)ₙ and (R^{L})ₘ corresponds to line D1-42 of Table D1 and the meaning for the combination of R¹, R² and R³ for each individual compound corresponds in each case to one line of Table B (compounds I.Bb.D1-42.B1 to I.Bb.D1-42.B442).
- Table 43d: Compounds of the formula I.Bb in which the combination of (R⁴)ₙ and (R^{L})ₘ corresponds to line D1-43 of Table D1 and the meaning for the combination of R¹, R² and R³ for each individual compound corresponds in each case to one line of Table B (compounds I.Bb.D1-43.B1 to I.Bb.D1-43.B442).
- Table 44d: Compounds of the formula I.Bb in which the combination of (R⁴)ₙ and (R^{L})ₘ corresponds to line D1-44 of Table D1 and the meaning for the combination of R¹, R² and R³ for each individual compound corresponds in each case to one line of Table B (compounds I.Bb.D1-44.B1 to I.Bb.D1-44.B442).
- Table 45d: Compounds of the formula I.Bb in which the combination of (R⁴)ₙ and (R^{L})ₘ corresponds to line D1-45 of Table D1 and the meaning for the combination of R¹, R² and R³ for each individual compound corresponds in each case to one line of Table B (compounds I.Bb.D1-45.B1 to I.Bb.D1-45.B442).
- Table 46d: Compounds of the formula I.Bb in which the combination of (R⁴)ₙ and (R^{L})ₘ corresponds to line D1-46 of Table D1 and the meaning for the combination of R¹, R² and R³ for each individual compound corresponds in each case to one line of Table B (compounds I.Bb.D1-46.B1 to I.Bb.D1-46.B442).
- Table 47d: Compounds of the formula I.Bb in which the combination of (R⁴)ₙ and (R^{L})ₘ corresponds to line D1-47 of Table D1 and the meaning for the combination of R¹, R² and R³ for each individual compound corresponds in each case to one line of Table B (compounds I.Bb.D1-47.B1 to I.Bb.D1-47.B442).
- Table 48d: Compounds of the formula I.Bb in which the combination of (R⁴)ₙ and (R^{L})ₘ corresponds to line D1-48 of Table D1 and the meaning for the combination of R¹, R² and R³ for each individual compound corresponds in each case to one line of Table B (compounds I.Bb.D1-48.B1 to I.Bb.D1-48.B442).
- Table 49d: Compounds of the formula I.Bb in which the combination of (R⁴)ₙ and (R^{L})ₘ corresponds to line D1-49 of Table D1 and the meaning for the combination of R¹, R² and R³ for each individual compound corresponds in each case to one line of Table B (compounds I.Bb.D1-49.B1 to I.Bb.D1-49.B442).
- Table 50d: Compounds of the formula I.Bb in which the combination of (R⁴)ₙ and (R^{L})ₘ corresponds to line D1-50 of Table D1 and the meaning for the combination of R¹, R² and R³ for each individual compound corresponds in each case to one line of Table B (compounds I.Bb.D1-50.B1 to I.Bb.D1-50.B442).
- Table 51d: Compounds of the formula I.Bb in which the combination of (R⁴)ₙ and (R^{L})ₘ corresponds to line D1-51 of Table D1 and the meaning for the combination of R¹, R² and R³ for each individual compound corresponds in each case to one line of Table B (compounds I.Bb.D1-51.B1 to I.Bb.D1-51.B442).
- Table 52d: Compounds of the formula I.Bb in which the combination of (R⁴)ₙ and (R^{L})ₘ corresponds to line D1-52 of Table D1 and the meaning for the combination of R¹, R² and R³ for each individual compound corresponds in each case to one line of Table B (compounds I.Bb.D1-52.B1 to I.Bb.D1-52.B442).
- Table 53d: Compounds of the formula I.Bb in which the combination of (R⁴)ₙ and (R^{L})ₘ corresponds to line D1-53 of Table D1 and the meaning for the combination of R¹, R² and R³ for each individual compound corresponds in each case to one line of Table B (compounds I.Bb.D1-53.B1 to I.Bb.D1-53.B442).
- Table 54d: Compounds of the formula I.Bb in which the combination of (R⁴)ₙ and (R^{L})ₘ corresponds to line D1-54 of Table D1 and the meaning for the combination of R¹, R² and R³ for each individual compound corresponds in each case to one line of Table B (compounds I.Bb.D1-54.B1 to I.Bb.D1-54.B442).
- Table 55d: Compounds of the formula I.Bb in which the combination of (R⁴)ₙ and (R^{L})ₘ corresponds to line D1-55 of Table D1 and the meaning for the combination of R¹, R² and R³ for each individual compound corresponds in each case to one line of Table B (compounds I.Bb.D1-55.B1 to I.Bb.D1-55.B442).
- Table 56d: Compounds of the formula I.Bb in which the combination of (R⁴)ₙ and (R^{L})ₘ corresponds to line D1-56 of Table D1 and the meaning for the combination of R¹, R² and R³ for each individual compound corresponds in each case to one line of Table B (compounds I.Bb.D1-56.B1 to I.Bb.D1-56.B442).
- Table 57d: Compounds of the formula I.Bb in which the combination of (R⁴)ₙ and (R^{L})ₘ corresponds to line D1-57 of Table D1 and the meaning for the combination of R¹, R² and R³ for each individual compound corresponds in each case to one line of Table B (compounds I.Bb.D1-57.B1 to I.Bb.D1-57.B442).
- Table 58d: Compounds of the formula I.Bb in which the combination of (R⁴)ₙ and (R^{L})ₘ corresponds to line D1-58 of Table D1 and the meaning for the combination of R¹, R² and R³ for each individual compound corresponds in each case to one line of Table B (compounds I.Bb.D1-58.B1 to I.Bb.D1-58.B442).
- Table 59d: Compounds of the formula I.Bb in which the combination of (R⁴)ₙ and (R^{L})ₘ corresponds to line D1-59 of Table D1 and the meaning for the combination of R¹, R² and R³ for each individual compound corresponds in each case to one line of Table B (compounds I.Bb.D1-59.B1 to I.Bb.D1-59.B442).
- Table 60d: Compounds of the formula I.Bb in which the combination of (R⁴)ₙ and (R^{L})ₘ corresponds to line D1-60 of Table D1 and the meaning for the combination of R¹, R² and R³ for each individual compound corresponds in each case to one line of Table B (compounds I.Bb.D1-60.B1 to I.Bb.D1-60.B442).
- Table 61d: Compounds of the formula I.Bb in which the combination of (R⁴)ₙ and (R^{L})ₘ corresponds to line D1-61 of Table D1 and the meaning for the combination of R¹, R² and R³ for each individual compound corresponds in each case to one line of Table B (compounds I.Bb.D1-61.B1 to I.Bb.D1-61.B442).
- Table 62d: Compounds of the formula I.Bb in which the combination of (R⁴)ₙ and (R^{L})ₘ corresponds to line D1-62 of Table D1 and the meaning for the combination of R¹, R² and R³ for each individual compound corresponds in each case to one line of Table B (compounds I.Bb.D1-62.B1 to I.Bb.D1-62.B442).
- Table 63d: Compounds of the formula I.Bb in which the combination of (R⁴)ₙ and (R^{L})ₘ corresponds to line D1-63 of Table D1 and the meaning for the combination of R¹, R² and R³ for each individual compound corresponds in each case to one line of Table B (compounds I.Bb.D1-63.B1 to I.Bb.D1-63.B442).
- Table 64d: Compounds of the formula I.Bb in which the combination of (R⁴)ₙ and (R^{L})ₘ corresponds to line D1-64 of Table D1 and the meaning for the combination of R¹, R² and R³ for each individual compound corresponds in each case to one line of Table B (compounds I.Bb.D1-64.B1 to I.Bb.D1-64.B442).
- Table 65d: Compounds of the formula I.Bb in which the combination of (R⁴)ₙ and (R^{L})ₘ corresponds to line D1-65 of Table D1 and the meaning for the combination of R¹, R² and R³ for each individual compound corresponds in each case to one line of Table B (compounds I.Bb.D1-65.B1 to I.Bb.D1-65.B442).
- Table 66d: Compounds of the formula I.Bb in which the combination of (R⁴)ₙ and (R^{L})ₘ corresponds to line D1-66 of Table D1 and the meaning for the combination of R¹, R² and R³ for each individual compound corresponds in each case to one line of Table B (compounds I.Bb.D1-66.B1 to I.Bb.D1-66.B442).
- Table 67d: Compounds of the formula I.Bb in which the combination of (R⁴)ₙ and (R^{L})ₘ corresponds to line D1-67 of Table D1 and the meaning for the combination of R¹, R² and R³ for each individual compound corresponds in each case to one line of Table B (compounds I.Bb.D1-67.B1 to I.Bb.D1-67.B442).
- Table 68d: Compounds of the formula I.Bb in which the combination of (R⁴)ₙ and (R^{L})ₘ corresponds to line D1-68 of Table D1 and the meaning for the combination of R¹, R² and R³ for each individual compound corresponds in each case to one line of Table B (compounds I.Bb.D1-68.B1 to I.Bb.D1-68.B442).
- Table 69d: Compounds of the formula I.Bb in which the combination of (R⁴)ₙ and (R^{L})ₘ corresponds to line D1-69 of Table D1 and the meaning for the combination of R¹, R² and R³ for each individual compound corresponds in each case to one line of Table B (compounds I.Bb.D1-69.B1 to I.Bb.D1-69.B442).
- Table 70d: Compounds of the formula I.Bb in which the combination of (R⁴)ₙ and (R^{L})ₘ corresponds to line D1-70 of Table D1 and the meaning for the combination of R¹, R² and R³ for each individual compound corresponds in each case to one line of Table B (compounds I.Bb.D1-70.B1 to I.Bb.D1-70.B442).
- Table 71d: Compounds of the formula I.Bb in which the combination of (R⁴)ₙ and (R^{L})ₘ corresponds to line D2-1 of Table D2 and the meaning for the combination of R¹, R² and R³ for each individual compound corresponds in each case to one line of Table B (compounds I.Bb.D2-1.B1 to I.Bb.D2-1.B442).
- Table 72d: Compounds of the formula I.Bb in which the combination of (R⁴)ₙ and (R^{L})ₘ corresponds to line D2-2 of Table D2 and the meaning for the combination of R¹, R² and R³ for each individual compound corresponds in each case to one line of Table B (compounds I.Bb.D2-2.B1 to I.Bb.D2-2.B442).
- Table 73d: Compounds of the formula I.Bb in which the combination of (R⁴)ₙ and (R^{L})ₘ corresponds to line D2-3 of Table D2 and the meaning for the combination of R¹, R² and R³ for each individual compound corresponds in each case to one line of Table B (compounds I.Bb.D2-3.B1 to I.Bb.D2-3.B442).
- Table 74d: Compounds of the formula I.Bb in which the combination of (R⁴)ₙ and (R^{L})ₘ corresponds to line D2-4 of Table D2 and the meaning for the combination of R¹, R² and R³ for each individual compound corresponds in each case to one line of Table B (compounds I.Bb.D2-4.B1 to I.Bb.D2-4.B442).
- Table 75d: Compounds of the formula I.Bb in which the combination of (R⁴)ₙ and (R^{L})ₘ corresponds to line D2-5 of Table D2 and the meaning for the combination of R¹, R² and R³ for each individual compound corresponds in each case to one line of Table B (compounds I.Bb.D2-5.B1 to I.Bb.D2-5.B442).
- Table 76d: Compounds of the formula I.Bb in which the combination of (R⁴)ₙ and (R^{L})ₘ corresponds to line D2-6 of Table D2 and the meaning for the combination of R¹, R² and R³ for each individual compound corresponds in each case to one line of Table B (compounds I.Bb.D2-6.B1 to I.Bb.D2-6.B442).
- Table 77d: Compounds of the formula I.Bb in which the combination of (R⁴)ₙ and (R^{L})ₘ corresponds to line D2-7 of Table D2 and the meaning for the combination of R¹, R² and R³ for each individual compound corresponds in each case to one line of Table B (compounds I.Bb.D2-7.B1 to I.Bb.D2-7.B442).
- Table 78d: Compounds of the formula I.Bb in which the combination of (R⁴)ₙ and (R^{L})ₘ corresponds to line D2-8 of Table D2 and the meaning for the combination of R¹, R² and R³ for each individual compound corresponds in each case to one line of Table B (compounds I.Bb.D2-8.B1 to I.Bb.D2-8.B442).
- Table 79d: Compounds of the formula I.Bb in which the combination of (R⁴)ₙ and (R^{L})ₘ corresponds to line D2-9 of Table D2 and the meaning for the combination of R¹, R² and R³ for each individual compound corresponds in each case to one line of Table B (compounds I.Bb.D2-9.B1 to I.Bb.D2-9.B442).
- Table 80d: Compounds of the formula I.Bb in which the combination of (R⁴)ₙ and (R^{L})ₘ corresponds to line D2-10 of Table D2 and the meaning for the combination of R¹, R² and R³ for each individual compound corresponds in each case to one line of Table B (compounds I.Bb.D2-10.B1 to I.Bb.D2-10.B442).
- Table 81d: Compounds of the formula I.Bb in which the combination of (R⁴)ₙ and (R^{L})ₘ corresponds to line D2-11 of Table D2 and the meaning for the combination of R¹, R² and R³ for each individual compound corresponds in each case to one line of Table B (compounds I.Bb.D2-11.B1 to I.Bb.D2-11.B442).
- Table 82d: Compounds of the formula I.Bb in which the combination of (R⁴)ₙ and (R^{L})ₘ corresponds to line D2-12 of Table D2 and the meaning for the combination of R¹, R² and R³ for each individual compound corresponds in each case to one line of Table B (compounds I.Bb.D2-12.B1 to I.Bb.D2-12.B442).
- Table 83d: Compounds of the formula I.Bb in which the combination of (R⁴)ₙ and (R^{L})ₘ corresponds to line D2-13 of Table D2 and the meaning for the combination of R¹, R² and R³ for each individual compound corresponds in each case to one line of Table B (compounds I.Bb.D2-13.B1 to I.Bb.D2-13.B442).
- Table 84d: Compounds of the formula I.Bb in which the combination of (R⁴)ₙ and (R^{L})ₘ corresponds to line D2-14 of Table D2 and the meaning for the combination of R¹, R² and R³ for each individual compound corresponds in each case to one line of Table B (compounds I.Bb.D2-14.B1 to I.Bb.D2-14.B442).
- Table 85d: Compounds of the formula I.Bb in which the combination of (R⁴)ₙ and (R^{L})ₘ corresponds to line D2-15 of Table D2 and the meaning for the combination of R¹, R² and R³ for each individual compound corresponds in each case to one line of Table B (compounds I.Bb.D2-15.B1 to I.Bb.D2-15.B442).
- Table 86d: Compounds of the formula I.Bb in which the combination of (R⁴)ₙ and (R^{L})ₘ corresponds to line D2-16 of Table D2 and the meaning for the combination of R¹, R² and R³ for each individual compound corresponds in each case to one line of Table B (compounds I.Bb.D2-16.B1 to I.Bb.D2-16.B442).
- Table 87d: Compounds of the formula I.Bb in which the combination of (R⁴)ₙ and (R^{L})ₘ corresponds to line D2-17 of Table D2 and the meaning for the combination of R¹, R² and R³ for each individual compound corresponds in each case to one line of Table B (compounds I.Bb.D2-17.B1 to I.Bb.D2-17.B442).
- Table 88d: Compounds of the formula I.Bb in which the combination of (R⁴)ₙ and (R^{L})ₘ corresponds to line D2-18 of Table D2 and the meaning for the combination of R¹, R² and R³ for each individual compound corresponds in each case to one line of Table B (compounds I.Bb.D2-18.B1 to I.Bb.D2-18.B442).
- Table 89d: Compounds of the formula I.Bb in which the combination of (R⁴)ₙ and (R^{L})ₘ corresponds to line D2-19 of Table D2 and the meaning for the combination of R¹, R² and R³ for each individual compound corresponds in each case to one line of Table B (compounds I.Bb.D2-19.B1 to I.Bb.D2-19.B442).
- Table 90d: Compounds of the formula I.Bb in which the combination of (R⁴)ₙ and (R^{L})ₘ corresponds to line D2-20 of Table D2 and the meaning for the combination of R¹, R² and R³ for each individual compound corresponds in each case to one line of Table B (compounds I.Bb.D2-20.B1 to I.Bb.D2-20.B442).
- Table 91d: Compounds of the formula I.Bb in which the combination of (R⁴)ₙ and (R^{L})ₘ corresponds to line D2-21 of Table D2 and the meaning for the combination of R¹, R² and R³ for each individual compound corresponds in each case to one line of Table B (compounds I.Bb.D2-21.B1 to I.Bb.D2-21.B442).
- Table 92d: Compounds of the formula I.Bb in which the combination of (R⁴)ₙ and (R^{L})ₘ corresponds to line D2-22 of Table D2 and the meaning for the combination of R¹, R² and R³ for each individual compound corresponds in each case to one line of Table B (compounds I.Bb.D2-22.B1 to I.Bb.D2-22.B442).
- Table 93d: Compounds of the formula I.Bb in which the combination of (R⁴)ₙ and (R^{L})ₘ corresponds to line D2-23 of Table D2 and the meaning for the combination of R¹, R² and R³ for each individual compound corresponds in each case to one line of Table B (compounds I.Bb.D2-23.B1 to I.Bb.D2-23.B442).
- Table 94d: Compounds of the formula I.Bb in which the combination of (R⁴)ₙ and (R^{L})ₘ corresponds to line D2-24 of Table D2 and the meaning for the combination of R¹, R² and R³ for each individual compound corresponds in each case to one line of Table B (compounds I.Bb.D2-24.B1 to I.Bb.D2-24.B442).
- Table 95d: Compounds of the formula I.Bb in which the combination of (R⁴)ₙ and (R^{L})ₘ corresponds to line D2-25 of Table D2 and the meaning for the combination of R¹, R² and R³ for each individual compound corresponds in each case to one line of Table B (compounds I.Bb.D2-25.B1 to I.Bb.D2-25.B442).
- Table 96d: Compounds of the formula I.Bb in which the combination of (R⁴)ₙ and (R^{L})ₘ corresponds to line D2-26 of Table D2 and the meaning for the combination of R¹, R² and R³ for each individual compound corresponds in each case to one line of Table B (compounds I.Bb.D2-26.B1 to I.Bb.D2-26.B442).
- Table 97d: Compounds of the formula I.Bb in which the combination of (R⁴)ₙ and (R^{L})ₘ corresponds to line D2-27 of Table D2 and the meaning for the combination of R¹, R² and R³ for each individual compound corresponds in each case to one line of Table B (compounds I.Bb.D2-27.B1 to I.Bb.D2-27.B442).
- Table 98d: Compounds of the formula I.Bb in which the combination of (R⁴)ₙ and (R^{L})ₘ corresponds to line D2-28 of Table D2 and the meaning for the combination of R¹, R² and R³ for each individual compound corresponds in each case to one line of Table B (compounds I.Bb.D2-28.B1 to I.Bb.D2-28.B442).
- Table 99d: Compounds of the formula I.Bb in which the combination of (R⁴)ₙ and (R^{L})ₘ corresponds to line D2-29 of Table D2 and the meaning for the combination of R¹, R² and R³ for each individual compound corresponds in each case to one line of Table B (compounds I.Bb.D2-29.B1 to I.Bb.D2-29.B442).
- Table 100d: Compounds of the formula I.Bb in which the combination of (R⁴)ₙ and (R^{L})ₘ corresponds to line D2-30 of Table D2 and the meaning for the combination of R¹, R² and R³ for each individual compound corresponds in each case to one line of Table B (compounds I.Bb.D2-30.B1 to I.Bb.D2-30.B442).
- Table 101d: Compounds of the formula I.Bb in which the combination of (R⁴)ₙ and (R^{L})ₘ corresponds to line D2-31 of Table D2 and the meaning for the combination of R¹, R² and R³ for each individual compound corresponds in each case to one line of Table B (compounds I.Bb.D2-31.B1 to I.Bb.D2-31.B442).
- Table 102d: Compounds of the formula I.Bb in which the combination of (R⁴)ₙ and (R^{L})ₘ corresponds to line D2-32 of Table D2 and the meaning for the combination of R¹, R² and R³ for each individual compound corresponds in each case to one line of Table B (compounds I.Bb.D2-32.B1 to I.Bb.D2-32.B442).
- Table 103d: Compounds of the formula I.Bb in which the combination of (R⁴)ₙ and (R^{L})ₘ corresponds to line D2-33 of Table D2 and the meaning for the combination of R¹, R² and R³ for each individual compound corresponds in each case to one line of Table B (compounds I.Bb.D2-33.B1 to I.Bb.D2-33.B442).
- Table 104d: Compounds of the formula I.Bb in which the combination of (R⁴)ₙ and (R^{L})ₘ corresponds to line D2-34 of Table D2 and the meaning for the combination of R¹, R² and R³ for each individual compound corresponds in each case to one line of Table B (compounds I.Bb.D2-34.B1 to I.Bb.D2-34.B442).
- Table 105d: Compounds of the formula I.Bb in which the combination of (R⁴)ₙ and (R^{L})ₘ corresponds to line D2-35 of Table D2 and the meaning for the combination of R¹, R² and R³ for each individual compound corresponds in each case to one line of Table B (compounds I.Bb.D2-35.B1 to I.Bb.D2-35.B442).
- Table 106d: Compounds of the formula I.Bb in which the combination of (R⁴)ₙ and (R^{L})ₘ corresponds to line D2-36 of Table D2 and the meaning for the combination of R¹, R² and R³ for each individual compound corresponds in each case to one line of Table B (compounds I.Bb.D2-36.B1 to I.Bb.D2-36.B442).
- Table 107d: Compounds of the formula I.Bb in which the combination of (R⁴)ₙ and (R^{L})ₘ corresponds to line D2-37 of Table D2 and the meaning for the combination of R¹, R² and R³ for each individual compound corresponds in each case to one line of Table B (compounds I.Bb.D2-37.B1 to I.Bb.D2-37.B442).
- Table 108d: Compounds of the formula I.Bb in which the combination of (R⁴)ₙ and (R^{L})ₘ corresponds to line D2-38 of Table D2 and the meaning for the combination of R¹, R² and R³ for each individual compound corresponds in each case to one line of Table B (compounds I.Bb.D2-38.B1 to I.Bb.D2-38.B442).
- Table 109d: Compounds of the formula I.Bb in which the combination of (R⁴)ₙ and (R^{L})ₘ corresponds to line D2-39 of Table D2 and the meaning for the combination of R¹, R² and R³ for each individual compound corresponds in each case to one line of Table B (compounds I.Bb.D2-39.B1 to I.Bb.D2-39.B442).
- Table 110d: Compounds of the formula I.Bb in which the combination of (R⁴)ₙ and (R^{L})ₘ corresponds to line D2-40 of Table D2 and the meaning for the combination of R¹, R² and R³ for each individual compound corresponds in each case to one line of Table B (compounds I.Bb.D2-40.B1 to I.Bb.D2-40.B442).
- Table 111d: Compounds of the formula I.Bb in which the combination of (R⁴)ₙ and (R^{L})ₘ corresponds to line D2-41 of Table D2 and the meaning for the combination of R¹, R² and R³ for each individual compound corresponds in each case to one line of Table B (compounds I.Bb.D2-41.B1 to I.Bb.D2-41.B442).
- Table 112d: Compounds of the formula I.Bb in which the combination of (R⁴)ₙ and (R^{L})ₘ corresponds to line D2-42 of Table D2 and the meaning for the combination of R¹, R² and R³ for each individual compound corresponds in each case to one line of Table B (compounds I.Bb.D2-42.B1 to I.Bb.D2-42.B442).
- Table 113d: Compounds of the formula I.Bb in which the combination of (R⁴)ₙ and (R^{L})ₘ corresponds to line D2-43 of Table D2 and the meaning for the combination of R¹, R² and R³ for each individual compound corresponds in each case to one line of Table B (compounds I.Bb.D2-43.B1 to I.Bb.D2-43.B442).
- Table 114d: Compounds of the formula I.Bb in which the combination of (R⁴)ₙ and (R^{L})ₘ corresponds to line D2-44 of Table D2 and the meaning for the combination of R¹, R² and R³ for each individual compound corresponds in each case to one line of Table B (compounds I.Bb.D2-44.B1 to I.Bb.D2-44.B442).
- Table 115d: Compounds of the formula I.Bb in which the combination of (R⁴)ₙ and (R^{L})ₘ corresponds to line D2-45 of Table D2 and the meaning for the combination of R¹, R² and R³ for each individual compound corresponds in each case to one line of Table B (compounds I.Bb.D2-45.B1 to I.Bb.D2-45.B442).
- Table 116d: Compounds of the formula I.Bb in which the combination of (R⁴)ₙ and (R^{L})ₘ corresponds to line D2-46 of Table D2 and the meaning for the combination of R¹, R² and R³ for each individual compound corresponds in each case to one line of Table B (compounds I.Bb.D2-46.B1 to I.Bb.D2-46.B442).
- Table 117d: Compounds of the formula I.Bb in which the combination of (R⁴)ₙ and (R^{L})ₘ corresponds to line D2-47 of Table D2 and the meaning for the combination of R¹, R² and R³ for each individual compound corresponds in each case to one line of Table B (compounds I.Bb.D2-47.B1 to I.Bb.D2-47.B442).
- Table 118d: Compounds of the formula I.Bb in which the combination of (R⁴)ₙ and (R^{L})ₘ corresponds to line D2-48 of Table D2 and the meaning for the combination of R¹, R² and R³ for each individual compound corresponds in each case to one line of Table B (compounds I.Bb.D2-48.B1 to I.Bb.D2-48.B442).
- Table 119d: Compounds of the formula I.Bb in which the combination of (R⁴)ₙ and (R^{L})ₘ corresponds to line D2-49 of Table D2 and the meaning for the combination of R¹, R² and R³ for each individual compound corresponds in each case to one line of Table B (compounds I.Bb.D2-49.B1 to I.Bb.D2-49.B442).
- Table 120d: Compounds of the formula I.Bb in which the combination of (R⁴)ₙ and (R^{L})ₘ corresponds to line D2-50 of Table D2 and the meaning for the combination of R¹, R² and R³ for each individual compound corresponds in each case to one line of Table B (compounds I.Bb.D2-50.B1 to I.Bb.D2-50.B442).
- Table 121d: Compounds of the formula I.Bb in which the combination of (R⁴)ₙ and (R^{L})ₘ corresponds to line D2-51 of Table D2 and the meaning for the combination of R¹, R² and R³ for each individual compound corresponds in each case to one line of Table B (compounds I.Bb.D2-51.B1 to I.Bb.D2-51.B442).
- Table 122d: Compounds of the formula I.Bb in which the combination of (R⁴)ₙ and (R^{L})ₘ corresponds to line D2-52 of Table D2 and the meaning for the combination of R¹, R² and R³ for each individual compound corresponds in each case to one line of Table B (compounds I.Bb.D2-52.B1 to I.Bb.D2-52.B442).
- Table 123d: Compounds of the formula I.Bb in which the combination of (R⁴)ₙ and (R^{L})ₘ corresponds to line D2-53 of Table D2 and the meaning for the combination of R¹, R² and R³ for each individual compound corresponds in each case to one line of Table B (compounds I.Bb.D2-53.B1 to I.Bb.D2-53.B442).
- Table 124d: Compounds of the formula I.Bb in which the combination of (R⁴)ₙ and (R^{L})ₘ corresponds to line D2-54 of Table D2 and the meaning for the combination of R¹, R² and R³ for each individual compound corresponds in each case to one line of Table B (compounds I.Bb.D2-54.B1 to I.Bb.D2-54.B442).
- Table 125d: Compounds of the formula I.Bb in which the combination of (R⁴)ₙ and (R^{L})ₘ corresponds to line D2-55 of Table D2 and the meaning for the combination of R¹, R² and R³ for each individual compound corresponds in each case to one line of Table B (compounds I.Bb.D2-55.B1 to I.Bb.D2-55.B442).
- Table 126d: Compounds of the formula I.Bb in which the combination of (R⁴)ₙ and (R^{L})ₘ corresponds to line D2-56 of Table D2 and the meaning for the combination of R¹, R² and R³ for each individual compound corresponds in each case to one line of Table B (compounds I.Bb.D2-56.B1 to I.Bb.D2-56.B442).
- Table 127d: Compounds of the formula I.Bb in which the combination of (R⁴)ₙ and (R^{L})ₘ corresponds to line D2-57 of Table D2 and the meaning for the combination of R¹, R² and R³ for each individual compound corresponds in each case to one line of Table B (compounds I.Bb.D2-57.B1 to I.Bb.D2-57.B442).
- Table 128d: Compounds of the formula I.Bb in which the combination of (R⁴)ₙ and (R^{L})ₘ corresponds to line D2-58 of Table D2 and the meaning for the combination of R¹, R² and R³ for each individual compound corresponds in each case to one line of Table B (compounds I.Bb.D2-58.B1 to I.Bb.D2-58.B442).
- Table 129d: Compounds of the formula I.Bb in which the combination of (R⁴)ₙ and (R^{L})ₘ corresponds to line D2-59 of Table D2 and the meaning for the combination of R¹, R² and R³ for each individual compound corresponds in each case to one line of Table B (compounds I.Bb.D2-59.B1 to I.Bb.D2-59.B442).
- Table 130d: Compounds of the formula I.Bb in which the combination of (R⁴)ₙ and (R^{L})ₘ corresponds to line D2-60 of Table D2 and the meaning for the combination of R¹, R² and R³ for each individual compound corresponds in each case to one line of Table B (compounds I.Bb.D2-60.B1 to I.Bb.D2-60.B442).
- Table 131d: Compounds of the formula I.Bb in which the combination of (R⁴)ₙ and (R^{L})ₘ corresponds to line D2-61 of Table D2 and the meaning for the combination of R¹, R² and R³ for each individual compound corresponds in each case to one line of Table B (compounds I.Bb.D2-61.B1 to I.Bb.D2-61.B442).
- Table 132d: Compounds of the formula I.Bb in which the combination of (R⁴)ₙ and (R^{L})ₘ corresponds to line D2-62 of Table D2 and the meaning for the combination of R¹, R² and R³ for each individual compound corresponds in each case to one line of Table B (compounds I.Bb.D2-62.B1 to I.Bb.D2-62.B442).
- Table 133d: Compounds of the formula I.Bb in which the combination of (R⁴)ₙ and (R^{L})ₘ corresponds to line D2-63 of Table D2 and the meaning for the combination of R¹, R² and R³ for each individual compound corresponds in each case to one line of Table B (compounds I.Bb.D2-63.B1 to I.Bb.D2-63.B442).
- Table 134d: Compounds of the formula I.Bb in which the combination of (R⁴)ₙ and (R^{L})ₘ corresponds to line D2-64 of Table D2 and the meaning for the combination of R¹, R² and R³ for each individual compound corresponds in each case to one line of Table B (compounds I.Bb.D2-64.B1 to I.Bb.D2-64.B442).
- Table 135d: Compounds of the formula I.Bb in which the combination of (R⁴)ₙ and (R^{L})ₘ corresponds to line D2-65 of Table D2 and the meaning for the combination of R¹, R² and R³ for each individual compound corresponds in each case to one line of Table B (compounds I.Bb.D2-65.B1 to I.Bb.D2-65.B442).
- Table 136d: Compounds of the formula I.Bb in which the combination of (R⁴)ₙ and (R^{L})ₘ corresponds to line D2-66 of Table D2 and the meaning for the combination of R¹, R² and R³ for each individual compound corresponds in each case to one line of Table B (compounds I.Bb.D2-66.B1 to I.Bb.D2-66.B442).
- Table 137d: Compounds of the formula I.Bb in which the combination of (R⁴)ₙ and (R^{L})ₘ corresponds to line D2-67 of Table D2 and the meaning for the combination of R¹, R² and R³ for each individual compound corresponds in each case to one line of Table B (compounds I.Bb.D2-67.B1 to I.Bb.D2-67.B442).
- Table 138d: Compounds of the formula I.Bb in which the combination of (R⁴)ₙ and (R^{L})ₘ corresponds to line D2-68 of Table D2 and the meaning for the combination of R¹, R² and R³ for each individual compound corresponds in each case to one line of Table B (compounds I.Bb.D2-68.B1 to I.Bb.D2-68.B442).
- Table 139d: Compounds of the formula I.Bb in which the combination of (R⁴)ₙ and (R^{L})ₘ corresponds to line D2-69 of Table D2 and the meaning for the combination of R¹, R² and R³ for each individual compound corresponds in each case to one line of Table B (compounds I.Bb.D2-69.B1 to I.Bb.D2-69.B442).
- Table 140d: Compounds of the formula I.Bb in which the combination of (R⁴)ₙ and (R^{L})ₘ corresponds to line D2-70 of Table D2 and the meaning for the combination of R¹, R² and R³ for each individual compound corresponds in each case to one line of Table B (compounds I.Bb.D2-70.B1 to I.Bb.D2-70.B442).
- Table 1e: Compounds of the formula I.Bc in which the combination of (R⁴)ₙ and (R^{L})ₘ corresponds to line D1-1 of Table D1 and the meaning for the combination of R¹, R² and R³ for each individual compound corresponds in each case to one line of Table B (compounds I.Bc.D1-1.B1 to I.Bc.D1.B442).
- Table 2e: Compounds of the formula I.Bc in which the combination of (R⁴)ₙ and (R^{L})ₘ corresponds to line D1-2 of Table D1 and the meaning for the combination of R¹, R² and R³ for each individual compound corresponds in each case to one line of Table B (compounds I.Bc.D1-2.B1 to I.Bc.D1-2.B442).
- Table 3e: Compounds of the formula I.Bc in which the combination of (R⁴)ₙ and (R^{L})ₘ corresponds to line D1-3 of Table D1 and the meaning for the combination of R¹, R² and R³ for each individual compound corresponds in each case to one line of Table B (compounds I.Bc.D1-3.B1 to I.Bc.D1-3.B442).
- Table 4e: Compounds of the formula I.Bc in which the combination of (R⁴)ₙ and (R^{L})ₘ corresponds to line D1-4 of Table D1 and the meaning for the combination of R¹, R² and R³ for each individual compound corresponds in each case to one line of Table B (compounds I.Bc.D1-4.B1 to I.Bc.D1-4.B442).
- Table 5e: Compounds of the formula I.Bc in which the combination of (R⁴)ₙ and (R^{L})ₘ corresponds to line D1-5 of Table D1 and the meaning for the combination of R¹, R² and R³ for each individual compound corresponds in each case to one line of Table B (compounds I.Bc.D1-5.B1 to I.Bc.D1-5.B442).
- Table 6e: Compounds of the formula I.Bc in which the combination of (R⁴)ₙ and (R^{L})ₘ corresponds to line D1-6 of Table D1 and the meaning for the combination of R¹, R² and R³ for each individual compound corresponds in each case to one line of Table B (compounds I.Bc.D1-6.B1 to I.Bc.D1-6.B442).
- Table 7e: Compounds of the formula I.Bc in which the combination of (R⁴)ₙ and (R^{L})ₘ corresponds to line D1-7 of Table D1 and the meaning for the combination of R¹, R² and R³ for each individual compound corresponds in each case to one line of Table B (compounds I.Bc.D1-7.B1 to I.Bc.D1-7.B442).
- Table 8e: Compounds of the formula I.Bc in which the combination of (R⁴)ₙ and (R^{L})ₘ corresponds to line D1-8 of Table D1 and the meaning for the combination of R¹, R² and R³ for each individual compound corresponds in each case to one line of Table B (compounds I.Bc.D1-8.B1 to I.Bc.D1-8.B442).
- Table 9e: Compounds of the formula I.Bc in which the combination of (R⁴)ₙ and (R^{L})ₘ corresponds to line D1-9 of Table D1 and the meaning for the combination of R¹, R² and R³ for each individual compound corresponds in each case to one line of Table B (compounds I.Bc.D1-9.B1 to I.Bc.D1-9.B442).
- Table 10e: Compounds of the formula I.Bc in which the combination of (R⁴)ₙ and (R^{L})ₘ corresponds to line D1-10 of Table D1 and the meaning for the combination of R¹, R² and R³ for each individual compound corresponds in each case to one line of Table B (compounds I.Bc.D1-10.B1 to I.Bc.D1-10.B442).
- Table 11e: Compounds of the formula I.Bc in which the combination of (R⁴)ₙ and (R^{L})ₘ corresponds to line D1-11 of Table D1 and the meaning for the combination of R¹, R² and R³ for each individual compound corresponds in each case to one line of Table B (compounds I.Bc.D1-11.B1 to I.Bc.D1-11.B442).
- Table 12e: Compounds of the formula I.Bc in which the combination of (R⁴)ₙ and (R^{L})ₘ corresponds to line D1-12 of Table D1 and the meaning for the combination of R¹, R² and R³ for each individual compound corresponds in each case to one line of Table B (compounds I.Bc.D1-12.B1 to I.Bc.D1-12.B442).
- Table 13e: Compounds of the formula I.Bc in which the combination of (R⁴)ₙ and (R^{L})ₘ corresponds to line D1-13 of Table D1 and the meaning for the combination of R¹, R² and R³ for each individual compound corresponds in each case to one line of Table B (compounds I.Bc.D1-13.B1 to I.Bc.D1-13.B442).
- Table 14e: Compounds of the formula I.Bc in which the combination of (R⁴)ₙ and (R^{L})ₘ corresponds to line D1-14 of Table D1 and the meaning for the combination of R¹, R² and R³ for each individual compound corresponds in each case to one line of Table B (compounds I.Bc.D1-14.B1 to I.Bc.D1-14.B442).
- Table 15e: Compounds of the formula I.Bc in which the combination of (R⁴)ₙ and (R^{L})ₘ corresponds to line D1-15 of Table D1 and the meaning for the combination of R¹, R² and R³ for each individual compound corresponds in each case to one line of Table B (compounds I.Bc.D1-15.B1 to I.Bc.D1-15.B442).
- Table 16e: Compounds of the formula I.Bc in which the combination of (R⁴)ₙ and (R^{L})ₘ corresponds to line D1-16 of Table D1 and the meaning for the combination of R¹, R² and R³ for each individual compound corresponds in each case to one line of Table B (compounds I.Bc.D1-16.B1 to I.Bc.D1-16.B442).
- Table 17e: Compounds of the formula I.Bc in which the combination of (R⁴)ₙ and (R^{L})ₘ corresponds to line D1-17 of Table D1 and the meaning for the combination of R¹, R² and R³ for each individual compound corresponds in each case to one line of Table B (compounds I.Bc.D1-17.B1 to I.Bc.D1-17.B442).
- Table 18e: Compounds of the formula I.Bc in which the combination of (R⁴)ₙ and (R^{L})ₘ corresponds to line D1-18 of Table D1 and the meaning for the combination of R¹, R² and R³ for each individual compound corresponds in each case to one line of Table B (compounds I.Bc.D1-18.B1 to I.Bc.D1-18.B442).
- Table 19e: Compounds of the formula I.Bc in which the combination of (R⁴)ₙ and (R^{L})ₘ corresponds to line D1-19 of Table D1 and the meaning for the combination of R¹, R² and R³ for each individual compound corresponds in each case to one line of Table B (compounds I.Bc.D1-19.B1 to I.Bc.D1-19.B442).
- Table 20e: Compounds of the formula I.Bc in which the combination of (R⁴)ₙ and (R^{L})ₘ corresponds to line D1-20 of Table D1 and the meaning for the combination of R¹, R² and R³ for each individual compound corresponds in each case to one line of Table B (compounds I.Bc.D1-20.B1 to I.Bc.D1-20.B442).
- Table 21e: Compounds of the formula I.Bc in which the combination of (R⁴)ₙ and (R^{L})ₘ corresponds to line D1-21 of Table D1 and the meaning for the combination of R¹, R² and R³ for each individual compound corresponds in each case to one line of Table B (compounds I.Bc.D1-21.B1 to I.Bc.D1-21.B442).
- Table 22e: Compounds of the formula I.Bc in which the combination of (R⁴)ₙ and (R^{L})ₘ corresponds to line D1-22 of Table D1 and the meaning for the combination of R¹, R² and R³ for each individual compound corresponds in each case to one line of Table B (compounds I.Bc.D1-22.B1 to I.Bc.D1-22.B442).
- Table 23e: Compounds of the formula I.Bc in which the combination of (R⁴)ₙ and (R^{L})ₘ corresponds to line D1-23 of Table D1 and the meaning for the combination of R¹, R² and R³ for each individual compound corresponds in each case to one line of Table B (compounds I.Bc.D1-23.B1 to I.Bc.D1-23.B442).
- Table 24e: Compounds of the formula I.Bc in which the combination of (R⁴)ₙ and (R^{L})ₘ corresponds to line D1-24 of Table D1 and the meaning for the combination of R¹, R² and R³ for each individual compound corresponds in each case to one line of Table B (compounds I.Bc.D1-24.B1 to I.Bc.D1-24.B442).
- Table 25e: Compounds of the formula I.Bc in which the combination of (R⁴)ₙ and (R^{L})ₘ corresponds to line D1-25 of Table D1 and the meaning for the combination of R¹, R² and R³ for each individual compound corresponds in each case to one line of Table B (compounds I.Bc.D1-25.B1 to I.Bc.D1-25.B442).
- Table 26e: Compounds of the formula I.Bc in which the combination of (R⁴)ₙ and (R^{L})ₘ corresponds to line D1-26 of Table D1 and the meaning for the combination of R¹, R² and R³ for each individual compound corresponds in each case to one line of Table B (compounds I.Bc.D1-26.B1 to I.Bc.D1-26.B442).
- Table 27e: Compounds of the formula I.Bc in which the combination of (R⁴)ₙ and (R^{L})ₘ corresponds to line D1-27 of Table D1 and the meaning for the combination of R¹, R² and R³ for each individual compound corresponds in each case to one line of Table B (compounds I.Bc.D1-27.B1 to I.Bc.D1-27.B442).
- Table 28e: Compounds of the formula I.Bc in which the combination of (R⁴)ₙ and (R^{L})ₘ corresponds to line D1-28 of Table D1 and the meaning for the combination of R¹, R² and R³ for each individual compound corresponds in each case to one line of Table B (compounds I.Bc.D1-28.B1 to I.Bc.D1-28.B442).
- Table 29e: Compounds of the formula I.Bc in which the combination of (R⁴)ₙ and (R^{L})ₘ corresponds to line D1-29 of Table D1 and the meaning for the combination of R¹, R² and R³ for each individual compound corresponds in each case to one line of Table B (compounds I.Bc.D1-29.B1 to I.Bc.D1-29.B442).
- Table 30e: Compounds of the formula I.Bc in which the combination of (R⁴)ₙ and (R^{L})ₘ corresponds to line D1-30 of Table D1 and the meaning for the combination of R¹, R² and R³ for each individual compound corresponds in each case to one line of Table B (compounds I.Bc.D1-30.B1 to I.Bc.D1-30.B442).
- Table 31e: Compounds of the formula I.Bc in which the combination of (R⁴)ₙ and (R^{L})ₘ corresponds to line D1-31 of Table D1 and the meaning for the combination of R¹, R² and R³ for each individual compound corresponds in each case to one line of Table B (compounds I.Bc.D1-31.B1 to I.Bc.D1-31.B442).
- Table 32e: Compounds of the formula I.Bc in which the combination of (R⁴)ₙ and (R^{L})ₘ corresponds to line D1-32 of Table D1 and the meaning for the combination of R¹, R² and R³ for each individual compound corresponds in each case to one line of Table B (compounds I.Bc.D1-32.B1 to I.Bc.D1-32.B442).
- Table 33e: Compounds of the formula I.Bc in which the combination of (R⁴)ₙ and (R^{L})ₘ corresponds to line D1-33 of Table D1 and the meaning for the combination of R¹, R² and R³ for each individual compound corresponds in each case to one line of Table B (compounds I.Bc.D1-33.B1 to I.Bc.D1-33.B442).
- Table 34e: Compounds of the formula I.Bc in which the combination of (R⁴)ₙ and (R^{L})ₘ corresponds to line D1-34 of Table D1 and the meaning for the combination of R¹, R² and R³ for each individual compound corresponds in each case to one line of Table B (compounds I.Bc.D1-34.B1 to I.Bc.D1-34.B442).
- Table 35e: Compounds of the formula I.Bc in which the combination of (R⁴)ₙ and (R^{L})ₘ corresponds to line D1-35 of Table D1 and the meaning for the combination of R¹, R² and R³ for each individual compound corresponds in each case to one line of Table B (compounds I.Bc.D1-35.B1 to I.Bc.D1-35.B442).
- Table 36e: Compounds of the formula I.Bc in which the combination of (R⁴)ₙ and (R^{L})ₘ corresponds to line D1-36 of Table D1 and the meaning for the combination of R¹, R² and R³ for each individual compound corresponds in each case to one line of Table B (compounds I.Bc.D1-36.B1 to I.Bc.D1-36.B442).
- Table 37e: Compounds of the formula I.Bc in which the combination of (R⁴)ₙ and (R^{L})ₘ corresponds to line D1-37 of Table D1 and the meaning for the combination of R¹, R² and R³ for each individual compound corresponds in each case to one line of Table B (compounds I.Bc.D1-37.B1 to I.Bc.D1-37.B442).
- Table 38e: Compounds of the formula I.Bc in which the combination of (R⁴)ₙ and (R^{L})ₘ corresponds to line D1-38 of Table D1 and the meaning for the combination of R¹, R² and R³ for each individual compound corresponds in each case to one line of Table B (compounds I.Bc.D1-38.B1 to I.Bc.D1-38.B442).
- Table 39e: Compounds of the formula I.Bc in which the combination of (R⁴)ₙ and (R^{L})ₘ corresponds to line D1-39 of Table D1 and the meaning for the combination of R¹, R² and R³ for each individual compound corresponds in each case to one line of Table B (compounds I.Bc.D1-39.B1 to I.Bc.D1-39.B442).
- Table 40e: Compounds of the formula I.Bc in which the combination of (R⁴)ₙ and (R^{L})ₘ corresponds to line D1-40 of Table D1 and the meaning for the combination of R¹, R² and R³ for each individual compound corresponds in each case to one line of Table B (compounds I.Bc.D1-40.B1 to I.Bc.D1-40.B442).
- Table 41e: Compounds of the formula I.Bc in which the combination of (R⁴)ₙ and (R^{L})ₘ corresponds to line D1-41 of Table D1 and the meaning for the combination of R¹, R² and R³ for each individual compound corresponds in each case to one line of Table B (compounds I.Bc.D1-41.B1 to I.Bc.D1-41.B442).
- Table 42e: Compounds of the formula I.Bc in which the combination of (R⁴)ₙ and (R^{L})ₘ corresponds to line D1-42 of Table D1 and the meaning for the combination of R¹, R² and R³ for each individual compound corresponds in each case to one line of Table B (compounds I.Bc.D1-42.B1 to I.Bc.D1-42.B442).
- Table 43e: Compounds of the formula I.Bc in which the combination of (R⁴)ₙ and (R^{L})ₘ corresponds to line D1-43 of Table D1 and the meaning for the combination of R¹, R² and R³ for each individual compound corresponds in each case to one line of Table B (compounds I.Bc.D1-43.B1 to I.Bc.D1-43.B442).
- Table 44e: Compounds of the formula I.Bc in which the combination of (R⁴)ₙ and (R^{L})ₘ corresponds to line D1-44 of Table D1 and the meaning for the combination of R¹, R² and R³ for each individual compound corresponds in each case to one line of Table B (compounds I.Bc.D1-44.B1 to I.Bc.D1-44.B442).
- Table 45e: Compounds of the formula I.Bc in which the combination of (R⁴)ₙ and (R^{L})ₘ corresponds to line D1-45 of Table D1 and the meaning for the combination of R¹, R² and R³ for each individual compound corresponds in each case to one line of Table B (compounds I.Bc.D1-45.B1 to I.Bc.D1-45.B442).
- Table 46e: Compounds of the formula I.Bc in which the combination of (R⁴)ₙ and (R^{L})ₘ corresponds to line D1-46 of Table D1 and the meaning for the combination of R¹, R² and R³ for each individual compound corresponds in each case to one line of Table B (compounds I.Bc.D1-46.B1 to I.Bc.D1-46.B442).
- Table 47e: Compounds of the formula I.Bc in which the combination of (R⁴)ₙ and (R^{L})ₘ corresponds to line D1-47 of Table D1 and the meaning for the combination of R¹, R² and R³ for each individual compound corresponds in each case to one line of Table B (compounds I.Bc.D1-47.B1 to I.Bc.D1-47.B442).
- Table 48e: Compounds of the formula I.Bc in which the combination of (R⁴)ₙ and (R^{L})ₘ corresponds to line D1-48 of Table D1 and the meaning for the combination of R¹, R² and R³ for each individual compound corresponds in each case to one line of Table B (compounds I.Bc.D1-48.B1 to I.Bc.D1-48.B442).
- Table 49e: Compounds of the formula I.Bc in which the combination of (R⁴)ₙ and (R^{L})ₘ corresponds to line D1-49 of Table D1 and the meaning for the combination of R¹, R² and R³ for each individual compound corresponds in each case to one line of Table B (compounds I.Bc.D1-49.B1 to I.Bc.D1-49.B442).
- Table 50e: Compounds of the formula I.Bc in which the combination of (R⁴)ₙ and (R^{L})ₘ corresponds to line D1-50 of Table D1 and the meaning for the combination of R¹, R² and R³ for each individual compound corresponds in each case to one line of Table B (compounds I.Bc.D1-50.B1 to I.Bc.D1-50.B442).
- Table 51e: Compounds of the formula I.Bc in which the combination of (R⁴)ₙ and (R^{L})ₘ corresponds to line D1-51 of Table D1 and the meaning for the combination of R¹, R² and R³ for each individual compound corresponds in each case to one line of Table B (compounds I.Bc.D1-51.B1 to I.Bc.D1-51.B442).
- Table 52e: Compounds of the formula I.Bc in which the combination of (R⁴)ₙ and (R^{L})ₘ corresponds to line D1-52 of Table D1 and the meaning for the combination of R¹, R² and R³ for each individual compound corresponds in each case to one line of Table B (compounds I.Bc.D1-52.B1 to I.Bc.D1-52.B442).
- Table 53e: Compounds of the formula I.Bc in which the combination of (R⁴)ₙ and (R^{L})ₘ corresponds to line D1-53 of Table D1 and the meaning for the combination of R¹, R² and R³ for each individual compound corresponds in each case to one line of Table B (compounds I.Bc.D1-53.B1 to I.Bc.D1-53.B442).
- Table 54e: Compounds of the formula I.Bc in which the combination of (R⁴)ₙ and (R^{L})ₘ corresponds to line D1-54 of Table D1 and the meaning for the combination of R¹, R² and R³ for each individual compound corresponds in each case to one line of Table B (compounds I.Bc.D1-54.B1 to I.Bc.D1-54.B442).
- Table 55e: Compounds of the formula I.Bc in which the combination of (R⁴)ₙ and (R^{L})ₘ corresponds to line D1-55 of Table D1 and the meaning for the combination of R¹, R² and R³ for each individual compound corresponds in each case to one line of Table B (compounds I.Bc.D1-55.B1 to I.Bc.D1-55.B442).
- Table 56e: Compounds of the formula I.Bc in which the combination of (R⁴)ₙ and (R^{L})ₘ corresponds to line D1-56 of Table D1 and the meaning for the combination of R¹, R² and R³ for each individual compound corresponds in each case to one line of Table B (compounds I.Bc.D1-56.B1 to I.Bc.D1-56.B442).
- Table 57e: Compounds of the formula I.Bc in which the combination of (R⁴)ₙ and (R^{L})ₘ corresponds to line D1-57 of Table D1 and the meaning for the combination of R¹, R² and R³ for each individual compound corresponds in each case to one line of Table B (compounds I.Bc.D1-57.B1 to I.Bc.D1-57.B442).
- Table 58e: Compounds of the formula I.Bc in which the combination of (R⁴)ₙ and (R^{L})ₘ corresponds to line D1-58 of Table D1 and the meaning for the combination of R¹, R² and R³ for each individual compound corresponds in each case to one line of Table B (compounds I.Bc.D1-58.B1 to I.Bc.D1-58.B442).
- Table 59e: Compounds of the formula I.Bc in which the combination of (R⁴)ₙ and (R^{L})ₘ corresponds to line D1-59 of Table D1 and the meaning for the combination of R¹, R² and R³ for each individual compound corresponds in each case to one line of Table B (compounds I.Bc.D1-59.B1 to I.Bc.D1-59.B442).
- Table 60e: Compounds of the formula I.Bc in which the combination of (R⁴)ₙ and (R^{L})ₘ corresponds to line D1-60 of Table D1 and the meaning for the combination of R¹, R² and R³ for each individual compound corresponds in each case to one line of Table B (compounds I.Bc.D1-60.B1 to I.Bc.D1-60.B442).
- Table 61e: Compounds of the formula I.Bc in which the combination of (R⁴)ₙ and (R^{L})ₘ corresponds to line D1-61 of Table D1 and the meaning for the combination of R¹, R² and R³ for each individual compound corresponds in each case to one line of Table B (compounds I.Bc.D1-61.B1 to I.Bc.D1-61.B442).
- Table 62e: Compounds of the formula I.Bc in which the combination of (R⁴)ₙ and (R^{L})ₘ corresponds to line D1-62 of Table D1 and the meaning for the combination of R¹, R² and R³ for each individual compound corresponds in each case to one line of Table B (compounds I.Bc.D1-62.B1 to I.Bc.D1-62.B442).
- Table 63e: Compounds of the formula I.Bc in which the combination of (R⁴)ₙ and (R^{L})ₘ corresponds to line D1-63 of Table D1 and the meaning for the combination of R¹, R² and R³ for each individual compound corresponds in each case to one line of Table B (compounds I.Bc.D1-63.B1 to I.Bc.D1-63.B442).
- Table 64e: Compounds of the formula I.Bc in which the combination of (R⁴)ₙ and (R^{L})ₘ corresponds to line D1-64 of Table D1 and the meaning for the combination of R¹, R² and R³ for each individual compound corresponds in each case to one line of Table B (compounds I.Bc.D1-64.B1 to I.Bc.D1-64.B442).
- Table 65e: Compounds of the formula I.Bc in which the combination of (R⁴)ₙ and (R^{L})ₘ corresponds to line D1-65 of Table D1 and the meaning for the combination of R¹, R² and R³ for each individual compound corresponds in each case to one line of Table B (compounds I.Bc.D1-65.B1 to I.Bc.D1-65.B442).
- Table 66e: Compounds of the formula I.Bc in which the combination of (R⁴)ₙ and (R^{L})ₘ corresponds to line D1-66 of Table D1 and the meaning for the combination of R¹, R² and R³ for each individual compound corresponds in each case to one line of Table B (compounds I.Bc.D1-66.B1 to I.Bc.D1-66.B442).
- Table 67e: Compounds of the formula I.Bc in which the combination of (R⁴)ₙ and (R^{L})ₘ corresponds to line D1-67 of Table D1 and the meaning for the combination of R¹, R² and R³ for each individual compound corresponds in each case to one line of Table B (compounds I.Bc.D1-67.B1 to I.Bc.D1-67.B442).
- Table 68e: Compounds of the formula I.Bc in which the combination of (R⁴)ₙ and (R^{L})ₘ corresponds to line D1-68 of Table D1 and the meaning for the combination of R¹, R² and R³ for each individual compound corresponds in each case to one line of Table B (compounds I.Bc.D1-68.B1 to I.Bc.D1-68.B442).
- Table 69e: Compounds of the formula I.Bc in which the combination of (R⁴)ₙ and (R^{L})ₘ corresponds to line D1-69 of Table D1 and the meaning for the combination of R¹, R² and R³ for each individual compound corresponds in each case to one line of Table B (compounds I.Bc.D1-69.B1 to I.Bc.D1-69.B442).
- Table 70e: Compounds of the formula I.Bc in which the combination of (R⁴)ₙ and (R^{L})ₘ corresponds to line D1-70 of Table D1 and the meaning for the combination of R¹, R² and R³ for each individual compound corresponds in each case to one line of Table B (compounds I.Bc.D1-70.B1 to I.Bc.D1-70.B442).
- Table 71e: Compounds of the formula I.Bc in which the combination of (R⁴)ₙ and (R^{L})ₘ corresponds to line D2-1 of Table D2 and the meaning for the combination of R¹, R² and R³ for each individual compound corresponds in each case to one line of Table B (compounds I.Bc.D2-1.B1 to I.Bc.D2-1.B442).
- Table 72e: Compounds of the formula I.Bc in which the combination of (R⁴)ₙ and (R^{L})ₘ corresponds to line D2-2 of Table D2 and the meaning for the combination of R¹, R² and R³ for each individual compound corresponds in each case to one line of Table B (compounds I.Bc.D2-2.B1 to I.Bc.D2-2.B442).
- Table 73e: Compounds of the formula I.Bc in which the combination of (R⁴)ₙ and (R^{L})ₘ corresponds to line D2-3 of Table D2 and the meaning for the combination of R¹, R² and R³ for each individual compound corresponds in each case to one line of Table B (compounds I.Bc.D2-3.B1 to I.Bc.D2-3.B442).
- Table 74e: Compounds of the formula I.Bc in which the combination of (R⁴)ₙ and (R^{L})ₘ corresponds to line D2-4 of Table D2 and the meaning for the combination of R¹, R² and R³ for each individual compound corresponds in each case to one line of Table B (compounds I.Bc.D2-4.B1 to I.Bc.D2-4.B442).
- Table 75e: Compounds of the formula I.Bc in which the combination of (R⁴)ₙ and (R^{L})ₘ corresponds to line D2-5 of Table D2 and the meaning for the combination of R¹, R² and R³ for each individual compound corresponds in each case to one line of Table B (compounds I.Bc.D2-5.B1 to I.Bc.D2-5.B442).
- Table 76e: Compounds of the formula I.Bc in which the combination of (R⁴)ₙ and (R^{L})ₘ corresponds to line D2-6 of Table D2 and the meaning for the combination of R¹, R² and R³ for each individual compound corresponds in each case to one line of Table B (compounds I.Bc.D2-6.B1 to I.Bc.D2-6.B442).
- Table 77e: Compounds of the formula I.Bc in which the combination of (R⁴)ₙ and (R^{L})ₘ corresponds to line D2-7 of Table D2 and the meaning for the combination of R¹, R² and R³ for each individual compound corresponds in each case to one line of Table B (compounds I.Bc.D2-7.B1 to I.Bc.D2-7.B442).
- Table 78e: Compounds of the formula I.Bc in which the combination of (R⁴)ₙ and (R^{L})ₘ corresponds to line D2-8 of Table D2 and the meaning for the combination of R¹, R² and R³ for each individual compound corresponds in each case to one line of Table B (compounds I.Bc.D2-8.B1 to I.Bc.D2-8.B442).
- Table 79e: Compounds of the formula I.Bc in which the combination of (R⁴)ₙ and (R^{L})ₘ corresponds to line D2-9 of Table D2 and the meaning for the combination of R¹, R² and R³ for each individual compound corresponds in each case to one line of Table B (compounds I.Bc.D2-9.B1 to I.Bc.D2-9.B442).
- Table 80e: Compounds of the formula I.Bc in which the combination of (R⁴)ₙ and (R^{L})ₘ corresponds to line D2-10 of Table D2 and the meaning for the combination of R¹, R² and R³ for each individual compound corresponds in each case to one line of Table B (compounds I.Bc.D2-10.B1 to I.Bc.D2-10.B442).
- Table 81e: Compounds of the formula I.Bc in which the combination of (R⁴)ₙ and (R^{L})ₘ corresponds to line D2-11 of Table D2 and the meaning for the combination of R¹, R² and R³ for each individual compound corresponds in each case to one line of Table B (compounds I.Bc.D2-11.B1 to I.Bc.D2-11.B442).
- Table 82e: Compounds of the formula I.Bc in which the combination of (R⁴)ₙ and (R^{L})ₘ corresponds to line D2-12 of Table D2 and the meaning for the combination of R¹, R² and R³ for each individual compound corresponds in each case to one line of Table B (compounds I.Bc.D2-12.B1 to I.Bc.D2-12.B442).
- Table 83e: Compounds of the formula I.Bc in which the combination of (R⁴)ₙ and (R^{L})ₘ corresponds to line D2-13 of Table D2 and the meaning for the combination of R¹, R² and R³ for each individual compound corresponds in each case to one line of Table B (compounds I.Bc.D2-13.B1 to I.Bc.D2-13.B442).
- Table 84e: Compounds of the formula I.Bc in which the combination of (R⁴)ₙ and (R^{L})ₘ corresponds to line D2-14 of Table D2 and the meaning for the combination of R¹, R² and R³ for each individual compound corresponds in each case to one line of Table B (compounds I.Bc.D2-14.B1 to I.Bc.D2-14.B442).
- Table 85e: Compounds of the formula I.Bc in which the combination of (R⁴)ₙ and (R^{L})ₘ corresponds to line D2-15 of Table D2 and the meaning for the combination of R¹, R² and R³ for each individual compound corresponds in each case to one line of Table B (compounds I.Bc.D2-15.B1 to I.Bc.D2-15.B442).
- Table 86e: Compounds of the formula I.Bc in which the combination of (R⁴)ₙ and (R^{L})ₘ corresponds to line D2-16 of Table D2 and the meaning for the combination of R¹, R² and R³ for each individual compound corresponds in each case to one line of Table B (compounds I.Bc.D2-16.B1 to I.Bc.D2-16.B442).
- Table 87e: Compounds of the formula I.Bc in which the combination of (R⁴)ₙ and (R^{L})ₘ corresponds to line D2-17 of Table D2 and the meaning for the combination of R¹, R² and R³ for each individual compound corresponds in each case to one line of Table B (compounds I.Bc.D2-17.B1 to I.Bc.D2-17.B442).
- Table 88e: Compounds of the formula I.Bc in which the combination of (R⁴)ₙ and (R^{L})ₘ corresponds to line D2-18 of Table D2 and the meaning for the combination of R¹, R² and R³ for each individual compound corresponds in each case to one line of Table B (compounds I.Bc.D2-18.B1 to I.Bc.D2-18.B442).
- Table 89e: Compounds of the formula I.Bc in which the combination of (R⁴)ₙ and (R^{L})ₘ corresponds to line D2-19 of Table D2 and the meaning for the combination of R¹, R² and R³ for each individual compound corresponds in each case to one line of Table B (compounds I.Bc.D2-19.B1 to I.Bc.D2-19.B442).
- Table 90e: Compounds of the formula I.Bc in which the combination of (R⁴)ₙ and (R^{L})ₘ corresponds to line D2-20 of Table D2 and the meaning for the combination of R¹, R² and R³ for each individual compound corresponds in each case to one line of Table B (compounds I.Bc.D2-20.B1 to I.Bc.D2-20.B442).
- Table 91e: Compounds of the formula I.Bc in which the combination of (R⁴)ₙ and (R^{L})ₘ corresponds to line D2-21 of Table D2 and the meaning for the combination of R¹, R² and R³ for each individual compound corresponds in each case to one line of Table B (compounds I.Bc.D2-21.B1 to I.Bc.D2-21.B442).
- Table 92e: Compounds of the formula I.Bc in which the combination of (R⁴)ₙ and (R^{L})ₘ corresponds to line D2-22 of Table D2 and the meaning for the combination of R¹, R² and R³ for each individual compound corresponds in each case to one line of Table B (compounds I.Bc.D2-22.B1 to I.Bc.D2-22.B442).
- Table 93e: Compounds of the formula I.Bc in which the combination of (R⁴)ₙ and (R^{L})ₘ corresponds to line D2-23 of Table D2 and the meaning for the combination of R¹, R² and R³ for each individual compound corresponds in each case to one line of Table B (compounds I.Bc.D2-23.B1 to I.Bc.D2-23.B442).
- Table 94e: Compounds of the formula I.Bc in which the combination of (R⁴)ₙ and (R^{L})ₘ corresponds to line D2-24 of Table D2 and the meaning for the combination of R¹, R² and R³ for each individual compound corresponds in each case to one line of Table B (compounds I.Bc.D2-24.B1 to I.Bc.D2-24.B442).
- Table 95e: Compounds of the formula I.Bc in which the combination of (R⁴)ₙ and (R^{L})ₘ corresponds to line D2-25 of Table D2 and the meaning for the combination of R¹, R² and R³ for each individual compound corresponds in each case to one line of Table B (compounds I.Bc.D2-25.B1 to I.Bc.D2-25.B442).
- Table 96e: Compounds of the formula I.Bc in which the combination of (R⁴)ₙ and (R^{L})ₘ corresponds to line D2-26 of Table D2 and the meaning for the combination of R¹, R² and R³ for each individual compound corresponds in each case to one line of Table B (compounds I.Bc.D2-26.B1 to I.Bc.D2-26.B442).
- Table 97e: Compounds of the formula I.Bc in which the combination of (R⁴)ₙ and (R^{L})ₘ corresponds to line D2-27 of Table D2 and the meaning for the combination of R¹, R² and R³ for each individual compound corresponds in each case to one line of Table B (compounds I.Bc.D2-27.B1 to I.Bc.D2-27.B442).
- Table 98e: Compounds of the formula I.Bc in which the combination of (R⁴)ₙ and (R^{L})ₘ corresponds to line D2-28 of Table D2 and the meaning for the combination of R¹, R² and R³ for each individual compound corresponds in each case to one line of Table B (compounds I.Bc.D2-28.B1 to I.Bc.D2-28.B442).
- Table 99e: Compounds of the formula I.Bc in which the combination of (R⁴)ₙ and (R^{L})ₘ corresponds to line D2-29 of Table D2 and the meaning for the combination of R¹, R² and R³ for each individual compound corresponds in each case to one line of Table B (compounds I.Bc.D2-29.B1 to I.Bc.D2-29.B442).
- Table 100e: Compounds of the formula I.Bc in which the combination of (R⁴)ₙ and (R^{L})ₘ corresponds to line D2-30 of Table D2 and the meaning for the combination of R¹, R² and R³ for each individual compound corresponds in each case to one line of Table B (compounds I.Bc.D2-30.B1 to I.Bc.D2-30.B442).
- Table 101e: Compounds of the formula I.Bc in which the combination of (R⁴)ₙ and (R^{L})ₘ corresponds to line D2-31 of Table D2 and the meaning for the combination of R¹, R² and R³ for each individual compound corresponds in each case to one line of Table B (compounds I.Bc.D2-31.B1 to I.Bc.D2-31.B442).
- Table 102e: Compounds of the formula I.Bc in which the combination of (R⁴)ₙ and (R^{L})ₘ corresponds to line D2-32 of Table D2 and the meaning for the combination of R¹, R² and R³ for each individual compound corresponds in each case to one line of Table B (compounds I.Bc.D2-32.B1 to I.Bc.D2-32.B442).
- Table 103e: Compounds of the formula I.Bc in which the combination of (R⁴)ₙ and (R^{L})ₘ corresponds to line D2-33 of Table D2 and the meaning for the combination of R¹, R² and R³ for each individual compound corresponds in each case to one line of Table B (compounds I.Bc.D2-33.B1 to I.Bc.D2-33.B442).
- Table 104e: Compounds of the formula I.Bc in which the combination of (R⁴)ₙ and (R^{L})ₘ corresponds to line D2-34 of Table D2 and the meaning for the combination of R¹, R² and R³ for each individual compound corresponds in each case to one line of Table B (compounds I.Bc.D2-34.B1 to I.Bc.D2-34.B442).
- Table 105e: Compounds of the formula I.Bc in which the combination of (R⁴)ₙ and (R^{L})ₘ corresponds to line D2-35 of Table D2 and the meaning for the combination of R¹, R² and R³ for each individual compound corresponds in each case to one line of Table B (compounds I.Bc.D2-35.B1 to I.Bc.D2-35.B442).
- Table 106e: Compounds of the formula I.Bc in which the combination of (R⁴)ₙ and (R^{L})ₘ corresponds to line D2-36 of Table D2 and the meaning for the combination of R¹, R² and R³ for each individual compound corresponds in each case to one line of Table B (compounds I.Bc.D2-36.B1 to I.Bc.D2-36.B442).
- Table 107e: Compounds of the formula I.Bc in which the combination of (R⁴)ₙ and (R^{L})ₘ corresponds to line D2-37 of Table D2 and the meaning for the combination of R¹, R² and R³ for each individual compound corresponds in each case to one line of Table B (compounds I.Bc.D2-37.B1 to I.Bc.D2-37.B442).
- Table 108e: Compounds of the formula I.Bc in which the combination of (R⁴)ₙ and (R^{L})ₘ corresponds to line D2-38 of Table D2 and the meaning for the combination of R¹, R² and R³ for each individual compound corresponds in each case to one line of Table B (compounds I.Bc.D2-38.B1 to I.Bc.D2-38.B442).
- Table 109e: Compounds of the formula I.Bc in which the combination of (R⁴)ₙ and (R^{L})ₘ corresponds to line D2-39 of Table D2 and the meaning for the combination of R¹, R² and R³ for each individual compound corresponds in each case to one line of Table B (compounds I.Bc.D2-39.B1 to I.Bc.D2-39.B442).
- Table 110e: Compounds of the formula I.Bc in which the combination of (R⁴)ₙ and (R^{L})ₘ corresponds to line D2-40 of Table D2 and the meaning for the combination of R¹, R² and R³ for each individual compound corresponds in each case to one line of Table B (compounds I.Bc.D2-40.B1 to I.Bc.D2-40.B442).
- Table 111e: Compounds of the formula I.Bc in which the combination of (R⁴)ₙ and (R^{L})ₘ corresponds to line D2-41 of Table D2 and the meaning for the combination of R¹, R² and R³ for each individual compound corresponds in each case to one line of Table B (compounds I.Bc.D2-41.B1 to I.Bc.D2-41.B442).
- Table 112e: Compounds of the formula I.Bc in which the combination of (R⁴)ₙ and (R^{L})ₘ corresponds to line D2-42 of Table D2 and the meaning for the combination of R¹, R² and R³ for each individual compound corresponds in each case to one line of Table B (compounds I.Bc.D2-42.B1 to I.Bc.D2-42.B442).
- Table 113e: Compounds of the formula I.Bc in which the combination of (R⁴)ₙ and (R^{L})ₘ corresponds to line D2-43 of Table D2 and the meaning for the combination of R¹, R² and R³ for each individual compound corresponds in each case to one line of Table B (compounds I.Bc.D2-43.B1 to I.Bc.D2-43.B442).
- Table 114e: Compounds of the formula I.Bc in which the combination of (R⁴)ₙ and (R^{L})ₘ corresponds to line D2-44 of Table D2 and the meaning for the combination of R¹, R² and R³ for each individual compound corresponds in each case to one line of Table B (compounds I.Bc.D2-44.B1 to I.Bc.D2-44.B442).
- Table 115e: Compounds of the formula I.Bc in which the combination of (R⁴)ₙ and (R^{L})ₘ corresponds to line D2-45 of Table D2 and the meaning for the combination of R¹, R² and R³ for each individual compound corresponds in each case to one line of Table B (compounds I.Bc.D2-45.B1 to I.Bc.D2-45.B442).
- Table 116e: Compounds of the formula I.Bc in which the combination of (R⁴)ₙ and (R^{L})ₘ corresponds to line D2-46 of Table D2 and the meaning for the combination of R¹, R² and R³ for each individual compound corresponds in each case to one line of Table B (compounds I.Bc.D2-46.B1 to I.Bc.D2-46.B442).
- Table 117e: Compounds of the formula I.Bc in which the combination of (R⁴)ₙ and (R^{L})ₘ corresponds to line D2-47 of Table D2 and the meaning for the combination of R¹, R² and R³ for each individual compound corresponds in each case to one line of Table B (compounds I.Bc.D2-47.B1 to I.Bc.D2-47.B442).
- Table 118e: Compounds of the formula I.Bc in which the combination of (R⁴)ₙ and (R^{L})ₘ corresponds to line D2-48 of Table D2 and the meaning for the combination of R¹, R² and R³ for each individual compound corresponds in each case to one line of Table B (compounds I.Bc.D2-48.B1 to I.Bc.D2-48.B442).
- Table 119e: Compounds of the formula I.Bc in which the combination of (R⁴)ₙ and (R^{L})ₘ corresponds to line D2-49 of Table D2 and the meaning for the combination of R¹, R² and R³ for each individual compound corresponds in each case to one line of Table B (compounds I.Bc.D2-49.B1 to I.Bc.D2-49.B442).
- Table 120e: Compounds of the formula I.Bc in which the combination of (R⁴)ₙ and (R^{L})ₘ corresponds to line D2-50 of Table D2 and the meaning for the combination of R¹, R² and R³ for each individual compound corresponds in each case to one line of Table B (compounds I.Bc.D2-50.B1 to I.Bc.D2-50.B442).
- Table 121e: Compounds of the formula I.Bc in which the combination of (R⁴)ₙ and (R^{L})ₘ corresponds to line D2-51 of Table D2 and the meaning for the combination of R¹, R² and R³ for each individual compound corresponds in each case to one line of Table B (compounds I.Bc.D2-51.B1 to I.Bc.D2-51.B442).
- Table 122e: Compounds of the formula I.Bc in which the combination of (R⁴)ₙ and (R^{L})ₘ corresponds to line D2-52 of Table D2 and the meaning for the combination of R¹, R² and R³ for each individual compound corresponds in each case to one line of Table B (compounds I.Bc.D2-52.B1 to I.Bc.D2-52.B442).
- Table 123e: Compounds of the formula I.Bc in which the combination of (R⁴)ₙ and (R^{L})ₘ corresponds to line D2-53 of Table D2 and the meaning for the combination of R¹, R² and R³ for each individual compound corresponds in each case to one line of Table B (compounds I.Bc.D2-53.B1 to I.Bc.D2-53.B442).
- Table 124e: Compounds of the formula I.Bc in which the combination of (R⁴)ₙ and (R^{L})ₘ corresponds to line D2-54 of Table D2 and the meaning for the combination of R¹, R² and R³ for each individual compound corresponds in each case to one line of Table B (compounds I.Bc.D2-54.B1 to I.Bc.D2-54.B442).
- Table 125e: Compounds of the formula I.Bc in which the combination of (R⁴)ₙ and (R^{L})ₘ corresponds to line D2-55 of Table D2 and the meaning for the combination of R¹, R² and R³ for each individual compound corresponds in each case to one line of Table B (compounds I.Bc.D2-55.B1 to I.Bc.D2-55.B442).
- Table 126e: Compounds of the formula I.Bc in which the combination of (R⁴)ₙ and (R^{L})ₘ corresponds to line D2-56 of Table D2 and the meaning for the combination of R¹, R² and R³ for each individual compound corresponds in each case to one line of Table B (compounds I.Bc.D2-56.B1 to I.Bc.D2-56.B442).
- Table 127e: Compounds of the formula I.Bc in which the combination of (R⁴)ₙ and (R^{L})ₘ corresponds to line D2-57 of Table D2 and the meaning for the combination of R¹, R² and R³ for each individual compound corresponds in each case to one line of Table B (compounds I.Bc.D2-57.B1 to I.Bc.D2-57.B442).
- Table 128e: Compounds of the formula I.Bc in which the combination of (R⁴)ₙ and (R^{L})ₘ corresponds to line D2-58 of Table D2 and the meaning for the combination of R¹, R² and R³ for each individual compound corresponds in each case to one line of Table B (compounds I.Bc.D2-58.B1 to I.Bc.D2-58.B442).
- Table 129e: Compounds of the formula I.Bc in which the combination of (R⁴)ₙ and (R^{L})ₘ corresponds to line D2-59 of Table D2 and the meaning for the combination of R¹, R² and R³ for each individual compound corresponds in each case to one line of Table B (compounds I.Bc.D2-59.B1 to I.Bc.D2-59.B442).
- Table 130e: Compounds of the formula I.Bc in which the combination of (R⁴)ₙ and (R^{L})ₘ corresponds to line D2-60 of Table D2 and the meaning for the combination of R¹, R² and R³ for each individual compound corresponds in each case to one line of Table B (compounds I.Bc.D2-60.B1 to I.Bc.D2-60.B442).
- Table 131e: Compounds of the formula I.Bc in which the combination of (R⁴)ₙ and (R^{L})ₘ corresponds to line D2-61 of Table D2 and the meaning for the combination of R¹, R² and R³ for each individual compound corresponds in each case to one line of Table B (compounds I.Bc.D2-61.B1 to I.Bc.D2-61.B442).
- Table 132e: Compounds of the formula I.Bc in which the combination of (R⁴)ₙ and (R^{L})ₘ corresponds to line D2-62 of Table D2 and the meaning for the combination of R¹, R² and R³ for each individual compound corresponds in each case to one line of Table B (compounds I.Bc.D2-62.B1 to I.Bc.D2-62.B442).
- Table 133e: Compounds of the formula I.Bc in which the combination of (R⁴)ₙ and (R^{L})ₘ corresponds to line D2-63 of Table D2 and the meaning for the combination of R¹, R² and R³ for each individual compound corresponds in each case to one line of Table B (compounds I.Bc.D2-63.B1 to I.Bc.D2-63.B442).
- Table 134e: Compounds of the formula I.Bc in which the combination of (R⁴)ₙ and (R^{L})ₘ corresponds to line D2-64 of Table D2 and the meaning for the combination of R¹, R² and R³ for each individual compound corresponds in each case to one line of Table B (compounds I.Bc.D2-64.B1 to I.Bc.D2-64.B442).
- Table 135e: Compounds of the formula I.Bc in which the combination of (R⁴)ₙ and (R^{L})ₘ corresponds to line D2-65 of Table D2 and the meaning for the combination of R¹, R² and R³ for each individual compound corresponds in each case to one line of Table B (compounds I.Bc.D2-65.B1 to I.Bc.D2-65.B442).
- Table 136e: Compounds of the formula I.Bc in which the combination of (R⁴)ₙ and (R^{L})ₘ corresponds to line D2-66 of Table D2 and the meaning for the combination of R¹, R² and R³ for each individual compound corresponds in each case to one line of Table B (compounds I.Bc.D2-66.B1 to I.Bc.D2-66.B442).
- Table 137e: Compounds of the formula I.Bc in which the combination of (R⁴)ₙ and (R^{L})ₘ corresponds to line D2-67 of Table D2 and the meaning for the combination of R¹, R² and R³ for each individual compound corresponds in each case to one line of Table B (compounds I.Bc.D2-67.B1 to I.Bc.D2-67.B442).
- Table 138e: Compounds of the formula I.Bc in which the combination of (R⁴)ₙ and (R^{L})ₘ corresponds to line D2-68 of Table D2 and the meaning for the combination of R¹, R² and R³ for each individual compound corresponds in each case to one line of Table B (compounds I.Bc.D2-68.B1 to I.Bc.D2-68.B442).
- Table 139e: Compounds of the formula I.Bc in which the combination of (R⁴)ₙ and (R^{L})ₘ corresponds to line D2-69 of Table D2 and the meaning for the combination of R¹, R² and R³ for each individual compound corresponds in each case to one line of Table B (compounds I.Bc.D2-69.B1 to I.Bc.D2-69.B442).
- Table 140e: Compounds of the formula I.Bc in which the combination of (R⁴)ₙ and (R^{L})ₘ corresponds to line D2-70 of Table D2 and the meaning for the combination of R¹, R² and R³ for each individual compound corresponds in each case to one line of Table B (compounds I.Bc.D2-70.B1 to I.Bc.D2-70.B442).
- Table 1f: Compounds of the formula I.Bd in which the combination of (R⁴)ₙ and (R^{L})ₘ corresponds to line D1-1 of Table D1 and the meaning for the combination of R¹, R² and R³ for each individual compound corresponds in each case to one line of Table B (compounds I.Bd.D1-1.B1 to I.Bd.D1.B442).
- Table 2f: Compounds of the formula I.Bd in which the combination of (R⁴)ₙ and (R^{L})ₘ corresponds to line D1-2 of Table D1 and the meaning for the combination of R¹, R² and R³ for each individual compound corresponds in each case to one line of Table B (compounds I.Bd.D1-2.B1 to I.Bd.D1-2.B442).
- Table 3f: Compounds of the formula I.Bd in which the combination of (R⁴)ₙ and (R^{L})ₘ corresponds to line D1-3 of Table D1 and the meaning for the combination of R¹, R² and R³ for each individual compound corresponds in each case to one line of Table B (compounds I.Bd.D1-3.B1 to I.Bd.D1-3.B442).
- Table 4f: Compounds of the formula I.Bd in which the combination of (R⁴)ₙ and (R^{L})ₘ corresponds to line D1-4 of Table D1 and the meaning for the combination of R¹, R² and R³ for each individual compound corresponds in each case to one line of Table B (compounds I.Bd.D1-4.B1 to I.Bd.D1-4.B442).
- Table 5f: Compounds of the formula I.Bd in which the combination of (R⁴)ₙ and (R^{L})ₘ corresponds to line D1-5 of Table D1 and the meaning for the combination of R¹, R² and R³ for each individual compound corresponds in each case to one line of Table B (compounds I.Bd.D1-5.B1 to I.Bd.D1-5.B442).
- Table 6f: Compounds of the formula I.Bd in which the combination of (R⁴)ₙ and (R^{L})ₘ corresponds to line D1-6 of Table D1 and the meaning for the combination of R¹, R² and R³ for each individual compound corresponds in each case to one line of Table B (compounds I.Bd.D1-6.B1 to I.Bd.D1-6.B442).
- Table 7f: Compounds of the formula I.Bd in which the combination of (R⁴)ₙ and (R^{L})ₘ corresponds to line D1-7 of Table D1 and the meaning for the combination of R¹, R² and R³ for each individual compound corresponds in each case to one line of Table B (compounds I.Bd.D1-7.B1 to I.Bd.D1-7.B442).
- Table 8f: Compounds of the formula I.Bd in which the combination of (R⁴)ₙ and (R^{L})ₘ corresponds to line D1-8 of Table D1 and the meaning for the combination of R¹, R² and R³ for each individual compound corresponds in each case to one line of Table B (compounds I.Bd.D1-8.B1 to I.Bd.D1-8.B442).
- Table 9f: Compounds of the formula I.Bd in which the combination of (R⁴)ₙ and (R^{L})ₘ corresponds to line D1-9 of Table D1 and the meaning for the combination of R¹, R² and R³ for each individual compound corresponds in each case to one line of Table B (compounds I.Bd.D1-9.B1 to I.Bd.D1-9.B442).
- Table 10f: Compounds of the formula I.Bd in which the combination of (R⁴)ₙ and (R^{L})ₘ corresponds to line D1-10 of Table D1 and the meaning for the combination of R¹, R² and R³ for each individual compound corresponds in each case to one line of Table B (compounds I.Bd.D1-10.B1 to I.Bd.D1-10.B442).
- Table 11f: Compounds of the formula I.Bd in which the combination of (R⁴)ₙ and (R^{L})ₘ corresponds to line D1-11 of Table D1 and the meaning for the combination of R¹, R² and R³ for each individual compound corresponds in each case to one line of Table B (compounds I.Bd.D1-11.B1 to I.Bd.D1-11.B442).
- Table 12f: Compounds of the formula I.Bd in which the combination of (R⁴)ₙ and (R^{L})ₘ corresponds to line D1-12 of Table D1 and the meaning for the combination of R¹, R² and R³ for each individual compound corresponds in each case to one line of Table B (compounds I.Bd.D1-12.B1 to I.Bd.D1-12.B442).
- Table 13f: Compounds of the formula I.Bd in which the combination of (R⁴)ₙ and (R^{L})ₘ corresponds to line D1-13 of Table D1 and the meaning for the combination of R¹, R² and R³ for each individual compound corresponds in each case to one line of Table B (compounds I.Bd.D1-13.B1 to I.Bd.D1-13.B442).
- Table 14f: Compounds of the formula I.Bd in which the combination of (R⁴)ₙ and (R^{L})ₘ corresponds to line D1-14 of Table D1 and the meaning for the combination of R¹, R² and R³ for each individual compound corresponds in each case to one line of Table B (compounds I.Bd.D1-14.B1 to I.Bd.D1-14.B442).
- Table 15f: Compounds of the formula I.Bd in which the combination of (R⁴)ₙ and (R^{L})ₘ corresponds to line D1-15 of Table D1 and the meaning for the combination of R¹, R² and R³ for each individual compound corresponds in each case to one line of Table B (compounds I.Bd.D1-15.B1 to I.Bd.D1-15.B442).
- Table 16f: Compounds of the formula I.Bd in which the combination of (R⁴)ₙ and (R^{L})ₘ corresponds to line D1-16 of Table D1 and the meaning for the combination of R¹, R² and R³ for each individual compound corresponds in each case to one line of Table B (compounds I.Bd.D1-16.B1 to I.Bd.D1-16.B442).
- Table 17f: Compounds of the formula I.Bd in which the combination of (R⁴)ₙ and (R^{L})ₘ corresponds to line D1-17 of Table D1 and the meaning for the combination of R¹, R² and R³ for each individual compound corresponds in each case to one line of Table B (compounds I.Bd.D1-17.B1 to I.Bd.D1-17.B442).
- Table 18f: Compounds of the formula I.Bd in which the combination of (R⁴)ₙ and (R^{L})ₘ corresponds to line D1-18 of Table D1 and the meaning for the combination of R¹, R² and R³ for each individual compound corresponds in each case to one line of Table B (compounds I.Bd.D1-18.B1 to I.Bd.D1-18.B442).
- Table 19f: Compounds of the formula I.Bd in which the combination of (R⁴)ₙ and (R^{L})ₘ corresponds to line D1-19 of Table D1 and the meaning for the combination of R¹, R² and R³ for each individual compound corresponds in each case to one line of Table B (compounds I.Bd.D1-19.B1 to I.Bd.D1-19.B442).
- Table 20f: Compounds of the formula I.Bd in which the combination of (R⁴)ₙ and (R^{L})ₘ corresponds to line D1-20 of Table D1 and the meaning for the combination of R¹, R² and R³ for each individual compound corresponds in each case to one line of Table B (compounds I.Bd.D1-20.B1 to I.Bd.D1-20.B442).
- Table 21f: Compounds of the formula I.Bd in which the combination of (R⁴)ₙ and (R^{L})ₘ corresponds to line D1-21 of Table D1 and the meaning for the combination of R¹, R² and R³ for each individual compound corresponds in each case to one line of Table B (compounds I.Bd.D1-21.B1 to I.Bd.D1-21.B442).
- Table 22f: Compounds of the formula I.Bd in which the combination of (R⁴)ₙ and (R^{L})ₘ corresponds to line D1-22 of Table D1 and the meaning for the combination of R¹, R² and R³ for each individual compound corresponds in each case to one line of Table B (compounds I.Bd.D1-22.B1 to I.Bd.D1-22.B442).
- Table 23f: Compounds of the formula I.Bd in which the combination of (R⁴)ₙ and (R^{L})ₘ corresponds to line D1-23 of Table D1 and the meaning for the combination of R¹, R² and R³ for each individual compound corresponds in each case to one line of Table B (compounds I.Bd.D1-23.B1 to I.Bd.D1-23.B442).
- Table 24f: Compounds of the formula I.Bd in which the combination of (R⁴)ₙ and (R^{L})ₘ corresponds to line D1-24 of Table D1 and the meaning for the combination of R¹, R² and R³ for each individual compound corresponds in each case to one line of Table B (compounds I.Bd.D1-24.B1 to I.Bd.D1-24.B442).
- Table 25f: Compounds of the formula I.Bd in which the combination of (R⁴)ₙ and (R^{L})ₘ corresponds to line D1-25 of Table D1 and the meaning for the combination of R¹, R² and R³ for each individual compound corresponds in each case to one line of Table B (compounds I.Bd.D1-25.B1 to I.Bd.D1-25.B442).
- Table 26f: Compounds of the formula I.Bd in which the combination of (R⁴)ₙ and (R^{L})ₘ corresponds to line D1-26 of Table D1 and the meaning for the combination of R¹, R² and R³ for each individual compound corresponds in each case to one line of Table B (compounds I.Bd.D1-26.B1 to I.Bd.D1-26.B442).
- Table 27f: Compounds of the formula I.Bd in which the combination of (R⁴)ₙ and (R^{L})ₘ corresponds to line D1-27 of Table D1 and the meaning for the combination of R¹, R² and R³ for each individual compound corresponds in each case to one line of Table B (compounds I.Bd.D1-27.B1 to I.Bd.D1-27.B442).
- Table 28f: Compounds of the formula I.Bd in which the combination of (R⁴)ₙ and (R^{L})ₘ corresponds to line D1-28 of Table D1 and the meaning for the combination of R¹, R² and R³ for each individual compound corresponds in each case to one line of Table B (compounds I.Bd.D1-28.B1 to I.Bd.D1-28.B442).
- Table 29f: Compounds of the formula I.Bd in which the combination of (R⁴)ₙ and (R^{L})ₘ corresponds to line D1-29 of Table D1 and the meaning for the combination of R¹, R² and R³ for each individual compound corresponds in each case to one line of Table B (compounds I.Bd.D1-29.B1 to I.Bd.D1-29.B442).
- Table 30f: Compounds of the formula I.Bd in which the combination of (R⁴)ₙ and (R^{L})ₘ corresponds to line D1-30 of Table D1 and the meaning for the combination of R¹, R² and R³ for each individual compound corresponds in each case to one line of Table B (compounds I.Bd.D1-30.B1 to I.Bd.D1-30.B442).
- Table 31f: Compounds of the formula I.Bd in which the combination of (R⁴)ₙ and (R^{L})ₘ corresponds to line D1-31 of Table D1 and the meaning for the combination of R¹, R² and R³ for each individual compound corresponds in each case to one line of Table B (compounds I.Bd.D1-31.B1 to I.Bd.D1-31.B442).
- Table 32f: Compounds of the formula I.Bd in which the combination of (R⁴)ₙ and (R^{L})ₘ corresponds to line D1-32 of Table D1 and the meaning for the combination of R¹, R² and R³ for each individual compound corresponds in each case to one line of Table B (compounds I.Bd.D1-32.B1 to I.Bd.D1-32.B442).
- Table 33f: Compounds of the formula I.Bd in which the combination of (R⁴)ₙ and (R^{L})ₘ corresponds to line D1-33 of Table D1 and the meaning for the combination of R¹, R² and R³ for each individual compound corresponds in each case to one line of Table B (compounds I.Bd.D1-33.B1 to I.Bd.D1-33.B442).
- Table 34f: Compounds of the formula I.Bd in which the combination of (R⁴)ₙ and (R^{L})ₘ corresponds to line D1-34 of Table D1 and the meaning for the combination of R¹, R² and R³ for each individual compound corresponds in each case to one line of Table B (compounds I.Bd.D1-34.B1 to I.Bd.D1-34.B442).
- Table 35f: Compounds of the formula I.Bd in which the combination of (R⁴)ₙ and (R^{L})ₘ corresponds to line D1-35 of Table D1 and the meaning for the combination of R¹, R² and R³ for each individual compound corresponds in each case to one line of Table B (compounds I.Bd.D1-35.B1 to I.Bd.D1-35.B442).
- Table 36f: Compounds of the formula I.Bd in which the combination of (R⁴)ₙ and (R^{L})ₘ corresponds to line D1-36 of Table D1 and the meaning for the combination of R¹, R² and R³ for each individual compound corresponds in each case to one line of Table B (compounds I.Bd.D1-36.B1 to I.Bd.D1-36.B442).
- Table 37f: Compounds of the formula I.Bd in which the combination of (R⁴)ₙ and (R^{L})ₘ corresponds to line D1-37 of Table D1 and the meaning for the combination of R¹, R² and R³ for each individual compound corresponds in each case to one line of Table B (compounds I.Bd.D1-37.B1 to I.Bd.D1-37.B442).
- Table 38f: Compounds of the formula I.Bd in which the combination of (R⁴)ₙ and (R^{L})ₘ corresponds to line D1-38 of Table D1 and the meaning for the combination of R¹, R² and R³ for each individual compound corresponds in each case to one line of Table B (compounds I.Bd.D1-38.B1 to I.Bd.D1-38.B442).
- Table 39f: Compounds of the formula I.Bd in which the combination of (R⁴)ₙ and (R^{L})ₘ corresponds to line D1-39 of Table D1 and the meaning for the combination of R¹, R² and R³ for each individual compound corresponds in each case to one line of Table B (compounds I.Bd.D1-39.B1 to I.Bd.D1-39.B442).
- Table 40f: Compounds of the formula I.Bd in which the combination of (R⁴)ₙ and (R^{L})ₘ corresponds to line D1-40 of Table D1 and the meaning for the combination of R¹, R² and R³ for each individual compound corresponds in each case to one line of Table B (compounds I.Bd.D1-40.B1 to I.Bd.D1-40.B442).
- Table 41f: Compounds of the formula I.Bd in which the combination of (R⁴)ₙ and (R^{L})ₘ corresponds to line D1-41 of Table D1 and the meaning for the combination of R¹, R² and R³ for each individual compound corresponds in each case to one line of Table B (compounds I.Bd.D1-41.B1 to I.Bd.D1-41.B442).
- Table 42f: Compounds of the formula I.Bd in which the combination of (R⁴)ₙ and (R^{L})ₘ corresponds to line D1-42 of Table D1 and the meaning for the combination of R¹, R² and R³ for each individual compound corresponds in each case to one line of Table B (compounds I.Bd.D1-42.B1 to I.Bd.D1-42.B442).
- Table 43f: Compounds of the formula I.Bd in which the combination of (R⁴)ₙ and (R^{L})ₘ corresponds to line D1-43 of Table D1 and the meaning for the combination of R¹, R² and R³ for each individual compound corresponds in each case to one line of Table B (compounds I.Bd.D1-43.B1 to I.Bd.D1-43.B442).
- Table 44f: Compounds of the formula I.Bd in which the combination of (R⁴)ₙ and (R^{L})ₘ corresponds to line D1-44 of Table D1 and the meaning for the combination of R¹, R² and R³ for each individual compound corresponds in each case to one line of Table B (compounds I.Bd.D1-44.B1 to I.Bd.D1-44.B442).
- Table 45f: Compounds of the formula I.Bd in which the combination of (R⁴)ₙ and (R^{L})ₘ corresponds to line D1-45 of Table D1 and the meaning for the combination of R¹, R² and R³ for each individual compound corresponds in each case to one line of Table B (compounds I.Bd.D1-45.B1 to I.Bd.D1-45.B442).
- Table 46f: Compounds of the formula I.Bd in which the combination of (R⁴)ₙ and (R^{L})ₘ corresponds to line D1-46 of Table D1 and the meaning for the combination of R¹, R² and R³ for each individual compound corresponds in each case to one line of Table B (compounds I.Bd.D1-46.B1 to I.Bd.D1-46.B442).
- Table 47f: Compounds of the formula I.Bd in which the combination of (R⁴)ₙ and (R^{L})ₘ corresponds to line D1-47 of Table D1 and the meaning for the combination of R¹, R² and R³ for each individual compound corresponds in each case to one line of Table B (compounds I.Bd.D1-47.B1 to I.Bd.D1-47.B442).
- Table 48f: Compounds of the formula I.Bd in which the combination of (R⁴)ₙ and (R^{L})ₘ corresponds to line D1-48 of Table D1 and the meaning for the combination of R¹, R² and R³ for each individual compound corresponds in each case to one line of Table B (compounds I.Bd.D1-48.B1 to I.Bd.D1-48.B442).
- Table 49f: Compounds of the formula I.Bd in which the combination of (R⁴)ₙ and (R^{L})ₘ corresponds to line D1-49 of Table D1 and the meaning for the combination of R¹, R² and R³ for each individual compound corresponds in each case to one line of Table B (compounds I.Bd.D1-49.B1 to I.Bd.D1-49.B442).
- Table 50f: Compounds of the formula I.Bd in which the combination of (R⁴)ₙ and (R^{L})ₘ corresponds to line D1-50 of Table D1 and the meaning for the combination of R¹, R² and R³ for each individual compound corresponds in each case to one line of Table B (compounds I.Bd.D1-50.B1 to I.Bd.D1-50.B442).
- Table 51f: Compounds of the formula I.Bd in which the combination of (R⁴)ₙ and (R^{L})ₘ corresponds to line D1-51 of Table D1 and the meaning for the combination of R¹, R² and R³ for each individual compound corresponds in each case to one line of Table B (compounds I.Bd.D1-51.B1 to I.Bd.D1-51.B442).
- Table 52f: Compounds of the formula I.Bd in which the combination of (R⁴)ₙ and (R^{L})ₘ corresponds to line D1-52 of Table D1 and the meaning for the combination of R¹, R² and R³ for each individual compound corresponds in each case to one line of Table B (compounds I.Bd.D1-52.B1 to I.Bd.D1-52.B442).
- Table 53f: Compounds of the formula I.Bd in which the combination of (R⁴)ₙ and (R^{L})ₘ corresponds to line D1-53 of Table D1 and the meaning for the combination of R¹, R² and R³ for each individual compound corresponds in each case to one line of Table B (compounds I.Bd.D1-53.B1 to I.Bd.D1-53.B442).
- Table 54f: Compounds of the formula I.Bd in which the combination of (R⁴)ₙ and (R^{L})ₘ corresponds to line D1-54 of Table D1 and the meaning for the combination of R¹, R² and R³ for each individual compound corresponds in each case to one line of Table B (compounds I.Bd.D1-54.B1 to I.Bd.D1-54.B442).
- Table 55f: Compounds of the formula I.Bd in which the combination of (R⁴)ₙ and (R^{L})ₘ corresponds to line D1-55 of Table D1 and the meaning for the combination of R¹, R² and R³ for each individual compound corresponds in each case to one line of Table B (compounds I.Bd.D1-55.B1 to I.Bd.D1-55.B442).
- Table 56f: Compounds of the formula I.Bd in which the combination of (R⁴)ₙ and (R^{L})ₘ corresponds to line D1-56 of Table D1 and the meaning for the combination of R¹, R² and R³ for each individual compound corresponds in each case to one line of Table B (compounds I.Bd.D1-56.B1 to I.Bd.D1-56.B442).
- Table 57f: Compounds of the formula I.Bd in which the combination of (R⁴)ₙ and (R^{L})ₘ corresponds to line D1-57 of Table D1 and the meaning for the combination of R¹, R² and R³ for each individual compound corresponds in each case to one line of Table B (compounds I.Bd.D1-57.B1 to I.Bd.D1-57.B442).
- Table 58f: Compounds of the formula I.Bd in which the combination of (R⁴)ₙ and (R^{L})ₘ corresponds to line D1-58 of Table D1 and the meaning for the combination of R¹, R² and R³ for each individual compound corresponds in each case to one line of Table B (compounds I.Bd.D1-58.B1 to I.Bd.D1-58.B442).
- Table 59f: Compounds of the formula I.Bd in which the combination of (R⁴)ₙ and (R^{L})ₘ corresponds to line D1-59 of Table D1 and the meaning for the combination of R¹, R² and R³ for each individual compound corresponds in each case to one line of Table B (compounds I.Bd.D1-59.B1 to I.Bd.D1-59.B442).
- Table 60f: Compounds of the formula I.Bd in which the combination of (R⁴)ₙ and (R^{L})ₘ corresponds to line D1-60 of Table D1 and the meaning for the combination of R¹, R² and R³ for each individual compound corresponds in each case to one line of Table B (compounds I.Bd.D1-60.B1 to I.Bd.D1-60.B442).
- Table 61f: Compounds of the formula I.Bd in which the combination of (R⁴)ₙ and (R^{L})ₘ corresponds to line D1-61 of Table D1 and the meaning for the combination of R¹, R² and R³ for each individual compound corresponds in each case to one line of Table B (compounds I.Bd.D1-61.B1 to I.Bd.D1-61.B442).
- Table 62f: Compounds of the formula I.Bd in which the combination of (R⁴)ₙ and (R^{L})ₘ corresponds to line D1-62 of Table D1 and the meaning for the combination of R¹, R² and R³ for each individual compound corresponds in each case to one line of Table B (compounds I.Bd.D1-62.B1 to I.Bd.D1-62.B442).
- Table 63f: Compounds of the formula I.Bd in which the combination of (R⁴)ₙ and (R^{L})ₘ corresponds to line D1-63 of Table D1 and the meaning for the combination of R¹, R² and R³ for each individual compound corresponds in each case to one line of Table B (compounds I.Bd.D1-63.B1 to I.Bd.D1-63.B442).
- Table 64f: Compounds of the formula I.Bd in which the combination of (R⁴)ₙ and (R^{L})ₘ corresponds to line D1-64 of Table D1 and the meaning for the combination of R¹, R² and R³ for each individual compound corresponds in each case to one line of Table B (compounds I.Bd.D1-64.B1 to I.Bd.D1-64.B442).
- Table 65f: Compounds of the formula I.Bd in which the combination of (R⁴)ₙ and (R^{L})ₘ corresponds to line D1-65 of Table D1 and the meaning for the combination of R¹, R² and R³ for each individual compound corresponds in each case to one line of Table B (compounds I.Bd.D1-65.B1 to I.Bd.D1-65.B442).
- Table 66f: Compounds of the formula I.Bd in which the combination of (R⁴)ₙ and (R^{L})ₘ corresponds to line D1-66 of Table D1 and the meaning for the combination of R¹, R² and R³ for each individual compound corresponds in each case to one line of Table B (compounds I.Bd.D1-66.B1 to I.Bd.D1-66.B442).
- Table 67f: Compounds of the formula I.Bd in which the combination of (R⁴)ₙ and (R^{L})ₘ corresponds to line D1-67 of Table D1 and the meaning for the combination of R¹, R² and R³ for each individual compound corresponds in each case to one line of Table B (compounds I.Bd.D1-67.B1 to I.Bd.D1-67.B442).
- Table 68f: Compounds of the formula I.Bd in which the combination of (R⁴)ₙ and (R^{L})ₘ corresponds to line D1-68 of Table D1 and the meaning for the combination of R¹, R² and R³ for each individual compound corresponds in each case to one line of Table B (compounds I.Bd.D1-68.B1 to I.Bd.D1-68.B442).
- Table 69f: Compounds of the formula I.Bd in which the combination of (R⁴)ₙ and (R^{L})ₘ corresponds to line D1-69 of Table D1 and the meaning for the combination of R¹, R² and R³ for each individual compound corresponds in each case to one line of Table B (compounds I.Bd.D1-69.B1 to I.Bd.D1-69.B442).
- Table 70f: Compounds of the formula I.Bd in which the combination of (R⁴)ₙ and (R^{L})ₘ corresponds to line D1-70 of Table D1 and the meaning for the combination of R¹, R² and R³ for each individual compound corresponds in each case to one line of Table B (compounds I.Bd.D1-70.B1 to I.Bd.D1-70.B442).
- Table 71f: Compounds of the formula I.Bd in which the combination of (R⁴)ₙ and (R^{L})ₘ corresponds to line D2-1 of Table D2 and the meaning for the combination of R¹, R² and R³ for each individual compound corresponds in each case to one line of Table B (compounds I.Bd.D2-1.B1 to I.Bd.D2-1.B442).
- Table 72f: Compounds of the formula I.Bd in which the combination of (R⁴)ₙ and (R^{L})ₘ corresponds to line D2-2 of Table D2 and the meaning for the combination of R¹, R² and R³ for each individual compound corresponds in each case to one line of Table B (compounds I.Bd.D2-2.B1 to I.Bd.D2-2.B442).
- Table 73f: Compounds of the formula I.Bd in which the combination of (R⁴)ₙ and (R^{L})ₘ corresponds to line D2-3 of Table D2 and the meaning for the combination of R¹, R² and R³ for each individual compound corresponds in each case to one line of Table B (compounds I.Bd.D2-3.B1 to I.Bd.D2-3.B442).
- Table 74f: Compounds of the formula I.Bd in which the combination of (R⁴)ₙ and (R^{L})ₘ corresponds to line D2-4 of Table D2 and the meaning for the combination of R¹, R² and R³ for each individual compound corresponds in each case to one line of Table B (compounds I.Bd.D2-4.B1 to I.Bd.D2-4.B442).
- Table 75f: Compounds of the formula I.Bd in which the combination of (R⁴)ₙ and (R^{L})ₘ corresponds to line D2-5 of Table D2 and the meaning for the combination of R¹, R² and R³ for each individual compound corresponds in each case to one line of Table B (compounds I.Bd.D2-5.B1 to I.Bd.D2-5.B442).
- Table 76f: Compounds of the formula I.Bd in which the combination of (R⁴)ₙ and (R^{L})ₘ corresponds to line D2-6 of Table D2 and the meaning for the combination of R¹, R² and R³ for each individual compound corresponds in each case to one line of Table B (compounds I.Bd.D2-6.B1 to I.Bd.D2-6.B442).
- Table 77f: Compounds of the formula I.Bd in which the combination of (R⁴)ₙ and (R^{L})ₘ corresponds to line D2-7 of Table D2 and the meaning for the combination of R¹, R² and R³ for each individual compound corresponds in each case to one line of Table B (compounds I.Bd.D2-7.B1 to I.Bd.D2-7.B442).
- Table 78f: Compounds of the formula I.Bd in which the combination of (R⁴)ₙ and (R^{L})ₘ corresponds to line D2-8 of Table D2 and the meaning for the combination of R¹, R² and R³ for each individual compound corresponds in each case to one line of Table B (compounds I.Bd.D2-8.B1 to I.Bd.D2-8.B442).
- Table 79f: Compounds of the formula I.Bd in which the combination of (R⁴)ₙ and (R^{L})ₘ corresponds to line D2-9 of Table D2 and the meaning for the combination of R¹, R² and R³ for each individual compound corresponds in each case to one line of Table B (compounds I.Bd.D2-9.B1 to I.Bd.D2-9.B442).
- Table 80f: Compounds of the formula I.Bd in which the combination of (R⁴)ₙ and (R^{L})ₘ corresponds to line D2-10 of Table D2 and the meaning for the combination of R¹, R² and R³ for each individual compound corresponds in each case to one line of Table B (compounds I.Bd.D2-10.B1 to I.Bd.D2-10.B442).
- Table 81f: Compounds of the formula I.Bd in which the combination of (R⁴)ₙ and (R^{L})ₘ corresponds to line D2-11 of Table D2 and the meaning for the combination of R¹, R² and R³ for each individual compound corresponds in each case to one line of Table B (compounds I.Bd.D2-11.B1 to I.Bd.D2-11.B442).
- Table 82f: Compounds of the formula I.Bd in which the combination of (R⁴)ₙ and (R^{L})ₘ corresponds to line D2-12 of Table D2 and the meaning for the combination of R¹, R² and R³ for each individual compound corresponds in each case to one line of Table B (compounds I.Bd.D2-12.B1 to I.Bd.D2-12.B442).
- Table 83f: Compounds of the formula I.Bd in which the combination of (R⁴)ₙ and (R^{L})ₘ corresponds to line D2-13 of Table D2 and the meaning for the combination of R¹, R² and R³ for each individual compound corresponds in each case to one line of Table B (compounds I.Bd.D2-13.B1 to I.Bd.D2-13.B442).
- Table 84f: Compounds of the formula I.Bd in which the combination of (R⁴)ₙ and (R^{L})ₘ corresponds to line D2-14 of Table D2 and the meaning for the combination of R¹, R² and R³ for each individual compound corresponds in each case to one line of Table B (compounds I.Bd.D2-14.B1 to I.Bd.D2-14.B442).
- Table 85f: Compounds of the formula I.Bd in which the combination of (R⁴)ₙ and (R^{L})ₘ corresponds to line D2-15 of Table D2 and the meaning for the combination of R¹, R² and R³ for each individual compound corresponds in each case to one line of Table B (compounds I.Bd.D2-15.B1 to I.Bd.D2-15.B442).
- Table 86f: Compounds of the formula I.Bd in which the combination of (R⁴)ₙ and (R^{L})ₘ corresponds to line D2-16 of Table D2 and the meaning for the combination of R¹, R² and R³ for each individual compound corresponds in each case to one line of Table B (compounds I.Bd.D2-16.B1 to I.Bd.D2-16.B442).
- Table 87f: Compounds of the formula I.Bd in which the combination of (R⁴)ₙ and (R^{L})ₘ corresponds to line D2-17 of Table D2 and the meaning for the combination of R¹, R² and R³ for each individual compound corresponds in each case to one line of Table B (compounds I.Bd.D2-17.B1 to I.Bd.D2-17.B442).
- Table 88f: Compounds of the formula I.Bd in which the combination of (R⁴)ₙ and (R^{L})ₘ corresponds to line D2-18 of Table D2 and the meaning for the combination of R¹, R² and R³ for each individual compound corresponds in each case to one line of Table B (compounds I.Bd.D2-18.B1 to I.Bd.D2-18.B442).
- Table 89f: Compounds of the formula I.Bd in which the combination of (R⁴)ₙ and (R^{L})ₘ corresponds to line D2-19 of Table D2 and the meaning for the combination of R¹, R² and R³ for each individual compound corresponds in each case to one line of Table B (compounds I.Bd.D2-19.B1 to I.Bd.D2-19.B442).
- Table 90f: Compounds of the formula I.Bd in which the combination of (R⁴)ₙ and (R^{L})ₘ corresponds to line D2-20 of Table D2 and the meaning for the combination of R¹, R² and R³ for each individual compound corresponds in each case to one line of Table B (compounds I.Bd.D2-20.B1 to I.Bd.D2-20.B442).
- Table 91f: Compounds of the formula I.Bd in which the combination of (R⁴)ₙ and (R^{L})ₘ corresponds to line D2-21 of Table D2 and the meaning for the combination of R¹, R² and R³ for each individual compound corresponds in each case to one line of Table B (compounds I.Bd.D2-21.B1 to I.Bd.D2-21.B442).
- Table 92f: Compounds of the formula I.Bd in which the combination of (R⁴)ₙ and (R^{L})ₘ corresponds to line D2-22 of Table D2 and the meaning for the combination of R¹, R² and R³ for each individual compound corresponds in each case to one line of Table B (compounds I.Bd.D2-22.B1 to I.Bd.D2-22.B442).
- Table 93f: Compounds of the formula I.Bd in which the combination of (R⁴)ₙ and (R^{L})ₘ corresponds to line D2-23 of Table D2 and the meaning for the combination of R¹, R² and R³ for each individual compound corresponds in each case to one line of Table B (compounds I.Bd.D2-23.B1 to I.Bd.D2-23.B442).
- Table 94f: Compounds of the formula I.Bd in which the combination of (R⁴)ₙ and (R^{L})ₘ corresponds to line D2-24 of Table D2 and the meaning for the combination of R¹, R² and R³ for each individual compound corresponds in each case to one line of Table B (compounds I.Bd.D2-24.B1 to I.Bd.D2-24.B442).
- Table 95f: Compounds of the formula I.Bd in which the combination of (R⁴)ₙ and (R^{L})ₘ corresponds to line D2-25 of Table D2 and the meaning for the combination of R¹, R² and R³ for each individual compound corresponds in each case to one line of Table B (compounds I.Bd.D2-25.B1 to I.Bd.D2-25.B442).
- Table 96f: Compounds of the formula I.Bd in which the combination of (R⁴)ₙ and (R^{L})ₘ corresponds to line D2-26 of Table D2 and the meaning for the combination of R¹, R² and R³ for each individual compound corresponds in each case to one line of Table B (compounds I.Bd.D2-26.B1 to I.Bd.D2-26.B442).
- Table 97f: Compounds of the formula I.Bd in which the combination of (R⁴)ₙ and (R^{L})ₘ corresponds to line D2-27 of Table D2 and the meaning for the combination of R¹, R² and R³ for each individual compound corresponds in each case to one line of Table B (compounds I.Bd.D2-27.B1 to I.Bd.D2-27.B442).
- Table 98f: Compounds of the formula I.Bd in which the combination of (R⁴)ₙ and (R^{L})ₘ corresponds to line D2-28 of Table D2 and the meaning for the combination of R¹, R² and R³ for each individual compound corresponds in each case to one line of Table B (compounds I.Bd.D2-28.B1 to I.Bd.D2-28.B442).
- Table 99f: Compounds of the formula I.Bd in which the combination of (R⁴)ₙ and (R^{L})ₘ corresponds to line D2-29 of Table D2 and the meaning for the combination of R¹, R² and R³ for each individual compound corresponds in each case to one line of Table B (compounds I.Bd.D2-29.B1 to I.Bd.D2-29.B442).
- Table 100f: Compounds of the formula I.Bd in which the combination of (R⁴)ₙ and (R^{L})ₘ corresponds to line D2-30 of Table D2 and the meaning for the combination of R¹, R² and R³ for each individual compound corresponds in each case to one line of Table B (compounds I.Bd.D2-30.B1 to I.Bd.D2-30.B442).
- Table 101f: Compounds of the formula I.Bd in which the combination of (R⁴)ₙ and (R^{L})ₘ corresponds to line D2-31 of Table D2 and the meaning for the combination of R¹, R² and R³ for each individual compound corresponds in each case to one line of Table B (compounds I.Bd.D2-31.B1 to I.Bd.D2-31.B442).
- Table 102f: Compounds of the formula I.Bd in which the combination of (R⁴)ₙ and (R^{L})ₘ corresponds to line D2-32 of Table D2 and the meaning for the combination of R¹, R² and R³ for each individual compound corresponds in each case to one line of Table B (compounds I.Bd.D2-32.B1 to I.Bd.D2-32.B442).
- Table 103f: Compounds of the formula I.Bd in which the combination of (R⁴)ₙ and (R^{L})ₘ corresponds to line D2-33 of Table D2 and the meaning for the combination of R¹, R² and R³ for each individual compound corresponds in each case to one line of Table B (compounds I.Bd.D2-33.B1 to I.Bd.D2-33.B442).
- Table 104f: Compounds of the formula I.Bd in which the combination of (R⁴)ₙ and (R^{L})ₘ corresponds to line D2-34 of Table D2 and the meaning for the combination of R¹, R² and R³ for each individual compound corresponds in each case to one line of Table B (compounds I.Bd.D2-34.B1 to I.Bd.D2-34.B442).
- Table 105f: Compounds of the formula I.Bd in which the combination of (R⁴)ₙ and (R^{L})ₘ corresponds to line D2-35 of Table D2 and the meaning for the combination of R¹, R² and R³ for each individual compound corresponds in each case to one line of Table B (compounds I.Bd.D2-35.B1 to I.Bd.D2-35.B442).
- Table 106f: Compounds of the formula I.Bd in which the combination of (R⁴)ₙ and (R^{L})ₘ corresponds to line D2-36 of Table D2 and the meaning for the combination of R¹, R² and R³ for each individual compound corresponds in each case to one line of Table B (compounds I.Bd.D2-36.B1 to I.Bd.D2-36.B442).
- Table 107f: Compounds of the formula I.Bd in which the combination of (R⁴)ₙ and (R^{L})ₘ corresponds to line D2-37 of Table D2 and the meaning for the combination of R¹, R² and R³ for each individual compound corresponds in each case to one line of Table B (compounds I.Bd.D2-37.B1 to I.Bd.D2-37.B442).
- Table 108f: Compounds of the formula I.Bd in which the combination of (R⁴)ₙ and (R^{L})ₘ corresponds to line D2-38 of Table D2 and the meaning for the combination of R¹, R² and R³ for each individual compound corresponds in each case to one line of Table B (compounds I.Bd.D2-38.B1 to I.Bd.D2-38.B442).
- Table 109f: Compounds of the formula I.Bd in which the combination of (R⁴)ₙ and (R^{L})ₘ corresponds to line D2-39 of Table D2 and the meaning for the combination of R¹, R² and R³ for each individual compound corresponds in each case to one line of Table B (compounds I.Bd.D2-39.B1 to I.Bd.D2-39.B442).
- Table 110f: Compounds of the formula I.Bd in which the combination of (R⁴)ₙ and (R^{L})ₘ corresponds to line D2-40 of Table D2 and the meaning for the combination of R¹, R² and R³ for each individual compound corresponds in each case to one line of Table B (compounds I.Bd.D2-40.B1 to I.Bd.D2-40.B442).
- Table 111f: Compounds of the formula I.Bd in which the combination of (R⁴)ₙ and (R^{L})ₘ corresponds to line D2-41 of Table D2 and the meaning for the combination of R¹, R² and R³ for each individual compound corresponds in each case to one line of Table B (compounds I.Bd.D2-41.B1 to I.Bd.D2-41.B442).
- Table 112f: Compounds of the formula I.Bd in which the combination of (R⁴)ₙ and (R^{L})ₘ corresponds to line D2-42 of Table D2 and the meaning for the combination of R¹, R² and R³ for each individual compound corresponds in each case to one line of Table B (compounds I.Bd.D2-42.B1 to I.Bd.D2-42.B442).
- Table 113f: Compounds of the formula I.Bd in which the combination of (R⁴)ₙ and (R^{L})ₘ corresponds to line D2-43 of Table D2 and the meaning for the combination of R¹, R² and R³ for each individual compound corresponds in each case to one line of Table B (compounds I.Bd.D2-43.B1 to I.Bd.D2-43.B442).
- Table 114f: Compounds of the formula I.Bd in which the combination of (R⁴)ₙ and (R^{L})ₘ corresponds to line D2-44 of Table D2 and the meaning for the combination of R¹, R² and R³ for each individual compound corresponds in each case to one line of Table B (compounds I.Bd.D2-44.B1 to I.Bd.D2-44.B442).
- Table 115f: Compounds of the formula I.Bd in which the combination of (R⁴)ₙ and (R^{L})ₘ corresponds to line D2-45 of Table D2 and the meaning for the combination of R¹, R² and R³ for each individual compound corresponds in each case to one line of Table B (compounds I.Bd.D2-45.B1 to I.Bd.D2-45.B442).
- Table 116f: Compounds of the formula I.Bd in which the combination of (R⁴)ₙ and (R^{L})ₘ corresponds to line D2-46 of Table D2 and the meaning for the combination of R¹, R² and R³ for each individual compound corresponds in each case to one line of Table B (compounds I.Bd.D2-46.B1 to I.Bd.D2-46.B442).
- Table 117f: Compounds of the formula I.Bd in which the combination of (R⁴)ₙ and (R^{L})ₘ corresponds to line D2-47 of Table D2 and the meaning for the combination of R¹, R² and R³ for each individual compound corresponds in each case to one line of Table B (compounds I.Bd.D2-47.B1 to I.Bd.D2-47.B442).
- Table 118f: Compounds of the formula I.Bd in which the combination of (R⁴)ₙ and (R^{L})ₘ corresponds to line D2-48 of Table D2 and the meaning for the combination of R¹, R² and R³ for each individual compound corresponds in each case to one line of Table B (compounds I.Bd.D2-48.B1 to I.Bd.D2-48.B442).
- Table 119f: Compounds of the formula I.Bd in which the combination of (R⁴)ₙ and (R^{L})ₘ corresponds to line D2-49 of Table D2 and the meaning for the combination of R¹, R² and R³ for each individual compound corresponds in each case to one line of Table B (compounds I.Bd.D2-49.B1 to I.Bd.D2-49.B442).
- Table 120f: Compounds of the formula I.Bd in which the combination of (R⁴)ₙ and (R^{L})ₘ corresponds to line D2-50 of Table D2 and the meaning for the combination of R¹, R² and R³ for each individual compound corresponds in each case to one line of Table B (compounds I.Bd.D2-50.B1 to I.Bd.D2-50.B442).
- Table 121f: Compounds of the formula I.Bd in which the combination of (R⁴)ₙ and (R^{L})ₘ corresponds to line D2-51 of Table D2 and the meaning for the combination of R¹, R² and R³ for each individual compound corresponds in each case to one line of Table B (compounds I.Bd.D2-51.B1 to I.Bd.D2-51.B442).
- Table 122f: Compounds of the formula I.Bd in which the combination of (R⁴)ₙ and (R^{L})ₘ corresponds to line D2-52 of Table D2 and the meaning for the combination of R¹, R² and R³ for each individual compound corresponds in each case to one line of Table B (compounds I.Bd.D2-52.B1 to I.Bd.D2-52.B442).
- Table 123f: Compounds of the formula I.Bd in which the combination of (R⁴)ₙ and (R^{L})ₘ corresponds to line D2-53 of Table D2 and the meaning for the combination of R¹, R² and R³ for each individual compound corresponds in each case to one line of Table B (compounds I.Bd.D2-53.B1 to I.Bd.D2-53.B442).
- Table 124f: Compounds of the formula I.Bd in which the combination of (R⁴)ₙ and (R^{L})ₘ corresponds to line D2-54 of Table D2 and the meaning for the combination of R¹, R² and R³ for each individual compound corresponds in each case to one line of Table B (compounds I.Bd.D2-54.B1 to I.Bd.D2-54.B442).
- Table 125f: Compounds of the formula I.Bd in which the combination of (R⁴)ₙ and (R^{L})ₘ corresponds to line D2-55 of Table D2 and the meaning for the combination of R¹, R² and R³ for each individual compound corresponds in each case to one line of Table B (compounds I.Bd.D2-55.B1 to I.Bd.D2-55.B442).
- Table 126f: Compounds of the formula I.Bd in which the combination of (R⁴)ₙ and (R^{L})ₘ corresponds to line D2-56 of Table D2 and the meaning for the combination of R¹, R² and R³ for each individual compound corresponds in each case to one line of Table B (compounds I.Bd.D2-56.B1 to I.Bd.D2-56.B442).
- Table 127f: Compounds of the formula I.Bd in which the combination of (R⁴)ₙ and (R^{L})ₘ corresponds to line D2-57 of Table D2 and the meaning for the combination of R¹, R² and R³ for each individual compound corresponds in each case to one line of Table B (compounds I.Bd.D2-57.B1 to I.Bd.D2-57.B442).
- Table 128f: Compounds of the formula I.Bd in which the combination of (R⁴)ₙ and (R^{L})ₘ corresponds to line D2-58 of Table D2 and the meaning for the combination of R¹, R² and R³ for each individual compound corresponds in each case to one line of Table B (compounds I.Bd.D2-58.B1 to I.Bd.D2-58.B442).
- Table 129f: Compounds of the formula I.Bd in which the combination of (R⁴)ₙ and (R^{L})ₘ corresponds to line D2-59 of Table D2 and the meaning for the combination of R¹, R² and R³ for each individual compound corresponds in each case to one line of Table B (compounds I.Bd.D2-59.B1 to I.Bd.D2-59.B442).
- Table 130f: Compounds of the formula I.Bd in which the combination of (R⁴)ₙ and (R^{L})ₘ corresponds to line D2-60 of Table D2 and the meaning for the combination of R¹, R² and R³ for each individual compound corresponds in each case to one line of Table B (compounds I.Bd.D2-60.B1 to I.Bd.D2-60.B442).
- Table 131f: Compounds of the formula I.Bd in which the combination of (R⁴)ₙ and (R^{L})ₘ corresponds to line D2-61 of Table D2 and the meaning for the combination of R¹, R² and R³ for each individual compound corresponds in each case to one line of Table B (compounds I.Bd.D2-61.B1 to I.Bd.D2-61.B442).
- Table 132f: Compounds of the formula I.Bd in which the combination of (R⁴)ₙ and (R^{L})ₘ corresponds to line D2-62 of Table D2 and the meaning for the combination of R¹, R² and R³ for each individual compound corresponds in each case to one line of Table B (compounds I.Bd.D2-62.B1 to I.Bd.D2-62.B442).
- Table 133f: Compounds of the formula I.Bd in which the combination of (R⁴)ₙ and (R^{L})ₘ corresponds to line D2-63 of Table D2 and the meaning for the combination of R¹, R² and R³ for each individual compound corresponds in each case to one line of Table B (compounds I.Bd.D2-63.B1 to I.Bd.D2-63.B442).
- Table 134f: Compounds of the formula I.Bd in which the combination of (R⁴)ₙ and (R^{L})ₘ corresponds to line D2-64 of Table D2 and the meaning for the combination of R¹, R² and R³ for each individual compound corresponds in each case to one line of Table B (compounds I.Bd.D2-64.B1 to I.Bd.D2-64.B442).
- Table 135f: Compounds of the formula I.Bd in which the combination of (R⁴)ₙ and (R^{L})ₘ corresponds to line D2-65 of Table D2 and the meaning for the combination of R¹, R² and R³ for each individual compound corresponds in each case to one line of Table B (compounds I.Bd.D2-65.B1 to I.Bd.D2-65.B442).
- Table 136f: Compounds of the formula I.Bd in which the combination of (R⁴)ₙ and (R^{L})ₘ corresponds to line D2-66 of Table D2 and the meaning for the combination of R¹, R² and R³ for each individual compound corresponds in each case to one line of Table B (compounds I.Bd.D2-66.B1 to I.Bd.D2-66.B442).
- Table 137f: Compounds of the formula I.Bd in which the combination of (R⁴)ₙ and (R^{L})ₘ corresponds to line D2-67 of Table D2 and the meaning for the combination of R¹, R² and R³ for each individual compound corresponds in each case to one line of Table B (compounds I.Bd.D2-67.B1 to I.Bd.D2-67.B442).
- Table 138f: Compounds of the formula I.Bd in which the combination of (R⁴)ₙ and (R^{L})ₘ corresponds to line D2-68 of Table D2 and the meaning for the combination of R¹, R² and R³ for each individual compound corresponds in each case to one line of Table B (compounds I.Bd.D2-68.B1 to I.Bd.D2-68.B442).
- Table 139f: Compounds of the formula I.Bd in which the combination of (R⁴)ₙ and (R^{L})ₘ corresponds to line D2-69 of Table D2 and the meaning for the combination of R¹, R² and R³ for each individual compound corresponds in each case to one line of Table B (compounds I.Bd.D2-69.B1 to I.Bd.D2-69.B442).
- Table 140f: Compounds of the formula I.Bd in which the combination of (R⁴)ₙ and (R^{L})ₘ corresponds to line D2-70 of Table D2 and the meaning for the combination of R¹, R² and R³ for each individual compound corresponds in each case to one line of Table B (compounds I.Bd.D2-70.B1 to I.Bd.D2-70.B442).

**Table D1:**

| **line** | **(R⁴)ₙ** | **(R^{L})ₘ** |
|---|---|---|
| D1-1 | -* | -* |
| D1-2 | 2-Cl | -* |
| D1-3 | 2-F | -* |
| D1-4 | 2-CF₃ | -* |
| D1-5 | 3-Cl | -* |
| D1-6 | 3-F | -* |
| D1-7 | 3-CF₃ | -* |
| D1-8 | -* | 2-Cl |
| D1-9 | 2-Cl | 2-Cl |
| D1-10 | 2-F | 2-Cl |
| D1-11 | 2-CF₃ | 2-Cl |
| D1-12 | 3-Cl | 2-Cl |
| D1-13 | 3-F | 2-Cl |
| D1-14 | 3-CF₃ | 2-Cl |
| D1-15 | -* | 3-Cl |
| D1-16 | 2-Cl | 3-Cl |
| D1-17 | 2-F | 3-Cl |
| D1-18 | 2-CF₃ | 3-Cl |
| D1-19 | 3-Cl | 3-Cl |
| D1-20 | 3-F | 3-Cl |
| D1-21 | 3-CF₃ | 3-Cl |
| D1-22 | -* | 4-Cl |
| D1-23 | 2-Cl | 4-Cl |
| D1-24 | 2-F | 4-Cl |
| D1-25 | 2-CF₃ | 4-Cl |
| D1-26 | 3-Cl | 4-Cl |
| D1-27 | 3-F | 4-Cl |
| D1-28 | 3-CF₃ | 4-Cl |
| D1-29 | -* | 2-F |
| D1-30 | 2-Cl | 2-F |
| D1-31 | 2-F | 2-F |
| D1-32 | 2-CF₃ | 2-F |
| D1-33 | 3-Cl | 2-F |
| D1-34 | 3-F | 2-F |
| D1-35 | 3-CF₃ | 2-F |
| D1-36 | -* | 3-F |
| D1-37 | 2-Cl | 3-F |
| D1-38 | 2-F | 3-F |
| D1-39 | 2-CF₃ | 3-F |
| D1-40 | 3-Cl | 3-F |
| D1-41 | 3-F | 3-F |
| D1-42 | 3-CF₃ | 3-F |
| D1-43 | -* | 4-F |
| D1-44 | 2-Cl | 4-F |
| D1-45 | 2-F | 4-F |
| D1-46 | 2-CF₃ | 4-F |
| D1-47 | 3-Cl | 4-F |
| D1-48 | 3-F | 4-F |
| D1-49 | 3-CF₃ | 4-F |
| D1-50 | -* | 2-CN |
| D1-51 | 2-Cl | 2-CN |
| D1-52 | 2-F | 2-CN |
| D1-53 | 2-CF₃ | 2-CN |
| D1-54 | 3-Cl | 2-CN |
| D1-55 | 3-F | 2-CN |
| D1-56 | 3-CF₃ | 2-CN |
| D1-57 | -* | 3-CN |
| D1-58 | 2-Cl | 3-CN |
| D1-59 | 2-F | 3-CN |
| D1-60 | 2-CF₃ | 3-CN |
| D1-61 | 3-Cl | 3-CN |
| D1-62 | 3-F | 3-CN |
| D1-63 | 3-CF₃ | 3-CN |
| D1-64 | -* | 4-CN |
| D1-65 | 2-Cl | 4-CN |
| D1-66 | 2-F | 4-CN |
| D1-67 | 2-CF₃ | 4-CN |
| D1-68 | 3-Cl | 4-CN |
| D1-69 | 3-F | 4-CN |
| D1-70 | 3-CF₃ | 4-CN |

| | | |
|---|---|---|
| * means that n=0 or m=0, respectively | | |

**Table D2:**

| **line** | **(R⁴)ₙ** | **(R^{L})ₘ** |
|---|---|---|
| D2-1 | -* | 2-CF₃ |
| D2-2 | 2-Cl | 2-CF₃ |
| D2-3 | 2-F | 2-CF₃ |
| D2-4 | 2-CF₃ | 2-CF₃ |
| D2-5 | 3-Cl | 2-CF₃ |
| D2-6 | 3-F | 2-CF₃ |
| D2-7 | 3-CF₃ | 2-CF₃ |
| D2-8 | -* | 3-CF₃ |
| D2-9 | 2-Cl | 3-CF₃ |
| D2-10 | 2-F | 3-CF₃ |
| D2-11 | 2-CF₃ | 3-CF₃ |
| D2-12 | 3-Cl | 3-CF₃ |
| D2-13 | 3-F | 3-CF₃ |
| D2-14 | 3-CF₃ | 3-CF₃ |
| D2-15 | -* | 4-CF₃ |
| D2-16 | 2-Cl | 4-CF₃ |
| D2-17 | 2-F | 4-CF₃ |
| D2-18 | 2-CF₃ | 4-CF₃ |
| D2-19 | 3-Cl | 4-CF₃ |
| D2-20 | 3-F | 4-CF₃ |
| D2-21 | 3-CF₃ | 4-CF₃ |
| D2-22 | -* | 2,4-Cl₂ |
| D2-23 | 2-Cl | 2,4-Cl₂ |
| D2-24 | 2-F | 2,4-Cl₂ |
| D2-25 | 2-CF₃ | 2,4-Cl₂ |
| D2-26 | 3-Cl | 2,4-Cl₂ |
| D2-27 | 3-F | 2,4-Cl₂ |
| D2-28 | 3-CF₃ | 2,4-Cl₂ |
| D2-29 | -* | 2,6-Cl₂ |
| D2-30 | 2-Cl | 2,6-Cl₂ |
| D2-31 | 2-F | 2,6-Cl₂ |
| D2-32 | 2-CF₃ | 2,6-Cl₂ |
| D2-33 | 3-Cl | 2,6-Cl₂ |
| D2-34 | 3-F | 2,6-Cl₂ |
| D2-35 | 3-CF₃ | 2,6-Cl₂ |
| D2-36 | -* | 2,4-F₂ |
| D2-37 | 2-Cl | 2,4-F₂ |
| D2-38 | 2-F | 2,4-F₂ |
| D2-39 | 2-CF₃ | 2,4-F₂ |
| D2-40 | 3-Cl | 2,4-F₂ |
| D2-41 | 3-F | 2,4-F₂ |
| D2-42 | 3-CF₃ | 2,4-F₂ |
| D2-43 | -* | 2,4-F₂ |
| D2-44 | 2-Cl | 2-F-4-CN |
| D2-45 | 2-F | 2-F-4-CN |
| D2-46 | 2-CF₃ | 2-F-4-CN |
| D2-47 | 3-Cl | 2-F-4-CN |
| D2-48 | 3-F | 2-F-4-CN |
| D2-49 | 3-CF₃ | 2-F-4-CN |
| D2-50 | -* | 2-Cl-4-CN |
| D2-51 | 2-Cl | 2-Cl-4-CN |
| D2-52 | 2-F | 2-Cl-4-CN |
| D2-53 | 2-CF₃ | 2-Cl-4-CN |
| D2-54 | 3-Cl | 2-Cl-4-CN |
| D2-55 | 3-F | 2-Cl-4-CN |
| D2-56 | 3-CF₃ | 2-Cl-4-CN |
| D2-57 | -* | 2-Cl-4-CF₃ |
| D2-58 | 2-Cl | 2-Cl-4-CF₃ |
| D2-59 | 2-F | 2-Cl-4-CF₃ |
| D2-60 | 2-CF₃ | 2-Cl-4-CF₃ |
| D2-61 | 3-Cl | 2-Cl-4-CF₃ |
| D2-62 | 3-F | 2-Cl-4-CF₃ |
| D2-63 | 3-CF₃ | 2-Cl-4-CF₃ |
| D2-64 | -* | 2-F-4-CF₃ |
| D2-65 | 2-Cl | 2-F-4-CF₃ |
| D2-66 | 2-F | 2-F-4-CF₃ |
| D2-67 | 2-CF₃ | 2-F-4-CF₃ |
| D2-68 | 3-Cl | 2-F-4-CF₃ |
| D2-69 | 3-F | 2-F-4-CF₃ |
| D2-70 | 3-CF₃ | 2-F-4-CF₃ |

| | | |
|---|---|---|
| * means that n=0 or m=0, respectively | | |

In particular with a view to their use, preference is also given to the compounds of the formula I.Be, I.Bf, I.Bg and I.Bh, that are compiled in the Tables 1 g to 70g, Tables 1 h to 70h, Tables 1i to 70i and Tables 1j to 70j below. Each of the groups mentioned for a substituent in the tables is furthermore per se, independently of the combination in which it is mentioned, a particularly preferred aspect of the substituent in question.
- Table 1g: Compounds of the formula I.Be in which the combination of (R⁴)ₙ and (R^{L})ₘ corresponds to line E-1 of Table E and the meaning for the combination of R¹, R² and R³ for each individual compound corresponds in each case to one line of Table B (compounds I.Be.E-1.B1 to I.Be.E-1.B442).
- Table 2g: Compounds of the formula I.Be in which the combination of (R⁴)ₙ and (R^{L})ₘ corresponds to line E-2 of Table E and the meaning for the combination of R¹, R² and R³ for each individual compound corresponds in each case to one line of Table B (compounds I.Be.E-2.B1 to I.Be.E-2.B442).
- Table 3g: Compounds of the formula I.Be in which the combination of (R⁴)ₙ and (R^{L})ₘ corresponds to line E-3 of Table E and the meaning for the combination of R¹, R² and R³ for each individual compound corresponds in each case to one line of Table B (compounds I.Be.E-3.B1 to I.Be.E-3.B442).
- Table 4g: Compounds of the formula I.Be in which the combination of (R⁴)ₙ and (R^{L})ₘ corresponds to line E-4 of Table E and the meaning for the combination of R¹, R² and R³ for each individual compound corresponds in each case to one line of Table B (compounds I.Be.E-4.B1 to I.Be.E-4.B442).
- Table 5g: Compounds of the formula I.Be in which the combination of (R⁴)ₙ and (R^{L})ₘ corresponds to line E-5 of Table E and the meaning for the combination of R¹, R² and R³ for each individual compound corresponds in each case to one line of Table B (compounds I.Be.E-5.B1 to I.Be.E-5.B442).
- Table 6g: Compounds of the formula I.Be in which the combination of (R⁴)ₙ and (R^{L})ₘ corresponds to line E-6 of Table E and the meaning for the combination of R¹, R² and R³ for each individual compound corresponds in each case to one line of Table B (compounds I.Be.E-6.B1 to I.Be.E-6.B442).
- Table 7g: Compounds of the formula I.Be in which the combination of (R⁴)ₙ and (R^{L})ₘ corresponds to line E-7 of Table E and the meaning for the combination of R¹, R² and R³ for each individual compound corresponds in each case to one line of Table B (compounds I.Be.E-7.B1 to I.Be.E-7.B442).
- Table 8g: Compounds of the formula I.Be in which the combination of (R⁴)ₙ and (R^{L})ₘ corresponds to line E-8 of Table E and the meaning for the combination of R¹, R² and R³ for each individual compound corresponds in each case to one line of Table B (compounds I.Be.E-8.B1 to I.Be.E-8.B442).
- Table 9g: Compounds of the formula I.Be in which the combination of (R⁴)ₙ and (R^{L})ₘ corresponds to line E-9 of Table E and the meaning for the combination of R¹, R² and R³ for each individual compound corresponds in each case to one line of Table B (compounds I.Be.E-9.B1 to I.Be.E-9.B442).
- Table 10g: Compounds of the formula I.Be in which the combination of (R⁴)ₙ and (R^{L})ₘ corresponds to line E-10 of Table E and the meaning for the combination of R¹, R² and R³ for each individual compound corresponds in each case to one line of Table B (compounds I.Be.E-10.B1 to I.Be.E-10.B442).
- Table 11g: Compounds of the formula I.Be in which the combination of (R⁴)ₙ and (R^{L})ₘ corresponds to line E-11 of Table E and the meaning for the combination of R¹, R² and R³ for each individual compound corresponds in each case to one line of Table B (compounds I.Be.E-11.B1 to I.Be.E-11.B442).
- Table 12g: Compounds of the formula I.Be in which the combination of (R⁴)ₙ and (R^{L})ₘ corresponds to line E-12 of Table E and the meaning for the combination of R¹, R² and R³ for each individual compound corresponds in each case to one line of Table B (compounds I.Be.E-12.B1 to I.Be.E-12.B442).
- Table 13g: Compounds of the formula I.Be in which the combination of (R⁴)ₙ and (R^{L})ₘ corresponds to line E-13 of Table E and the meaning for the combination of R¹, R² and R³ for each individual compound corresponds in each case to one line of Table B (compounds I.Be.E-13.B1 to I.Be.E-13.B442).
- Table 14g: Compounds of the formula I.Be in which the combination of (R⁴)ₙ and (R^{L})ₘ corresponds to line E-14 of Table E and the meaning for the combination of R¹, R² and R³ for each individual compound corresponds in each case to one line of Table B (compounds I.Be.E-14.B1 to I.Be.E-14.B442).
- Table 15g: Compounds of the formula I.Be in which the combination of (R⁴)ₙ and (R^{L})ₘ corresponds to line E-15 of Table E and the meaning for the combination of R¹, R² and R³ for each individual compound corresponds in each case to one line of Table B (compounds I.Be.E-15.B1 to I.Be.E-15.B442).
- Table 16g: Compounds of the formula I.Be in which the combination of (R⁴)ₙ and (R^{L})ₘ corresponds to line E-16 of Table E and the meaning for the combination of R¹, R² and R³ for each individual compound corresponds in each case to one line of Table B (compounds I.Be.E-16.B1 to I.Be.E-16.B442).
- Table 17g: Compounds of the formula I.Be in which the combination of (R⁴)ₙ and (R^{L})ₘ corresponds to line E-17 of Table E and the meaning for the combination of R¹, R² and R³ for each individual compound corresponds in each case to one line of Table B (compounds I.Be.E-17.B1 to I.Be.E-17.B442).
- Table 18g: Compounds of the formula I.Be in which the combination of (R⁴)ₙ and (R^{L})ₘ corresponds to line E-18 of Table E and the meaning for the combination of R¹, R² and R³ for each individual compound corresponds in each case to one line of Table B (compounds I.Be.E-18.B1 to I.Be.E-18.B442).
- Table 19g: Compounds of the formula I.Be in which the combination of (R⁴)ₙ and (R^{L})ₘ corresponds to line E-19 of Table E and the meaning for the combination of R¹, R² and R³ for each individual compound corresponds in each case to one line of Table B (compounds I.Be.E-19.B1 to I.Be.E-19.B442).
- Table 20g: Compounds of the formula I.Be in which the combination of (R⁴)ₙ and (R^{L})ₘ corresponds to line E-20 of Table E and the meaning for the combination of R¹, R² and R³ for each individual compound corresponds in each case to one line of Table B (compounds I.Be.E-20.B1 to I.Be.E-20.B442).
- Table 21g: Compounds of the formula I.Be in which the combination of (R⁴)ₙ and (R^{L})ₘ corresponds to line E-21 of Table E and the meaning for the combination of R¹, R² and R³ for each individual compound corresponds in each case to one line of Table B (compounds I.Be.E-21.B1 to I.Be.E-21.B442).
- Table 22g: Compounds of the formula I.Be in which the combination of (R⁴)ₙ and (R^{L})ₘ corresponds to line E-22 of Table E and the meaning for the combination of R¹, R² and R³ for each individual compound corresponds in each case to one line of Table B (compounds I.Be.E-22.B1 to I.Be.E-22.B442).
- Table 23g: Compounds of the formula I.Be in which the combination of (R⁴)ₙ and (R^{L})ₘ corresponds to line E-23 of Table E and the meaning for the combination of R¹, R² and R³ for each individual compound corresponds in each case to one line of Table B (compounds I.Be.E-23.B1 to I.Be.E-23.B442).
- Table 24g: Compounds of the formula I.Be in which the combination of (R⁴)ₙ and (R^{L})ₘ corresponds to line E-24 of Table E and the meaning for the combination of R¹, R² and R³ for each individual compound corresponds in each case to one line of Table B (compounds I.Be.E-24.B1 to I.Be.E-24.B442).
- Table 25g: Compounds of the formula I.Be in which the combination of (R⁴)ₙ and (R^{L})ₘ corresponds to line E-25 of Table E and the meaning for the combination of R¹, R² and R³ for each individual compound corresponds in each case to one line of Table B (compounds I.Be.E-25.B1 to I.Be.E-25.B442).
- Table 26g: Compounds of the formula I.Be in which the combination of (R⁴)ₙ and (R^{L})ₘ corresponds to line E-26 of Table E and the meaning for the combination of R¹, R² and R³ for each individual compound corresponds in each case to one line of Table B (compounds I.Be.E-26.B1 to I.Be.E-26.B442).
- Table 27g: Compounds of the formula I.Be in which the combination of (R⁴)ₙ and (R^{L})ₘ corresponds to line E-27 of Table E and the meaning for the combination of R¹, R² and R³ for each individual compound corresponds in each case to one line of Table B (compounds I.Be.E-27.B1 to I.Be.E-27.B442).
- Table 28g: Compounds of the formula I.Be in which the combination of (R⁴)ₙ and (R^{L})ₘ corresponds to line E-28 of Table E and the meaning for the combination of R¹, R² and R³ for each individual compound corresponds in each case to one line of Table B (compounds I.Be.E-28.B1 to I.Be.E-28.B442).
- Table 29g: Compounds of the formula I.Be in which the combination of (R⁴)ₙ and (R^{L})ₘ corresponds to line E-29 of Table E and the meaning for the combination of R¹, R² and R³ for each individual compound corresponds in each case to one line of Table B (compounds I.Be.E-29.B1 to I.Be.E-29.B442).
- Table 30g: Compounds of the formula I.Be in which the combination of (R⁴)ₙ and (R^{L})ₘ corresponds to line E-30 of Table E and the meaning for the combination of R¹, R² and R³ for each individual compound corresponds in each case to one line of Table B (compounds I.Be.E-30.B1 to I.Be.E-30.B442).
- Table 31g: Compounds of the formula I.Be in which the combination of (R⁴)ₙ and (R^{L})ₘ corresponds to line E-31 of Table E and the meaning for the combination of R¹, R² and R³ for each individual compound corresponds in each case to one line of Table B (compounds I.Be.E-31.B1 to I.Be.E-31.B442).
- Table 32g: Compounds of the formula I.Be in which the combination of (R⁴)ₙ and (R^{L})ₘ corresponds to line E-32 of Table E and the meaning for the combination of R¹, R² and R³ for each individual compound corresponds in each case to one line of Table B (compounds I.Be.E-32.B1 to I.Be.E-32.B442).
- Table 33g: Compounds of the formula I.Be in which the combination of (R⁴)ₙ and (R^{L})ₘ corresponds to line E-33 of Table E and the meaning for the combination of R¹, R² and R³ for each individual compound corresponds in each case to one line of Table B (compounds I.Be.E-33.B1 to I.Be.E-33.B442).
- Table 34g: Compounds of the formula I.Be in which the combination of (R⁴)ₙ and (R^{L})ₘ corresponds to line E-34 of Table E and the meaning for the combination of R¹, R² and R³ for each individual compound corresponds in each case to one line of Table B (compounds I.Be.E-34.B1 to I.Be.E-34.B442).
- Table 35g: Compounds of the formula I.Be in which the combination of (R⁴)ₙ and (R^{L})ₘ corresponds to line E-35 of Table E and the meaning for the combination of R¹, R² and R³ for each individual compound corresponds in each case to one line of Table B (compounds I.Be.E-35.B1 to I.Be.E-35.B442).
- Table 36g: Compounds of the formula I.Be in which the combination of (R⁴)ₙ and (R^{L})ₘ corresponds to line E-36 of Table E and the meaning for the combination of R¹, R² and R³ for each individual compound corresponds in each case to one line of Table B (compounds I.Be.E-36.B1 to I.Be.E-36.B442).
- Table 37g: Compounds of the formula I.Be in which the combination of (R⁴)ₙ and (R^{L})ₘ corresponds to line E-37 of Table E and the meaning for the combination of R¹, R² and R³ for each individual compound corresponds in each case to one line of Table B (compounds I.Be.E-37.B1 to I.Be.E-37.B442).
- Table 38g: Compounds of the formula I.Be in which the combination of (R⁴)ₙ and (R^{L})ₘ corresponds to line E-38 of Table E and the meaning for the combination of R¹, R² and R³ for each individual compound corresponds in each case to one line of Table B (compounds I.Be.E-38.B1 to I.Be.E-38.B442).
- Table 39g: Compounds of the formula I.Be in which the combination of (R⁴)ₙ and (R^{L})ₘ corresponds to line E-39 of Table E and the meaning for the combination of R¹, R² and R³ for each individual compound corresponds in each case to one line of Table B (compounds I.Be.E-39.B1 to I.Be.E-39.B442).
- Table 40g: Compounds of the formula I.Be in which the combination of (R⁴)ₙ and (R^{L})ₘ corresponds to line E-40 of Table E and the meaning for the combination of R¹, R² and R³ for each individual compound corresponds in each case to one line of Table B (compounds I.Be.E-40.B1 to I.Be.E-40.B442).
- Table 41g: Compounds of the formula I.Be in which the combination of (R⁴)ₙ and (R^{L})ₘ corresponds to line E-41 of Table E and the meaning for the combination of R¹, R² and R³ for each individual compound corresponds in each case to one line of Table B (compounds I.Be.E-41.B1 to I.Be.E-41.B442).
- Table 42g: Compounds of the formula I.Be in which the combination of (R⁴)ₙ and (R^{L})ₘ corresponds to line E-42 of Table E and the meaning for the combination of R¹, R² and R³ for each individual compound corresponds in each case to one line of Table B (compounds I.Be.E-42.B1 to I.Be.E-42.B442).
- Table 43g: Compounds of the formula I.Be in which the combination of (R⁴)ₙ and (R^{L})ₘ corresponds to line E-43 of Table E and the meaning for the combination of R¹, R² and R³ for each individual compound corresponds in each case to one line of Table B (compounds I.Be.E-43.B1 to I.Be.E-43.B442).
- Table 44g: Compounds of the formula I.Be in which the combination of (R⁴)ₙ and (R^{L})ₘ corresponds to line E-44 of Table E and the meaning for the combination of R¹, R² and R³ for each individual compound corresponds in each case to one line of Table B (compounds I.Be.E-44.B1 to I.Be.E-44.B442).
- Table 45g: Compounds of the formula I.Be in which the combination of (R⁴)ₙ and (R^{L})ₘ corresponds to line E-45 of Table E and the meaning for the combination of R¹, R² and R³ for each individual compound corresponds in each case to one line of Table B (compounds I.Be.E-45.B1 to I.Be.E-45.B442).
- Table 46g: Compounds of the formula I.Be in which the combination of (R⁴)ₙ and (R^{L})ₘ corresponds to line E-46 of Table E and the meaning for the combination of R¹, R² and R³ for each individual compound corresponds in each case to one line of Table B (compounds I.Be.E-46.B1 to I.Be.E-46.B442).
- Table 47g: Compounds of the formula I.Be in which the combination of (R⁴)ₙ and (R^{L})ₘ corresponds to line E-47 of Table E and the meaning for the combination of R¹, R² and R³ for each individual compound corresponds in each case to one line of Table B (compounds I.Be.E-47.B1 to I.Be.E-47.B442).
- Table 48g: Compounds of the formula I.Be in which the combination of (R⁴)ₙ and (R^{L})ₘ corresponds to line E-48 of Table E and the meaning for the combination of R¹, R² and R³ for each individual compound corresponds in each case to one line of Table B (compounds I.Be.E-48.B1 to I.Be.E-48.B442).
- Table 49g: Compounds of the formula I.Be in which the combination of (R⁴)ₙ and (R^{L})ₘ corresponds to line E-49 of Table E and the meaning for the combination of R¹, R² and R³ for each individual compound corresponds in each case to one line of Table B (compounds I.Be.E-49.B1 to I.Be.E-49.B442).
- Table 50g: Compounds of the formula I.Be in which the combination of (R⁴)ₙ and (R^{L})ₘ corresponds to line E-50 of Table E and the meaning for the combination of R¹, R² and R³ for each individual compound corresponds in each case to one line of Table B (compounds I.Be.E-50.B1 to I.Be.E-50.B442).
- Table 51g: Compounds of the formula I.Be in which the combination of (R⁴)ₙ and (R^{L})ₘ corresponds to line E-51 of Table E and the meaning for the combination of R¹, R² and R³ for each individual compound corresponds in each case to one line of Table B (compounds I.Be.E-51.B1 to I.Be.E-51.B442).
- Table 52g: Compounds of the formula I.Be in which the combination of (R⁴)ₙ and (R^{L})ₘ corresponds to line E-52 of Table E and the meaning for the combination of R¹, R² and R³ for each individual compound corresponds in each case to one line of Table B (compounds I.Be.E-52.B1 to I.Be.E-52.B442).
- Table 53g: Compounds of the formula I.Be in which the combination of (R⁴)ₙ and (R^{L})ₘ corresponds to line E-53 of Table E and the meaning for the combination of R¹, R² and R³ for each individual compound corresponds in each case to one line of Table B (compounds I.Be.E-53.B1 to I.Be.E-53.B442).
- Table 54g: Compounds of the formula I.Be in which the combination of (R⁴)ₙ and (R^{L})ₘ corresponds to line E-54 of Table E and the meaning for the combination of R¹, R² and R³ for each individual compound corresponds in each case to one line of Table B (compounds I.Be.E-54.B1 to I.Be.E-54.B442).
- Table 55g: Compounds of the formula I.Be in which the combination of (R⁴)ₙ and (R^{L})ₘ corresponds to line E-55 of Table E and the meaning for the combination of R¹, R² and R³ for each individual compound corresponds in each case to one line of Table B (compounds I.Be.E-55.B1 to I.Be.E-55.B442).
- Table 56g: Compounds of the formula I.Be in which the combination of (R⁴)ₙ and (R^{L})ₘ corresponds to line E-56 of Table E and the meaning for the combination of R¹, R² and R³ for each individual compound corresponds in each case to one line of Table B (compounds I.Be.E-56.B1 to I.Be.E-56.B442).
- Table 57g: Compounds of the formula I.Be in which the combination of (R⁴)ₙ and (R^{L})ₘ corresponds to line E-57 of Table E and the meaning for the combination of R¹, R² and R³ for each individual compound corresponds in each case to one line of Table B (compounds I.Be.E-57.B1 to I.Be.E-57.B442).
- Table 58g: Compounds of the formula I.Be in which the combination of (R⁴)ₙ and (R^{L})ₘ corresponds to line E-58 of Table E and the meaning for the combination of R¹, R² and R³ for each individual compound corresponds in each case to one line of Table B (compounds I.Be.E-58.B1 to I.Be.E-58.B442).
- Table 59g: Compounds of the formula I.Be in which the combination of (R⁴)ₙ and (R^{L})ₘ corresponds to line E-59 of Table E and the meaning for the combination of R¹, R² and R³ for each individual compound corresponds in each case to one line of Table B (compounds I.Be.E-59.B1 to I.Be.E-59.B442).
- Table 60g: Compounds of the formula I.Be in which the combination of (R⁴)ₙ and (R^{L})ₘ corresponds to line E-60 of Table E and the meaning for the combination of R¹, R² and R³ for each individual compound corresponds in each case to one line of Table B (compounds I.Be.E-60.B1 to I.Be.E-60.B442).
- Table 61g: Compounds of the formula I.Be in which the combination of (R⁴)ₙ and (R^{L})ₘ corresponds to line E-61 of Table E and the meaning for the combination of R¹, R² and R³ for each individual compound corresponds in each case to one line of Table B (compounds I.Be.E-61.B1 to I.Be.E-61.B442).
- Table 62g: Compounds of the formula I.Be in which the combination of (R⁴)ₙ and (R^{L})ₘ corresponds to line E-62 of Table E and the meaning for the combination of R¹, R² and R³ for each individual compound corresponds in each case to one line of Table B (compounds I.Be.E-62.B1 to I.Be.E-62.B442).
- Table 63g: Compounds of the formula I.Be in which the combination of (R⁴)ₙ and (R^{L})ₘ corresponds to line E-63 of Table E and the meaning for the combination of R¹, R² and R³ for each individual compound corresponds in each case to one line of Table B (compounds I.Be.E-63.B1 to I.Be.E-63.B442).
- Table 64g: Compounds of the formula I.Be in which the combination of (R⁴)ₙ and (R^{L})ₘ corresponds to line E-64 of Table E and the meaning for the combination of R¹, R² and R³ for each individual compound corresponds in each case to one line of Table B (compounds I.Be.E-64.B1 to I.Be.E-64.B442).
- Table 65g: Compounds of the formula I.Be in which the combination of (R⁴)ₙ and (R^{L})ₘ corresponds to line E-65 of Table E and the meaning for the combination of R¹, R² and R³ for each individual compound corresponds in each case to one line of Table B (compounds I.Be.E-65.B1 to I.Be.E-65.B442).
- Table 66g: Compounds of the formula I.Be in which the combination of (R⁴)ₙ and (R^{L})ₘ corresponds to line E-66 of Table E and the meaning for the combination of R¹, R² and R³ for each individual compound corresponds in each case to one line of Table B (compounds I.Be.E-66.B1 to I.Be.E-66.B442).
- Table 67g: Compounds of the formula I.Be in which the combination of (R⁴)ₙ and (R^{L})ₘ corresponds to line E-67 of Table E and the meaning for the combination of R¹, R² and R³ for each individual compound corresponds in each case to one line of Table B (compounds I.Be.E-67.B1 to I.Be.E-67.B442).
- Table 68g: Compounds of the formula I.Be in which the combination of (R⁴)ₙ and (R^{L})ₘ corresponds to line E-68 of Table E and the meaning for the combination of R¹, R² and R³ for each individual compound corresponds in each case to one line of Table B (compounds I.Be.E-68.B1 to I.Be.E-68.B442).
- Table 69g: Compounds of the formula I.Be in which the combination of (R⁴)ₙ and (R^{L})ₘ corresponds to line E-69 of Table E and the meaning for the combination of R¹, R² and R³ for each individual compound corresponds in each case to one line of Table B (compounds I.Be.E-69.B1 to I.Be.E-69.B442).
- Table 70g: Compounds of the formula I.Be in which the combination of (R⁴)ₙ and (R^{L})ₘ corresponds to line E-70 of Table E and the meaning for the combination of R¹, R² and R³ for each individual compound corresponds in each case to one line of Table B (compounds I.Be.E-70.B1 to I.Be.E-70.B442).
- Table 1h: Compounds of the formula I.Bf in which the combination of (R⁴)ₙ and (R^{L})ₘ corresponds to line E-1 of Table E and the meaning for the combination of R¹, R² and R³ for each individual compound corresponds in each case to one line of Table B (compounds I.Bf.E-1.B1 to I.Bf.E-1.B442).
- Table 2h: Compounds of the formula I.Bf in which the combination of (R⁴)ₙ and (R^{L})ₘ corresponds to line E-2 of Table E and the meaning for the combination of R¹, R² and R³ for each individual compound corresponds in each case to one line of Table B (compounds I.Bf.E-2.B1 to I.Bf.E-2.B442).
- Table 3h: Compounds of the formula I.Bf in which the combination of (R⁴)ₙ and (R^{L})ₘ corresponds to line E-3 of Table E and the meaning for the combination of R¹, R² and R³ for each individual compound corresponds in each case to one line of Table B (compounds I.Bf.E-3.B1 to I.Bf.E-3.B442).
- Table 4h: Compounds of the formula I.Bf in which the combination of (R⁴)ₙ and (R^{L})ₘ corresponds to line E-4 of Table E and the meaning for the combination of R¹, R² and R³ for each individual compound corresponds in each case to one line of Table B (compounds I.Bf.E-4.B1 to I.Bf.E-4.B442).
- Table 5h: Compounds of the formula I.Bf in which the combination of (R⁴)ₙ and (R^{L})ₘ corresponds to line E-5 of Table E and the meaning for the combination of R¹, R² and R³ for each individual compound corresponds in each case to one line of Table B (compounds I.Bf.E-5.B1 to I.Bf.E-5.B442).
- Table 6h: Compounds of the formula I.Bf in which the combination of (R⁴)ₙ and (R^{L})ₘ corresponds to line E-6 of Table E and the meaning for the combination of R¹, R² and R³ for each individual compound corresponds in each case to one line of Table B (compounds I.Bf.E-6.B1 to I.Bf.E-6.B442).
- Table 7h: Compounds of the formula I.Bf in which the combination of (R⁴)ₙ and (R^{L})ₘ corresponds to line E-7 of Table E and the meaning for the combination of R¹, R² and R³ for each individual compound corresponds in each case to one line of Table B (compounds I.Bf.E-7.B1 to I.Bf.E-7.B442).
- Table 8h: Compounds of the formula I.Bf in which the combination of (R⁴)ₙ and (R^{L})ₘ corresponds to line E-8 of Table E and the meaning for the combination of R¹, R² and R³ for each individual compound corresponds in each case to one line of Table B (compounds I.Bf.E-8.B1 to I.Bf.E-8.B442).
- Table 9h: Compounds of the formula I.Bf in which the combination of (R⁴)ₙ and (R^{L})ₘ corresponds to line E-9 of Table E and the meaning for the combination of R¹, R² and R³ for each individual compound corresponds in each case to one line of Table B (compounds I.Bf.E-9.B1 to I.Bf.E-9.B442).
- Table 10h: Compounds of the formula I.Bf in which the combination of (R⁴)ₙ and (R^{L})ₘ corresponds to line E-10 of Table E and the meaning for the combination of R¹, R² and R³ for each individual compound corresponds in each case to one line of Table B (compounds I.Bf.E-10.B1 to I.Bf.E-10.B442).
- Table 11h: Compounds of the formula I.Bf in which the combination of (R⁴)ₙ and (R^{L})ₘ corresponds to line E-11 of Table E and the meaning for the combination of R¹, R² and R³ for each individual compound corresponds in each case to one line of Table B (compounds I.Bf.E-11.B1 to I.Bf.E-11.B442).
- Table 12h: Compounds of the formula I.Bf in which the combination of (R⁴)ₙ and (R^{L})ₘ corresponds to line E-12 of Table E and the meaning for the combination of R¹, R² and R³ for each individual compound corresponds in each case to one line of Table B (compounds I.Bf.E-12.B1 to I.Bf.E-12.B442).
- Table 13h: Compounds of the formula I.Bf in which the combination of (R⁴)ₙ and (R^{L})ₘ corresponds to line E-13 of Table E and the meaning for the combination of R¹, R² and R³ for each individual compound corresponds in each case to one line of Table B (compounds I.Bf.E-13.B1 to I.Bf.E-13.B442).
- Table 14h: Compounds of the formula I.Bf in which the combination of (R⁴)ₙ and (R^{L})ₘ corresponds to line E-14 of Table E and the meaning for the combination of R¹, R² and R³ for each individual compound corresponds in each case to one line of Table B (compounds I.Bf.E-14.B1 to I.Bf.E-14.B442).
- Table 15h: Compounds of the formula I.Bf in which the combination of (R⁴)ₙ and (R^{L})ₘ corresponds to line E-15 of Table E and the meaning for the combination of R¹, R² and R³ for each individual compound corresponds in each case to one line of Table B (compounds I.Bf.E-15.B1 to I.Bf.E-15.B442).
- Table 16h: Compounds of the formula I.Bf in which the combination of (R⁴)ₙ and (R^{L})ₘ corresponds to line E-16 of Table E and the meaning for the combination of R¹, R² and R³ for each individual compound corresponds in each case to one line of Table B (compounds I.Bf.E-16.B1 to I.Bf.E-16.B442).
- Table 17h: Compounds of the formula I.Bf in which the combination of (R⁴)ₙ and (R^{L})ₘ corresponds to line E-17 of Table E and the meaning for the combination of R¹, R² and R³ for each individual compound corresponds in each case to one line of Table B (compounds I.Bf.E-17.B1 to I.Bf.E-17.B442).
- Table 18h: Compounds of the formula I.Bf in which the combination of (R⁴)ₙ and (R^{L})ₘ corresponds to line E-18 of Table E and the meaning for the combination of R¹, R² and R³ for each individual compound corresponds in each case to one line of Table B (compounds I.Bf.E-18.B1 to I.Bf.E-18.B442).
- Table 19h: Compounds of the formula I.Bf in which the combination of (R⁴)ₙ and (R^{L})ₘ corresponds to line E-19 of Table E and the meaning for the combination of R¹, R² and R³ for each individual compound corresponds in each case to one line of Table B (compounds I.Bf.E-19.B1 to I.Bf.E-19.B442).
- Table 20h: Compounds of the formula I.Bf in which the combination of (R⁴)ₙ and (R^{L})ₘ corresponds to line E-20 of Table E and the meaning for the combination of R¹, R² and R³ for each individual compound corresponds in each case to one line of Table B (compounds I.Bf.E-20.B1 to I.Bf.E-20.B442).
- Table 21h: Compounds of the formula I.Bf in which the combination of (R⁴)ₙ and (R^{L})ₘ corresponds to line E-21 of Table E and the meaning for the combination of R¹, R² and R³ for each individual compound corresponds in each case to one line of Table B (compounds I.Bf.E-21.B1 to I.Bf.E-21.B442).
- Table 22h: Compounds of the formula I.Bf in which the combination of (R⁴)ₙ and (R^{L})ₘ corresponds to line E-22 of Table E and the meaning for the combination of R¹, R² and R³ for each individual compound corresponds in each case to one line of Table B (compounds I.Bf.E-22.B1 to I.Bf.E-22.B442).
- Table 23h: Compounds of the formula I.Bf in which the combination of (R⁴)ₙ and (R^{L})ₘ corresponds to line E-23 of Table E and the meaning for the combination of R¹, R² and R³ for each individual compound corresponds in each case to one line of Table B (compounds I.Bf.E-23.B1 to I.Bf.E-23.B442).
- Table 24h: Compounds of the formula I.Bf in which the combination of (R⁴)ₙ and (R^{L})ₘ corresponds to line E-24 of Table E and the meaning for the combination of R¹, R² and R³ for each individual compound corresponds in each case to one line of Table B (compounds I.Bf.E-24.B1 to I.Bf.E-24.B442).
- Table 25h: Compounds of the formula I.Bf in which the combination of (R⁴)ₙ and (R^{L})ₘ corresponds to line E-25 of Table E and the meaning for the combination of R¹, R² and R³ for each individual compound corresponds in each case to one line of Table B (compounds I.Bf.E-25.B1 to I.Bf.E-25.B442).
- Table 26h: Compounds of the formula I.Bf in which the combination of (R⁴)ₙ and (R^{L})ₘ corresponds to line E-26 of Table E and the meaning for the combination of R¹, R² and R³ for each individual compound corresponds in each case to one line of Table B (compounds I.Bf.E-26.B1 to I.Bf.E-26.B442).
- Table 27h: Compounds of the formula I.Bf in which the combination of (R⁴)ₙ and (R^{L})ₘ corresponds to line E-27 of Table E and the meaning for the combination of R¹, R² and R³ for each individual compound corresponds in each case to one line of Table B (compounds I.Bf.E-27.B1 to I.Bf.E-27.B442).
- Table 28h: Compounds of the formula I.Bf in which the combination of (R⁴)ₙ and (R^{L})ₘ corresponds to line E-28 of Table E and the meaning for the combination of R¹, R² and R³ for each individual compound corresponds in each case to one line of Table B (compounds I.Bf.E-28.B1 to I.Bf.E-28.B442).
- Table 29h: Compounds of the formula I.Bf in which the combination of (R⁴)ₙ and (R^{L})ₘ corresponds to line E-29 of Table E and the meaning for the combination of R¹, R² and R³ for each individual compound corresponds in each case to one line of Table B (compounds I.Bf.E-29.B1 to I.Bf.E-29.B442).
- Table 30h: Compounds of the formula I.Bf in which the combination of (R⁴)ₙ and (R^{L})ₘ corresponds to line E-30 of Table E and the meaning for the combination of R¹, R² and R³ for each individual compound corresponds in each case to one line of Table B (compounds I.Bf.E-30.B1 to I.Bf.E-30.B442).
- Table 31h: Compounds of the formula I.Bf in which the combination of (R⁴)ₙ and (R^{L})ₘ corresponds to line E-31 of Table E and the meaning for the combination of R¹, R² and R³ for each individual compound corresponds in each case to one line of Table B (compounds I.Bf.E-31.B1 to I.Bf.E-31.B442).
- Table 32h: Compounds of the formula I.Bf in which the combination of (R⁴)ₙ and (R^{L})ₘ corresponds to line E-32 of Table E and the meaning for the combination of R¹, R² and R³ for each individual compound corresponds in each case to one line of Table B (compounds I.Bf.E-32.B1 to I.Bf.E-32.B442).
- Table 33h: Compounds of the formula I.Bf in which the combination of (R⁴)ₙ and (R^{L})ₘ corresponds to line E-33 of Table E and the meaning for the combination of R¹, R² and R³ for each individual compound corresponds in each case to one line of Table B (compounds I.Bf.E-33.B1 to I.Bf.E-33.B442).
- Table 34h: Compounds of the formula I.Bf in which the combination of (R⁴)ₙ and (R^{L})ₘ corresponds to line E-34 of Table E and the meaning for the combination of R¹, R² and R³ for each individual compound corresponds in each case to one line of Table B (compounds I.Bf.E-34.B1 to I.Bf.E-34.B442).
- Table 35h: Compounds of the formula I.Bf in which the combination of (R⁴)ₙ and (R^{L})ₘ corresponds to line E-35 of Table E and the meaning for the combination of R¹, R² and R³ for each individual compound corresponds in each case to one line of Table B (compounds I.Bf.E-35.B1 to I.Bf.E-35.B442).
- Table 36h: Compounds of the formula I.Bf in which the combination of (R⁴)ₙ and (R^{L})ₘ corresponds to line E-36 of Table E and the meaning for the combination of R¹, R² and R³ for each individual compound corresponds in each case to one line of Table B (compounds I.Bf.E-36.B1 to I.Bf.E-36.B442).
- Table 37h: Compounds of the formula I.Bf in which the combination of (R⁴)ₙ and (R^{L})ₘ corresponds to line E-37 of Table E and the meaning for the combination of R¹, R² and R³ for each individual compound corresponds in each case to one line of Table B (compounds I.Bf.E-37.B1 to I.Bf.E-37.B442).
- Table 38h: Compounds of the formula I.Bf in which the combination of (R⁴)ₙ and (R^{L})ₘ corresponds to line E-38 of Table E and the meaning for the combination of R¹, R² and R³ for each individual compound corresponds in each case to one line of Table B (compounds I.Bf.E-38.B1 to I.Bf.E-38.B442).
- Table 39h: Compounds of the formula I.Bf in which the combination of (R⁴)ₙ and (R^{L})ₘ corresponds to line E-39 of Table E and the meaning for the combination of R¹, R² and R³ for each individual compound corresponds in each case to one line of Table B (compounds I.Bf.E-39.B1 to I.Bf.E-39.B442).
- Table 40h: Compounds of the formula I.Bf in which the combination of (R⁴)ₙ and (R^{L})ₘ corresponds to line E-40 of Table E and the meaning for the combination of R¹, R² and R³ for each individual compound corresponds in each case to one line of Table B (compounds I.Bf.E-40.B1 to I.Bf.E-40.B442).
- Table 41h: Compounds of the formula I.Bf in which the combination of (R⁴)ₙ and (R^{L})ₘ corresponds to line E-41 of Table E and the meaning for the combination of R¹, R² and R³ for each individual compound corresponds in each case to one line of Table B (compounds I.Bf.E-41.B1 to I.Bf.E-41.B442).
- Table 42h: Compounds of the formula I.Bf in which the combination of (R⁴)ₙ and (R^{L})ₘ corresponds to line E-42 of Table E and the meaning for the combination of R¹, R² and R³ for each individual compound corresponds in each case to one line of Table B (compounds I.Bf.E-42.B1 to I.Bf.E-42.B442).
- Table 43h: Compounds of the formula I.Bf in which the combination of (R⁴)ₙ and (R^{L})ₘ corresponds to line E-43 of Table E and the meaning for the combination of R¹, R² and R³ for each individual compound corresponds in each case to one line of Table B (compounds I.Bf.E-43.B1 to I.Bf.E-43.B442).
- Table 44h: Compounds of the formula I.Bf in which the combination of (R⁴)ₙ and (R^{L})ₘ corresponds to line E-44 of Table E and the meaning for the combination of R¹, R² and R³ for each individual compound corresponds in each case to one line of Table B (compounds I.Bf.E-44.B1 to I.Bf.E-44.B442).
- Table 45h: Compounds of the formula I.Bf in which the combination of (R⁴)ₙ and (R^{L})ₘ corresponds to line E-45 of Table E and the meaning for the combination of R¹, R² and R³ for each individual compound corresponds in each case to one line of Table B (compounds I.Bf.E-45.B1 to I.Bf.E-45.B442).
- Table 46h: Compounds of the formula I.Bf in which the combination of (R⁴)ₙ and (R^{L})ₘ corresponds to line E-46 of Table E and the meaning for the combination of R¹, R² and R³ for each individual compound corresponds in each case to one line of Table B (compounds I.Bf.E-46.B1 to I.Bf.E-46.B442).
- Table 47h: Compounds of the formula I.Bf in which the combination of (R⁴)ₙ and (R^{L})ₘ corresponds to line E-47 of Table E and the meaning for the combination of R¹, R² and R³ for each individual compound corresponds in each case to one line of Table B (compounds I.Bf.E-47.B1 to I.Bf.E-47.B442).
- Table 48h: Compounds of the formula I.Bf in which the combination of (R⁴)ₙ and (R^{L})ₘ corresponds to line E-48 of Table E and the meaning for the combination of R¹, R² and R³ for each individual compound corresponds in each case to one line of Table B (compounds I.Bf.E-48.B1 to I.Bf.E-48.B442).
- Table 49h: Compounds of the formula I.Bf in which the combination of (R⁴)ₙ and (R^{L})ₘ corresponds to line E-49 of Table E and the meaning for the combination of R¹, R² and R³ for each individual compound corresponds in each case to one line of Table B (compounds I.Bf.E-49.B1 to I.Bf.E-49.B442).
- Table 50h: Compounds of the formula I.Bf in which the combination of (R⁴)ₙ and (R^{L})ₘ corresponds to line E-50 of Table E and the meaning for the combination of R¹, R² and R³ for each individual compound corresponds in each case to one line of Table B (compounds I.Bf.E-50.B1 to I.Bf.E-50.B442).
- Table 51h: Compounds of the formula I.Bf in which the combination of (R⁴)ₙ and (R^{L})ₘ corresponds to line E-51 of Table E and the meaning for the combination of R¹, R² and R³ for each individual compound corresponds in each case to one line of Table B (compounds I.Bf.E-51.B1 to I.Bf.E-51.B442).
- Table 52h: Compounds of the formula I.Bf in which the combination of (R⁴)ₙ and (R^{L})ₘ corresponds to line E-52 of Table E and the meaning for the combination of R¹, R² and R³ for each individual compound corresponds in each case to one line of Table B (compounds I.Bf.E-52.B1 to I.Bf.E-52.B442).
- Table 53h: Compounds of the formula I.Bf in which the combination of (R⁴)ₙ and (R^{L})ₘ corresponds to line E-53 of Table E and the meaning for the combination of R¹, R² and R³ for each individual compound corresponds in each case to one line of Table B (compounds I.Bf.E-53.B1 to I.Bf.E-53.B442).
- Table 54h: Compounds of the formula I.Bf in which the combination of (R⁴)ₙ and (R^{L})ₘ corresponds to line E-54 of Table E and the meaning for the combination of R¹, R² and R³ for each individual compound corresponds in each case to one line of Table B (compounds I.Bf.E-54.B1 to I.Bf.E-54.B442).
- Table 55h: Compounds of the formula I.Bf in which the combination of (R⁴)ₙ and (R^{L})ₘ corresponds to line E-55 of Table E and the meaning for the combination of R¹, R² and R³ for each individual compound corresponds in each case to one line of Table B (compounds I.Bf.E-55.B1 to I.Bf.E-55.B442).
- Table 56h: Compounds of the formula I.Bf in which the combination of (R⁴)ₙ and (R^{L})ₘ corresponds to line E-56 of Table E and the meaning for the combination of R¹, R² and R³ for each individual compound corresponds in each case to one line of Table B (compounds I.Bf.E-56.B1 to I.Bf.E-56.B442).
- Table 57h: Compounds of the formula I.Bf in which the combination of (R⁴)ₙ and (R^{L})ₘ corresponds to line E-57 of Table E and the meaning for the combination of R¹, R² and R³ for each individual compound corresponds in each case to one line of Table B (compounds I.Bf.E-57.B1 to I.Bf.E-57.B442).
- Table 58h: Compounds of the formula I.Bf in which the combination of (R⁴)ₙ and (R^{L})ₘ corresponds to line E-58 of Table E and the meaning for the combination of R¹, R² and R³ for each individual compound corresponds in each case to one line of Table B (compounds I.Bf.E-58.B1 to I.Bf.E-58.B442).
- Table 59h: Compounds of the formula I.Bf in which the combination of (R⁴)ₙ and (R^{L})ₘ corresponds to line E-59 of Table E and the meaning for the combination of R¹, R² and R³ for each individual compound corresponds in each case to one line of Table B (compounds I.Bf.E-59.B1 to I.Bf.E-59.B442).
- Table 60h: Compounds of the formula I.Bf in which the combination of (R⁴)ₙ and (R^{L})ₘ corresponds to line E-60 of Table E and the meaning for the combination of R¹, R² and R³ for each individual compound corresponds in each case to one line of Table B (compounds I.Bf.E-60.B1 to I.Bf.E-60.B442).
- Table 61h: Compounds of the formula I.Bf in which the combination of (R⁴)ₙ and (R^{L})ₘ corresponds to line E-61 of Table E and the meaning for the combination of R¹, R² and R³ for each individual compound corresponds in each case to one line of Table B (compounds I.Bf.E-61.B1 to I.Bf.E-61.B442).
- Table 62h: Compounds of the formula I.Bf in which the combination of (R⁴)ₙ and (R^{L})ₘ corresponds to line E-62 of Table E and the meaning for the combination of R¹, R² and R³ for each individual compound corresponds in each case to one line of Table B (compounds I.Bf.E-62.B1 to I.Bf.E-62.B442).
- Table 63h: Compounds of the formula I.Bf in which the combination of (R⁴)ₙ and (R^{L})ₘ corresponds to line E-63 of Table E and the meaning for the combination of R¹, R² and R³ for each individual compound corresponds in each case to one line of Table B (compounds I.Bf.E-63.B1 to I.Bf.E-63.B442).
- Table 64h: Compounds of the formula I.Bf in which the combination of (R⁴)ₙ and (R^{L})ₘ corresponds to line E-64 of Table E and the meaning for the combination of R¹, R² and R³ for each individual compound corresponds in each case to one line of Table B (compounds I.Bf.E-64.B1 to I.Bf.E-64.B442).
- Table 65h: Compounds of the formula I.Bf in which the combination of (R⁴)ₙ and (R^{L})ₘ corresponds to line E-65 of Table E and the meaning for the combination of R¹, R² and R³ for each individual compound corresponds in each case to one line of Table B (compounds I.Bf.E-65.B1 to I.Bf.E-65.B442).
- Table 66h: Compounds of the formula I.Bf in which the combination of (R⁴)ₙ and (R^{L})ₘ corresponds to line E-66 of Table E and the meaning for the combination of R¹, R² and R³ for each individual compound corresponds in each case to one line of Table B (compounds I.Bf.E-66.B1 to I.Bf.E-66.B442).
- Table 67h: Compounds of the formula I.Bf in which the combination of (R⁴)ₙ and (R^{L})ₘ corresponds to line E-67 of Table E and the meaning for the combination of R¹, R² and R³ for each individual compound corresponds in each case to one line of Table B (compounds I.Bf.E-67.B1 to I.Bf.E-67.B442).
- Table 68h: Compounds of the formula I.Bf in which the combination of (R⁴)ₙ and (R^{L})ₘ corresponds to line E-68 of Table E and the meaning for the combination of R¹, R² and R³ for each individual compound corresponds in each case to one line of Table B (compounds I.Bf.E-68.B1 to I.Bf.E-68.B442).
- Table 69h: Compounds of the formula I.Bf in which the combination of (R⁴)ₙ and (R^{L})ₘ corresponds to line E-69 of Table E and the meaning for the combination of R¹, R² and R³ for each individual compound corresponds in each case to one line of Table B (compounds I.Bf.E-69.B1 to I.Bf.E-69.B442).
- Table 70h: Compounds of the formula I.Bf in which the combination of (R⁴)ₙ and (R^{L})ₘ corresponds to line E-70 of Table E and the meaning for the combination of R¹, R² and R³ for each individual compound corresponds in each case to one line of Table B (compounds I.Bf.E-70.B1 to I.Bf.E-70.B442).
- Table 1i: Compounds of the formula I.Bg in which the combination of (R⁴)ₙ and (R^{L})ₘ corresponds to line E-1 of Table E and the meaning for the combination of R¹, R² and R³ for each individual compound corresponds in each case to one line of Table B (compounds I.Bg.E-1.B1 to I.Bg.E-1.B442).
- Table 2i: Compounds of the formula I.Bg in which the combination of (R⁴)ₙ and (R^{L})ₘ corresponds to line E-2 of Table E and the meaning for the combination of R¹, R² and R³ for each individual compound corresponds in each case to one line of Table B (compounds I.Bg.E-2.B1 to I.Bg.E-2.B442).
- Table 3i: Compounds of the formula I.Bg in which the combination of (R⁴)ₙ and (R^{L})ₘ corresponds to line E-3 of Table E and the meaning for the combination of R¹, R² and R³ for each individual compound corresponds in each case to one line of Table B (compounds I.Bg.E-3.B1 to I.Bg.E-3.B442).
- Table 4i: Compounds of the formula I.Bg in which the combination of (R⁴)ₙ and (R^{L})ₘ corresponds to line E-4 of Table E and the meaning for the combination of R¹, R² and R³ for each individual compound corresponds in each case to one line of Table B (compounds I.Bg.E-4.B1 to I.Bg.E-4.B442).
- Table 5i: Compounds of the formula I.Bg in which the combination of (R⁴)ₙ and (R^{L})ₘ corresponds to line E-5 of Table E and the meaning for the combination of R¹, R² and R³ for each individual compound corresponds in each case to one line of Table B (compounds I.Bg.E-5.B1 to I.Bg.E-5.B442).
- Table 6i: Compounds of the formula I.Bg in which the combination of (R⁴)ₙ and (R^{L})ₘ corresponds to line E-6 of Table E and the meaning for the combination of R¹, R² and R³ for each individual compound corresponds in each case to one line of Table B (compounds I.Bg.E-6.B1 to I.Bg.E-6.B442).
- Table 7i: Compounds of the formula I.Bg in which the combination of (R⁴)ₙ and (R^{L})ₘ corresponds to line E-7 of Table E and the meaning for the combination of R¹, R² and R³ for each individual compound corresponds in each case to one line of Table B (compounds I.Bg.E-7.B1 to I.Bg.E-7.B442).
- Table 8i: Compounds of the formula I.Bg in which the combination of (R⁴)ₙ and (R^{L})ₘ corresponds to line E-8 of Table E and the meaning for the combination of R¹, R² and R³ for each individual compound corresponds in each case to one line of Table B (compounds I.Bg.E-8.B1 to I.Bg.E-8.B442).
- Table 9i: Compounds of the formula I.Bg in which the combination of (R⁴)ₙ and (R^{L})ₘ corresponds to line E-9 of Table E and the meaning for the combination of R¹, R² and R³ for each individual compound corresponds in each case to one line of Table B (compounds I.Bg.E-9.B1 to I.Bg.E-9.B442).
- Table 10i: Compounds of the formula I.Bg in which the combination of (R⁴)ₙ and (R^{L})ₘ corresponds to line E-10 of Table E and the meaning for the combination of R¹, R² and R³ for each individual compound corresponds in each case to one line of Table B (compounds I.Bg.E-10.B1 to I.Bg.E-10.B442).
- Table 11i: Compounds of the formula I.Bg in which the combination of (R⁴)ₙ and (R^{L})ₘ corresponds to line E-11 of Table E and the meaning for the combination of R¹, R² and R³ for each individual compound corresponds in each case to one line of Table B (compounds I.Bg.E-11.B1 to I.Bg.E-11.B442).
- Table 12i: Compounds of the formula I.Bg in which the combination of (R⁴)ₙ and (R^{L})ₘ corresponds to line E-12 of Table E and the meaning for the combination of R¹, R² and R³ for each individual compound corresponds in each case to one line of Table B (compounds I.Bg.E-12.B1 to I.Bg.E-12.B442).
- Table 13i: Compounds of the formula I.Bg in which the combination of (R⁴)ₙ and (R^{L})ₘ corresponds to line E-13 of Table E and the meaning for the combination of R¹, R² and R³ for each individual compound corresponds in each case to one line of Table B (compounds I.Bg.E-13.B1 to I.Bg.E-13.B442).
- Table 14i: Compounds of the formula I.Bg in which the combination of (R⁴)ₙ and (R^{L})ₘ corresponds to line E-14 of Table E and the meaning for the combination of R¹, R² and R³ for each individual compound corresponds in each case to one line of Table B (compounds I.Bg.E-14.B1 to I.Bg.E-14.B442).
- Table 15i: Compounds of the formula I.Bg in which the combination of (R⁴)ₙ and (R^{L})ₘ corresponds to line E-15 of Table E and the meaning for the combination of R¹, R² and R³ for each individual compound corresponds in each case to one line of Table B (compounds I.Bg.E-15.B1 to I.Bg.E-15.B442).
- Table 16i: Compounds of the formula I.Bg in which the combination of (R⁴)ₙ and (R^{L})ₘ corresponds to line E-16 of Table E and the meaning for the combination of R¹, R² and R³ for each individual compound corresponds in each case to one line of Table B (compounds I.Bg.E-16.B1 to I.Bg.E-16.B442).
- Table 17i: Compounds of the formula I.Bg in which the combination of (R⁴)ₙ and (R^{L})ₘ corresponds to line E-17 of Table E and the meaning for the combination of R¹, R² and R³ for each individual compound corresponds in each case to one line of Table B (compounds I.Bg.E-17.B1 to I.Bg.E-17.B442).
- Table 18i: Compounds of the formula I.Bg in which the combination of (R⁴)ₙ and (R^{L})ₘ corresponds to line E-18 of Table E and the meaning for the combination of R¹, R² and R³ for each individual compound corresponds in each case to one line of Table B (compounds I.Bg.E-18.B1 to I.Bg.E-18.B442).
- Table 19i: Compounds of the formula I.Bg in which the combination of (R⁴)ₙ and (R^{L})ₘ corresponds to line E-19 of Table E and the meaning for the combination of R¹, R² and R³ for each individual compound corresponds in each case to one line of Table B (compounds I.Bg.E-19.B1 to I.Bg.E-19.B442).
- Table 20i: Compounds of the formula I.Bg in which the combination of (R⁴)ₙ and (R^{L})ₘ corresponds to line E-20 of Table E and the meaning for the combination of R¹, R² and R³ for each individual compound corresponds in each case to one line of Table B (compounds I.Bg.E-20.B1 to I.Bg.E-20.B442).
- Table 21i: Compounds of the formula I.Bg in which the combination of (R⁴)ₙ and (R^{L})ₘ corresponds to line E-21 of Table E and the meaning for the combination of R¹, R² and R³ for each individual compound corresponds in each case to one line of Table B (compounds I.Bg.E-21.B1 to I.Bg.E-21.B442).
- Table 22i: Compounds of the formula I.Bg in which the combination of (R⁴)ₙ and (R^{L})ₘ corresponds to line E-22 of Table E and the meaning for the combination of R¹, R² and R³ for each individual compound corresponds in each case to one line of Table B (compounds I.Bg.E-22.B1 to I.Bg.E-22.B442).
- Table 23i: Compounds of the formula I.Bg in which the combination of (R⁴)ₙ and (R^{L})ₘ corresponds to line E-23 of Table E and the meaning for the combination of R¹, R² and R³ for each individual compound corresponds in each case to one line of Table B (compounds I.Bg.E-23.B1 to I.Bg.E-23.B442).
- Table 24i: Compounds of the formula I.Bg in which the combination of (R⁴)ₙ and (R^{L})ₘ corresponds to line E-24 of Table E and the meaning for the combination of R¹, R² and R³ for each individual compound corresponds in each case to one line of Table B (compounds I.Bg.E-24.B1 to I.Bg.E-24.B442).
- Table 25i: Compounds of the formula I.Bg in which the combination of (R⁴)ₙ and (R^{L})ₘ corresponds to line E-25 of Table E and the meaning for the combination of R¹, R² and R³ for each individual compound corresponds in each case to one line of Table B (compounds I.Bg.E-25.B1 to I.Bg.E-25.B442).
- Table 26i: Compounds of the formula I.Bg in which the combination of (R⁴)ₙ and (R^{L})ₘ corresponds to line E-26 of Table E and the meaning for the combination of R¹, R² and R³ for each individual compound corresponds in each case to one line of Table B (compounds I.Bg.E-26.B1 to I.Bg.E-26.B442).
- Table 27i: Compounds of the formula I.Bg in which the combination of (R⁴)ₙ and (R^{L})ₘ corresponds to line E-27 of Table E and the meaning for the combination of R¹, R² and R³ for each individual compound corresponds in each case to one line of Table B (compounds I.Bg.E-27.B1 to I.Bg.E-27.B442).
- Table 28i: Compounds of the formula I.Bg in which the combination of (R⁴)ₙ and (R^{L})ₘ corresponds to line E-28 of Table E and the meaning for the combination of R¹, R² and R³ for each individual compound corresponds in each case to one line of Table B (compounds I.Bg.E-28.B1 to I.Bg.E-28.B442).
- Table 29i: Compounds of the formula I.Bg in which the combination of (R⁴)ₙ and (R^{L})ₘ corresponds to line E-29 of Table E and the meaning for the combination of R¹, R² and R³ for each individual compound corresponds in each case to one line of Table B (compounds I.Bg.E-29.B1 to I.Bg.E-29.B442).
- Table 30i: Compounds of the formula I.Bg in which the combination of (R⁴)ₙ and (R^{L})ₘ corresponds to line E-30 of Table E and the meaning for the combination of R¹, R² and R³ for each individual compound corresponds in each case to one line of Table B (compounds I.Bg.E-30.B1 to I.Bg.E-30.B442).
- Table 31i: Compounds of the formula I.Bg in which the combination of (R⁴)ₙ and (R^{L})ₘ corresponds to line E-31 of Table E and the meaning for the combination of R¹, R² and R³ for each individual compound corresponds in each case to one line of Table B (compounds I.Bg.E-31.B1 to I.Bg.E-31.B442).
- Table 32i: Compounds of the formula I.Bg in which the combination of (R⁴)ₙ and (R^{L})ₘ corresponds to line E-32 of Table E and the meaning for the combination of R¹, R² and R³ for each individual compound corresponds in each case to one line of Table B (compounds I.Bg.E-32.B1 to I.Bg.E-32.B442).
- Table 33i: Compounds of the formula I.Bg in which the combination of (R⁴)ₙ and (R^{L})ₘ corresponds to line E-33 of Table E and the meaning for the combination of R¹, R² and R³ for each individual compound corresponds in each case to one line of Table B (compounds I.Bg.E-33.B1 to I.Bg.E-33.B442).
- Table 34i: Compounds of the formula I.Bg in which the combination of (R⁴)ₙ and (R^{L})ₘ corresponds to line E-34 of Table E and the meaning for the combination of R¹, R² and R³ for each individual compound corresponds in each case to one line of Table B (compounds I.Bg.E-34.B1 to I.Bg.E-34.B442).
- Table 35i: Compounds of the formula I.Bg in which the combination of (R⁴)ₙ and (R^{L})ₘ corresponds to line E-35 of Table E and the meaning for the combination of R¹, R² and R³ for each individual compound corresponds in each case to one line of Table B (compounds I.Bg.E-35.B1 to I.Bg.E-35.B442).
- Table 36i: Compounds of the formula I.Bg in which the combination of (R⁴)ₙ and (R^{L})ₘ corresponds to line E-36 of Table E and the meaning for the combination of R¹, R² and R³ for each individual compound corresponds in each case to one line of Table B (compounds I.Bg.E-36.B1 to I.Bg.E-36.B442).
- Table 37i: Compounds of the formula I.Bg in which the combination of (R⁴)ₙ and (R^{L})ₘ corresponds to line E-37 of Table E and the meaning for the combination of R¹, R² and R³ for each individual compound corresponds in each case to one line of Table B (compounds I.Bg.E-37.B1 to I.Bg.E-37.B442).
- Table 38i: Compounds of the formula I.Bg in which the combination of (R⁴)ₙ and (R^{L})ₘ corresponds to line E-38 of Table E and the meaning for the combination of R¹, R² and R³ for each individual compound corresponds in each case to one line of Table B (compounds I.Bg.E-38.B1 to I.Bg.E-38.B442).
- Table 39i: Compounds of the formula I.Bg in which the combination of (R⁴)ₙ and (R^{L})ₘ corresponds to line E-39 of Table E and the meaning for the combination of R¹, R² and R³ for each individual compound corresponds in each case to one line of Table B (compounds I.Bg.E-39.B1 to I.Bg.E-39.B442).
- Table 40i: Compounds of the formula I.Bg in which the combination of (R⁴)ₙ and (R^{L})ₘ corresponds to line E-40 of Table E and the meaning for the combination of R¹, R² and R³ for each individual compound corresponds in each case to one line of Table B (compounds I.Bg.E-40.B1 to I.Bg.E-40.B442).
- Table 41i: Compounds of the formula I.Bg in which the combination of (R⁴)ₙ and (R^{L})ₘ corresponds to line E-41 of Table E and the meaning for the combination of R¹, R² and R³ for each individual compound corresponds in each case to one line of Table B (compounds I.Bg.E-41.B1 to I.Bg.E-41.B442).
- Table 42i: Compounds of the formula I.Bg in which the combination of (R⁴)ₙ and (R^{L})ₘ corresponds to line E-42 of Table E and the meaning for the combination of R¹, R² and R³ for each individual compound corresponds in each case to one line of Table B (compounds I.Bg.E-42.B1 to I.Bg.E-42.B442).
- Table 43i: Compounds of the formula I.Bg in which the combination of (R⁴)ₙ and (R^{L})ₘ corresponds to line E-43 of Table E and the meaning for the combination of R¹, R² and R³ for each individual compound corresponds in each case to one line of Table B (compounds I.Bg.E-43.B1 to I.Bg.E-43.B442).
- Table 44i: Compounds of the formula I.Bg in which the combination of (R⁴)ₙ and (R^{L})ₘ corresponds to line E-44 of Table E and the meaning for the combination of R¹, R² and R³ for each individual compound corresponds in each case to one line of Table B (compounds I.Bg.E-44.B1 to I.Bg.E-44.B442).
- Table 45i: Compounds of the formula I.Bg in which the combination of (R⁴)ₙ and (R^{L})ₘ corresponds to line E-45 of Table E and the meaning for the combination of R¹, R² and R³ for each individual compound corresponds in each case to one line of Table B (compounds I.Bg.E-45.B1 to I.Bg.E-45.B442).
- Table 46i: Compounds of the formula I.Bg in which the combination of (R⁴)ₙ and (R^{L})ₘ corresponds to line E-46 of Table E and the meaning for the combination of R¹, R² and R³ for each individual compound corresponds in each case to one line of Table B (compounds I.Bg.E-46.B1 to I.Bg.E-46.B442).
- Table 47i: Compounds of the formula I.Bg in which the combination of (R⁴)ₙ and (R^{L})ₘ corresponds to line E-47 of Table E and the meaning for the combination of R¹, R² and R³ for each individual compound corresponds in each case to one line of Table B (compounds I.Bg.E-47.B1 to I.Bg.E-47.B442).
- Table 48i: Compounds of the formula I.Bg in which the combination of (R⁴)ₙ and (R^{L})ₘ corresponds to line E-48 of Table E and the meaning for the combination of R¹, R² and R³ for each individual compound corresponds in each case to one line of Table B (compounds I.Bg.E-48.B1 to I.Bg.E-48.B442).
- Table 49i: Compounds of the formula I.Bg in which the combination of (R⁴)ₙ and (R^{L})ₘ corresponds to line E-49 of Table E and the meaning for the combination of R¹, R² and R³ for each individual compound corresponds in each case to one line of Table B (compounds I.Bg.E-49.B1 to I.Bg.E-49.B442).
- Table 50i: Compounds of the formula I.Bg in which the combination of (R⁴)ₙ and (R^{L})ₘ corresponds to line E-50 of Table E and the meaning for the combination of R¹, R² and R³ for each individual compound corresponds in each case to one line of Table B (compounds I.Bg.E-50.B1 to I.Bg.E-50.B442).
- Table 51i: Compounds of the formula I.Bg in which the combination of (R⁴)ₙ and (R^{L})ₘ corresponds to line E-51 of Table E and the meaning for the combination of R¹, R² and R³ for each individual compound corresponds in each case to one line of Table B (compounds I.Bg.E-51.B1 to I.Bg.E-51.B442).
- Table 52i: Compounds of the formula I.Bg in which the combination of (R⁴)ₙ and (R^{L})ₘ corresponds to line E-52 of Table E and the meaning for the combination of R¹, R² and R³ for each individual compound corresponds in each case to one line of Table B (compounds I.Bg.E-52.B1 to I.Bg.E-52.B442).
- Table 53i: Compounds of the formula I.Bg in which the combination of (R⁴)ₙ and (R^{L})ₘ corresponds to line E-53 of Table E and the meaning for the combination of R¹, R² and R³ for each individual compound corresponds in each case to one line of Table B (compounds I.Bg.E-53.B1 to I.Bg.E-53.B442).
- Table 54i: Compounds of the formula I.Bg in which the combination of (R⁴)ₙ and (R^{L})ₘ corresponds to line E-54 of Table E and the meaning for the combination of R¹, R² and R³ for each individual compound corresponds in each case to one line of Table B (compounds I.Bg.E-54.B1 to I.Bg.E-54.B442).
- Table 55i: Compounds of the formula I.Bg in which the combination of (R⁴)ₙ and (R^{L})ₘ corresponds to line E-55 of Table E and the meaning for the combination of R¹, R² and R³ for each individual compound corresponds in each case to one line of Table B (compounds I.Bg.E-55.B1 to I.Bg.E-55.B442).
- Table 56i: Compounds of the formula I.Bg in which the combination of (R⁴)ₙ and (R^{L})ₘ corresponds to line E-56 of Table E and the meaning for the combination of R¹, R² and R³ for each individual compound corresponds in each case to one line of Table B (compounds I.Bg.E-56.B1 to I.Bg.E-56.B442).
- Table 57i: Compounds of the formula I.Bg in which the combination of (R⁴)ₙ and (R^{L})ₘ corresponds to line E-57 of Table E and the meaning for the combination of R¹, R² and R³ for each individual compound corresponds in each case to one line of Table B (compounds I.Bg.E-57.B1 to I.Bg.E-57.B442).
- Table 58i: Compounds of the formula I.Bg in which the combination of (R⁴)ₙ and (R^{L})ₘ corresponds to line E-58 of Table E and the meaning for the combination of R¹, R² and R³ for each individual compound corresponds in each case to one line of Table B (compounds I.Bg.E-58.B1 to I.Bg.E-58.B442).
- Table 59i: Compounds of the formula I.Bg in which the combination of (R⁴)ₙ and (R^{L})ₘ corresponds to line E-59 of Table E and the meaning for the combination of R¹, R² and R³ for each individual compound corresponds in each case to one line of Table B (compounds I.Bg.E-59.B1 to I.Bg.E-59.B442).
- Table 60i: Compounds of the formula I.Bg in which the combination of (R⁴)ₙ and (R^{L})ₘ corresponds to line E-60 of Table E and the meaning for the combination of R¹, R² and R³ for each individual compound corresponds in each case to one line of Table B (compounds I.Bg.E-60.B1 to I.Bg.E-60.B442).
- Table 61i: Compounds of the formula I.Bg in which the combination of (R⁴)ₙ and (R^{L})ₘ corresponds to line E-61 of Table E and the meaning for the combination of R¹, R² and R³ for each individual compound corresponds in each case to one line of Table B (compounds I.Bg.E-61.B1 to I.Bg.E-61.B442).
- Table 62i: Compounds of the formula I.Bg in which the combination of (R⁴)ₙ and (R^{L})ₘ corresponds to line E-62 of Table E and the meaning for the combination of R¹, R² and R³ for each individual compound corresponds in each case to one line of Table B (compounds I.Bg.E-62.B1 to I.Bg.E-62.B442).
- Table 63i: Compounds of the formula I.Bg in which the combination of (R⁴)ₙ and (R^{L})ₘ corresponds to line E-63 of Table E and the meaning for the combination of R¹, R² and R³ for each individual compound corresponds in each case to one line of Table B (compounds I.Bg.E-63.B1 to I.Bg.E-63.B442).
- Table 64i: Compounds of the formula I.Bg in which the combination of (R⁴)ₙ and (R^{L})ₘ corresponds to line E-64 of Table E and the meaning for the combination of R¹, R² and R³ for each individual compound corresponds in each case to one line of Table B (compounds I.Bg.E-64.B1 to I.Bg.E-64.B442).
- Table 65i: Compounds of the formula I.Bg in which the combination of (R⁴)ₙ and (R^{L})ₘ corresponds to line E-65 of Table E and the meaning for the combination of R¹, R² and R³ for each individual compound corresponds in each case to one line of Table B (compounds I.Bg.E-65.B1 to I.Bg.E-65.B442).
- Table 66i: Compounds of the formula I.Bg in which the combination of (R⁴)ₙ and (R^{L})ₘ corresponds to line E-66 of Table E and the meaning for the combination of R¹, R² and R³ for each individual compound corresponds in each case to one line of Table B (compounds I.Bg.E-66.B1 to I.Bg.E-66.B442).
- Table 67i: Compounds of the formula I.Bg in which the combination of (R⁴)ₙ and (R^{L})ₘ corresponds to line E-67 of Table E and the meaning for the combination of R¹, R² and R³ for each individual compound corresponds in each case to one line of Table B (compounds I.Bg.E-67.B1 to I.Bg.E-67.B442).
- Table 68i: Compounds of the formula I.Bg in which the combination of (R⁴)ₙ and (R^{L})ₘ corresponds to line E-68 of Table E and the meaning for the combination of R¹, R² and R³ for each individual compound corresponds in each case to one line of Table B (compounds I.Bg.E-68.B1 to I.Bg.E-68.B442).
- Table 69i: Compounds of the formula I.Bg in which the combination of (R⁴)ₙ and (R^{L})ₘ corresponds to line E-69 of Table E and the meaning for the combination of R¹, R² and R³ for each individual compound corresponds in each case to one line of Table B (compounds I.Bg.E-69.B1 to I.Bg.E-69.B442).
- Table 70i: Compounds of the formula I.Bg in which the combination of (R⁴)ₙ and (R^{L})ₘ corresponds to line E-70 of Table E and the meaning for the combination of R¹, R² and R³ for each individual compound corresponds in each case to one line of Table B (compounds I.Bg.E-70.B1 to I.Bg.E-70.B442).
- Table 1j: Compounds of the formula I.Bh in which the combination of (R⁴)ₙ and (R^{L})ₘ corresponds to line E-1 of Table E and the meaning for the combination of R¹, R² and R³ for each individual compound corresponds in each case to one line of Table B (compounds I.Bh.E-1.B1 to I.Bh.E-1.B442).
- Table 2j: Compounds of the formula I.Bh in which the combination of (R⁴)ₙ and (R^{L})ₘ corresponds to line E-2 of Table E and the meaning for the combination of R¹, R² and R³ for each individual compound corresponds in each case to one line of Table B (compounds I.Bh.E-2.B1 to I.Bh.E-2.B442).
- Table 3j: Compounds of the formula I.Bh in which the combination of (R⁴)ₙ and (R^{L})ₘ corresponds to line E-3 of Table E and the meaning for the combination of R¹, R² and R³ for each individual compound corresponds in each case to one line of Table B (compounds I.Bh.E-3.B1 to I.Bh.E-3.B442).
- Table 4j: Compounds of the formula I.Bh in which the combination of (R⁴)ₙ and (R^{L})ₘ corresponds to line E-4 of Table E and the meaning for the combination of R¹, R² and R³ for each individual compound corresponds in each case to one line of Table B (compounds I.Bh.E-4.B1 to I.Bh.E-4.B442).
- Table 5j: Compounds of the formula I.Bh in which the combination of (R⁴)ₙ and (R^{L})ₘ corresponds to line E-5 of Table E and the meaning for the combination of R¹, R² and R³ for each individual compound corresponds in each case to one line of Table B (compounds I.Bh.E-5.B1 to I.Bh.E-5.B442).
- Table 6j: Compounds of the formula I.Bh in which the combination of (R⁴)ₙ and (R^{L})ₘ corresponds to line E-6 of Table E and the meaning for the combination of R¹, R² and R³ for each individual compound corresponds in each case to one line of Table B (compounds I.Bh.E-6.B1 to I.Bh.E-6.B442).
- Table 7j: Compounds of the formula I.Bh in which the combination of (R⁴)ₙ and (R^{L})ₘ corresponds to line E-7 of Table E and the meaning for the combination of R¹, R² and R³ for each individual compound corresponds in each case to one line of Table B (compounds I.Bh.E-7.B1 to I.Bh.E-7.B442).
- Table 8j: Compounds of the formula I.Bh in which the combination of (R⁴)ₙ and (R^{L})ₘ corresponds to line E-8 of Table E and the meaning for the combination of R¹, R² and R³ for each individual compound corresponds in each case to one line of Table B (compounds I.Bh.E-8.B1 to I.Bh.E-8.B442).
- Table 9j: Compounds of the formula I.Bh in which the combination of (R⁴)ₙ and (R^{L})ₘ corresponds to line E-9 of Table E and the meaning for the combination of R¹, R² and R³ for each individual compound corresponds in each case to one line of Table B (compounds I.Bh.E-9.B1 to I.Bh.E-9.B442).
- Table 10j: Compounds of the formula I.Bh in which the combination of (R⁴)ₙ and (R^{L})ₘ corresponds to line E-10 of Table E and the meaning for the combination of R¹, R² and R³ for each individual compound corresponds in each case to one line of Table B (compounds I.Bh.E-10.B1 to I.Bh.E-10.B442).
- Table 11j: Compounds of the formula I.Bh in which the combination of (R⁴)ₙ and (R^{L})ₘ corresponds to line E-11 of Table E and the meaning for the combination of R¹, R² and R³ for each individual compound corresponds in each case to one line of Table B (compounds I.Bh.E-11.B1 to I.Bh.E-11.B442).
- Table 12j: Compounds of the formula I.Bh in which the combination of (R⁴)ₙ and (R^{L})ₘ corresponds to line E-12 of Table E and the meaning for the combination of R¹, R² and R³ for each individual compound corresponds in each case to one line of Table B (compounds I.Bh.E-12.B1 to I.Bh.E-12.B442).
- Table 13j: Compounds of the formula I.Bh in which the combination of (R⁴)ₙ and (R^{L})ₘ corresponds to line E-13 of Table E and the meaning for the combination of R¹, R² and R³ for each individual compound corresponds in each case to one line of Table B (compounds I.Bh.E-13.B1 to I.Bh.E-13.B442).
- Table 14j: Compounds of the formula I.Bh in which the combination of (R⁴)ₙ and (R^{L})ₘ corresponds to line E-14 of Table E and the meaning for the combination of R¹, R² and R³ for each individual compound corresponds in each case to one line of Table B (compounds I.Bh.E-14.B1 to I.Bh.E-14.B442).
- Table 15j: Compounds of the formula I.Bh in which the combination of (R⁴)ₙ and (R^{L})ₘ corresponds to line E-15 of Table E and the meaning for the combination of R¹, R² and R³ for each individual compound corresponds in each case to one line of Table B (compounds I.Bh.E-15.B1 to I.Bh.E-15.B442).
- Table 16j: Compounds of the formula I.Bh in which the combination of (R⁴)ₙ and (R^{L})ₘ corresponds to line E-16 of Table E and the meaning for the combination of R¹, R² and R³ for each individual compound corresponds in each case to one line of Table B (compounds I.Bh.E-16.B1 to I.Bh.E-16.B442).
- Table 17j: Compounds of the formula I.Bh in which the combination of (R⁴)ₙ and (R^{L})ₘ corresponds to line E-17 of Table E and the meaning for the combination of R¹, R² and R³ for each individual compound corresponds in each case to one line of Table B (compounds I.Bh.E-17.B1 to I.Bh.E-17.B442).
- Table 18j: Compounds of the formula I.Bh in which the combination of (R⁴)ₙ and (R^{L})ₘ corresponds to line E-18 of Table E and the meaning for the combination of R¹, R² and R³ for each individual compound corresponds in each case to one line of Table B (compounds I.Bh.E-18.B1 to I.Bh.E-18.B442).
- Table 19j: Compounds of the formula I.Bh in which the combination of (R⁴)ₙ and (R^{L})ₘ corresponds to line E-19 of Table E and the meaning for the combination of R¹, R² and R³ for each individual compound corresponds in each case to one line of Table B (compounds I.Bh.E-19.B1 to I.Bh.E-19.B442).
- Table 20j: Compounds of the formula I.Bh in which the combination of (R⁴)ₙ and (R^{L})ₘ corresponds to line E-20 of Table E and the meaning for the combination of R¹, R² and R³ for each individual compound corresponds in each case to one line of Table B (compounds I.Bh.E-20.B1 to I.Bh.E-20.B442).
- Table 21j: Compounds of the formula I.Bh in which the combination of (R⁴)ₙ and (R^{L})ₘ corresponds to line E-21 of Table E and the meaning for the combination of R¹, R² and R³ for each individual compound corresponds in each case to one line of Table B (compounds I.Bh.E-21.B1 to I.Bh.E-21.B442).
- Table 22j: Compounds of the formula I.Bh in which the combination of (R⁴)ₙ and (R^{L})ₘ corresponds to line E-22 of Table E and the meaning for the combination of R¹, R² and R³ for each individual compound corresponds in each case to one line of Table B (compounds I.Bh.E-22.B1 to I.Bh.E-22.B442).
- Table 23j: Compounds of the formula I.Bh in which the combination of (R⁴)ₙ and (R^{L})ₘ corresponds to line E-23 of Table E and the meaning for the combination of R¹, R² and R³ for each individual compound corresponds in each case to one line of Table B (compounds I.Bh.E-23.B1 to I.Bh.E-23.B442).
- Table 24j: Compounds of the formula I.Bh in which the combination of (R⁴)ₙ and (R^{L})ₘ corresponds to line E-24 of Table E and the meaning for the combination of R¹, R² and R³ for each individual compound corresponds in each case to one line of Table B (compounds I.Bh.E-24.B1 to I.Bh.E-24.B442).
- Table 25j: Compounds of the formula I.Bh in which the combination of (R⁴)ₙ and (R^{L})ₘ corresponds to line E-25 of Table E and the meaning for the combination of R¹, R² and R³ for each individual compound corresponds in each case to one line of Table B (compounds I.Bh.E-25.B1 to I.Bh.E-25.B442).
- Table 26j: Compounds of the formula I.Bh in which the combination of (R⁴)ₙ and (R^{L})ₘ corresponds to line E-26 of Table E and the meaning for the combination of R¹, R² and R³ for each individual compound corresponds in each case to one line of Table B (compounds I.Bh.E-26.B1 to I.Bh.E-26.B442).
- Table 27j: Compounds of the formula I.Bh in which the combination of (R⁴)ₙ and (R^{L})ₘ corresponds to line E-27 of Table E and the meaning for the combination of R¹, R² and R³ for each individual compound corresponds in each case to one line of Table B (compounds I.Bh.E-27.B1 to I.Bh.E-27.B442).
- Table 28j: Compounds of the formula I.Bh in which the combination of (R⁴)ₙ and (R^{L})ₘ corresponds to line E-28 of Table E and the meaning for the combination of R¹, R² and R³ for each individual compound corresponds in each case to one line of Table B (compounds I.Bh.E-28.B1 to I.Bh.E-28.B442).
- Table 29j: Compounds of the formula I.Bh in which the combination of (R⁴)ₙ and (R^{L})ₘ corresponds to line E-29 of Table E and the meaning for the combination of R¹, R² and R³ for each individual compound corresponds in each case to one line of Table B (compounds I.Bh.E-29.B1 to I.Bh.E-29.B442).
- Table 30j: Compounds of the formula I.Bh in which the combination of (R⁴)ₙ and (R^{L})ₘ corresponds to line E-30 of Table E and the meaning for the combination of R¹, R² and R³ for each individual compound corresponds in each case to one line of Table B (compounds I.Bh.E-30.B1 to I.Bh.E-30.B442).
- Table 31j: Compounds of the formula I.Bh in which the combination of (R⁴)ₙ and (R^{L})ₘ corresponds to line E-31 of Table E and the meaning for the combination of R¹, R² and R³ for each individual compound corresponds in each case to one line of Table B (compounds I.Bh.E-31.B1 to I.Bh.E-31.B442).
- Table 32j: Compounds of the formula I.Bh in which the combination of (R⁴)ₙ and (R^{L})ₘ corresponds to line E-32 of Table E and the meaning for the combination of R¹, R² and R³ for each individual compound corresponds in each case to one line of Table B (compounds I.Bh.E-32.B1 to I.Bh.E-32.B442).
- Table 33j: Compounds of the formula I.Bh in which the combination of (R⁴)ₙ and (R^{L})ₘ corresponds to line E-33 of Table E and the meaning for the combination of R¹, R² and R³ for each individual compound corresponds in each case to one line of Table B (compounds I.Bh.E-33.B1 to I.Bh.E-33.B442).
- Table 34j: Compounds of the formula I.Bh in which the combination of (R⁴)ₙ and (R^{L})ₘ corresponds to line E-34 of Table E and the meaning for the combination of R¹, R² and R³ for each individual compound corresponds in each case to one line of Table B (compounds I.Bh.E-34.B1 to I.Bh.E-34.B442).
- Table 35j: Compounds of the formula I.Bh in which the combination of (R⁴)ₙ and (R^{L})ₘ corresponds to line E-35 of Table E and the meaning for the combination of R¹, R² and R³ for each individual compound corresponds in each case to one line of Table B (compounds I.Bh.E-35.B1 to I.Bh.E-35.B442).
- Table 36j: Compounds of the formula I.Bh in which the combination of (R⁴)ₙ and (R^{L})ₘ corresponds to line E-36 of Table E and the meaning for the combination of R¹, R² and R³ for each individual compound corresponds in each case to one line of Table B (compounds I.Bh.E-36.B1 to I.Bh.E-36.B442).
- Table 37j: Compounds of the formula I.Bh in which the combination of (R⁴)ₙ and (R^{L})ₘ corresponds to line E-37 of Table E and the meaning for the combination of R¹, R² and R³ for each individual compound corresponds in each case to one line of Table B (compounds I.Bh.E-37.B1 to I.Bh.E-37.B442).
- Table 38j: Compounds of the formula I.Bh in which the combination of (R⁴)ₙ and (R^{L})ₘ corresponds to line E-38 of Table E and the meaning for the combination of R¹, R² and R³ for each individual compound corresponds in each case to one line of Table B (compounds I.Bh.E-38.B1 to I.Bh.E-38.B442).
- Table 39j: Compounds of the formula I.Bh in which the combination of (R⁴)ₙ and (R^{L})ₘ corresponds to line E-39 of Table E and the meaning for the combination of R¹, R² and R³ for each individual compound corresponds in each case to one line of Table B (compounds I.Bh.E-39.B1 to I.Bh.E-39.B442).
- Table 40j: Compounds of the formula I.Bh in which the combination of (R⁴)ₙ and (R^{L})ₘ corresponds to line E-40 of Table E and the meaning for the combination of R¹, R² and R³ for each individual compound corresponds in each case to one line of Table B (compounds I.Bh.E-40.B1 to I.Bh.E-40.B442).
- Table 41j: Compounds of the formula I.Bh in which the combination of (R⁴)ₙ and (R^{L})ₘ corresponds to line E-41 of Table E and the meaning for the combination of R¹, R² and R³ for each individual compound corresponds in each case to one line of Table B (compounds I.Bh.E-41.B1 to I.Bh.E-41.B442).
- Table 42j: Compounds of the formula I.Bh in which the combination of (R⁴)ₙ and (R^{L})ₘ corresponds to line E-42 of Table E and the meaning for the combination of R¹, R² and R³ for each individual compound corresponds in each case to one line of Table B (compounds I.Bh.E-42.B1 to I.Bh.E-42.B442).
- Table 43j: Compounds of the formula I.Bh in which the combination of (R⁴)ₙ and (R^{L})ₘ corresponds to line E-43 of Table E and the meaning for the combination of R¹, R² and R³ for each individual compound corresponds in each case to one line of Table B (compounds I.Bh.E-43.B1 to I.Bh.E-43.B442).
- Table 44j: Compounds of the formula I.Bh in which the combination of (R⁴)ₙ and (R^{L})ₘ corresponds to line E-44 of Table E and the meaning for the combination of R¹, R² and R³ for each individual compound corresponds in each case to one line of Table B (compounds I.Bh.E-44.B1 to I.Bh.E-44.B442).
- Table 45j: Compounds of the formula I.Bh in which the combination of (R⁴)ₙ and (R^{L})ₘ corresponds to line E-45 of Table E and the meaning for the combination of R¹, R² and R³ for each individual compound corresponds in each case to one line of Table B (compounds I.Bh.E-45.B1 to I.Bh.E-45.B442).
- Table 46j: Compounds of the formula I.Bh in which the combination of (R⁴)ₙ and (R^{L})ₘ corresponds to line E-46 of Table E and the meaning for the combination of R¹, R² and R³ for each individual compound corresponds in each case to one line of Table B (compounds I.Bh.E-46.B1 to I.Bh.E-46.B442).
- Table 47j: Compounds of the formula I.Bh in which the combination of (R⁴)ₙ and (R^{L})ₘ corresponds to line E-47 of Table E and the meaning for the combination of R¹, R² and R³ for each individual compound corresponds in each case to one line of Table B (compounds I.Bh.E-47.B1 to I.Bh.E-47.B442).
- Table 48j: Compounds of the formula I.Bh in which the combination of (R⁴)ₙ and (R^{L})ₘ corresponds to line E-48 of Table E and the meaning for the combination of R¹, R² and R³ for each individual compound corresponds in each case to one line of Table B (compounds I.Bh.E-48.B1 to I.Bh.E-48.B442).
- Table 49j: Compounds of the formula I.Bh in which the combination of (R⁴)ₙ and (R^{L})ₘ corresponds to line E-49 of Table E and the meaning for the combination of R¹, R² and R³ for each individual compound corresponds in each case to one line of Table B (compounds I.Bh.E-49.B1 to I.Bh.E-49.B442).
- Table 50j: Compounds of the formula I.Bh in which the combination of (R⁴)ₙ and (R^{L})ₘ corresponds to line E-50 of Table E and the meaning for the combination of R¹, R² and R³ for each individual compound corresponds in each case to one line of Table B (compounds I.Bh.E-50.B1 to I.Bh.E-50.B442).
- Table 51j: Compounds of the formula I.Bh in which the combination of (R⁴)ₙ and (R^{L})ₘ corresponds to line E-51 of Table E and the meaning for the combination of R¹, R² and R³ for each individual compound corresponds in each case to one line of Table B (compounds I.Bh.E-51.B1 to I.Bh.E-51.B442).
- Table 52j: Compounds of the formula I.Bh in which the combination of (R⁴)ₙ and (R^{L})ₘ corresponds to line E-52 of Table E and the meaning for the combination of R¹, R² and R³ for each individual compound corresponds in each case to one line of Table B (compounds I.Bh.E-52.B1 to I.Bh.E-52.B442).
- Table 53j: Compounds of the formula I.Bh in which the combination of (R⁴)ₙ and (R^{L})ₘ corresponds to line E-53 of Table E and the meaning for the combination of R¹, R² and R³ for each individual compound corresponds in each case to one line of Table B (compounds I.Bh.E-53.B1 to I.Bh.E-53.B442).
- Table 54j: Compounds of the formula I.Bh in which the combination of (R⁴)ₙ and (R^{L})ₘ corresponds to line E-54 of Table E and the meaning for the combination of R¹, R² and R³ for each individual compound corresponds in each case to one line of Table B (compounds I.Bh.E-54.B1 to I.Bh.E-54.B442).
- Table 55j: Compounds of the formula I.Bh in which the combination of (R⁴)ₙ and (R^{L})ₘ corresponds to line E-55 of Table E and the meaning for the combination of R¹, R² and R³ for each individual compound corresponds in each case to one line of Table B (compounds I.Bh.E-55.B1 to I.Bh.E-55.B442).
- Table 56j: Compounds of the formula I.Bh in which the combination of (R⁴)ₙ and (R^{L})ₘ corresponds to line E-56 of Table E and the meaning for the combination of R¹, R² and R³ for each individual compound corresponds in each case to one line of Table B (compounds I.Bh.E-56.B1 to I.Bh.E-56.B442).
- Table 57j: Compounds of the formula I.Bh in which the combination of (R⁴)ₙ and (R^{L})ₘ corresponds to line E-57 of Table E and the meaning for the combination of R¹, R² and R³ for each individual compound corresponds in each case to one line of Table B (compounds I.Bh.E-57.B1 to I.Bh.E-57.B442).
- Table 58j: Compounds of the formula I.Bh in which the combination of (R⁴)ₙ and (R^{L})ₘ corresponds to line E-58 of Table E and the meaning for the combination of R¹, R² and R³ for each individual compound corresponds in each case to one line of Table B (compounds I.Bh.E-58.B1 to I.Bh.E-58.B442).
- Table 59j: Compounds of the formula I.Bh in which the combination of (R⁴)ₙ and (R^{L})ₘ corresponds to line E-59 of Table E and the meaning for the combination of R¹, R² and R³ for each individual compound corresponds in each case to one line of Table B (compounds I.Bh.E-59.B1 to I.Bh.E-59.B442).
- Table 60j: Compounds of the formula I.Bh in which the combination of (R⁴)ₙ and (R^{L})ₘ corresponds to line E-60 of Table E and the meaning for the combination of R¹, R² and R³ for each individual compound corresponds in each case to one line of Table B (compounds I.Bh.E-60.B1 to I.Bh.E-60.B442).
- Table 61j: Compounds of the formula I.Bh in which the combination of (R⁴)ₙ and (R^{L})ₘ corresponds to line E-61 of Table E and the meaning for the combination of R¹, R² and R³ for each individual compound corresponds in each case to one line of Table B (compounds I.Bh.E-61.B1 to I.Bh.E-61.B442).
- Table 62j: Compounds of the formula I.Bh in which the combination of (R⁴)ₙ and (R^{L})ₘ corresponds to line E-62 of Table E and the meaning for the combination of R¹, R² and R³ for each individual compound corresponds in each case to one line of Table B (compounds I.Bh.E-62.B1 to I.Bh.E-62.B442).
- Table 63j: Compounds of the formula I.Bh in which the combination of (R⁴)ₙ and (R^{L})ₘ corresponds to line E-63 of Table E and the meaning for the combination of R¹, R² and R³ for each individual compound corresponds in each case to one line of Table B (compounds I.Bh.E-63.B1 to I.Bh.E-63.B442).
- Table 64j: Compounds of the formula I.Bh in which the combination of (R⁴)ₙ and (R^{L})ₘ corresponds to line E-64 of Table E and the meaning for the combination of R¹, R² and R³ for each individual compound corresponds in each case to one line of Table B (compounds I.Bh.E-64.B1 to I.Bh.E-64.B442).
- Table 65j: Compounds of the formula I.Bh in which the combination of (R⁴)ₙ and (R^{L})ₘ corresponds to line E-65 of Table E and the meaning for the combination of R¹, R² and R³ for each individual compound corresponds in each case to one line of Table B (compounds I.Bh.E-65.B1 to I.Bh.E-65.B442).
- Table 66j: Compounds of the formula I.Bh in which the combination of (R⁴)ₙ and (R^{L})ₘ corresponds to line E-66 of Table E and the meaning for the combination of R¹, R² and R³ for each individual compound corresponds in each case to one line of Table B (compounds I.Bh.E-66.B1 to I.Bh.E-66.B442).
- Table 67j: Compounds of the formula I.Bh in which the combination of (R⁴)ₙ and (R^{L})ₘ corresponds to line E-67 of Table E and the meaning for the combination of R¹, R² and R³ for each individual compound corresponds in each case to one line of Table B (compounds I.Bh.E-67.B1 to I.Bh.E-67.B442).
- Table 68j: Compounds of the formula I.Bh in which the combination of (R⁴)ₙ and (R^{L})ₘ corresponds to line E-68 of Table E and the meaning for the combination of R¹, R² and R³ for each individual compound corresponds in each case to one line of Table B (compounds I.Bh.E-68.B1 to I.Bh.E-68.B442).
- Table 69j: Compounds of the formula I.Bh in which the combination of (R⁴)ₙ and (R^{L})ₘ corresponds to line E-69 of Table E and the meaning for the combination of R¹, R² and R³ for each individual compound corresponds in each case to one line of Table B (compounds I.Bh.E-69.B1 to I.Bh.E-69.B442).
- Table 70j: Compounds of the formula I.Bh in which the combination of (R⁴)ₙ and (R^{L})ₘ corresponds to line E-70 of Table E and the meaning for the combination of R¹, R² and R³ for each individual compound corresponds in each case to one line of Table B (compounds I.Bh.E-70.B1 to I.Bh.E-70.B442).

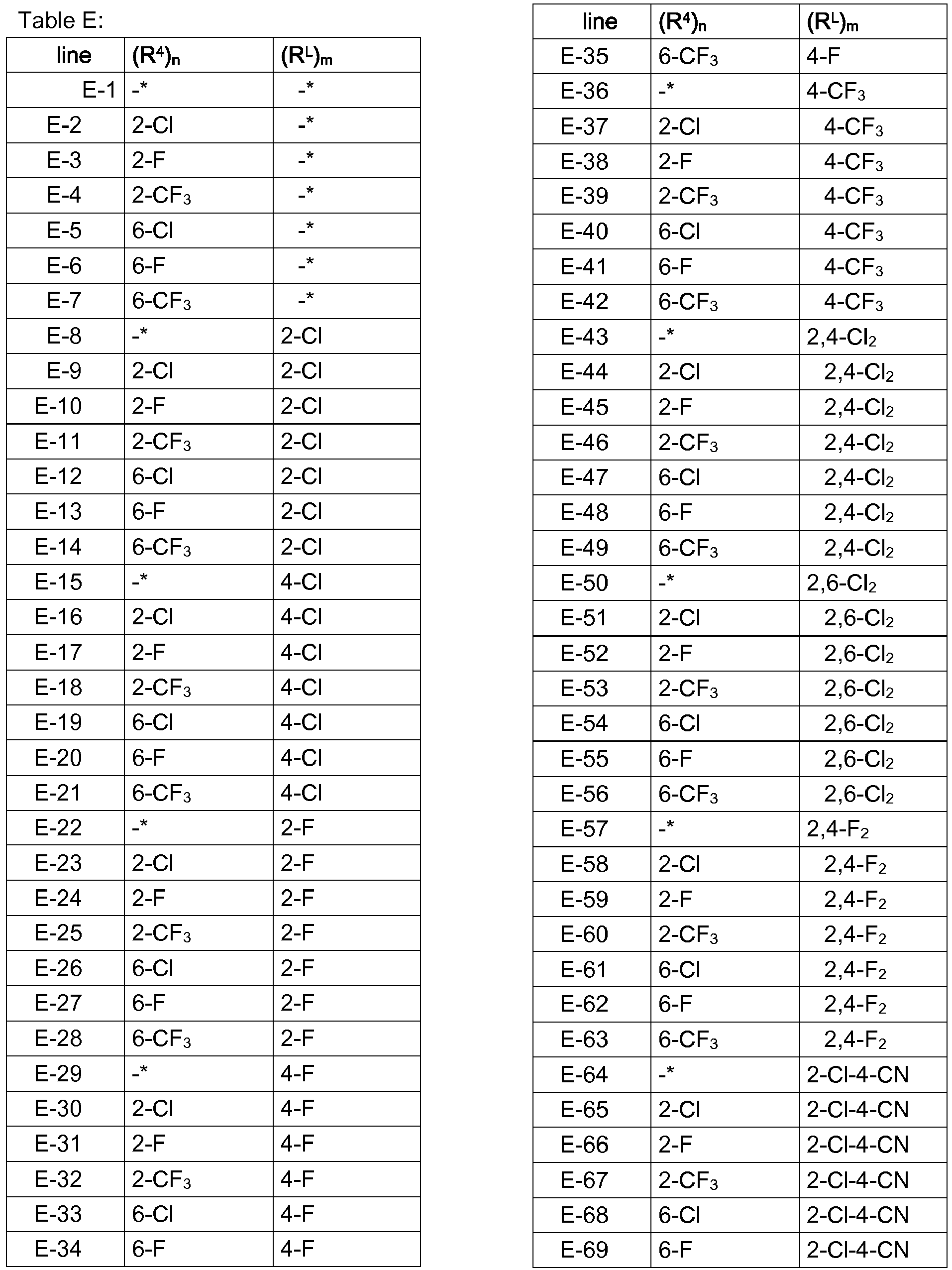

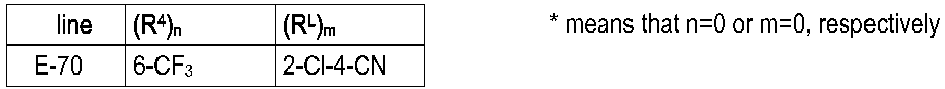

The compounds I and the compositions according to the invention, respectively, are suitable as fungicides. They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoAreductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesisrelated proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds I and compositions thereof, respectively, are particularly suitable for controlling the following plant diseases:
*Albugo* spp. (white rust) on ornamentals, vegetables (e. g. A. *candida)* and sunflowers (e. g. A. *tragopogonis*); *Alternaria* spp. (Alternaria leaf spot) on vegetables, rape (A. *brassicola* or *brassicae*), sugar beets (A. *tenuis),* fruits, rice, soybeans, potatoes (e. g. A. *solani* or A. *alternata*), tomatoes (e. g. A. *solani* or A. *alternata*) and wheat; *Aphanomyces* spp. on sugar beets and vegetables; *Ascochyta* spp. on cereals and vegetables, e. g. A. *tritici* (anthracnose) on wheat and A. *hordei* on barley; *Bipolaris* and *Drechslera* spp. (teleomorph: *Cochliobolus* spp.), e. g. Southern leaf blight (*D. maydis*) or Northern leaf blight (*B. zeicola)* on corn, e. g. spot blotch (*B. sorokiniana*) on cereals and e.g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma)* spp. (rot or wilt) on broad-leaved trees and evergreens, e. g. *C*. *ulmi* (Dutch elm disease) on elms; *Cercospora* spp. (Cercospora leaf spots) on corn (e.g. Gray leaf spot: *C. zeae-maydis*), rice, sugar beets (e. g. *C. beticola*), sugar cane, vegetables, coffee, soybeans (e. g. *C. sojina* or C. *kikuchii*) and rice; *Cladosporium* spp. on tomatoes (e. g. *C. fulvum:* leaf mold) and cereals, e. g. *C. herbarum* (black ear) on wheat; *Claviceps purpurea* (ergot) on cereals; *Cochliobolus* (anamorph: *Helminthosporium* of *Bipolaris)* spp. (leaf spots) on corn (*C. carbonum*)*,* cereals (e. g. *C. sativus,* anamorph: *B. sorokiniana*) and rice (e. g. *C. miyabeanus,* anamorph: H. *oryzae*)*; Colletotrichum* (teleomorph: *Glomerella)* spp. (anthracnose) on cotton (e. g. *C*. *gossypii*), corn (e. g. *C. graminicola:* Anthracnose stalk rot), soft fruits, potatoes (e. g. *C*. *coccodes:* black dot), beans (e. g. *C. lindemuthianum*) and soybeans (e. g. *C. truncatum* or C. *gloeosporioides*); *Corticium* spp., e. g. *C*. *sasakii* (sheath blight) on rice; *Corynespora cassiicola* (leaf spots) on soybeans and ornamentals; *Cycloconium* spp., e. g. *C*. *oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri:* Black Foot Disease) and ornamentals; *Dematophora* (teleomorph: *Rosellinia*) necatrix (root and stem rot) on soybeans; *Diaporthe* spp., e. g. *D. phaseolorum* (damping off) on soybeans; *Drechslera* (*syn. Helminthosporium,* teleomorph: *Pyrenophora)* spp. on corn, cereals, such as barley (e. g. *D. teres,* net blotch) and wheat (e. g. *D. tritici-repentis:* tan spot), rice and turf; Esca (dieback, apoplexy) on vines, caused by *Formitiporia (syn. Phellinus) punctata, F. mediterranea, Phaeomoniella chlamydospora* (earlier *Phaeoacremonium chlamydosporum*), *Phaeoacremonium aleophilum* and/or *Botryosphaeria obtusa*; *Elsinoe* spp. on pome fruits (*E. pyri*), soft fruits *(E. veneta:* anthracnose) and vines *(E. ampelina:* anthracnose); *Entyloma oryzae* (leaf smut) on rice; *Epicoccum* spp. (black mold) on wheat; *Erysiphe* spp. (powdery mildew) on sugar beets (*E. betae*)*,* vegetables (e. g. *E. pisi*)*,* such as cucurbits (e. g. *E. cichoracearum*)*,* cabbages, rape (e. g. *E. cruciferarum*); *Eutypa lata* (Eutypa canker or dieback, anamorph: *Cytosporina lata, syn. Libertella blepharis*) on fruit trees, vines and ornamental woods; *Exserohilum* (*syn. Helminthosporium*) spp. on corn (e. g. *E. turcicum*); *Fusarium* (teleomorph: *Gibberella)* spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme*) and *F. tucumaniae* and *F*. *brasiliense* each causing sudden death syndrome on soybeans and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi:* Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora (syn. Cladosporium vitis)* on vines; *Macrophomina phaseolina* (*syn. phaseoli*) (root and stem rot) on soybeans and cotton; *Microdochium* (*syn. Fusarium*) *nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M*. *fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P. tabacina*) and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P*. *tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola:* can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P*. *megasperma, syn. P. sojae*), potatoes and tomatoes (e. g. *P. infestans:* late blight) and broadleaved trees (e. g. *P. ramorum:* sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P*. *cubensis* on cucurbits or *P*. *humili* on hop; *Pseudopezicula tracheiphila* (red fire disease or ,rotbrenner', anamorph: *Phialophora)* on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P*. *recondita* (brown or leaf rust) on cereals, such as e. g. wheat, barley or rye, *P. kuehnii* (orange rust) on sugar cane and *P. asparagi* on asparagus; *Pyrenophora* (anamorph: *Drechslera*) *tritici-repentis* (tan spot) on wheat or *P*. *teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P*. *aphanidermatum*); *Ramularia* spp., e. g. *R. collo-cygni* (Ramularia leaf spots, Physiological leaf spots) on barley and *R*. *beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and S. *attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S*. ***sclerotiorum*);** *Septoria* spp. on various plants, e. g. *S*. *glycines* (brown spot) on soybeans, *S*. *tritici* (Septoria blotch) on wheat and *S*. (syn. *Stagonospora*) nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S. turcicum,* syn. *Helminthosporium turcicum)* and turf; *Sphacelotheca* spp. (smut) on corn, (e. g. *S. reiliana:* head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S*. *nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T*. *basicola* (syn. *Chalara elegans*); *Tilletia* spp. (common bunt or stinking smut) on cereals, such as e. g. *T. tritici* (syn. *T*. *caries,* wheat bunt) and *T*. *controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds I and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichorma* spp., *Alternaria* spp., *Paecilomyces* spp. and Zygomycetes such as Mucor spp., and in addition in the protection of stored products and harvest the following yeast fungi are worthy of note: *Candida* spp. and *Saccharomyces cerevisae.*

The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds I and compositions thereof, resepectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting.

The invention also relates to compositions comprising one compound I according to the invention.

In particular, such composition further comprises an auxiliary as defined below.

The term "effective amount" used denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkyl-polyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound I and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of a compound I and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of a compound I and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e.g. sodium lignosulfonate and alcohol ethoxylate), 0.1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e.g. finely divided kaolin) ad 100 wt%.
xii) Granules (GR, FG)
   0.5-30 wt% of a compound I is ground finely and associated with solid carrier (e.g. silicate) ad 100 wt%. Granulation is achieved by extrusion, spray-drying or fluidized bed.
xiii) Ultra-low volume liquids (UL)
   1-50 wt% of a compound I are dissolved in organic solvent (e.g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 g to 10 kg, in particular 0.1 g to 1000 g, more particularly from 1 to 1000 g, specifically from 1 to 100 g and most specifically from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as a virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term pesticides includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology to increase plant growth, biomass, yield or any other quality parameter of the harvestable goods of acrop plant.

Biopesticides are typically created by growing and concentrating naturally occurring organisms and/or their metabolites including bacteria and other microbes, fungi, viruses, nematodes, proteins, etc. They are often considered to be important components of integrated pest management (IPM) programmes.

Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classed as microbial pesticides, even though they are multi-cellular.
(2) Biochemical pesticides are naturally occurring substances that control pests or provide other crop protection uses as defined below, but are relatively non-toxic to mammals.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a composition comprising two or three active ingredients may be mixed by the user himself in a spray tank and further auxiliaries may be added, if appropriate.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides (e.g. pesticidally active substances and biopesticides), in conjunction with which the compounds I can be used, is intended to illustrate the possible combinations but does not limit them:
A) Respiration inhibitors
   - Inhibitors of complex III at Q₀ site (e.g. strobilurins): azoxystrobin, coumethoxystrobin, coumoxystrobin, dimoxystrobin, enestroburin, fenaminstrobin, fenoxy-strobin/flufenoxystrobin, fluoxastrobin, kresoxim-methyl, metominostrobin, orysastrobin, picoxystrobin, pyraclostrobin, pyrametostrobin, pyraoxystrobin, trifloxystrobin, 2-[2-(2,5-dimethyl-phenoxymethyl)-phenyl]-3-methoxy-acrylic acid methyl ester and 2-(2-(3-(2,6-di-chlorophenyl)-1-methyl-allylideneaminooxymethyl)-phenyl)-2-methoxyimino-N-methyl-acetamide, pyribencarb, triclopyricarb/chlorodincarb, famoxadone, fenamidone;
   - inhibitors of complex III at Qᵢ site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate; (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate; (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate;
   - inhibitors of complex II (e. g. carboxamides): benodanil, benzovindiflupyr, bixafen, boscalid, carboxin, fenfuram, fluopyram, flutolanil, fluxapyroxad, furametpyr, isofetamid, isopyrazam, mepronil, oxycarboxin, penflufen, penthiopyrad, sedaxane, tecloftalam, thifluzamide, N-(4'-trifluoromethylthiobiphenyl-2-yl)-3-difluoromethyl-1-methyl-1H-pyrazole-4-carboxamide, N-(2-(1,3,3-trimethyl-butyl)-phenyl)-1,3-dimethyl-5-fluoro-1H-pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide; N-(7-fluoro-1,1,3-trimethyl-indan-4-yl)-1,3-dimethyl-pyrazole-4-carboxamide, N-[2-(2,4-dichlorophenyl)-2-methoxy-1-methyl-ethyl]-3-(difluoromethyl)-1-methyl-pyrazole-4-carboxamide;
   - other respiration inhibitors (e.g. complex I, uncouplers): diflumetorim, (5,8-difluoroquinazolin-4-yl)-{2-[2-fluoro-4-(4-trifluoromethylpyridin-2-yloxy)-phenyl]-ethyl}-amine; nitrophenyl derivates: binapacryl, dinobuton, dinocap, fluazinam; ferimzone; organometal compounds: fentin salts, such as fentin-acetate, fentin chloride or fentin hydroxide; ametoctradin; and silthiofam;
B) Sterol biosynthesis inhibitors (SBI fungicides)
   - C14 demethylase inhibitors (DMI fungicides): triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole, 1-[*rel*-(2*S*;3*R*)-3-(2-chlorophenyl)-2-(2,4-difluorophenyl)-oxiranylmethyl]-5-thiocyanato-1H-[1,2,4]triazole, 2-[*rel*-(2*S*;3*R*)-3-(2-chlorophenyl)-2-(2,4-difluorophenyl)-oxiranylmethyl]-2H-[1,2,4]triazole-3-thiol; 2-[2-chloro-4-(4-chlorophenoxy)phenyl]-1-(1,2,4-triazol-1-yl)pentan-2-ol, 1-[4-(4-chlorophenoxy)-2-(trifluoromethyl)phenyl]-1-cyclopropyl-2-(1,2,4-triazol-1-yl)ethanol, 2-[4-(4-chlorophenoxy)-2-(trifluoromethyl)phenyl]-1-(1,2,4-triazol-1-yl)butan-2-ol, 2-[2-chloro-4-(4-chlorophenoxy)phenyl]-1-(1,2,4-triazol-1-yl)butan-2-ol, 2-[4-(4-chloro-phenoxy)-2-(trifluoromethyl)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)propan-2-ol, 2-[2-chloro-4-(4-chlorophenoxy)phenyl]-3-methyl-1-(1,2,4-triazol-1-yl)butan-2-ol, 2-[4-(4-chlorophenoxy)-2-(trifluoromethyl)phenyl]-1-(1,2,4-triazol-1-yl)pentan-2-ol, 2-[4-(4-fluorophenoxy)-2-(trifluoromethyl)phenyl]-1-(1,2,4-triazol-1-yl)propan-2-ol; imidazoles: imazalil, pefurazoate, prochloraz, triflumizol; pyrimidines, pyridines and piperazines: fenarimol, nuarimol, pyrifenox, triforine, 3-(4-chloro-2-fluoro-phenyl)-5-(2,4-difluorophenyl)isoxazol-4-yl]-(3-pyridyl)methanol;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
C) Nucleic acid synthesis inhibitors
   - phenylamides or acyl amino acid fungicides: benalaxyl, benalaxyl-M, kiralaxyl, metalaxyl, metalaxyl-M (mefenoxam), ofurace, oxadixyl;
   - others: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine;
D) Inhibitors of cell division and cytoskeleton
   - tubulin inhibitors, such as benzimidazoles, thiophanates: benomyl, carbendazim, fuberidazole, thiabendazole, thiophanate-methyl; triazolopyrimidines: 5-chloro-7-(4-methyl-piperidin-1-yl)-6-(2,4,6-trifluorophenyl)-[1,2,4]triazolo[1,5-a]pyrimidine
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors (anilino-pyrimidines): cyprodinil, mepanipyrim, pyrimethanil;
   - protein synthesis inhibitors: blasticidin-S, kasugamycin, kasugamycin hydrochloride-hydrate, mildiomycin, streptomycin, oxytetracyclin, polyoxine, validamycin A;
F) Signal transduction inhibitors
   - MAP / histidine kinase inhibitors: fluoroimid, iprodione, procymidone, vinclozolin, fenpiclonil, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate and N-(1-(1-(4-cyano-phenyl)ethanesulfonyl)-but-2-yl) carbamic acid-(4-fluorophenyl) ester;
   - compounds affecting cell membrane permeability and fatty acides: propamocarb, propamo-carb-hydrochlorid
   - fatty acid amide hydrolase inhibitors: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone; 2-{3-[2-(1-{[3,5-bis(difluoromethyl-1H-pyrazol-1-yl]acetyl}piperidin-4-yl)-1,3-thiazol-4-yl]-4,5-dihydro-1,2-oxazol-5-yl}phenyl methanesulfonate, 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5-yl}-3-chlorophenyl methanesulfonate;
H) Inhibitors with Multi Site Action
   - inorganic active substances: Bordeaux mixture, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds (e.g. phthalimides, sulfamides, chloronitriles): anilazine, chlorothalonil, captafol, captan, folpet, dichlofluanid, dichlorophen, hexachlorobenzene, pentachlorphenole and its salts, phthalide, tolylfluanid, N-(4-chloro-2-nitro-phenyl)-N-ethyl-4-methyl-benzenesulfonamide;
   - guanidines and others: guanidine, dodine, dodine free base, guazatine, guazatine-acetate, iminoctadine, iminoctadine-triacetate, iminoctadine-tris(albesilate), dithianon, 2,6-dimethyl-1 H,5H-[1,4]dithiino[2,3-c:5,6-c']dipyrrole-1,3,5,7(2H,6H)-tetraone;
I) Cell wall synthesis inhibitors
   - inhibitors of glucan synthesis: validamycin, polyoxin B;
   - melanin synthesis inhibitors: pyroquilon, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum, phosphorous acid and its salts;
K) Unknown mode of action
   - bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, tolprocarb, oxin-copper, proquinazid, tebufloquin, tecloftalam, triazoxide, 2-butoxy-6-iodo-3-propylchromen-4-one, 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, 2-[3,5-bis(difluoromethyl)-1 H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, methoxyacetic acid 6-tert-butyl-8-fluoro-2,3-dimethyl-quinolin-4-yl ester, 3-[5-(4-methylphenyl)-2,3-dimethyl-isoxazolidin-3-yl]-pyridine, 3-[5-(4-chloro-phenyl)-2,3-dimethyl-isoxazolidin-3-yl]-pyridine (pyrisoxazole),
      N-(6-methoxy-pyridin-3-yl) cyclopropanecarboxylic acid amide, 5-chloro-1-(4,6-dimethoxy-pyrimidin-2-yl)-2-methyl-1 H-benzoimidazole, 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate , picarbutrazox, pentyl N-[6-[[(Z)-[(1-methyltetrazol-5-yl)-phenyl-methylene]amino]oxymethyl]-2-pyridyl]carbamate, 2-[2-[(7,8-difluoro-2-methyl-3-quinolyl)oxy]-6-fluoro-phenyl]propan-2-ol, 2-[2-fluoro-6-[(8-fluoro-2-methyl-3-quinolyl)oxy]-phen-yl]propan-2-ol, 3-(5-fluoro-3,3,4,4-tetramethyl-3,4-dihydroisoquinolin-1-yl)quinoline, 3-(4,4-difluoro-3,3-dimethyl-3,4-dihydroisoquinolin-1-yl)quinoline, 3-(4,4,5-trifluoro-3,3-dimethyl-3,4-dihydroisoquinolin-1-yl)quinoline;
L) Biopesticides
   L1) Microbial pesticides with fungicidal, bactericidal, viricidal and/or plant defense activator activity: Ampelomyces quisqualis, Aspergillus flavus, Aureobasidium pullulans, Bacillus amyloliquefaciens, B. mojavensis, B. pumilus, B. simplex, B. solisalsi, B. subtilis, B. subtilis var. amyloliquefaciens, Candida oleophila, C. saitoana, Clavibacter michiganensis (bacteriophages), Coniothyrium minitans, Cryphonectria parasitica, Cryptococcus albidus, Dilophosphora alopecuri, Fusarium oxysporum, Clonostachys rosea f. catenulate (also named Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus polymyxa, Pantoea vagans, Phlebiopsis gigantea, Pseudomonas sp., Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperellum, T. atroviride, T. fertile, T. gamsii, T. harmatum, T. harzianum; mixture of T. harzianum and T. viride; mixture of T. polysporum and T. harzianum; T. stromaticum, T. virens (also named Gliocladium virens), T. viride, Typhula phacorrhiza, Ulocladium oudemansii, Verticillium dahlia, zucchini yellow mosaic virus (avirulent strain);
   L2) Biochemical pesticides with fungicidal, bactericidal, viricidal and/or plant defense activator activity: chitosan (hydrolysate), harpin protein, laminarin, Menhaden fish oil, natamycin, Plum pox virus coat protein, potassium or sodium bicarbonate, Reynoutria sachlinensis extract, salicylic acid, tea tree oil;
   L3) Microbial pesticides with insecticidal, acaricidal, molluscidal and/or nematicidal activity: Agrobacterium radiobacter, Bacillus cereus, B. firmus, B. thuringiensis, B. thuringiensis ssp. aizawai, B. t. ssp. israelensis, B. t. ssp. galleriae, B. t. ssp. kurstaki, B. t. ssp. tenebrionis, Beauveria bassiana, B. brongniartii , Burkholderia sp., Chromobacterium subtsugae, Cydia pomonella granulosis virus, Cryptophlebia leucotreta granulovirus (CrIeGV), Isaria fumosorosea, Heterorhabditis bacteriophora, Lecanicillium longisporum, L. muscarium (formerly Verticillium lecanii), Metarhizium anisopliae, M. anisopliae var. acridum, Nomuraea rileyi, Paecilomyces fumosoroseus, P. lilacinus, Paenibacillus popilliae, Pasteuria spp., P. nishizawae, P. penetrans, P. ramose, P. reneformis, P. thornea, P. usgae, Pseudomonas fluorescens, Steinernema carpocapsae, S. feltiae, S. kraussei;
   L4) Biochemical pesticides with insecticidal, acaricidal, molluscidal, pheromone and/or nematicidal activity: L-carvone, citral, (E,Z)-7,9-dodecadien-1-yl acetate, ethyl formate, (E,Z)-2,4-ethyl decadienoate (pear ester), (Z,Z,E)-7,11,13-hexadecatrienal, heptyl butyrate, isopropyl myristate, lavanulyl senecioate, cis-jasmone, 2-methyl 1-butanol, methyl eugenol, methyl jasmonate, (E,Z)-2,13-octadecadien-1-ol, (E,Z)-2,13-octadecadien-1-ol acetate, (E,Z)-3,13-octadecadien-1-ol, R-1-octen-3-ol, pentatermanone, potassium silicate, sorbitol actanoate, (E,Z,Z)-3,8,11-tetradecatrienyl acetate, (Z,E)-9,12-tetradecadien-1-yl acetate, Z-7-tetradecen-2-one, Z-9-tetradecen-1-yl acetate, Z-11-tetradecenal, Z-11-tetradecen-1-ol, Acacia negra extract, extract of grapefruit seeds and pulp, extract of Chenopodium ambrosiodae, Catnip oil, Neem oil, Quillay extract, Tagetes oil;
   L5) Microbial pesticides with plant stress reducing, plant growth regulator, plant growth promoting and/or yield enhancing activity: Azospirillum amazonense, A. brasilense, A. lipoferum, A. irakense, A. halopraeferens, Bradyrhizobium sp., B. elkanii, B. japonicum, B. liaoningense, B. lupini, Delftia acidovorans, Glomus intraradices, Mesorhizobium sp., Paenibacillus alvei, Penicillium bilaiae, Rhizobium leguminosarum bv. phaseoli, R. I. trifolii, R. I. bv. viciae, R. tropici, Sinorhizobium meliloti;
   L6) Biochemical pesticides with plant stress reducing, plant growth regulator and/or plant yield enhancing activity: abscisic acid, aluminium silicate (kaolin), 3-decen-2-one, formononetin, genistein, hesperetin, homobrassinlide, humates, jasmonic acid or salts or derivatives thereof, lysophosphatidyl ethanolamine, naringenin, polymeric polyhydroxy acid, Ascophyllum nodosum (Norwegian kelp, Brown kelp) extract and Ecklonia maxima (kelp) extract;
M) Growth regulators
   abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonyl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron,tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethyl, chlorthal, cinmethylin, clomazone, cumyluron, cyprosulfamide, dicamba, difenzoquat, diflufenzopyr, *Drechslera monoceras,* endothal, ethofumesate, etobenzanid, fenoxasulfone, fentrazamide, flumicloracpentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester.
O) Insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, e-thion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alphacypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) e-cdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, flupyradifurone, imidacloprid, thiamethoxam, nitenpyram, acetamiprid, thiacloprid, 1-2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperonyl butoxide;
   - sodium channel blockers: indoxacarb, metaflumizone;
   - ryanodine receptor inhibitors: chlorantraniliprole, cyantraniliprole, flubendiamide, N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide; N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide; N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide; N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide; N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide; N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide; N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide);
   - others: benclothiaz, bifenazate, cartap, flonicamid, pyridalyl, pymetrozine, sulfur, thiocyclam, cyenopyrafen, flupyrazofos, cyflumetofen, amidoflumet, imicyafos, bistrifluron, pyrifluquinazon and 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester.

The present invention furthermore relates to compositions comprising a compound I (component 1) and at least one further active substance useful for plant protection, e. g. selected from the groups A) to O) (component 2), in particular one further fungicide, e. g. fungicide from the groups A) to K), as described above, and if desired one suitable solvent or solid carrier. Those compositions are of particular interest, since many of them at the same application rate show higher efficiencies against harmful fungi. Furthermore, combating harmful fungi with a composition comprising a compoundl and a fungicide from groups A) to K), as described above, is more efficient than combating those fungi with individual compounds I or individual fungicides from groups A) to K). By applying compounds I together with at least one active substance from groups A) to O) a synergistic effect can be obtained, i.e. more then simple addition of the individual effects is obtained (synergistic compositions).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying a compound of the present invention and a pesticide II sequentially the time between both applications may vary e.g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day. In case of a composition or mixture comprising a pesticide II selected from group L), it is preferred that the pesticide II is applied as last treatment.

According to the invention, the solid material (dry matter) of the biopesticides (with the exception of oils such as Neem oil, Tagetes oil, etc.) are considered as active components (e.g. to be obtained after drying or evaporation of the extraction medium or the suspension medium in case of liquid formulations of the microbial pesticides).

In accordance with the present invention, the weight ratios and percentages used herein for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calclulate the total weight of the respective active component with the following equation that 1 x 10⁹ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomopathogenic) nematode biopesticides, such as Steinernema feltiae.

In the binary mixtures and compositions according to the invention the weight ratio of the component 1) and the component 2) generally depends from the properties of the active components used, usually it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to a further embodiments of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 1 to 1:1, even more preferably in the range of from 4:1 to 1:1 and in particular in the range of from 2:1 to 1:1.

According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

In compositions according to the invention comprising one compound I (component 1) and one further pesticidally active substance (component 2), e. g. one active substance from groups A) to K), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:3 to 3:1.

In compositions according to the invention comprising one compound I (component 1) and a first further pesticidally active substance (component 2) and a second further pesticidally active substance (component 3), e. g. two active substances from groups A) to K), the weight ratio of component 1 and component 2 depends from the properties of the active substances used, preferably it is in the range of from 1:50 to 50:1 and particularly in the range of from 1:10 to 10:1, and the weight ratio of component 1 and component 3 preferably is in the range of from 1:50 to 50:1 and particularly in the range of from 1:10 to 10:1.

Preference is also given to compositions comprising a compound I (component 1) and at least one active substance selected from group A) (component 2) and particularly selected from azoxystrobin, dimoxystrobin, fluoxastrobin, kresoxim-methyl, orysastrobin, picoxystrobin, pyraclostrobin, trifloxystrobin; famoxadone, fenamidone; benzovindiflupyr, bixafen, boscalid, fluopyram, fluxapyroxad, isopyrazam, penflufen, penthiopyrad, sedaxane; ametoctradin, cyazofamid, fluazinam, fentin salts, such as fentin acetate.

Preference is given to compositions comprising a compound of formula I (component 1) and at least one active substance selected from group B) (component 2) and particularly selected from cyproconazole, difenoconazole, epoxiconazole, fluquinconazole, flusilazole, flutriafol, metconazole, myclobutanil, penconazole, propiconazole, prothioconazole, triadimefon, triadimenol, tebuconazole, tetraconazole, triticonazole, prochloraz, fenarimol, triforine; dodemorph, fenpropimorph, tridemorph, fenpropidin, spiroxamine; fenhexamid.

Preference is given to compositions comprising a compound of formula I (component 1) and at least one active substance selected from group C) (component 2) and particularly selected from metalaxyl, (metalaxyl-M) mefenoxam, ofurace.

Preference is given to compositions comprising a compound of formula I (component 1) and at least one active substance selected from group D) (component 2) and particularly selected from benomyl, carbendazim, thiophanate-methyl, ethaboxam, fluopicolide, zoxamide, metrafenone, pyriofenone.

Preference is also given to compositions comprising a compound I (component 1) and at least one active substance selected from group E) (component 2) and particularly selected from cyprodinil, mepanipyrim, pyrimethanil.

Preference is also given to compositions comprising a compound I (component 1) and at least one active substance selected from group F) (component 2) and particularly selected from iprodione, fludioxonil, vinclozolin, quinoxyfen.

Preference is also given to compositions comprising a compound I (component 1) and at least one active substance selected from group G) (component 2) and particularly selected from dimethomorph, flumorph, iprovalicarb, benthiavalicarb, mandipropamid, propamocarb.

Preference is also given to compositions comprising a compound I (component 1) and at least one active substance selected from group H) (component 2) and particularly selected from copper acetate, copper hydroxide, copper oxychloride, copper sulfate, sulfur, mancozeb, metiram, propineb, thiram, captafol, folpet, chlorothalonil, dichlofluanid, dithianon.

Preference is also given to compositions comprising a compound I (component 1) and at least one active substance selected from group I) (component 2) and particularly selected from carpropamid and fenoxanil.

Preference is also given to compositions comprising a compound I (component 1) and at least one active substance selected from group J) (component 2) and particularly selected from acibenzolar-S-methyl, probenazole, tiadinil, fosetyl, fosetyl-aluminium, H₃PO₃ and salts thereof.

Preference is also given to compositions comprising a compound I (component 1) and at least one active substance selected from group K) (component 2) and particularly selected from cymoxanil, proquinazid and *N*-methyl-2-{1-[(5-methyl-3-trifluoromethyl-1H-pyrazol-1-yl)-acetyl]-piperidin-4-yl}-*N*-[(1R)-1,2,3,4-tetrahydronaphthalen-1-yl]-4-thiazolecarboxamide.

The biopesticides from group L) of pesticides II, their preparation and their pesticidal activity e.g. against harmful fungi or insects are known (e-Pesticide Manual V 5.2 (ISBN 978 1 901396 85 0) (2008-2011); http://www.epa.gov/opp00001/biopesticides/, see product lists therein; http://www.omri.org/omri-lists, see lists therein; Bio-Pesticides Database BPDB http://sitem.herts.ac.uk/aeru/bpdb/, see A to Z link therein).

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) and/or L6) may also have fungicidal, bactericidal, viricidal, plant defense activator, insecticidal, acaricidal, molluscidal, pheromone and/or nematicidal activity.

Many of these biopesticides are registered and/or are commercially available: aluminium silicate (Screen™ Duo from Certis LLC, USA), Agrobacterium radiobacter K1026 (e.g. NoGall® from Becker Underwood Pty Ltd., Australia), A. radiobacter K84 (Nature 280, 697-699, 1979; e.g. GallTroll® from AG Biochem, Inc., C, USA), Ampelomyces quisqualis M-10 (e.g. AQ 10® from Intrachem Bio GmbH & Co. KG, Germany), Ascophyllum nodosum (Norwegian kelp, Brown kelp) extract or filtrate (e.g. ORKA GOLD from Becker Underwood, South Africa; or Goemar® from Laboratoires Goemar, France), Aspergillus flavus NRRL 21882 isolated from a peanut in Georgia in 1991 by the USDA, National Peanut Research Laboratory (e.g. in Afla-Guard® from Syngenta, CH), mixtures of Aureobasidium pullulans DSM14940 and DSM 14941 (e.g. blastospores in BlossomProtect® from bio-ferm GmbH, Germany), Azospirillum amazonense BR 11140 (SpY2^{T}) (Proc. 9th Int. and 1st Latin American PGPR meeting, Quimara, Medellín, Colombia 2012, p. 60, ISBN 978-958-46-0908-3), A. brasilense AZ39 (Eur. J. Soil Biol 45(1), 28-35, 2009), A. brasilense XOH (e.g. AZOS from Xtreme Gardening, USA or RTI Reforestation Technologies International; USA), A. brasilense BR 11002 (Proc. 9^{th} Int. and 1^{st} Latin American PGPR meeting, Quimara, Medellín, Colombia 2012, p. 60, ISBN 978-958-46-0908-3), A. brasilense BR 11005 (SP245; e.g. in GELFIX Gramíneas from BASF Agricultural Specialties Ltd., Brazil), A. lipoferum BR 11646 (Sp31) (Proc. 9^{th} Int. and 1^{st} Latin American PGPR meeting, Quimara, Medellín, Colombia 2012, p. 60), Bacillus amyloliquefaciens FZB42 (e.g. in RhizoVital® 42 from AbiTEP GmbH, Berlin, Germany), B. amyloliquefaciens IN937a (J. Microbiol. Biotechnol. 17(2), 280-286, 2007; e.g. in BioYield® from Gustafson LLC, TX, USA), B. amyloliquefaciens IT-45 (CNCM I-3800) (e.g. Rhizocell C from ITHEC, France), B. amyloliquefaciens subsp. plantarum MBI600 (NRRL B-50595, deposited at United States Department of Agriculture) (e.g. Integral®, Subtilex® NG from Becker Underwood, USA), B. cereus CNCM I-1562 (US 6,406,690), B. firmus CNCM I-1582 (WO 2009/126473, WO 2009/124707, US 6,406,690; Votivo® from Bayer Crop Science LP, USA), B. pumilus GB34 (ATCC 700814; e.g. in YieldShield® from Gustafson LLC, TX, USA), and Bacillus pumilus KFP9F (NRRL B-50754) (e.g. in BAC-UP or FUSION-P from Becker Underwood South Africa), B. pumilus QST 2808 (NRRL B-30087) (e.g. Sonata® and Ballad® Plus from AgraQuest Inc., USA), B. subtilis GB03 (e.g. Kodiak® or BioYield® from Gustafson, Inc., USA; or Companion® from Growth Products, Ltd., White Plains, NY 10603, USA), B. subtilis GB07 (Epic® from Gustafson, Inc., USA), B. subtilis QST-713 (NRRL B-21661 in Rhapsody®, Serenade® MAX and Serenade® ASO from AgraQuest Inc., USA), B. subtilis var. amyloliquefaciens FZB24 (e.g. Taegro® from Novozyme Biologicals, Inc., USA), B. subtilis var. amyloliquefaciens D747 (e.g. Double Nickel 55 from Certis LLC, USA), B. thuringiensis ssp. aizawai ABTS-1857 (e.g. in XenTari® from BioFa AG, Münsingen, Germany), B. t. ssp. aizawai SAN 401 I, ABG-6305 and ABG-6346, Bacillus t. ssp. israelensis AM65-52 (e.g. in VectoBac® from Valent BioSciences, IL, USA), Bacillus thuringiensis ssp. kurstaki SB4 (NRRL B-50753; e.g. Beta Pro® from Becker Underwood, South Africa), B. t. ssp. kurstaki ABTS-351 identical to HD-1 (ATCC SD-1275; e.g. in Dipel® DF from Valent BioSciences, IL, USA), B. t. ssp. kurstaki EG 2348 (e.g. in Lepinox® or Rapax® from CBC (Europe) S.r.l., Italy), B. t. ssp. tenebrionis DSM 2803 (EP 0 585 215 B1; identical to NRRL B-15939; Mycogen Corp.), B. t. ssp. tenebrionis NB-125 (DSM 5526; EP 0 585 215 B1; also referred to as SAN 418 I or ABG-6479; former production strain of Novo-Nordisk), B. t. ssp. tenebrionis NB-176 (or NB-176-1) a gamma-irridated, induced high-yielding mutant of strain NB-125 (DSM 5480; EP 585 215 B1; Novodor® from Valent BioSciences, Switzerland), Beauveria bassiana ATCC 74040 (e.g. in Naturalis® from CBC (Europe) S.r.l., Italy), B. bassiana DSM 12256 (US 200020031495; e.g. BioExpert® SC from Live Sytems Technology S.A., Colombia), B. bassiana GHA (BotaniGard® 22WGP from Laverlam Int. Corp., USA), B. bassiana PPRI 5339 (ARSEF number 5339 in the USDA ARS collection of entomopathogenic fungal cultures; NRRL 50757) (e.g. BroadBand® from Becker Underwood, South Africa), B. brongniartii (e.g. in Melocont® from Agrifutur, Agrianello, Italy, for control of cockchafer; J. Appl. Microbiol. 100(5),1063-72, 2006), Bradyrhizobium sp. (e.g. Vault® from Becker Underwood, USA), B. japonicum (e.g. VAULT® from Becker Underwood, USA), Candida oleophila I-182 (NRRL Y-18846; e.g. Aspire® from Ecogen Inc., USA, Phytoparasitica 23(3), 231-234, 1995), C. oleophila strain O (NRRL Y-2317; Biological Control 51, 403-408, 2009),, Candida saitoana (e.g. Biocure® (in mixture with lysozyme) and BioCoat® from Micro Flo Company, USA (BASF SE) and Arysta), Chitosan (e.g. Armour-Zen® from BotriZen Ltd., NZ), Clonostachys rosea f. catenulata, also named Gliocladium catenulatum (e.g. isolate J 1446: Prestop® from Verdera Oy, Finland), Chromobacterium subtsugae PRAA4-1 isolated from soil under an eastern hemlock (Tsuga canadensis) in the Catoctin Mountain region of central Maryland (e.g. in GRANDEVO from Marrone Bio Innovations, USA), Coniothyrium minitans CON/M/91-08 (e.g. Contans® WG from Prophyta, Germany), Cryphonectria parasitica (e.g. Endothia parasitica from CNICM, France), Cryptococcus albidus (e.g. YIELD PLUS® from Anchor Bio-Technologies, South Africa), Cryptophlebia leucotreta granulovirus (CrleGV) (e.g. in CRYPTEX from Adermatt Biocontrol, Switzerland), Cydia pomonella granulovirus (CpGV) V03 (DSM GV-0006; e.g. in MADEX Max from Andermatt Biocontrol, Switzerland), CpGV V22 (DSM GV-0014; e.g. in MADEX Twin from Adermatt Biocontrol, Switzerland), Delftia acidovorans RAY209 (ATCC PTA-4249; WO 2003/57861; e.g. in BIOBOOST from Brett Young, Winnipeg, Canada), Dilophosphora alopecuri (Twist Fungus from Becker Underwood, Australia), Ecklonia maxima (kelp) extract (e.g. KELPAK SL from Kelp Products Ltd, South Africa), formononetin (e.g. in MYCONATE from Plant Health Care plc, U.K.), Fusarium oxysporum (e.g. BIOFOX® from S.I.A.P.A., Italy, FUSACLEAN® from Natural Plant Protection, France), Glomus intraradices (e.g. MYC 4000 from ITHEC, France), Glomus intraradices RTI-801 (e.g. MYKOS from Xtreme Gardening, USA or RTI Reforestation Technologies International; USA), grapefruit seeds and pulp extract (e.g. BC-1 000 from Chemie S.A., Chile), harpin (alpha-beta) protein (e.g. MESSENGER or HARP-N-Tek from Plant Health Care plc, U.K.; Science 257, 1-132, 1992), Heterorhabditis bacteriophaga (e.g. Nemasys® G from Becker Underwood Ltd., UK), Isaria fumosorosea Apopka-97 (ATCC 20874) (PFR-97™ from Certis LLC, USA), cis-jasmone (US 8,221,736), laminarin (e.g. in VACCIPLANT from Laboratoires Goemar, St. Malo, France or Stähler SA, Switzerland), Lecanicillium longisporum KV42 and KV71 (e.g. VERTALEC® from Koppert BV, Netherlands), L. muscarium KV01 (formerly Verticillium lecanii) (e.g. MYCOTAL from Koppert BV, Netherlands), Lysobacter antibioticus 13-1 (Biological Control 45, 288-296, 2008), L. antibioticus HS124 (Curr. Microbiol. 59(6), 608-615, 2009), L. enzymogenes 3.1T8 (Microbiol. Res. 158, 107-115; Biological Control 31(2), 145-154, 2004), Metarhizium anisopliae var. acridum IMI 330189 (isolated from Ornithacris cavroisi in Niger; also NRRL 50758) (e.g. GREEN MUSCLE® from Becker Underwood, South Africa), M. a. var. acridum FI-985 (e.g. GREEN GUARD® SC from Becker Underwood Pty Ltd, Australia), M. anisopliae FI-1045 (e.g. BIOCANE® from Becker Underwood Pty Ltd, Australia), M. anisopliae F52 (DSM 3884, ATCC 90448; e.g. MET52® Novozymes Biologicals BioAg Group, Canada), M. anisopliae ICIPE 69 (e.g. METATHRIPOL from ICIPE, Nairobe, Kenya), Metschnikowia fructicola (NRRL Y-30752; e.g. SHEMER® from Agrogreen, Israel, now distributed by Bayer CropSciences, Germany; US 6,994,849), Microdochium dimerum (e.g. ANTIBOT® from Agrauxine, France), Microsphaeropsis ochracea P130A (ATCC 74412 isolated from apple leaves from an abandoned orchard, St-Joseph-du-Lac, Quebec, Canada in 1993; Mycologia 94(2), 297-301, 2002), Muscodor albus QST 20799 originally isolated from the bark of a cinnamon tree in Honduras (e.g. in development products Muscudor™ or QRD300 from AgraQuest, USA), Neem oil (e.g. TRILOGY®, TRIACT® 70 EC from Certis LLC, USA), Nomuraea rileyi strains SA86101, GU87401, SR86151, CG128 and VA9101, Paecilomyces fumosoroseus FE 9901 (e.g. NO FLY™ from Natural Industries, Inc., USA), P. lilacinus 251 (e.g. in BioAct®/MeloCon® from Prophyta, Germany; Crop Protection 27, 352-361, 2008; originally isolated from infected nematode eggs in the Philippines), P. lilacinus DSM 15169 (e.g. NEMATA® SC from Live Systems Technology S.A., Colombia), P. lilacinus BCP2 (NRRL 50756; e.g. PL GOLD from Becker Underwood BioAg SA Ltd, South Africa), mixture of Paenibacillus alvei NAS6G6 (NRRL B-50755), Pantoea vagans (formerly agglomerans) C9-1 (originally isolated in 1994 from apple stem tissue; BlightBan C9-1® from NuFrams America Inc., USA, for control of fire blight in apple; J. Bacteriol. 192(24) 6486-6487, 2010), Pasteuria spp. ATCC PTA-9643 (WO 2010/085795), Pasteuria spp. ATCC SD-5832 (WO 2012/064527), P. nishizawae (WO 2010/80169), P. penetrans (US 5,248,500), P. ramose (WO 2010/80619), P. thornea (WO 2010/80169), P. usgae (WO 2010/80169), Penicillium bilaiae (e.g. Jump Start® from Novozymes Biologicals BioAg Group, Canada, originally isolated from soil in southern Alberta; Fertilizer Res. 39, 97-103, 1994), Phlebiopsis gigantea (e.g. RotStop® from Verdera Oy, Finland), Pichia anomala WRL-076 (NRRL Y-30842; US 8,206,972), potassium bicarbonate (e.g. Amicarb® fromm Stähler SA, Switzerland), potassium silicate (e.g. Sil-MATRIX™ from Certis LLC, USA), Pseudozyma flocculosa PF-A22 UL (e.g. Sporodex® from Plant Products Co. Ltd., Canada), Pseudomonas sp. DSM 13134 (WO 2001/40441, e.g. in PRORADIX from Sourcon Padena GmbH & Co. KG, Hechinger Str. 262, 72072 Tübingen, Germany), P. chloraphis MA 342 (e.g. in CERALL or CEDEMON from BioAgri AB, Uppsala, Sweden), P. fluorescens CL 145A (e.g. in ZEQUANOX from Marrone Biolnnovations, Davis, CA, USA; J. Invertebr. Pathol. 113(1):104-14, 2013), Pythium oligandrum DV 74 (ATCC 38472; e.g. POLYVERSUM® from Remeslo SSRO, Biopreparaty, Czech Rep. and GOWAN, USA; US 2013/0035230), Reynoutria sachlinensis extract (e.g. REGALIA® SC from Marrone BioInnovations, Davis, CA, USA), Rhizobium leguminosarum bv. phaseoli (e.g. RHIZO-STICK from Becker Underwood, USA), R. I. trifolii RP113-7 (e.g. DORMAL from Becker Underwood, USA; Appl. Environ. Microbiol. 44(5), 1096-1101), R. I. bv. viciae P1NP3Cst (also referred to as 1435; New Phytol 179(1), 224-235, 2008; e.g. in NODULATOR PL Peat Granule from Becker Underwood, USA; or in NODULATOR XL PL bfrom Becker Underwood, Canada), R. I. bv. viciae SU303 (e.g. NODULAID Group E from Becker Underwood, Australia), R. I. bv. viciae WSM1455 (e.g. NODULAID Group F from Becker Underwood, Australia), R. tropici SEMIA 4080 (identical to PRF 81; Soil Biology & Biochemistry 39, 867-876, 2007), Sinorhizobium meliloti MSDJ0848 (INRA, France) also referred to as strain 2011 or RCR2011 (Mol Gen Genomics (2004) 272: 1-17; e.g. DORMAL ALFALFA from Becker Underwood, USA; NITRAGIN® Gold from Novozymes Biologicals BioAg Group, Canada), Sphaerodes mycoparasitica IDAC 301008-01 (WO 2011/022809), Steinernema carpocapsae (e.g. MILLENIUM® from Becker Underwood Ltd., UK), S. feltiae (NEMASHIELD® from BioWorks, Inc., USA; NEMASYS® from Becker Underwood Ltd., UK), S. kraussei L137 (NEMASYS® L from Becker Underwood Ltd., UK), Streptomyces griseoviridis K61 (e.g. MYCOSTOP® from Verdera Oy, Espoo, Finland; Crop Protection 25, 468-475, 2006), S. lydicus WYEC 108 (e.g. Actinovate® from Natural Industries, Inc., USA, US 5,403,584), S. violaceusniger YCED-9 (e.g. DT-9® from Natural Industries, Inc., USA, US 5,968,503), Talaromyces flavus V117b (e.g. PROTUS® from Prophyta, Germany), Trichoderma asperellum SKT-1 (e.g. ECO-HOPE® from Kumiai Chemical Industry Co., Ltd., Japan), T. asperellum ICC 012 (e.g. in TENET WP, REMDIER WP, BIOTEN WP from Isagro NC, USA, BIO-TAM from AgraQuest, USA), T. atroviride LC52 (e.g. SENTINEL® from Agrimm Technologies Ltd, NZ), T. atroviride CNCM I-1237 (e.g. in Esquive WG from Agrauxine S.A., France, e.g. against pruning wound diseases on vine and plant root pathogens), T. fertile JM41 R (NRRL 50759; e.g. RICHPLUS™ from Becker Underwood Bio Ag SA Ltd, South Africa), T. gamsii ICC 080 (e.g. in TENET WP, REMDIER WP, BIOTEN WP from Isagro NC, USA, BIO-TAM from AgraQuest, USA), T. harzianum T-22 (e.g. PLANTSHIELD® der Firma BioWorks Inc., USA), T. harzianum TH 35 (e.g. ROOT PRO® from Mycontrol Ltd., Israel), T. harzianum T-39 (e.g. TRICHODEX® and TRICHODERMA 2000® from Mycontrol Ltd., Israel and Makhteshim Ltd., Israel), T. harzianum and T. viride (e.g. TRICHOPEL from Agrimm Technologies Ltd, NZ), T. harzianum ICC012 and T. viride ICC080 (e.g. REMEDIER® WP from Isagro Ricerca, Italy), T. polysporum and T. harzianum (e.g. BINAB® from BINAB Bio-Innovation AB, Sweden), T. stromaticum (e.g. TRICOVAB® from C.E.P.L.A.C., Brazil), T. virens GL-21 (also named Gliocladium virens) (e.g. SOILGARD® from Certis LLC, USA), T. viride (e.g. TRIECO® from Ecosense Labs. (India) Pvt. Ltd., Indien, BIO-CURE® F from T. Stanes & Co. Ltd., Indien), T. viride TV1 (e.g. T. viride TV1 from Agribiotec srl, Italy) and Ulocladium oudemansii HRU3 (e.g. in BOTRY-ZEN® from Botry-Zen Ltd, NZ).

Strains can be sourced from genetic resource and deposition centers: American Type Culture Collection, 10801 University Blvd., Manassas, VA 20110-2209, USA (strains with ATCC prefic); CABI Europe - International Mycological Institute, Bakeham Lane, Egham, Surrey, TW20 9TYNRRL, UK (strains with prefices CABI and IMI); Centraalbureau voor Schimmelcultures, Fungal Biodiversity Centre, Uppsalaan 8, PO Box 85167, 3508 AD Utrecht, Netherlands (strains with prefic CBS); Division of Plant Industry, CSIRO, Canberra, Australia (strains with prefix CC); Collection Nationale de Cultures de Microorganismes, Institut Pasteur, 25 rue du Docteur Roux, F-75724 PARIS Cedex 15 (strains with prefix CNCM); Leibniz-Institut DSMZ-Deutsche Sammlung von Mikroorganismen und Zellkulturen GmbH, Inhoffenstraße 7 B, 38124 Braunschweig, Germany (strains with prefix DSM); International Depositary Authority of Canada Collection, Canada (strains with prefix IDAC); Interntional Collection of Micro-orgniasms from Plants, Landcare Research, Private Bag 92170, Auckland Mail Centre, Auckland 1142, New Zealand (strans with prefix ICMP); IITA, PMB 5320, Ibadan, Nigeria (straisn with prefix IITA); The National Collections of Industrial and Marine Bacteria Ltd., Torry Research Station, P.O. Box 31, 135 Abbey Road, Aberdeen, AB9 8DG, Scotland (strains with prefix NCIMB); ARS Culture Collection of the National Center for Agricultural Utilization Research, Agricultural Research Service, U.S. Department of Agriculture, 1815 North University Street, Peoria, Illinois 61604, USA (strains with prefix NRRL); Department of Scientific and Industrial Research Culture Collection, Applied Biochemistry Division, Palmerston North, New Zealand (strains with prefix NZP); FEPAGRO-Fundação Estadual de Pesquisa Agropecuária, Rua Gonçalves Dias, 570, Bairro Menino Deus, Porto Alegre/RS, Brazil (strains with prefix SEMIA); SARDI, Adelaide, South Australia (strains with prefix SRDI); U.S. Department of Agriculture, Agricultural Research Service, Soybean and Alfalfa Research Laboratory, BARC-West, 10300 Baltimore Boulevard, Building 011, Room 19-9, Beltsville, MD 20705, USA (strains with prefix USDA: Beltsville Rhizobium Culture Collection Catalog March 1987 USDA-ARS ARS-30: http://pdf.usaid.gov/pdf_docs/PNAAW891.pdf); and Murdoch University, Perth, Western Australia (strains with prefix WSM). Further strains may be found at the Global catalogue of Microorganisms: http://gcm.wfcc.info/ and http://www.landcareresearch.co.nz/resources/collections/icmp and further references to strain collections and their prefixes at http://refs.wdcm.org/collections.htm.

Bacillus amyloliquefaciens subsp. plantarum MBI600 (NRRL B-50595) is deposited under accession number NRRL B-50595 with the strain designation Bacillus subtilis 1430 (and identical to NCIMB 1237). Recently, MBI 600 has been re-classified as Bacillus amyloliquefaciens subsp. plantarum based on polyphasic testing which combines classical microbiological methods relying on a mixture of traditional tools (such as culture-based methods) and molecular tools (such as genotyping and fatty acids analysis). Thus, Bacillus subtilis MBI600 (or MBI 600 or MBI-600) is identical to Bacillus amyloliquefaciens subsp. plantarum MB1600, formerly Bacillus subtilis MB1600. Bacillus amyloliquefaciens MBI600 is known as plant growth-promoting rice seed treatment from Int. J. Microbiol. Res. 3(2) (2011), 120-130 and further described e.g. in US 2012/0149571 A1. This strain MBI600 is e.g. commercially available as liquid formulation product INTEGRAL® (Becker-Underwood Inc., USA).

Bacillus subtilis strain FB17 was originally isolated from red beet roots in North America (System Appl. Microbiol 27 (2004) 372-379). This B. subtilis strain promotes plant health (US 2010/0260735 A1; WO 2011/109395 A2). B. subtilis FB17 has also been deposited at ATCC under number PTA-11857 on April 26, 2011. Bacillus subtilis strain FB17 may be referred elsewhere to as UD1022 or UD10-22.

Bacillus amyloliquefaciens AP-136 (NRRL B-50614), B. amyloliquefaciens AP-188 (NRRL B-50615), B. amyloliquefaciens AP-218 (NRRL B-50618), B. amyloliquefaciens AP-219 (NRRL B-50619), B. amyloliquefaciens AP-295 (NRRL B-50620), B. japonicum SEMIA 5079 (e.g. Gelfix 5 or Adhere 60 from Nitral Urbana Laoboratories, Brazil, a BASF Company), B. japonicum SEMIA 5080 (e.g. GELFIX 5 or ADHERE 60 from Nitral Urbana Laoboratories, Brazil, a BASF Company), B. mojavensis AP-209 (NRRL B-50616), B. solisalsi AP-217 (NRRL B-50617), B. pumilus strain INR-7 (otherwise referred to as BU-F22 (NRRL B-50153) and BU-F33 (NRRL B-50185)), B. simplex ABU 288 (NRRL B-50340) and B. amyloliquefaciens subsp. plantarum MBI600 (NRRL B-50595) have been mentioned i.a. in US patent appl. 20120149571, US 8,445,255, WO 2012/079073. Bradyrhizobium japonicum USDA 3 is known from US patent 7,262,151.

Jasmonic acid or salts (jasmonates) or derivatives include without limitation potassium jasmonate, sodium jasmonate, lithium jasmonate, ammonium jasmonate, dimethylammonium jasmonate, isopropylammonium jasmonate, diolammonium jasmonate, diethtriethanolammonium jasmonate, jasmonic acid methyl ester, jasmonic acid amide, jasmonic acid methylamide, jasmonic acid-L-amino acid (amide-linked) conjugates (e.g., conjugates with L-isoleucine, L-valine, L-leucine, or L-phenylalanine), 12-oxo-phytodienoic acid, coronatine, coronafacoyl-L-serine, coronafacoyl-L-threonine, methyl esters of 1-oxo-indanoyl-isoleucine, methyl esters of 1-oxo-indanoyl-leucine, coronalon (2-[(6-ethyl-I-oxo-indane-4-carbonyl) -amino]-3-methyl -pentanoic acid methyl ester), linoleic acid or derivatives thereof and cis-jasmone, or combinations of any of the above.

Humates are humic and fulvic acids extracted from a form of lignite coal and clay, known as leonardite. Humic acids are organic acids that occur in humus and other organically derived materials such as peat and certain soft coal. They have been shown to increase fertilizer efficiency in phosphate and micro-nutrient uptake by plants as well as aiding in the development of plant root systems.

According to one embodiment, the microbial pesticides selected from groups L1), L3) and L5) embrace not only the isolated, pure cultures of the respective micro-organism as defined herein, but also its cell-free extract, its suspensions in a whole broth culture or as a metabolite-containing supernatant or a purified metabolite obtained from a whole broth culture of the microorganism or microorganism strain.

According to a further embodiment, the microbial pesticides selected from groups L1), L3 and L5) embraces not only the isolated, pure cultures of the respective micro-organism as defined herein, but also a cell-free extract thereof or at least one metabolite thereof, and/or a mutant of the respective micro-organism having all the identifying characteristics thereof and also a cell-free extract or at least one metabolite of the mutant.

"Whole broth culture" refers to a liquid culture containing both cells and media.

"Supernatant" refers to the liquid broth remaining when cells grown in broth are removed by centrifugation, filtration, sedimentation, or other means well known in the art.

The term "cell-free extract" refers to an extract of the vegetative cells, spores and/or the whole culture broth of a microorganism comprising cellular metabolites produced by the respective microorganism obtainable by cell disruption methods known in the art such as solvent-based (e.g. organic solvents such as alcohols sometimesin combination with suitable salts), temperature-based, application of shear forces, cell disrupotion with an ultrasonicator. The desired extract may be concentrated by conventional concentration techniques such as drying, evaporation, centrifugation or alike. Certain washing steps using organic solents and/or water-based media may also be applied to the crude extract preferably prior to use.

The term "metabolite" refers to any compound, substance or byproduct produced by a microorganism (such as fungi and bacteria) that has improves plant growth, water use efficiency of the plant, plant health, plant appearance, or the population of beneficial microorganisms in the soil around the plant activity.

The term "mutant" refers a microorganism obtained by direct mutant selection but also includes microorganisms that have been further mutagenized or otherwise manipulated (e.g., via the introduction of a plasmid). Accordingly, embodiments include mutants, variants, and or derivatives of the respective microorganism, both naturally occurring and artificially induced mutants. For example, mutants may be induced by subjecting the microorganism to known mutagens, such as N-methyl-nitrosoguanidine, using conventional methods.

Suitable bactericides are bronopol and isothiazolinone derivatives such as alkylisothiazolinones and benzisothiazolinones. Suitable anti-freezing agents are ethylene glycol, propylene glycol, urea and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants). Suitable tackifiers or binders are polyvinylpyrrolidons, polyvinylacetates, polyvinyl alcohols, polyacrylates, biological or synthetic waxes, and cellulose ethers.

In the case of mixtures comprising microbial pesticides II selected from groups L1), L3) and L5), the microorganisms as used according to the invention can be cultivated continuously or discontinuously in the batch process or in the fed batch or repeated fed batch process. A review of known methods of cultivation will be found in the textbook by Chmiel (Bioprozesstechnik 1. Ein-führung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

When living microorganisms, such as pesticides II from groups L1), L3) and L5), form part of the compositions, such compositions can be prepared as compositions comprising besides the active ingredients at least one auxiliary (inert ingredient) by usual means (see e.g. H.D. Burges: Formulation of Micobial Biopestcides, Springer, 1998). Suitable customary types of such compositions are suspensions, dusts, powders, pastes, granules, pressings, capsules, and mixtures thereof. Examples for composition types are suspensions (e.g. SC, OD, FS), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). Herein, it has to be taken into account that each formulation type or choice of auxiliary should not influence the viability of the microorganism during storage of thecomposition and when final-ly applied to the soil, plant or plant propagation material. Suitable formulations are e.g. mentioned in WO 2008/002371, US 6955,912, US 5,422,107.

Examples for suitable auxiliaries are those mentioned earlier herein, wherein it must be taken care that choice and amounts of such auxiliaries should not influence the viability of the micro-bial pesticides in the composition. Especially for bactericides and solvents, compatibility with the respective microorganism of the respective microbial pesticide has to be taken into account. In addition, compositions with microbial pesticides may further contain stabilizers or nutrients and UV protectants. Suitable stabilzers or nutrients are e.g. alpha-tocopherol, trehalose, glutamate, potassium sorbate, various sugars like glucose, sucrose, lactose and maltodextrine (H.D. Burges: Formulation of Micobial Biopestcides, Springer, 1998). Suitable UV protectants are e.g. inorganic compouns like titan dioxide, zinc oxide and iron oxide pigments or organic compounds like benzophenones, benzotriazoles and phenyltriazines. The compositions may in addition to auxiliaries mentioned for compositions comprising compounds I herein optionally comprise 0.1 - 80% stabilizers or nutrients and 0.1-10% UV protectants.

When mixtures comprising microbial pesticides are employed in crop protection, the application rates preferably range from about 1 x 10⁶ to 5 x 10¹⁵ (or more) CFU/ha. Preferably, the spore concentration is about 1 x 10⁷ to about 1 x 10¹¹ CFU/ha. In the case of (entomopathogenic) nematodes as microbial pesticides (e.g. Steinernema feltiae), the application rates preferably range inform about 1 x 10⁵ to 1 x 10¹² (or more), more preferably from 1 x 10⁸ to 1 x 10¹¹, even more preferably from 5 x 10⁸ to 1 x 10¹⁰ individuals (e.g. in the form of eggs, juvenile or any other live stages, preferably in an infetive juvenile stage) per ha.

When mixtures comprising microbial pesticides are employed in seed treatment, the application rates with respect to plant propagation material preferably range from about 1 x 10⁶ to 1 x 10¹² (or more) CFU/seed. Preferably, the concentration is about 1 x 10⁶ to about 1 x 10¹¹ CFU/seed. In the case of the microbial pesticides II, the application rates with respect to plant propagation material also preferably range from about 1 x 10⁷ to 1 x 10¹⁴ (or more) CFU per 100 kg of seed, preferably from 1 x 10⁹ to about 1 x 10¹¹ CFU per 100 kg of seed.

Accordingly, the present invention furthermore relates to compositions comprising one compound I (component 1) and one further active substance (component 2), which further active substance is selected from the column "Component 2" of the lines C-1 to C-398 of Table C.

A further embodiment relates to the compositions C-1 to C-398 listed in Table C, wherein one row of Table C corresponds in each case to a composition comprising one of the compounds I that are individualized compounds of formula I (component 1) and the respective further active substance from groups A) to O) (component 2) stated in the respective row. According to a preferred embodiment, the "individualized compound I" is one of the compounds as individualized in Tables 1 a to 64a, Tables 1 b to 64b, Tables 1 c to 140c, Tables 1 d to 140d, Tables 1 e to 140e, Tables 1f to 140f, Tables 1 g to 70g, Tables 1 h to 70h, Tables 1 i to 70i and Tables 1j to 70j. Preferably, the compositions described comprise the active substances in synergistically effective amounts.

**Table C: Composition comprising one individualized compound of the present invention and one further active substance from groups A) to O)**

| **Composition** | **Component 1** | **Component 2** |
|---|---|---|
| C-1 | one individualized compound I | Azoxystrobin |
| C-2 | one individualized compound I | Coumethoxystrobin |
| C-3 | one individualized compound I | Coumoxystrobin |
| C-4 | one individualized compound I | Dimoxystrobin |
| C-5 | one individualized compound I | Enestroburin |
| C-6 | one individualized compound I | Fenaminstrobin |
| C-7 | one individualized compound I | Fenoxystrobin/Flufenoxystrobin |
| C-8 | one individualized compound I | Fluoxastrobin |
| C-9 | one individualized compound I | Kresoxim-methyl |
| C-10 | one individualized compound I | Metominostrobin |
| C-11 | one individualized compound I | Orysastrobin |
| C-12 | one individualized compound I | Picoxystrobin |
| C-13 | one individualized compound I | Pyraclostrobin |
| C-14 | one individualized compound I | Pyrametostrobin |
| C-15 | one individualized compound I | Pyraoxystrobin |
| C-16 | one individualized compound I | Pyribencarb |
| C-17 | one individualized compound I | Trifloxystrobin |
| C-18 | one individualized compound I | Triclopyricarb/Chlorodincarb |
| C-19 | one individualized compound I | 2-[2-(2,5-dimethyl-phenoxymethyl)-phenyl]-3-methoxy-acrylic acid methyl ester |
| C-20 | one individualized compound I | 2-(2-(3-(2,6-dichlorophenyl)-1-methyl-allylideneaminooxymethyl)-phenyl)-2-methoxyimino-N-methyl-acetamide |
| C-21 | one individualized compound I | Benalaxyl |
| C-22 | one individualized compound I | Benalaxyl-M |
| C-23 | one individualized compound I | Benodanil |
| C-24 | one individualized compound I | Benzovindiflupyr |
| C-25 | one individualized compound I | Bixafen |
| C-26 | one individualized compound I | Boscalid |
| C-27 | one individualized compound I | Carboxin |
| C-28 | one individualized compound I | Fenfuram |
| C-29 | one individualized compound I | Fenhexamid |
| C-30 | one individualized compound I | Flutolanil |
| C-31 | one individualized compound I | Fluxapyroxad |
| C-32 | one individualized compound I | Furametpyr |
| C-33 | one individualized compound I | Isopyrazam |
| C-34 | one individualized compound I | Isotianil |
| C-35 | one individualized compound I | Kiralaxyl |
| C-36 | one individualized compound I | Mepronil |
| C-37 | one individualized compound I | Metalaxyl |
| C-38 | one individualized compound I | Metalaxyl-M |
| C-39 | one individualized compound I | Ofurace |
| C-40 | one individualized compound I | Oxadixyl |
| C-41 | one individualized compound I | Oxycarboxin |
| C-42 | one individualized compound I | Penflufen |
| C-43 | one individualized compound I | Penthiopyrad |
| C-44 | one individualized compound I | Sedaxane |
| C-45 | one individualized compound I | Tecloftalam |
| C-46 | one individualized compound I | Thifluzamide |
| C-47 | one individualized compound I | Tiadinil |
| C-48 | one individualized compound I | 2-Amino-4-methyl-thiazole-5-carboxylic acid anilide |
| C-49 | one individualized compound I | N-(4'-trifluoromethylthiobiphenyl-2-yl)-3-difluoromethyl-1-methyl-1 H-pyrazole-4-carboxamide |
| C-50 | one individualized compound I | N-(2-(1,3,3-trimethyl-butyl)-phenyl)-1,3-dimethyl-5-fluoro-1H-pyrazole-4-carboxamide |
| C-51 | one individualized compound I | 3-(difluoromethyl)-1-methyl-N-(1,1,3-trimethylindan-4-yl)pyrazole-4-carboxamide |
| C-52 | one individualized compound I | 3-(trifluoromethyl)-1-methyl-N-(1,1,3-trimethylindan-4-yl)pyrazole-4-carboxamide |
| C-53 | one individualized compound I | 1,3-dimethyl-N-(1,1,3-trimethylindan-4-yl)pyrazole-4-carboxamide |
| C-54 | one individualized compound I | 3-(trifluoromethyl)-1,5-dimethyl-N-(1,1,3-trimethylindan-4-yl)pyrazole-4-carboxamide |
| C-55 | one individualized compound I | 3-(difluoromethyl)-1,5-dimethyl-N-(1,1,3-trimethylindan-4-yl)pyrazole-4-carboxamide |
| C-56 | one individualized compound I | 1,3,5-trimethyl-N-(1,1,3-trimethylindan-4-yl)pyrazole-4-carboxamide |
| C-57 | one individualized compound I | Dimethomorph |
| C-58 | one individualized compound I | Flumorph |
| C-59 | one individualized compound I | Pyrimorph |
| C-60 | one individualized compound I | Flumetover |
| C-61 | one individualized compound I | Fluopicolide |
| C-62 | one individualized compound I | Fluopyram |
| C-63 | one individualized compound I | Zoxamide |
| C-64 | one individualized compound I | Carpropamid |
| C-65 | one individualized compound I | Diclocymet |
| C-66 | one individualized compound I | Mandipropamid |
| C-67 | one individualized compound I | Oxytetracyclin |
| C-68 | one individualized compound I | Silthiofam |
| C-69 | one individualized compound I | N-(6-methoxy-pyridin-3-yl) cyclopropanecarboxylic acid amide |
| C-70 | one individualized compound I | Azaconazole |
| C-71 | one individualized compound I | Bitertanol |
| C-72 | one individualized compound I | Bromuconazole |
| C-73 | one individualized compound I | Cyproconazole |
| C-74 | one individualized compound I | Difenoconazole |
| C-75 | one individualized compound I | Diniconazole |
| C-76 | one individualized compound I | Diniconazole-M |
| C-77 | one individualized compound I | Epoxiconazole |
| C-78 | one individualized compound I | Fenbuconazole |
| C-79 | one individualized compound I | Fluquinconazole |
| C-80 | one individualized compound I | Flusilazole |
| C-81 | one individualized compound I | Flutriafol |
| C-82 | one individualized compound I | Hexaconazol |
| C-83 | one individualized compound I | Imibenconazole |
| C-84 | one individualized compound I | Ipconazole |
| C-85 | one individualized compound I | Metconazole |
| C-86 | one individualized compound I | Myclobutanil |
| C-87 | one individualized compound I | Oxpoconazol |
| C-88 | one individualized compound I | Paclobutrazol |
| C-89 | one individualized compound I | Penconazole |
| C-90 | one individualized compound I | Propiconazole |
| C-91 | one individualized compound I | Prothioconazole |
| C-92 | one individualized compound I | Simeconazole |
| C-93 | one individualized compound I | Tebuconazole |
| C-94 | one individualized compound I | Tetraconazole |
| C-95 | one individualized compound I | Triadimefon |
| C-96 | one individualized compound I | Triadimenol |
| C-97 | one individualized compound I | Triticonazole |
| C-98 | one individualized compound I | Uniconazole |
| C-99 | one individualized compound I | 1-[*rel*-(2*S*;3*R*)-3-(2-chlorophenyl)-2-(2,4-difluorophenyl)-oxiranylmethyl]-5-thiocyanato-1H-[1,2,4]triazole, |
| C-100 | one individualized compound I | 2-[*rel*-(2*S*;3*R*)-3-(2-chlorophenyl)-2-(2,4-difluorophenyl)-oxiranylmethyl]-2H-[1,2,4]triazole-3-thiol |
| C-101 | one individualized compound I | Cyazofamid |
| C-102 | one individualized compound I | Amisulbrom |
| C-103 | one individualized compound I | Imazalil |
| C-104 | one individualized compound I | Imazalil-sulfate |
| C-105 | one individualized compound I | Pefurazoate |
| C-106 | one individualized compound I | Prochloraz |
| C-107 | one individualized compound I | Triflumizole |
| C-108 | one individualized compound I | Benomyl |
| C-109 | one individualized compound I | Carbendazim |
| C-110 | one individualized compound I | Fuberidazole |
| C-111 | one individualized compound I | Thiabendazole |
| C-112 | one individualized compound I | Ethaboxam |
| C-113 | one individualized compound I | Etridiazole |
| C-114 | one individualized compound I | Hymexazole |
| C-115 | one individualized compound I | 2-(4-Chloro-phenyl)-N-[4-(3,4-dimethoxy-phenyl)-isoxazol-5-yl]-2-prop-2-ynyloxy-acetamide |
| C-116 | one individualized compound I | Fluazinam |
| C-117 | one individualized compound I | Pyrifenox |
| C-118 | one individualized compound I | 3-[5-(4-Chloro-phenyl)-2,3-dimethyl-is-oxazolidin-3-yl]-pyridine (Pyrisoxazole) |
| C-119 | one individualized compound I | 3-[5-(4-Methyl-phenyl)-2,3-dimethyl-isoxazolidin-3-yl]-pyridine |
| C-120 | one individualized compound I | Bupirimate |
| C-121 | one individualized compound I | Cyprodinil |
| C-122 | one individualized compound I | 5-Fluorocytosine |
| C-123 | one individualized compound I | 5-Fluoro-2-(p-tolylmethoxy)pyrimidin-4-amine |
| C-124 | one individualized compound I | 5-Fluoro-2-(4-fluorophenylmethoxy)-pyrimidin-4-amine |
| C-125 | one individualized compound I | Diflumetorim |
| C-126 | one individualized compound I | (5,8-Difluoroquinazolin-4-yl)-{2-[2-fluoro-4-(4-trifluoromethylpyridin-2-yloxy)-phenyl]-ethyl}-amine |
| C-127 | one individualized compound I | Fenarimol |
| C-128 | one individualized compound I | Ferimzone |
| C-129 | one individualized compound I | Mepanipyrim |
| C-130 | one individualized compound I | Nitrapyrin |
| C-131 | one individualized compound I | Nuarimol |
| C-132 | one individualized compound I | Pyrimethanil |
| C-133 | one individualized compound I | Triforine |
| C-134 | one individualized compound I | Fenpiclonil |
| C-135 | one individualized compound I | Fludioxonil |
| C-136 | one individualized compound I | Aldimorph |
| C-137 | one individualized compound I | Dodemorph |
| C-138 | one individualized compound I | Dodemorph-acetate |
| C-139 | one individualized compound I | Fenpropimorph |
| C-140 | one individualized compound I | Tridemorph |
| C-141 | one individualized compound I | Fenpropidin |
| C-142 | one individualized compound I | Fluoroimid |
| C-143 | one individualized compound I | Iprodione |
| C-144 | one individualized compound I | Procymidone |
| C-145 | one individualized compound I | Vinclozolin |
| C-146 | one individualized compound I | Famoxadone |
| C-147 | one individualized compound I | Fenamidone |
| C-148 | one individualized compound I | Flutianil |
| C-149 | one individualized compound I | Octhilinone |
| C-150 | one individualized compound I | Probenazole |
| C-151 | one individualized compound I | Fenpyrazamine |
| C-152 | one individualized compound I | Acibenzolar-S-methyl |
| C-153 | one individualized compound I | Ametoctradin |
| C-154 | one individualized compound I | Amisulbrom |
| C-155 | one individualized compound I | [(3S,6S,7R,8R)-8-benzyl-3-[(3-isobuty-ryloxymethoxy-4-methoxypyridine-2-carbonyl)amino]-6-methyl-4,9-dioxo-[1,5]dioxonan-7-yl] 2-methylpropanoate |
| C-156 | one individualized compound I | [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate |
| C-157 | one individualized compound I | [(3S,6S,7R,8R)-8-benzyl-3-[[3-(acet-oxymethoxy)-4-methoxy-pyridine-2-carbonyl]amino]-6-methyl-4,9-dioxo-1,5-dioxonan-7-yl] 2-methylpropanoate |
| C-158 | one individualized compound I | [(3S,6S,7R,8R)-8-benzyl-3-[(3-isobut-oxycarbonyloxy-4-methoxy-pyridine-2-carbonyl)amino]-6-methyl-4,9-dioxo-1,5-dioxonan-7-yl] 2-methylpropanoate |
| C-159 | one individualized compound I | [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate |
| C-160 | one individualized compound I | (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate |
| C-161 | one individualized compound I | Anilazin |
| C-162 | one individualized compound I | Blasticidin-S |
| C-163 | one individualized compound I | Captafol |
| C-164 | one individualized compound I | Captan |
| C-165 | one individualized compound I | Chinomethionat |
| C-166 | one individualized compound I | Dazomet |
| C-167 | one individualized compound I | Debacarb |
| C-168 | one individualized compound I | Diclomezine |
| C-169 | one individualized compound I | Difenzoquat, |
| C-170 | one individualized compound I | Difenzoquat-methylsulfate |
| C-171 | one individualized compound I | Fenoxanil |
| C-172 | one individualized compound I | Folpet |
| C-173 | one individualized compound I | Oxolinsäure |
| C-174 | one individualized compound I | Piperalin |
| C-175 | one individualized compound I | Proquinazid |
| C-176 | one individualized compound I | Pyroquilon |
| C-177 | one individualized compound I | Quinoxyfen |
| C-178 | one individualized compound I | Triazoxid |
| C-179 | one individualized compound I | Tricyclazole |
| C-180 | one individualized compound I | 2-Butoxy-6-iodo-3-propyl-chromen-4-one |
| C-181 | one individualized compound I | 5-Chloro-1-(4,6-dimethoxy-pyrimidin-2-yl)-2-methyl-1 H-benzoimidazole |
| C-182 | one individualized compound I | 5-Ch loro-7-(4-methyl-pi perid i n-1-yl)-6-(2,4,6-trifluoro-phenyl)-[1,2,4]triazolo[1,5-a]pyrimidine |
| C-183 | one individualized compound I | Ferbam |
| C-184 | one individualized compound I | Mancozeb |
| C-185 | one individualized compound I | Maneb |
| C-186 | one individualized compound I | Metam |
| C-187 | one individualized compound I | Methasulphocarb |
| C-188 | one individualized compound I | Metiram |
| C-189 | one individualized compound I | Propineb |
| C-190 | one individualized compound I | Thiram |
| C-191 | one individualized compound I | Zineb |
| C-192 | one individualized compound I | Ziram |
| C-193 | one individualized compound I | Diethofencarb |
| C-194 | one individualized compound I | Benthiavalicarb |
| C-195 | one individualized compound I | Iprovalicarb |
| C-196 | one individualized compound I | Propamocarb |
| C-197 | one individualized compound I | Propamocarb hydrochlorid |
| C-198 | one individualized compound I | Valifenalate |
| C-199 | one individualized compound I | N-(1-(1-(4-cyanophenyl)ethanesulfonyl)-but-2-yl) carbamic acid-(4-fluorophenyl) ester |
| C-200 | one individualized compound I | Dodine |
| C-201 | one individualized compound I | Dodine free base |
| C-202 | one individualized compound I | Guazatine |
| C-203 | one individualized compound I | Guazatine-acetate |
| C-204 | one individualized compound I | Iminoctadine |
| C-205 | one individualized compound I | Iminoctadine-triacetate |
| C-206 | one individualized compound I | Iminoctadine-tris(albesilate) |
| C-207 | one individualized compound I | Kasugamycin |
| C-208 | one individualized compound I | Kasugamycin-hydrochloride-hydrate |
| C-209 | one individualized compound I | Polyoxine |
| C-210 | one individualized compound I | Streptomycin |
| C-211 | one individualized compound I | Validamycin A |
| C-212 | one individualized compound I | Binapacryl |
| C-213 | one individualized compound I | Dicloran |
| C-214 | one individualized compound I | Dinobuton |
| C-215 | one individualized compound I | Dinocap |
| C-216 | one individualized compound I | Nitrothal-isopropyl |
| C-217 | one individualized compound I | Tecnazen |
| C-218 | one individualized compound I | Fentin salts |
| C-219 | one individualized compound I | Dithianon |
| C-220 | one individualized compound I | 2,6-dimethyl-1 H,5H-[1,4]dithiino [2,3-c:5,6-c']dipyrrole-1,3,5,7(2H,6H)-tetraone |
| C-221 | one individualized compound I | Isoprothiolane |
| C-222 | one individualized compound I | Edifenphos |
| C-223 | one individualized compound I | Fosetyl, Fosetyl-aluminium |
| C-224 | one individualized compound I | Iprobenfos |
| C-225 | one individualized compound I | Phosphorous acid (H₃PO₃) and derivatives |
| C-226 | one individualized compound I | Pyrazophos |
| C-227 | one individualized compound I | Tolclofos-methyl |
| C-228 | one individualized compound I | Chlorothalonil |
| C-229 | one individualized compound I | Dichlofluanid |
| C-230 | one individualized compound I | Dichlorophen |
| C-231 | one individualized compound I | Flusulfamide |
| C-232 | one individualized compound I | Hexachlorbenzene |
| C-233 | one individualized compound I | Pencycuron |
| C-234 | one individualized compound I | Pentachlorophenol and salts |
| C-235 | one individualized compound I | Phthalide |
| C-236 | one individualized compound I | Quintozene |
| C-237 | one individualized compound I | Thiophanate Methyl |
| C-238 | one individualized compound I | Tolylfluanid |
| C-239 | one individualized compound I | N-(4-chloro-2-nitro-phenyl)-N-ethyl-4-methyl-benzenesulfonamide |
| C-240 | one individualized compound I | Bordeaux composition |
| C-241 | one individualized compound I | Copper acetate |
| C-242 | one individualized compound I | Copper hydroxide |
| C-243 | one individualized compound I | Copper oxychloride |
| C-244 | one individualized compound I | basic Copper sulfate |
| C-245 | one individualized compound I | Sulfur |
| C-246 | one individualized compound I | Biphenyl |
| C-247 | one individualized compound I | Bronopol |
| C-248 | one individualized compound I | Cyflufenamid |
| C-249 | one individualized compound I | Cymoxanil |
| C-250 | one individualized compound I | Diphenylamin |
| C-251 | one individualized compound I | Metrafenone |
| C-252 | one individualized compound I | Pyriofenone |
| C-253 | one individualized compound I | Mildiomycin |
| C-254 | one individualized compound I | Oxin-copper |
| C-255 | one individualized compound I | Oxathiapiprolin |
| C-256 | one individualized compound I | Prohexadione calcium |
| C-257 | one individualized compound I | Spiroxamine |
| C-258 | one individualized compound I | Tebufloquin |
| C-259 | one individualized compound I | Tolylfluanid |
| C-260 | one individualized compound I | N-(Cyclopropylmethoxyimino-(6-difluoro-methoxy-2,3-difluoro-phenyl)-methyl)-2-phenyl acetamide |
| C-261 | one individualized compound I | N'-(4-(4-chloro-3-trifluoromethyl-phenoxy)-2,5-dimethyl-phenyl)-N-ethyl-N-methyl formamidine |
| C-262 | one individualized compound I | N'-(4-(4-fluoro-3-trifluoromethyl-phenoxy)-2,5-dimethyl-phenyl)-N-ethyl-N-methyl formamidine |
| C-263 | one individualized compound I | N'-(2-methyl-5-trifluoromethyl-4-(3-trimethylsilanyl-propoxy)-phenyl)-N-ethyl-N-methyl formamidine |
| C-264 | one individualized compound I | N'-(5-difluoromethyl-2-methyl-4-(3-trimethylsilanyl-propoxy)-phenyl)-N-ethyl-N-methyl formamidine |
| C-265 | one individualized compound I | Methoxy-acetic acid 6-tert-butyl-8-fluoro-2,3-dimethyl-quinolin-4-yl ester |
| C-266 | one individualized compound I | *Bacillus subtilis* NRRL No. B-21661 |
| C-267 | one individualized compound I | *Bacillus pumilus* NRRL No. B-30087 |
| C-268 | one individualized compound I | *Ulocladium oudemansii* |
| C-269 | one individualized compound I | Carbaryl |
| C-270 | one individualized compound I | Carbofuran |
| C-271 | one individualized compound I | Carbosulfan |
| C-272 | one individualized compound I | Methomylthiodicarb |
| C-273 | one individualized compound I | Bifenthrin |
| C-274 | one individualized compound I | Cyfluthrin |
| C-275 | one individualized compound I | Cypermethrin |
| C-276 | one individualized compound I | alpha-Cypermethrin |
| C-277 | one individualized compound I | zeta-Cypermethrin |
| C-278 | one individualized compound I | Deltamethrin |
| C-279 | one individualized compound I | Esfenvalerate |
| C-280 | one individualized compound I | Lambda-cyhalothrin |
| C-281 | one individualized compound I | Permethrin |
| C-282 | one individualized compound I | Tefluthrin |
| C-283 | one individualized compound I | Diflubenzuron |
| C-284 | one individualized compound I | Flufenoxuron |
| C-285 | one individualized compound I | Lufenuron |
| C-286 | one individualized compound I | Teflubenzuron |
| C-287 | one individualized compound I | Spirotetramate |
| C-288 | one individualized compound I | Clothianidin |
| C-289 | one individualized compound I | Dinotefuran |
| C-290 | one individualized compound I | Imidacloprid |
| C-291 | one individualized compound I | Thiamethoxam |
| C-292 | one individualized compound I | Flupyradifurone |
| C-293 | one individualized compound I | Acetamiprid |
| C-294 | one individualized compound I | Thiacloprid |
| C-295 | one individualized compound I | Endosulfan |
| C-296 | one individualized compound I | Fipronil |
| C-297 | one individualized compound I | Abamectin |
| C-298 | one individualized compound I | Emamectin |
| C-299 | one individualized compound I | Spinosad |
| C-300 | one individualized compound I | Spinetoram |
| C-301 | one individualized compound I | Hydramethylnon |
| C-302 | one individualized compound I | Chlorfenapyr |
| C-303 | one individualized compound I | Fenbutatin oxide |
| C-304 | one individualized compound I | Indoxacarb |
| C-305 | one individualized compound I | Metaflumizone |
| C-306 | one individualized compound I | Flonicamid |
| C-307 | one individualized compound I | Lubendiamide |
| C-308 | one individualized compound I | Chlorantraniliprole |
| C-309 | one individualized compound I | Cyazypyr (HGW86) |
| C-310 | one individualized compound I | Cyflumetofen |
| C-311 | one individualized compound I | Acetochlor |
| C-312 | one individualized compound I | Dimethenamid |
| C-313 | one individualized compound I | metolachlor |
| C-314 | one individualized compound I | Metazachlor |
| C-315 | one individualized compound I | Glyphosate |
| C-316 | one individualized compound I | Glufosinate |
| C-317 | one individualized compound I | Sulfosate |
| C-318 | one individualized compound I | Clodinafop |
| C-319 | one individualized compound I | Fenoxaprop |
| C-320 | one individualized compound I | Fluazifop |
| C-321 | one individualized compound I | Haloxyfop |
| C-322 | one individualized compound I | Paraquat |
| C-323 | one individualized compound I | Phenmedipham |
| C-324 | one individualized compound I | Clethodim |
| C-325 | one individualized compound I | Cycloxydim |
| C-326 | one individualized compound I | Profoxydim |
| C-327 | one individualized compound I | Sethoxydim |
| C-328 | one individualized compound I | Tepraloxydim |
| C-329 | one individualized compound I | Pendimethalin |
| C-330 | one individualized compound I | Prodiamine |
| C-331 | one individualized compound I | Trifluralin |
| C-332 | one individualized compound I | Acifluorfen |
| C-333 | one individualized compound I | Bromoxynil |
| C-334 | one individualized compound I | Imazamethabenz |
| C-335 | one individualized compound I | Imazamox |
| C-336 | one individualized compound I | Imazapic |
| C-337 | one individualized compound I | Imazapyr |
| C-338 | one individualized compound I | Imazaquin |
| C-339 | one individualized compound I | Imazethapyr |
| C-340 | one individualized compound I | 2,4-Dichlorophenoxyacetic acid (2,4-D) |
| C-341 | one individualized compound I | Chloridazon |
| C-342 | one individualized compound I | Clopyralid |
| C-343 | one individualized compound I | Fluroxypyr |
| C-344 | one individualized compound I | Picloram |
| C-345 | one individualized compound I | Picolinafen |
| C-346 | one individualized compound I | Bensulfuron |
| C-347 | one individualized compound I | Chlorimuron-ethyl |
| C-348 | one individualized compound I | Cyclosulfamuron |
| C-349 | one individualized compound I | lodosulfuron |
| C-350 | one individualized compound I | Mesosulfuron |
| C-351 | one individualized compound I | Metsulfuron-methyl |
| C-352 | one individualized compound I | Nicosulfuron |
| C-353 | one individualized compound I | Rimsulfuron |
| C-354 | one individualized compound I | Triflusulfuron |
| C-355 | one individualized compound I | Atrazine |
| C-356 | one individualized compound I | Hexazinone |
| C-357 | one individualized compound I | Diuron |
| C-358 | one individualized compound I | Florasulam |
| C-359 | one individualized compound I | Pyroxasulfone |
| C-360 | one individualized compound I | Bentazone |
| C-361 | one individualized compound I | Cinidon-ethyl |
| C-362 | one individualized compound I | Cinmethylin |
| C-363 | one individualized compound I | Dicamba |
| C-364 | one individualized compound I | Diflufenzopyr |
| C-365 | one individualized compound I | Quinclorac |
| C-366 | one individualized compound I | Quinmerac |
| C-367 | one individualized compound I | Mesotrione |
| C-368 | one individualized compound I | Saflufenacil |
| C-369 | one individualized compound I | Topramezone |
| C-370 | one individualized compound I | 1,1'-[(3S,4R,4aR,6S,6aS,12R,12aS, 12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester |
| C-371 | one individualized compound I | (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate |
| C-372 | one individualized compound I | isofetamid |
| C-373 | one individualized compound I | N-(7-fluoro-1,1,3-trimethyl-indan-4-yl)-1,3-dimethyl-pyrazole-4-carboxamide |
| C-374 | one individualized compound I | N-[2-(2,4-dichlorophenyl)-2-methoxy-1-methyl-ethyl]-3-(difluoromethyl)-1-methylpyrazole-4-carboxamide |
| C-375 | one individualized compound I | 2-[2-chloro-4-(4-chlorophenoxy)phenyl]-1-(1,2,4-triazol-1-yl)pentan-2-ol |
| C-376 | one individualized compound I | 1-[4-(4-chlorophenoxy)-2-(trifluoromethyl)phenyl]-1-cyclopropyl-2-(1,2,4-triazol-1-yl)ethanol |
| C-377 | one individualized compound I | 2-[4-(4-chlorophenoxy)-2-(trifluoromethyl)phenyl]-1-(1,2,4-triazol-1-yl)butan-2-ol |
| C-378 | one individualized compound I | 2-[2-chloro-4-(4-chlorophenoxy)phenyl]-1-(1,2,4-triazol-1-yl)butan-2-ol |
| C-379 | one individualized compound I | 2-[4-(4-chlorophenoxy)-2-(trifluoromethyl)phenyl]-3-methyl-1-(1,2,4-triazol-1-yl)butan-2-ol |
| C-380 | one individualized compound I | 2-[4-(4-chlorophenoxy)-2-(trifluoromethyl)phenyl]-1-(1,2,4-triazol-1-yl)propan-2-ol |
| C-381 | one individualized compound I | 2-[2-chloro-4-(4-chlorophenoxy)phenyl]-3-methyl-1-(1,2,4-triazol-1-yl)butan-2-ol |
| C-382 | one individualized compound I | 2-[4-(4-chlorophenoxy)-2-(trifluoromethyl)phenyl]-1-(1,2,4-triazol-1-yl)pentan-2-ol |
| C-383 | one individualized compound I | 2-[4-(4-fluorophenoxy)-2-(trifluoromethyl)phenyl]-1-(1,2,4-triazol-1-yl)propan-2-ol |
| C-384 | one individualized compound I | 3-(4-chloro-2-fluoro-phenyl)-5-(2,4-difluorophenyl)isoxazol-4-yl]-(3-pyridyl)methanol |
| C-385 | one individualized compound I | 2-{3-[2-(1-{[3,5-bis(difluoromethyl-1H-pyrazol-1-yl]acetyl}piperidin-4-yl)-1,3-thiazol-4-yl]-4,5-dihydro-1,2-oxazol-5-yl}phenyl methanesulfonate |
| C-386 | one individualized compound I | 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5-yl}-3-chlorophenyl methanesulfonate |
| C-387 | one individualized compound I | tolprocarb |
| C-388 | one individualized compound I | 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone |
| C-389 | one individualized compound I | 2-[3,5-bis(difluoromethyl)-1H-pyrazol-1-yl]-1-[4-(4-{5-[2-fluoro-6-(prop-2-yn-1-yl-oxy)phenyl]-4,5-dihydro-1,2-oxazol-3-yl}-1,3-thiazol-2-yl)piperidin-1-yl]ethanone |
| C-390 | one individualized compound I | 2-[3,5-bis(difluoromethyl)-1H-pyrazol-1-yl]-1-[4-(4-{5-[2-chloro-6-(prop-2-yn-1-yl-oxy)phenyl]-4,5-dihydro-1,2-oxazol-3-yl}-1,3-thiazol-2-yl)piperidin-1-yl]ethanone |
| C-391 | one individualized compound I | ethyl (Z)-3-amino-2-cyano-3-phenyl-prop-2-enoate, |
| C-392 | one individualized compound I | picarbutrazox |
| C-393 | one individualized compound I | pentyl N-[6-[[(Z)-[(1-methyltetrazol-5-yl)-phenyl-methylene]amino]oxymethyl]-2-pyridyl]carbamate, |
| C-394 | one individualized compound I | 2-[2-[(7,8-difluoro-2-methyl-3-quinolyl)oxy]-6-fluoro-phenyl]propan-2-ol |
| C-395 | one individualized compound I | 2-[2-fluoro-6-[(8-fluoro-2-methyl-3-quinolyl)oxy]phen-yl]propan-2-ol, |
| C-396 | one individualized compound I | 3-(5-fluoro-3,3,4,4-tetramethyl-3,4-dihydroisoquinolin-1-yl)quinoline |
| C-397 | one individualized compound I | 3-(4,4-difluoro-3,3-dimethyl-3,4-dihydroisoquinolin-1-yl)quinoline |
| C-398 | one individualized compound I | 3-(4,4,5-trifluoro-3,3-dimethyl-3,4-dihydroisoquinolin-1-yl)quinoline; |

The active substances referred to as component 2, their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657, W02012/168188, WO 2007/006670, WO 2011/77514; WO13/047749, WO 10/069882, WO 13/047441, WO 03/16303, WO 09/90181, WO 13/007767, WO 13/010862, WO 13/127704, WO 13/024009 and WO 13/024010).

The composition of active substances can be prepared as compositions comprising besides the active ingredients at least one inert ingredient (auxiliary) by usual means, e. g. by the means given for the compositions of compounds I.

Concerning usual ingredients of such compositions reference is made to the explanations given for the compositions containing compounds I.

The compositions of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

## Claims

1. Compounds of the formula I 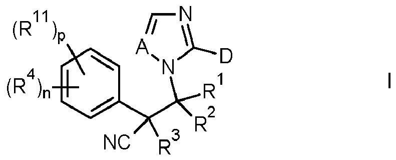 wherein
A is CH or N;
D is H, halogen or SR^{D}, wherein
R^{D} is hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl or CN;
R¹ is halogen, CN, NO₂, OH, SH, C₁-C₆-alkyl, C₁-C₆-alkoxy, C₁-C₆-alkylthio, , C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyloxy, C₃-C₈-cycloalkyl-C₁-C₄-alkyl, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl),C(=O)-N(C₁-C₄-alkyl)₂, C(=O)-NH(C₃-C₆-cycloalkyl), C(=O)-N(C₃-C₆-cycloalkyl)₂, heteroaryl, phenyl-C₁-C₄-alkyl or heteroaryl-C₁-C₄-alkyl, wherein the heteroaryl radical in the last-mentioned groups is 5- or 6-membered;;
and wherein the aliphatic, alicyclic and aromatic moieties of R¹ are unsubstituted or substituted by one, two, three or four or up to the maximum possible number of R^{1a}; wherein
R^{1a} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R² is hydrogen or is selected from the substituents defined for R¹; wherein the aliphatic, alicyclic and aromatic moieties of R² are unsubstituted or substituted by one, two, three or four or up to the maximum possible number of R^{2a}, wherein R^{2a} has the meaning as defined for R^{1a};
or R¹ and R² together with the carbon atom to which they are bound form a saturated or partially unsaturated three-, four-, five-, six- or seven-membered carbocycle or a saturated or partially unsaturated three-, four-, five-, six- or seven-membered heterocycle, wherein the heterocycle contains one, two, three or four heteroatoms selected from N, O and S, and wherein the carbo- or heterocycle is unsubstituted or carries one, two, three or four substituents R^{12a} independently selected from halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy; and wherein one or two CH₂ groups of the carbo- or heterocycle may be replaced by a group independently selected from C(=O) and C(=S);
or R¹ and R² together with the carbon atom to which they are bound (denominated C*) form a group (C*=CR¹⁸R¹⁹), wherein
R¹⁸, R¹⁹ are independently selected from hydrogen, halogen, CN, NO₂, OH, SH, NH₂, C₁-C₆-alkyl, C₁-C₆-haloalkyl, C₁-C₆-alkoxy, C₁-C₆-haloalkoxy, C₁-C₆-alkylthio, C₁-C₆-haloalkylthio and C₁-C₄-alkoxy-C₁-C₄-alkyl;
R³ is hydrogen, halogen, C₁-C₈-alkyl, C₂-C₈-alkenyl, C₂-C₈-alkinyl, C₁-C₆-alkoxy, C₃-C₈-cycloalkyl, C₃-C₈-cycloalkyl-C₁-C₄-alkyl, phenyl, phenyl-C₁-C₄-alkyl, phenyl-C₂-C₄-alkenyl or phenyl-C₂-C₄-alkynyl; wherein the aliphatic, alicyclic and aromatic moieties of R³ are unsubstituted or substituted by one, two, three or four or up to the maximum possible number of R^{3a}, wherein
R^{3a} is independently selected from halogen, CN, OH, nitro, C₁-C₄-alkyl, C₁-C₄-halogenalkyl, C₃-C₆-cycloalkyl, C₃-C₆-halocycloalkyl, C₁-C₄-alkoxy and C₁-C₄-halogenalkoxy;
p is 0 or1;
R¹¹ stands for a group Z-Y, wherein said group is bound to the phenyl via Y, wherein
Y is a direct bond or a divalent group selected from the group consisting of-O-, -S-, -SO-, -SO₂-, -NH-, -N(C₁-C₄-alkyl)-, -CR¹²R¹³-, -CR¹²R¹³-CR¹⁴R¹⁵-, -CR¹⁶=CR¹⁷-and -C≡C-; wherein
R¹²,R¹³,R¹⁴,R¹⁵,R¹⁶,R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy;
Z is five or six-membered heteroaryl or phenyl, wherein the heteroaryl contains 1, 2, 3 or 4 heteroatoms selected from the group consisting of O, N and S, and wherein the heteroaryl or phenyl is unsubstituted (m=0) or substituted by (R^{L})ₘ, wherein
m is 0, 1, 2, 3 , 4 or 5; and wherein
R^{L} is independently selected from halogen, CN, NO₂, OH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein
R^{La} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio;
R⁴ is independently selected from halogen, CN, NO₂, OH, SH, C₁-C₄-alkyl, C₁C₁-C₄-alkoxy, C₁-C₆-alkylthio, C₁-C₆-alkylsulfinyl, C₁-C₆-alkylsulfonyl, C₂-C₆-alk-enyl, C₂-C₆-alkynyl, C₃-C₈-cycloalkyl, C₃-C₈-cycloalkyl-C₁-C₄-alkyl, C₃-C₈-cycloalkyloxy, phenyl, phenoxy, a 5- or 6-membered heteroaryl, a 5- or 6-membered heteroaryloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic, alicyclic and aromatic moieties of R⁴ are unsubstituted or substituted by one, two, three or four or up to the maximum possible number of R^{4a}; wherein
R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy;
n is 0, 1, 2, 3 or 4;
with the proviso that for A = CH, R¹=n-C₃H₇, R²=hydrogen, p=0 and n=2 and (R⁴)₂=2,4-Cl₂, R³ is not hydrogen;
and the N-oxides and the agriculturally acceptable salts thereof.

2. The compounds of any of claims 1, wherein A is N.

3. The compounds of any of claims 1, wherein A is CH.

4. The compounds of any one of claims 1 to 3, wherein R³ is halogen, C₁-C₈-alkyl, C₂-C₈-alkenyl, C₂-C₈-alkinyl, C₁-C₆-alkoxy, C₃-C₈-cycloalkyl, C₃-C₈-cycloalkyl-C₁-C₄-alkyl, phenyl, phenyl-C₁-C₄-alkyl, phenyl-C₂-C₄-alkenyl or phenyl-C₂-C₄-alkynyl; wherein the aliphatic, alicyclic and aromatic moieties of R³ are unsubstituted or substituted by one, two, three or four or up to the maximum possible number of R^{3a}, wherein

5. R^{3a} is independently selected from halogen, CN, OH, nitro, C₁-C₄-alkyl, C₁-C₄-halogenalkyl, C₃-C₆-cycloalkyl, C₃-C₆-halocycloalkyl, C₁-C₄-alkoxy and C₁-C₄-halogenalkoxy. The compounds of any of claims 1 to 4, wherein Y is a direct bond.

6. The compounds of any of claims 1 to 4, wherein Y is O.

7. The compounds of any one of claims 1 to 6, wherein Z is phenyl that is unsubstituted or substituted by (R^{L})ₘ.

8. The compounds of any one of claims 1 to 4, wherein p is 0.

9. A composition, comprising one compound of formula I, as defined in any of the claims 1 to 8, an N-oxide or an agriculturally acceptable salt thereof.

10. The composition according to claim 9, comprising additionally a further active substance.

11. A use of a compound of the formula I, as defined in any of the claims 1 to 8, and/or of an agriculturally acceptable salt thereof or of the compositions, as defined in any of the claims 9 or 10, for combating phytopathogenic fungi.

12. A method for combating harmful fungi, comprising treating the fungi or the materials, plants, the soil or seeds to be protected against fungal attack with an effective amount of at least one compound of formula I, as defined in any of the claims 1 to 8, or with a composition, as defined in any of the claims 9 or 10.

13. Seed, coated with at least one compound of the formula I, as defined in any of the claims 1 to 8, and/or an agriculturally acceptable salt thereof or with a composition, as defined in any of the claims 8 or 9, in an amount of from 0.1 to 10 kg per 100 kg of seed.
